# EUROPEAN PATENT SPECIFICATION

(11) **EP 3 063 228 B1**
(45) Date of publication and mention of the grant of the patent: **01.04.2020**
(21) Application number: 14787176.8
(22) Date of filing: 22.10.2014
(51) Int. Cl.: C09B 29/44, C09B 29/36, C09B 29/42, C07D 215/38, C07D 401/12, C07D 409/12, C07D 417/12, C09B 29/01, C09B 29/033, C09B 29/46

(54) **DISPERSE DYES, THEIR PREPARATION AND THEIR USE**
DISPERGIERFARBSTOFFE, DEREN HERSTELLUNG UND DEREN VERWENDUNG
COLORANTS DISPERSÉS, LEUR PRÉPARATION ET LEUR UTILISATION

(30) Priority: 29.10.2013 EP 13190666
(43) Date of publication of application: 07.09.2016
(73) Proprietor: DyStar Colours Distribution GmbH, 65479 Raunheim (DE)
(72) Inventor: GAO, Yongnian, Singapore 650318 (SG); LIEW, Si SI, Singapore 510714 (SG); SHANTONG FU, Samuel, Singapore 117468 (SG); BARBIERU, Roxana, Lynsville Singapore 117468 (SG)
(86) International application number: PCT/EP2014/072654
(87) International publication number: WO 2015/062937

(56) References cited:
- DE-A1- 2 640 624
- US-A- 5 179 207
- GEOFFREY HALLAS ET AL: "Synthesis and Electronic Spectra of Some Disperse Dyes Derived from 9-Phenylazo-1-ketojulolidine", JOURNAL OF THE SOCIETY OF DYERS AND COLOURISTS, vol. 93, no. 7, 1 July 1977 (1977-07-01), pages 284-287, XP055164020, ISSN: 0037-9859, DOI: 10.1111/j.1478-4408.1977.tb03360.x
- P. E. MACEY ET AL: "492. Cinnolines and other heterocyclic types in relation to the chemotherapy of trypanosomiasis. Part III. Synthesis of 4 : 4?-diamino-6 : 6?-azoquinoline metho-salts", JOURNAL OF THE CHEMICAL SOCIETY (RESUMED), 1 January 1952 (1952-01-01), page 2602, XP055164640, ISSN: 0368-1769, DOI: 10.1039/jr9520002602

## Description

The present invention relates to disperse azo dyes comprising tetrahydroquinoline derivatives as coupling components, a process for the preparation of such dyes and the use thereof in dyeing or printing semi-synthetic and synthetic hydrophobic fibre materials, especially textile materials.

Azo compounds derived from substituted tetrahydroquinoline derivatives as coupling components are known to be used as intermediates for photoactivatable dyes used for non-textile applications such as cell staining (e.g. WO 2009/036351), dyes for photosensitive materials (e.g. JP2005/305835) or cationic dyes for dyeing keratin fibres (e.g. WO 2008/034650). Also a specific group of Thioazolyl-azo-tetrahydroquinolines is know to be useful for textile dyeing from US 3,699,092. Similar structures are also disclosed in DE 26 40 624 as dichroitic dyes useful e.g. in liquid crystal applications. And structures 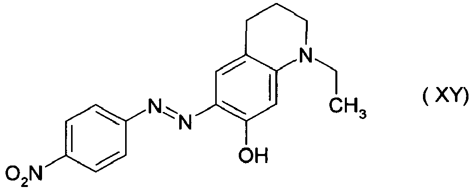 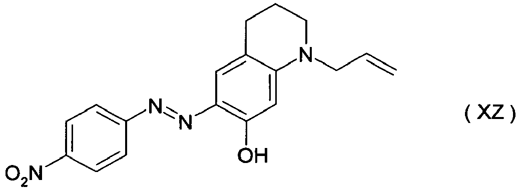 are disclosed in WO 2009/036351 on page 13 and 24 respectively as intermediates to photoactivatable dyes. However, in use for textile dyeing - and for red dyes in particular - are structures such as 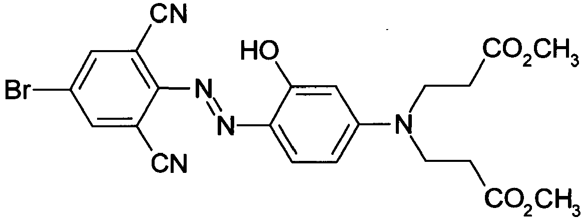 (xc1), which is commonly known as C. I. Disperse Red 369 or dyes based on the following chemical compound:

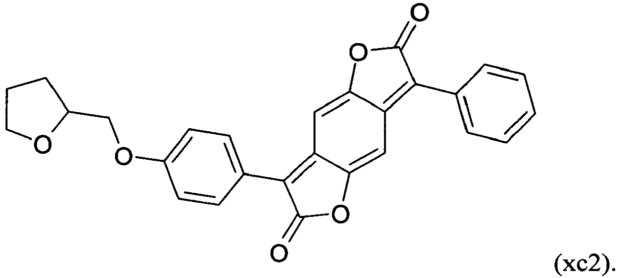

Surprisingly, it has now been found that disperse dyes of formula (I) show highly advantageous properties over the known disperse dyes for dyeing and printing textile materials. The superior properties include excellent wet contact fastness properties, good build-up, sublimation and light fastness properties both in the exhaust and thermosol process, and in textile printing.

The present invention refers to dyes of the formula (I) and mixtures thereof: 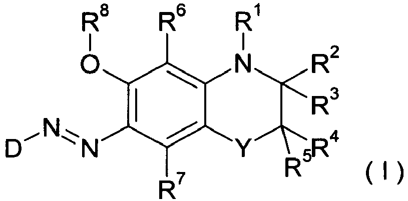 wherein
Y is a group of general formula (II) 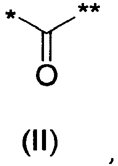
R¹ is alkyl, alkenyl, cycloalkyl, aryl, heteroaryl, heterocycloalkyl
   or
   alkyl interrupted by one or more heteroatoms selected from the group consisting of oxygen and sulphur
   or
   alkyl substituted by one or more substituents selected from the group consisting of hydroxy, aryl, cycloalkyl, alkoxy, thioalkoxy, amino, *N*-monoalkyl-amino, *N,N*-dialkyl-amino, *N-*monoaryl-amino, *N,N*-diaryl-amino, *N*-alkyl-*N*-aryl-amino, *N*-monocycloalkyl-amino, *N,N-*dicycloalkyl-amino, *N*-monoalkyl-monocycloalkyl-amino, *N,N-*monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, *N*-monoaryl-carbamoyl, *N,N*-diaryl-carbamoyl, *N*-monocycloalkyl-*N*-monoarylcarbamoyl, *N*-monoalkyl*-N*-monoaryl-carbamoyl, sulfamoyl, *N*-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²
   or
   alkyl interrupted by one or more heteroatoms selected from the group consisting of oxygen and sulphur and substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N*-diaryl-amino, *N*-alkyl-*N*-aryl-amino, *N*-monocycloalkyl-amino, *N,N-*dicycloalkyl-amino, *N*-monoalkyl-monocycloalkyl-amino, *N*,*N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxythiocarbonyl, acyloxy, aryloyloxy, carbamoyl, *N*-monocycloalkyl-carbamoyl, *N*-monoalkyl-carbamoyl, *N,N*-dicycloalkyl-carbamoyl, *N,N*-dialkyl-carbamoyl, *N*-monoaryl-carbamoyl, *N,N*-diaryl-carbamoyl, *N*-monocycloalkyl-*N*-monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²,
   or
   aryl substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N*-diaryl-amino, *N*-alkyl-*N*-aryl-amino, *N*-monocycloalkyl-amino, *N,N-*dicycloalkyl-amino, *N*-monoalkyl-monocycloalkyl-amino, *N,N-*monoaryl-monocycloalkyl-amino, *N-*acylamino, N-alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N-*dialkyl-carbamoyl, N-monoaryl-carbamoyl, *N,N*-diaryl-carbamoyl, *N*-monocycloalkyl-*N*-monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, N,N-dialkyl-sulfamoyl, *N*-monoaryl-sulfamoyl, *N,N*-diaryl-sulfamoyl, *N*-monocycloalkyl-*N*-monoarylsulfamoyl, *N*-monoalkyl-N-monoarylsulfamoyl, arylsulphonyloxy, alkylsulphonyloxy, aryloxysulfonyl, alkoxylsulfonyl and COOR¹²,
R² to R⁸ independent of each other is hydrogen, alkyl, aryl, alkoxyl, cycloalkyl, halogen
   or
   alkyl interrupted by one or more heteroatoms selected from the group consisting of oxygen and sulphur
   or
   alkyl substituted by one or more substituents selected from the group consisting of hydroxy, aryl, cycloalkyl, alkoxy, thioalkoxy, amino, *N*-monoalkyl-amino, *N,N-*dialkyl-amino, *N-*monoaryl-amino, *N,N*-diaryl-amino, *N*-alkyl-*N*-aryl-amino, *N*-monocycloalkyl-amino, *N,N-*dicycloalkyl-amino, *N*-monoalkyl-monocycloalkyl-amino, *N*,*N*-monoaryl-monocycloalkyl-amino, N-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, *N*-monoaryl-carbamoyl, *N,N*-diaryl-carbamoyl, *N*-monocycloalkyl-*N*-monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N*-monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²,
   or
   alkyl interrupted by one or more heteroatoms selected from the group consisting of oxygen and sulphur and substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N*-diaryl-amino, *N*-alkyl-*N*-aryl-amino, *N*-monocycloalkyl-amino, *N,N-*dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, *N-*monoalkyl-carbamoyl, *N,N*-dicycloalkyl-carbamoyl, *N,N*-dialkyl-carbamoyl, *N*-monoaryl-carbamoyl, *N,N*-diaryl-carbamoyl, *N*-monocycloalkyl-*N*-monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N*-monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²
   or
   aryl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N*-diaryl-amino, *N*-alkyl-*N*-aryl-amino, *N*-monocycloalkyl-amino, *N,N*-dicycloalkyl-amino, *N*-monoalkyl-monocycloalkyl-amino, *N,N-*monoaryl-monocycloalkyl-amino, *N-*acylamino, *N*-alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N-*dicycloalkyl-carbamoyl, *N,N*-dialkyl-carbamoyl, N-monoaryl-carbamoyl, *N,N*-diaryl-carbamoyl, *N*-monocycloalkyl-*N*-monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, N,N-dialkyl-sulfamoyl, *N*-monoaryl-sulfamoyl, *N,N*-diaryl-sulfamoyl, *N*-monocycloalkyl-*N*-monoarylsulfamoyl, *N*-monoalkyl-N-monoarylsulfamoyl, arylsulphonyloxy, alkylsulphonyloxy, aryloxysulfonyl, alkoxylsulfonyl and COOR¹²,
D represents a group of formula (VI) 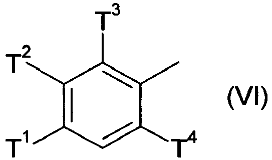 wherein
   T¹ to T⁴ independent from each other is
   hydrogen, alkyl, cycloalkyl, alkenyl, alkynyl, aryl, alkoxyl, halogen, cyano, nitro, acyl, aryloyl, arylsulfonyl, alkylsulfonyl, *N*-monoalkyl-sulfamoyl, *N,N*-dialkyl-sulfamoyl, alkoxylcarbonyl, aryloxycarbonyl, *N*-monoalkyl-carbamoyl, *N,N*-dialkyl-carbamoyl, aryloyloxy, acyloxy, aryloxy, thiocyano, hydroxyl, arylmethoxy, arylsulphonyloxy, alkylsulphonyloxy, aryloxysulfonyl, - [(alkoxycarbonyl)-methoxyl]carbonyl, [(aryloxycarbonyl)-methoxyl]carbonyl, [(alkylacyl)-methoxy]carbonyl, [(arylacyl)-methoxy]carbonyl, trifluoromethyl
   or
   alkyl interrupted by one or more heteroatoms selected from the group consisting of oxygen and sulfur
   or
   alkyl substituted by one or more substituents selected from the group consisting of hydroxy, aryl, cycloalkyl, alkoxy, thioalkoxy, amino, *N*-monoalkyl-amino, *N,N*-dialkyl-amino, *N-*monoaryl-amino, *N,N*-diaryl-amino, *N*-alkyl-*N*-aryl-amino, *N*-monocycloalkyl-amino, *N,N-*dicycloalkyl-amino, *N*-monoalkyl-monocycloalkyl-amino, *N,N-*monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, *N*-monoaryl-carbamoyl, *N,N*-diaryl-carbamoyl, *N*-monocycloalkyl-*N*-monoarylcarbamoyl, *N*-monoalkyl-*N*-monoaryl-carbamoyl, sulfamoyl, N-monocycloalkyl-sulfamoyl, *N*-monoalkyl-sulfamoyl, *N,N*-dicycloalkyl-sulfamoyl, *N,N-*dialkyl-sulfamoyl, *N*-monoaryl-sulfamoyl, *N,N*-diaryl-sulfamoyl, *N*-monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²
   or
   alkyl interrupted by one or more heteroatoms selected from the group consisting of oxygen and sulphur and substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N*-diaryl-amino, *N*-alkyl-*N*-aryl-amino, *N*-monocycloalkyl-amino, *N,N-*dicycloalkyl-amino, *N*-monoalkyl-monocycloalkyl-amino, *N*,*N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, *N-*monoalkyl-carbamoyl, *N,N*-dicycloalkyl-carbamoyl, *N,N-*dialkyl-carbamoyl, *N*-monoaryl-carbamoyl, *N,N*-diaryl-carbamoyl, *N*-monocycloalkyl-*N*-monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N*-monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²
   or
   aryl substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N*-diaryl-amino, *N*-alkyl-*N*-aryl-amino, *N*-monocycloalkyl-amino, *N,N-*dicycloalkyl-amino, *N*-monoalkyl-monocycloalkyl-amino, *N,N*-monoaryl-monocycloalkyl-amino, *N-*acylamino, *N*-alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, N-monoaryl-carbamoyl, *N,N*-diaryl-carbamoyl, *N*-monocycloalkyl-*N*-monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, N,N-dialkyl-sulfamoyl, N-monoaryl-sulfamoyl, *N,N*-diaryl-sulfamoyl, *N*-monocycloalkyl-*N*-monoarylsulfamoyl, *N*-monoalkyl-N-monoarylsulfamoyl, arylsulphonyloxy, alkylsulphonyloxy, aryloxysulfonyl, alkoxylsulfonyl and COOR¹², whereby at least one of T¹, T², T³ and T⁴ is not hydrogen
   or
D represents a group of formula (VII) 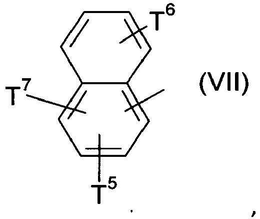 wherein
   T⁵ to T⁷ independent from each other is
   hydrogen, alkyl, cycloalkyl, alkenyl, alkynyl, aryl, alkoxyl, halogen, cyano, nitro, acyl, arylacyl, alkoxycarbonyl, aryloxycarbonyl, [(alkoxycarbonyl)-methoxyl]carbonyl, [(aryloxycarbonyl)-methoxyl]carbonyl, [(alkylacyl)-methoxy]carbonyl, [(arylacyl)-methoxy]carbonyl, *N-*monoalkyl-carbamoyl, *N,N*-dialkyl-carbamoyl
   or
   alkyl interrupted by one or more heteroatoms selected from the group consisting of oxygen and sulfur
   or
   alkyl substituted by one or more substituents selected from the group consisting of hydroxy, aryl, cycloalkyl, alkoxy, thioalkoxy, amino, *N*-monoalkyl-amino, *N,N*-dialkyl-amino, *N-*monoaryl-amino, *N,N*-diaryl-amino, *N*-alkyl-*N*-aryl-amino, *N*-monocycloalkyl-amino, *N,N-*dicycloalkyl-amino, *N*-monoalkyl-monocycloalkyl-amino, *N*,*N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N-*dialkyl-carbamoyl, *N*-monoaryl-carbamoyl, *N,N*-diaryl-carbamoyl, *N*-monocycloalkyl-*N*-monoarylcarbamoyl, *N*-monoalkyl-*N*-monoaryl-carbamoyl, sulfamoyl, N-monocycloalkyl-sulfamoyl, *N*-monoalkyl-sulfamoyl, *N,N-*dicycloalkyl-sulfamoyl*, N,N*-dialkyl-sulfamoyl, *N*-monoaryl-sulfamoyl, *N,N*-diaryl-sulfamoyl, *N*-monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²
   or
   alkyl interrupted by one or more heteroatoms selected from the group consisting of oxygen and sulphur and substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N*-diaryl-amino, *N*-alkyl-*N*-aryl-amino, *N*-monocycloalkyl-amino, *N,N-*dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, *N-*monoalkyl-carbamoyl, *N,N-*dicycloalkyl-carbamoyl, *N,N*-dialkyl-carbamoyl, *N*-monoaryl-carbamoyl, *N,N*-diaryl-carbamoyl, *N*-monocycloalkyl-*N*-monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N-*diaryl-sulfamoyl, *N*-monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²
   or
   aryl substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N*-diaryl-amino, *N*-alkyl-*N*-aryl-amino, *N*-monocycloalkyl-amino, *N,N*-dicycloalkyl-amino, *N*-monoalkyl-monocycloalkyl-amino, *N,N*-monoaryl-monocycloalkyl-amino, *N-*acylamino, N-alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, N-monoaryl-carbamoyl, *N,N*-diaryl-carbamoyl, *N*-monocycloalkyl-*N*-monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, N,N-dialkyl-sulfamoyl, *N*-monoaryl-sulfamoyl, *N,N*-diaryl-sulfamoyl, *N*-monocycloalkyl-*N*-monoarylsulfamoyl, *N*-monoalkyl-N-monoarylsulfamoyl, arylsulphonyloxy, alkylsulphonyloxy, aryloxysulfonyl, alkoxylsulfonyl and COOR¹²,
   whereby at least one of T⁵, T⁶ and T⁷ is not hydrogen,
   or
D represents a group of the formula (VIII) 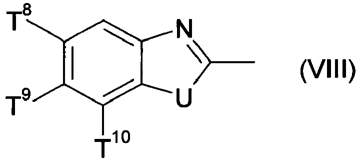 wherein
   U is oxygen, sulphur or N-R¹⁵,
   T⁸ and T¹⁰ independent of each other is hydrogen, fluorine, chlorine or bromine,
   T⁹ is hydrogen, alkylsulfonyl, thiocyano, alkoxy, halogen or nitro,
R¹⁵ is alkyl, aryl or cycloalkyl,
   or
D represents a group of the formula (IX) 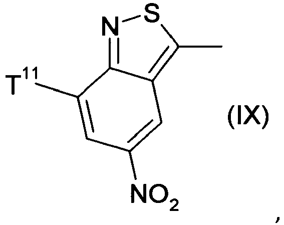 wherein
   T¹¹ is hydrogen, nitro or halogen,
   or
D represents a group of the formula (X) 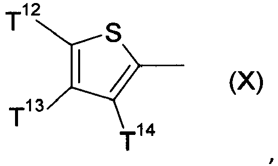 wherein
   T¹² and T¹⁴ independent from each other is nitro, cyano, acyl or alkoxycarbonyl,
   T¹³ is hydrogen, aryl, alkyl, halogen
   or
   alkyl interrupted by one or more heteroatoms selected from the group consisting of oxygen and sulfur
   or
   alkyl substituted by one or more substituents selected from the group consisting of hydroxy, aryl, cycloalkyl, alkoxy, thioalkoxy, amino, *N*-monoalkyl-amino, *N,N*-dialkyl-amino, *N-*monoaryl-amino, *N,N*-diaryl-amino, *N*-alkyl-*N*-aryl-amino, *N*-monocycloalkyl-amino, *N,N-*dicycloalkyl-amino, *N*-monoalkyl-monocycloalkyl-amino, *N*,*N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, *N*-monoaryl-carbamoyl, *N,N*-diaryl-carbamoyl, *N*-monocycloalkyl-*N*-monoarylcarbamoyl, *N*-monoalkyl-*N*-monoaryl-carbamoyl, sulfamoyl, N-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N*-monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²
   or
   alkyl interrupted by one or more heteroatoms selected from the group consisting of oxygen and sulphur and substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N*-diaryl-amino, *N*-alkyl-*N*-aryl-amino, *N*-monocycloalkyl-amino, *N,N-*dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, *N-*monoalkyl-carbamoyl, *N,N*-dicycloalkyl-carbamoyl, *N,N*-dialkyl-carbamoyl, *N*-monoaryl-carbamoyl, *N,N*-diaryl-carbamoyl, *N*-monocycloalkyl-*N*-monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N,N*-dicycloalkyl-sulfamoyl, *N,N-*dialkyl-sulfamoyl, *N*-monoaryl-sulfamoyl, *N,N*-diaryl-sulfamoyl, *N*-monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²
   or
   aryl substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N*-diaryl-amino, *N*-alkyl-*N*-aryl-amino, *N*-monocycloalkyl-amino, *N,N*-dicycloalkyl-amino, *N*-monoalkyl-monocycloalkyl-amino, *N,N*-monoaryl-monocycloalkyl-amino, *N-*acylamino, *N*-alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, N-monoaryl-carbamoyl, *N,N*-diaryl-carbamoyl, *N*-monocycloalkyl-*N*-monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, N,N-dialkyl-sulfamoyl, N-monoaryl-sulfamoyl, *N,N*-diaryl-sulfamoyl, *N*-monocycloalkyl-*N*-monoarylsulfamoyl, *N*-monoalkyl-N-monoarylsulfamoyl, arylsulphonyloxy, alkylsulphonyloxy, aryloxysulfonyl, alkoxylsulfonyl and COOR¹²,
   or
D represents a group of the formula (XI) 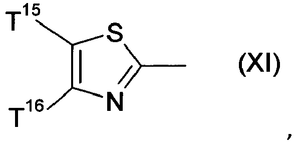 wherein
   T¹⁵ and T¹⁶ independent from each other is hydrogen, halogen, alkyl, nitro, cyano, acyl, alkoxycarbonyl, aryl
   or
   alkyl interrupted by one or more heteroatoms selected from the group consisting of oxygen and sulfur
   or
   alkyl substituted by one or more substituents selected from the group consisting of hydroxy, aryl, cycloalkyl, alkoxy, thioalkoxy, amino, *N*-monoalkyl-amino, *N,N*-dialkyl-amino, *N-*monoaryl-amino, *N,N*-diaryl-amino, *N*-alkyl-*N*-aryl-amino, *N*-monocycloalkyl-amino, *N,N-*dicycloalkyl-amino, *N*-monoalkyl-monocycloalkyl-amino, *N,N-*monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, *N*-monoaryl-carbamoyl, *N,N*-diaryl-carbamoyl, *N*-monocycloalkyl-*N*-monoarylcarbamoyl, *N*-monoalkyl-*N*-monoaryl-carbamoyl, sulfamoyl, N-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N*-monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²
   or
   alkyl interrupted by one or more heteroatoms selected from the group consisting of oxygen and sulphur and substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N*-diaryl-amino, *N*-alkyl-*N*-aryl-amino, *N*-monocycloalkyl-amino, *N,N-*dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, *N-*monoalkyl-carbamoyl, *N,N*-dicycloalkyl-carbamoyl, *N,N*-dialkyl-carbamoyl, *N*-monoaryl-carbamoyl, *N,N*-diaryl-carbamoyl, *N*-monocycloalkyl-*N*-monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N*-monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²
   or
   aryl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N*-diaryl-amino, *N*-alkyl-*N*-aryl-amino, *N*-monocycloalkyl-amino, *N,N*-dicycloalkyl-amino, *N*-monoalkyl-monocycloalkyl-amino, *N,N-*monoaryl-monocycloalkyl-amino, *N-*acylamino, *N*-alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, N-monoaryl-carbamoyl, *N,N*-diaryl-carbamoyl, *N*-monocycloalkyl-*N*-monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, N,N-dialkyl-sulfamoyl, *N*-monoaryl-sulfamoyl, *N,N*-diaryl-sulfamoyl, *N*-monocycloalkyl-*N*-monoarylsulfamoyl, *N*-monoalkyl-N-monoarylsulfamoyl, arylsulphonyloxy, alkylsulphonyloxy, aryloxysulfonyl, alkoxylsulfonyl and COOR¹²
   whereby at least one of T¹⁵ and T¹⁶ is not hydrogen,
   or
D represents a group of the formula (XII) 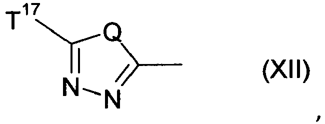 wherein
   T¹⁷ is aryl, thioalkoxyl
      or
      aryl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N*-diaryl-amino, *N*-alkyl-*N*-aryl-amino, *N*-monocycloalkyl-amino, *N,N*-dicycloalkyl-amino, *N*-monoalkyl-monocycloalkyl-amino, *N,N*-monoaryl-monocycloalkyl-amino, *N-*acylamino, *N*-alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N-*dicycloalkyl-carbamoyl, *N,N*-dialkyl-carbamoyl, N-monoaryl-carbamoyl, *N,N*-diaryl-carbamoyl, *N*-monocycloalkyl-*N*-monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, N,N-dialkyl-sulfamoyl, *N*-monoaryl-sulfamoyl, *N,N*-diaryl-sulfamoyl, *N*-monocycloalkyl-*N*-monoarylsulfamoyl, *N*-monoalkyl-N-monoarylsulfamoyl, arylsulphonyloxy, alkylsulphonyloxy, aryloxysulfonyl, alkoxylsulfonyl and COOR¹²,
   Q is oxygen or sulphur,
   or
D represents a group of the formula (XIII) 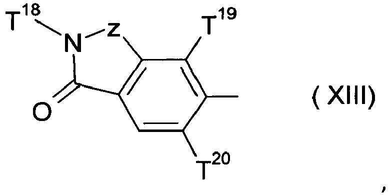 wherein
   T¹⁸ is alkyl, alkenyl, aryl
      or
      alkyl interrupted by one or more heteroatoms selected from the group consisting of oxygen and sulfur
      or
      alkyl substituted by one or more substituents selected from the group consisting of hydroxy, aryl, cycloalkyl, alkoxy, thioalkoxy, amino, *N*-monoalkyl-amino, *N,N*-dialkyl-amino, *N-*monoaryl-amino, *N,N*-diaryl-amino, *N*-alkyl-*N*-aryl-amino, *N*-monocycloalkyl-amino, *N,N-*dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, *N*-monoaryl-carbamoyl, *N,N*-diaryl-carbamoyl, *N*-monocycloalkyl-*N*-monoarylcarbamoyl, *N*-monoalkyl-*N*-monoaryl-carbamoyl, sulfamoyl, N-monocycloalkyl-sulfamoyl, *N*-monoalkyl-sulfamoyl, *N,N*-dicycloalkyl-sulfamoyl, *N,N-*dialkyl-sulfamoyl, *N*-monoaryl-sulfamoyl, *N,N-*diaryl-sulfamoyl, *N*-monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²
      or
      alkyl interrupted by one or more heteroatoms selected from the group consisting of oxygen and sulphur and substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N-*diaryl-amino, *N*-alkyl-*N*-aryl-amino, *N*-monocycloalkyl-amino, *N,N-*dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, *N-*monoalkyl-carbamoyl, *N,N*-dicycloalkyl-carbamoyl, *N,N*-dialkyl-carbamoyl, *N*-monoaryl-carbamoyl, *N,N*-diaryl-carbamoyl, *N*-monocycloalkyl-*N*-monoarylcarbamoyl, *N*-monoalkyl-N-monoaryl-carbamoyl, sulfamoyl, *N*-monocycloalkyl-sulfamoyl, *N*-monoalkyl-sulfamoyl, *N,N-*dicycloalkyl-sulfamoyl, *N,N*-dialkyl-sulfamoyl, *N*-monoaryl-sulfainoyl, *N,N-*diaryl-sulfamoyl, *N*-monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl, and COOR¹²
      or
      aryl substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N*-diaryl-amino, *N*-alkyl-*N*-aryl-amino, *N*-monocycloalkyl-amino, *N,N*-dicycloalkyl-amino, *N*-monoalkyl-monocycloalkyl-amino, *N,N-*monoaryl-monocycloalkyl-amino, *N-*acylamino, N-alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, N-monoaryl-carbamoyl, *N,N*-diaryl-carbamoyl, *N*-monocycloalkyl-*N*-monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, N,N-dialkyl-sulfamoyl, *N*-monoaryl-sulfamoyl, *N,N*-diaryl-sulfamoyl, *N*-monocycloalkyl-*N*-monoarylsulfamoyl, *N*-monoalkyl-N-monoarylsulfamoyl, arylsulphonyloxy, alkylsulphonyloxy, aryloxysulfonyl, alkoxylsulfonyl and COOR¹²,
   T¹⁹ and T²⁰ independent from each other is hydrogen, halogen, cyano, nitro or trifluoromethyl,
   Z is a carbonyl or a sulfonyl rest,
   or
D represents a group of the formula (XIV) 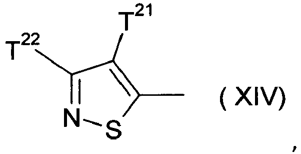 wherein
   T²¹ is cyano, nitro or alkoxycarbonyl,
   T²² is alkyl, hydrogen, halogen, aryl
   or
   alkyl interrupted by one or more heteroatoms selected from the group consisting of oxygen and sulfur
   or
   alkyl substituted by one or more substituents selected from the group consisting of hydroxy, aryl, cycloalkyl, alkoxy, thioalkoxy, amino, *N*-monoalkyl-amino, *N,N*-dialkyl-amino, *N-*monoaryl-amino, *N,N*-diaryl-amino, *N*-alkyl-*N*-aryl-amino, *N*-monocycloalkyl-amino, *N,N-*dicycloalkyl-amino, *N*-monoalkyl-monocycloalkyl-amino, *N,N-*monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, *N*-monoaryl-carbamoyl, *N,N*-diaryl-carbamoyl, N-monocycloalkyl-*N*-monoarylcarbamoyl, *N*-monoalkyl-*N*-monoaryl-carbamoyl, sulfamoyl, N-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N*-monocycloalkyl-*N*-monoarylsulfamoyl, *N-*monoalkyl-*N-*monoarylsulfamoyl, arylsulphonyloxy, alkylsulphonyloxy, aryloxysulfonyl, alkoxylsulfonyl and COOR¹²
   or
   alkyl interrupted by one or more heteroatoms selected from the group consisting of oxygen or sulphur and substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N*-diaryl-amino, *N*-alkyl-*N*-aryl-amino, *N*-monocycloalkyl-amino, *N,N-*dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, *N-*monoalkyl-carbamoyl, *N,N*-dicycloalkyl-carbamoyl, *N,N*-dialkyl-carbamoyl, *N*-monoaryl-carbamoyl, *N,N*-diaryl-carbamoyl, *N*-monocycloalkyl-*N*-monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N*,*N*-diaryl-sulfamoyl, *N*-monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²
   or
   aryl substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N*-diaryl-amino, *N*-alkyl-*N*-aryl-amino, *N*-monocycloalkyl-amino, *N,N*-dicycloalkyl-amino, *N*-monoalkyl-monocycloalkyl-amino, *N,N*-monoaryl-monocycloalkyl-amino, *N-*acylamino, *N*-alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N-*dialkyl-carbamoyl, N-monoaryl-carbamoyl, *N,N*-diaryl-carbamoyl, *N*-monocycloalkyl-*N*-monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, N,N-dialkyl-sulfamoyl, *N*-monoaryl-sulfamoyl, *N,N*-diaryl-sulfamoyl, *N*-monocycloalkyl-*N*-monoarylsulfamoyl, *N*-monoalkyl-N-monoarylsulfamoyl, arylsulphonyloxy, alkylsulphonyloxy, aryloxysulfonyl, alkoxylsulfonyl and COOR¹²,
   or
D represents a group of the formula (XV) 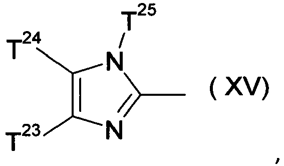 wherein
   T²³ and T²⁴ independent from each other is cyano, nitro, halogen, aryl or trifluoromethyl,
   T²⁵ is hydrogen, alkyl, alkenyl, aryl
   or
   alkyl interrupted by one or more heteroatoms selected from the group consisting of oxygen and sulphur
   or
   alkyl substituted by one or more substituents selected from the group consisting of hydroxy, aryl, cycloalkyl, alkoxy, thioalkoxy, amino, *N*-monoalkyl-amino, *N,N*-dialkyl-amino, *N-*monoaryl-amino, *N,N*-diaryl-amino, *N*-alkyl-*N*-aryl-amino, *N*-monocycloalkyl-amino, *N,N-*dicycloalkyl-amino, *N*-monoalkyl-monocycloalkyl-amino, *N*,*N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N-*dicycloalkyl-carbamoyl, *N,N*-dialkyl-carbamoyl, *N*-monoaryl-carbamoyl, *N,N*-diaryl-carbamoyl, *N*-monocycloalkyl-*N*-monoarylcarbamoyl, *N*-monoalkyl-*N*-monoaryl-carbamoyl, sulfamoyl, N-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N*-monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²
   or alkyl interrupted by one or more heteroatoms selected from the group consisting of oxygen and sulphur and substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N*-diaryl-amino, *N*-alkyl-*N*-aryl-amino, *N*-monocycloalkyl-amino, *N,N-*dicycloalkyl-amino, *N*-monoalkyl-monocycloalkyl-amino, *N*,*N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, *N-*monoalkyl-carbamoyl, *N,N*-dicycloalkyl-carbamoyl, *N,N*-dialkyl-carbamoyl, *N*-monoaryl-carbamoyl, *N,N*-diaryl-carbamoyl, *N*-monocycloalkyl-*N*-monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N-*diaryl-sulfamoyl, *N*-monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²
   or
   aryl substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N*-diaryl-amino, *N*-alkyl-*N*-aryl-amino, *N*-monocycloalkyl-amino, *N,N*-dicycloalkyl-amino, *N*-monoalkyl-monocycloalkyl-amino, *N,N*-monoaryl-monocycloalkyl-amino, *N-*acylamino, *N*-alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, N-monoaryl-carbamoyl, *N,N*-diaryl-carbamoyl, *N*-monocycloalkyl-*N*-monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, N,N-dialkyl-sulfamoyl, *N*-monoaryl-sulfamoyl, *N,N*-diaryl-sulfamoyl, *N*-monocycloalkyl-*N*-monoarylsulfamoyl, *N*-monoalkyl-N-monoarylsulfamoyl, arylsulphonyloxy, alkylsulphonyloxy, aryloxysulfonyl, alkoxylsulfonyl and COOR¹²
   or
D represents a group of the formula (XVI) 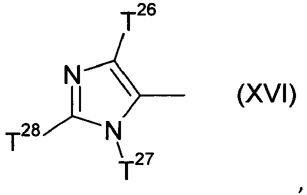 wherein
   T²⁶ is cyano, nitro, aryl or trifluoromethyl,
   T²⁷ is hydrogen, aryl, alkly or
      alkyl interrupted by one or more heteroatoms selected from the group consisting of oxygen and sulfur
      or
      alkyl substituted by one or more substituents selected from the group consisting of hydroxy, aryl, cycloalkyl, alkoxy, thioalkoxy, amino, *N*-monoalkyl-amino, *N,N*-dialkyl-amino, *N-*monoaryl-amino, *N,N*-diaryl-amino, *N*-alkyl-*N*-aryl-amino, *N*-monocycloalkyl-amino, *N,N-*dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, *N*-monoaryl-carbamoyl, *N,N*-diaryl-carbamoyl, *N*-monocycloalkyl-*N*-monoarylcarbamoyl, *N*-monoalkyl-*N*-monoaryl-carbamoyl, sulfamoyl, N-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N*-monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²
      or
      alkyl interrupted by one or more heteroatoms selected from the group consisting of oxygen and sulphur and substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N*-diaryl-amino, *N*-alkyl-*N*-aryl-amino, *N*-monocycloalkyl-amino, *N,N-*dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, *N-*monoalkyl-carbamoyl, *N,N*-dicycloalkyl-carbamoyl, *N,N*-dialkyl-carbamoyl, *N*-monoaryl-carbamoyl, *N,N*-diaryl-carbamoyl, *N*-monocycloalkyl-*N*-monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N*-monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²
      or
      aryl substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N*-diaryl-amino, *N*-alkyl-*N*-aryl-amino, N-monocycloalkyl-amino, *N,N*-dicycloalkyl-amino, *N*-monoalkyl-monocycloalkyl-amino, *N,N*-monoaryl-monocycloalkyl-amino, *N-*acylamino, *N*-alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, N-monoaryl-carbamoyl, *N,N*-diaryl-carbamoyl, *N*-monocycloalkyl-*N*-monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, N,N-dialkyl-sulfamoyl, *N*-monoaryl-sulfamoyl, *N,N*-diaryl-sulfamoyl, *N*-monocycloalkyl-*N*-monoarylsulfamoyl, *N*-monoalkyl-N-monoarylsulfamoyl, arylsulphonyloxy, alkylsulphonyloxy, aryloxysulfonyl, alkoxylsulfonyl and COOR¹²,
   T²⁸ is hydrogen, alkyl, alkenyl, alkynyl, aryl, alkoxycarbonyl
   or
   alkyl interrupted by one or more heteroatoms selected from the group consisting of oxygen and sulfur
   or
   alkyl substituted by one or more substituents selected from the group consisting of hydroxy, aryl, cycloalkyl, alkoxy, thioalkoxy, amino, *N*-monoalkyl-amino, *N,N*-dialkyl-amino, *N-*monoaryl-amino, *N,N*-diaryl-amino, *N*-alkyl-*N*-aryl-amino, *N*-monocycloalkyl-amino, *N,N-*dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N-*dicycloalkyl-carbamoyl, *N,N*-dialkyl-carbamoyl, *N*-monoaryl-carbamoyl, *N,N*-diaryl-carbamoyl, *N*-monocycloalkyl-*N*-monoarylcarbamoyl, *N*-monoalkyl-*N*-monoaryl-carbamoyl, sulfamoyl, N-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N*-monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²
   or
   alkyl interrupted by one or more heteroatoms selected from the group consisting of oxygen and sulphur and substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N*-diaryl-amino, *N*-alkyl-*N*-aryl-amino, *N*-monocycloalkyl-amino, *N,N-*dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, *N-*monoalkyl-carbamoyl, *N,N*-dicycloalkyl-carbamoyl, *N,N-*dialkyl-carbamoyl, *N*-monoaryl-carbamoyl, *N,N*-diaryl-carbamoyl, *N*-monocycloalkyl-*N*-monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N*-monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²
   or
   aryl substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N*-diaryl-amino, *N*-alkyl-*N*-aryl-amino, *N*-monocycloalkyl-amino, *N,N*-dicycloalkyl-amino, *N*-monoalkyl-monocycloalkyl-amino, *N,N*-monoaryl-monocycloalkyl-amino, *N-*acylamino, *N*-alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, N-monoaryl-carbamoyl, *N,N*-diaryl-carbamoyl, *N*-monocycloalkyl-*N*-monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, N,N-dialkyl-sulfamoyl, *N*-monoaryl-sulfamoyl, *N,N*-diaryl-sulfamoyl, *N*-monocycloalkyl-*N*-monoarylsulfamoyl, *N*-monoalkyl-N-monoarylsulfamoyl, arylsulphonyloxy, alkylsulphonyloxy, aryloxysulfonyl, alkoxylsulfonyl and COOR¹²,
   or
D represents a group of the formula (XVII) 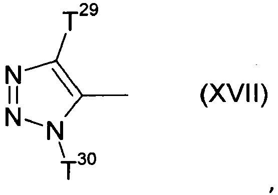 wherein
   T²⁹ is cyano, nitro, aryl or trifluoromethyl,
   T³⁰ is hydrogen, alkyl, alkenyl, alkenyl, alkynyl, aryl, arylmethyl, alkoxycarbonyl
   or
   alkyl interrupted by one or more heteroatoms selected from the group consisting of oxygen and sulfur
   or
   alkyl substituted by one or more substituents selected from the group consisting of hydroxy, aryl, cycloalkyl, alkoxy, thioalkoxy, amino, *N*-monoalkyl-amino, *N,N*-dialkyl-amino, *N-*monoaryl-amino, *N,N*-diaryl-amino, *N*-alkyl-*N*-aryl-amino, *N*-monocycloalkyl-amino, *N,N-*dicycloalkyl-amino, *N*-monoalkyl-monocycloalkyl-amino, *N*,*N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N-*dialkyl-carbamoyl, *N*-monoaryl-carbamoyl, *N,N*-diaryl-carbamoyl, *N*-monocycloalkyl-*N*-monoarylcarbamoyl, *N*-monoalkyl-*N*-monoaryl-carbamoyl, sulfamoyl, N-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N*-monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²
   or
   alkyl interrupted by one or more heteroatoms selected from the group consisting of oxygen and sulphur and substituted by one or more substituents selected from the group consisting of hydroxy, aryl, cycloalkyl, alkoxy, thioalkoxy, amino, *N*-monoalkyl-amino, *N*,*N*-dialkyl-amino, *N*-monoaryl-amino, *N,N*-diaryl-amino, *N*-alkyl-*N*-aryl-amino, *N*-monocycloalkyl-amino, *N,N-*dicycloalkyl-amino, *N*-monoalkyl-monocycloalkyl-amino, *N*,*N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, *N-*monoalkyl-carbamoyl, *N,N*-dicycloalkyl-carbamoyl, *N,N-*dialkyl-carbamoyl, *N*-monoaryl-carbamoyl, *N,N*-diaryl-carbamoyl, *N*-monocycloalkyl-*N*-monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N,N*-dicycloalkyl-sulfamoyl, *N*,*N*-dialkyl-sulfamoyl, *N*-monoaryl-sulfamoyl, *N,N-*diaryl-sulfamoyl, *N*-monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²
   or
   aryl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N*-diaryl-amino, *N*-alkyl-*N*-aryl-amino, *N*-monocycloalkyl-amino, *N,N*-dicycloalkyl-amino, *N*-monoalkyl-monocycloalkyl-amino, *N,N*-monoaryl-monocycloalkyl-amino, *N-*acylamino, *N*-alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, N-monoaryl-carbamoyl, *N,N*-diaryl-carbamoyl, *N*-monocycloalkyl-*N*-monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, N,N-dialkyl-sulfamoyl, *N*-monoaryl-sulfamoyl, *N,N*-diaryl-sulfamoyl, *N*-monocycloalkyl-*N*-monoarylsulfamoyl, *N*-monoalkyl-N-monoarylsulfamoyl, arylsulphonyloxy, alkylsulphonyloxy, aryloxysulfonyl, alkoxylsulfonyl and COOR¹²,
   or
D represents a group of the formula (XVIII) 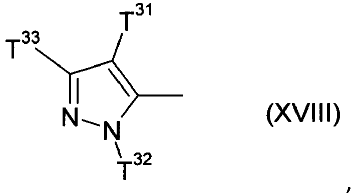 wherein
   T³¹ is cyano, nitro, aryl, trifluoromethyl
      or
      aryl substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N*-diaryl-amino, *N*-alkyl-*N*-aryl-amino, *N*-monocycloalkyl-amino, *N,N*-dicycloalkyl-amino, *N*-monoalkyl-monocycloalkyl-amino, *N,N*-monoaryl-monocycloalkyl-amino, *N-*acylamino, *N*-alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, N-monoarylcarbamoyl, *N,N*-diaryl-carbamoyl, *N*-monocycloalkyl-*N*-monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, N,N-dialkyl-sulfamoyl, *N*-monoaryl-sulfamoyl, *N,N*-diaryl-sulfamoyl, *N*-monocycloalkyl-*N*-monoarylsulfamoyl, *N*-monoalkyl-N-monoarylsulfamoyl, arylsulphonyloxy, alkylsulphonyloxy, aryloxysulfonyl, alkoxylsulfonyl and COOR¹²,
   T³² is hydrogen, alkyl, alkenyl, alkynyl, aryl, alkoxycarbonyl
      or
      alkyl interrupted by one or more heteroatoms selected from the group consisting of oxygen and sulphur
      or
      alkyl substituted by one or more substituents selected from the group consisting of hydroxy, aryl, cycloalkyl, alkoxy, thioalkoxy, amino, *N*-monoalkyl-amino, *N*,*N*-dialkyl-amino, *N-*monoaryl-amino, *N,N*-diaryl-amino, *N*-alkyl-*N*-aryl-amino, *N*-monocycloalkyl-amino, *N,N-*dicycloalkyl-amino, *N*-monoalkyl-monocycloalkyl-amino, *N*,*N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, *N*-monoarylcarbamoyl, *N*,*N*-diaryl-carbamoyl, *N*-monocycloalkyl-*N*-monoarylcarbamoyl, *N*-monoalkyl-*N*-monoaryl-carbamoyl, sulfamoyl, N-monocycloalkyl-sulfamoyl, *N*-monoalkyl-sulfamoyl, *N*,*N*-dicycloalkyl-sulfamoyl, *N*,*N*-dialkyl-sulfamoyl, *N*-monoaryl-sulfamoyl, *N,N*-diaryl-sulfamoyl, *N*-monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²
      or
      alkyl interrupted by one or more heteroatoms selected from the group consisting of oxygen and sulphur and substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N*-diaryl-amino, *N*-alkyl-*N*-aryl-amino, *N*-monocycloalkyl-amino, *N,N-*dicycloalkyl-amino, *N*-monoalkyl-monocycloalkyl-amino, *N*,*N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, *N-*monoalkyl-carbamoyl, *N,N*-dicycloalkyl-carbamoyl, *N,N*-dialkyl-carbamoyl, *N*-monoarylcarbamoyl, *N,N*-diaryl-carbamoyl, *N*-monocycloalkyl-*N*-monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N*,*N*-dicycloalkyl-sulfamoyl, *N*,*N*-dialkyl-sulfamoyl, *N*-monoaryl-sulfamoyl, *N*,*N*-diaryl-sulfamoyl, *N*-monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²
      or
      aryl substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N*,*N*-diaryl-amino, *N*-alkyl-*N*-aryl-amino, *N*-monocycloalkyl-amino, *N*,*N*-dicycloalkyl-amino, *N*-monoalkyl-monocycloalkyl-amino, *N*,*N*-monoaryl-monocycloalkyl-amino, *N-*acylamino, *N*-alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N*,*N*-dicycloalkyl-carbamoyl, *N*,*N*-dialkyl-carbamoyl, N-monoarylcarbamoyl, *N*,*N*-diaryl-carbamoyl, *N*-monocycloalkyl-*N*-monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, N,N-dialkyl-sulfamoyl, *N*-monoaryl-sulfamoyl, *N*,*N*-diaryl-sulfamoyl, *N*-monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²,
   T³³ is hydrogen, alkyl
   or
   alkyl interrupted by one or more heteroatoms selected from the group consisting of oxygen and sulphur
   or
   alkyl substituted by one or more substituents selected from the group consisting of hydroxy, aryl, cycloalkyl, alkoxy, thioalkoxy, amino, *N*-monoalkyl-amino, *N,N*-dialkyl-amino, *N-*monoaryl-amino, *N*,*N*-diaryl-amino, *N*-alkyl-*N*-aryl-amino, *N*-monocycloalkyl-amino, *N,N-*dicycloalkyl-amino, *N*-monoalkyl-monocycloalkyl-amino, *N*,*N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N*,*N*-dialkyl-carbamoyl, *N*-monoarylcarbamoyl, *N,N*-diaryl-carbamoyl, *N*-monocycloalkyl-*N*-monoarylcarbamoyl, *N*-monoalkyl-*N*-monoaryl-carbamoyl, sulfamoyl, N-monocycloalkyl-sulfamoyl, *N*-monoalkyl-sulfamoyl, *N*,*N*-dicycloalkyl-sulfamoyl, *N*,*N*-dialkyl-sulfamoyl, *N*-monoaryl-sulfamoyl, *N*,*N*-diaryl-sulfamoyl, *N*-monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²
   or
   alkyl interrupted by one or more heteroatoms selected from the group consisting of oxygen and sulphur and substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N*,*N*-diaryl-amino, *N*-alkyl-*N*-aryl-amino, *N*-monocycloalkyl-amino, *N,N-*dicycloalkyl-amino, *N*-monoalkyl-monocycloalkyl-amino, *N*,*N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, *N-*monoalkyl-carbamoyl, *N,N*-dicycloalkyl-carbamoyl, *N*,*N*-dialkyl-carbamoyl, *N*-monoarylcarbamoyl, *N*,*N*-diaryl-carbamoyl, *N*-monocycloalkyl-*N*-monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N*,*N*-dicycloalkyl-sulfamoyl, *N*,*N*-dialkyl-sulfamoyl, *N*-monoaryl-sulfamoyl, *N*,*N*-diaryl-sulfamoyl, *N*-monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²
   or
   aryl substituted by one or more substituents selected from the group consisting of hydroxy, aryl, cycloalkyl, alkoxy, thioalkoxy, amino, *N*-monoalkyl-amino, *N*,*N*-dialkyl-amino, *N*-monoaryl-amino, *N,N*-diaryl-amino, *N*-alkyl-*N*-aryl-amino, *N*-monocycloalkyl-amino, *N*,*N*-dicycloalkyl-amino, *N*-monoalkyl-monocycloalkyl-amino, *N*,*N*-monoaryl-monocycloalkyl-amino, *N-*acylamino, *N*-alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N*,*N*-dicycloalkyl-carbamoyl, *N,N*-dialkyl-carbamoyl, N-monoarylcarbamoyl, *N,N*-diaryl-carbamoyl, *N*-monocycloalkyl-*N*-monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, *N*,*N*-dialkyl-sulfamoyl, *N*-monoaryl-sulfamoyl, *N*,*N*-diaryl-sulfamoyl, *N*-monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²,
   or
D represents a group of the formula (XIX) 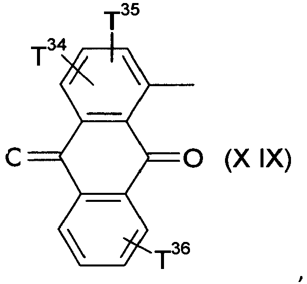 wherein
   T³⁴, T³⁵ and T³⁶ independent from each other is hydrogen, halogen, nitro, cyano, hydroxyl, alkoxylcarbonyl, alkyl, acyl
   or
   alkyl substituted by one or more substituents selected from the group consisting of hydroxy, aryl, cycloalkyl, alkoxy, thioalkoxy, amino, *N*-monoalkyl-amino, *N*,*N*-dialkyl-amino, *N-*monoaryl-amino, *N*,*N*-diaryl-amino, *N*-alkyl-*N*-aryl-amino, *N*-monocycloalkyl-amino, *N,N-*dicycloalkyl-amino, *N*-monoalkyl-monocycloalkyl-amino, *N*,*N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N*,*N*-dicycloalkyl-carbamoyl, *N*,*N*-dialkyl-carbamoyl, *N*-monoarylcarbamoyl, *N,N*-diaryl-carbamoyl, *N*-monocycloalkyl-*N*-monoarylcarbamoyl, *N*-monoalkyl-*N*-monoaryl-carbamoyl, sulfamoyl, N-monocycloalkyl-sulfamoyl, *N*-monoalkyl-sulfamoyl, *N*,*N*-dicycloalkyl-sulfamoyl, *N*,*N*-dialkyl-sulfamoyl, *N*-monoaryl-sulfamoyl, *N*,*N*-diaryl-sulfamoyl, *N*-monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²
   or
   alkyl interrupted by one or more heteroatoms selected from the group consisting of oxygen and sulphur and substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N*,*N*-diaryl-amino, *N*-alkyl-*N*-aryl-amino, *N*-monocycloalkyl-amino, *N,N-*dicycloalkyl-amino, *N*-monoalkyl-monocycloalkyl-amino, *N*,*N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, *N-*monoalkyl-carbamoyl, *N,N*-dicycloalkyl-carbamoyl, *N,N*-dialkyl-carbamoyl, *N*-monoarylcarbamoyl, *N,N*-diaryl-carbamoyl, *N*-monocycloalkyl-*N*-monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N*,*N*-dicycloalkyl-sulfamoyl, *N*,*N*-dialkyl-sulfamoyl, *N*-monoaryl-sulfamoyl, *N*,*N*-diaryl-sulfamoyl, *N*-monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²,
   or
D represents a group of the formula (XX) 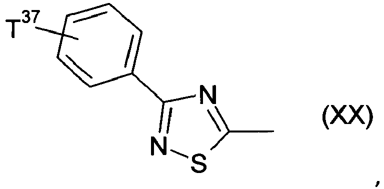 wherein
   T³⁷ is hydrogen, halogen or nitro,
   R¹² is hydrogen, alkyl, alkenyl, cycloalkyl, aryl, heteroaryl, heterocycloalkyl
   or
   alkyl interrupted by one or more heteroatoms selected from the group consisting of oxygen and sulfur
   or
   alkyl substituted by one or more substituents selected from the group consisting of hydroxy, aryl, cycloalkyl, alkoxy, thioalkoxy, halogen, cyano, amino, thiocyano, nitro, acyl, thioacyl, alkylsulfonyl, aryloyl, trifluoromethyl, heteroaryl, heterocycloalkyl, alkoxythiocarbonyl, acyloxy, aryloyloxy, carbamoyl, sulfamoyl, arylsulphonyloxy, alkylsulphonyloxy, aryloxysulfonyl and alkoxylsulfonyl
   or
   alkyl interrupted by one or more heteroatoms selected from the group consisting of oxygen and sulphur and substituted by one or more substituents selected from the group consisting of hydroxy, aryl, cycloalkyl, alkoxy, thioalkoxy, halogen, cyano, amino, thiocyano, nitro, acyl, thioacyl, alkylsulfonyl, aryloyl, trifluoromethyl, heteroaryl, heterocycloalkyl, alkoxythiocarbonyl, acyloxy, aryloyloxy, carbamoyl, sulfamoyl, arylsulphonyloxy, alkylsulphonyloxy, aryloxysulfonyl and alkoxylsulfonyl
   or
   aryl substituted by one or more substituents selected from the group consisting of hydroxy, aryl, cycloalkyl, alkoxy, thioalkoxy, amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, sulfamoyl, arylsulphonyloxy, alkylsulphonyloxy, aryloxysulfonyl and alkoxylsulfonyl,
   with the proviso that no fused-ring formation is allowed among R² to R¹⁴.

This invention refers to all tautomeric and geometric isomers of the dyes of formula (I) and mixtures thereof.

Alkyl groups appearing in this invention may be straight-chain or branched and are (C₁-C₁₂)-alkyl groups, preferably (C₁-C₆)-alkyl groups, for example methyl, ethyl, propyl, isopropyl, n-butyl, isobutyl, *tert*-butyl, *n*-pentyl, isopentyl or *n*-hexyl.

The same applies to alkoxy groups which accordingly are preferably (C₁-C₈)-alkoxy, for example methoxy and ethoxy, to thioalkoxy groups, which are preferably (C₁-C₈)-thioalkoxy, for example methylsulfanyl or ethylsulfanyl.

Cycloalkyl groups are preferably (C₃-C₈)-cycloalkyl and especially preferably cyclopentyl and cyclohexyl. The term cyloalkyl comprises for the purpose of the present invention substituted cycloalklyl groups and unsatured cycloalkyl groups as well. A preferred group of this type is cyclopentenyl. Preferred substituents are alkyl, hydroxyalkyl, halogen, hydroxyl, alkoxy, acyl, cyano, nitro, amino, monoalkylamino, dialkylamino, mono(hydroxyalkyl)amino, bis (hydroxyalkyl)amino, monoalkyl-mono(hydroxyalkyl)amino, carbamoyl, sulfamoyl, acylamino, aminosulfonylamino, alkoxycarbonyl and acyloxy.

Alkenyl groups may be straight-chain or branched and are preferably (C₂-C₆)-groups for example vinyl and allyl. The term alkenyl comprises for the purpose of the present invention alkynyl groups as well, for example ethynyl and propargyl.

Aryl groups appearing in this invention are preferably phenyl or naphthyl. The terms phenyl and naphthyl comprises unsubstituted as well as substituted phenyl and naphthyl. Preferred substituents are alkyl, cycloalkyl, heterocycloalkyl, hydroxyalkyl, halogen, hydroxyl, alkoxy, alkylthio, acyl, nitro, cyano, amino, monoalkylamino, dialkylamino, mono(hydroxyalkyl)amino, bis (hydroxyalkyl)amino, monoalkyl-mono(hydroxyalkyl)amino, carbamoyl, sulfamoyl, acylamino, aminosulfonylamino, alkoxycarbonyl, acyloxy, arylsulphonyloxy, alkylsulphonyloxy, aryloxysulfonyl or alkoxylsulfonyl.

Heteroaryl groups appearing in this invention are preferably pyridine, pyrimidine, pyridazine, pyrazine, pyrrole, imidazole, pyrazole, 1,2,4-thiadiazole, 1,2,4-triazole, tetrazole, thiophene, thiazole, isothiazole, benzothiazole, benzoisothiazole, 1,3,4-thiadiazole, furane, oxazole, benzoxazole or isoxazole. The terms heteroaryl comprises the above groups in unsubstituted as well as in substituted form. Preferred substituents are alkyl, hydroxyalkyl, halogen, hydroxyl, alkoxy, alkylthio, acyl, nitro, cyano, amino, monoalkylamino, dialkylamino, mono(hydroxyalkyl)amino, bis (hydroxyalkyl)amino, monoalkyl-mono(hydroxyalkyl)amino, carbamoyl, sulfamoyl, acylamino, aminosulfonylamino, alkoxycarbonyl, arylsulphonyloxy, alkylsulphonyloxy, aryloxysulfonyl, alkoxylsulfonyl or acyloxy.

Heterocycloalkyl groups are preferably pyrrolidine, piperidine, morpholine, tetrahydrofuran or piperazine. The terms heterocycloalkyl comprises the above groups in unsubstituted as well as in substituted form. Preferred substituents are alkyl, hydroxyalkyl, halogen, hydroxyl, alkoxy, alkylthio, acyl, nitro, cyano, amino, monoalkylamino, dialkylamino, mono(hydroxyalkyl)amino, bis(hydroxyalkyl)amino, monoalkyl-mono(hydroxyalkyl)amino, carbamoyl, sulfamoyl, acylamino, aminocarbonylamino, aminosulfonylamino, alkoxycarbonyl, arylsulphonyloxy, alkylsulphonyloxy, aryloxysulfonyl, alkoxylsulfonyl or acyloxy.

Halogen is preferably chlorine, bromine or fluorine.

Preferred are dyes as described above having formula (I-a) 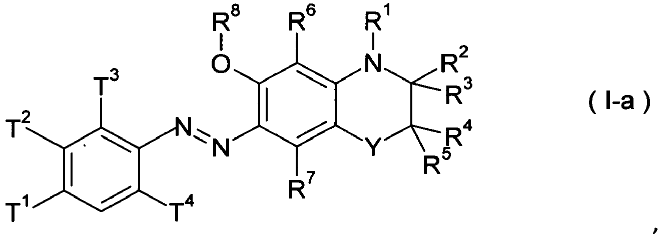 wherein
each of T¹ to T⁴ independent from each other is hydrogen, (C₁-C₆)-alkyl, (C₃-C₆)-cycloalkyl, substituted (C₁-C₆)-alkyl, (C₃-C₄)-alkenyl, (C₃-C₄)-alkynyl, aryl, O-interrupted (C₁-C₆)-alkyl, S-interrupted (C₁-C₆)-alkyl, (C₁-C₈)-alkoxyl, (C₁-C₆)-cycloalkyl, halogen, cyano, nitro, arylsulfonyl, arylsulfonyloxy, (C₁-C₆)-alkylsulfonyl, (C₁-C₆)-alkoxylsulfonyl, *N*-(C₁-C₆)-alkylsulfamoyl, *N*,*N*-bis(C₁-C₆)alkylsulfamoyl, *N*-(C₁-C₆)-alkyl carbamoyl, *N*-bis(C₁-C₆)alkyl-carbamoyl, (C₁-C₆)-alkylacyl, arylacyl, (C₁-C₆)-alkoxylcarbonyl, aryloxylcarbonyl, thiocyano, hydroxyl, aryloxy, arylmethoxyl, aryloyloxy, arylsulfonyloxy, {[(C₁-C₆)-alkoxylcarbonyl]methoxyl} carbonyl, [(aryloxycarbonyl)methoxy]carbonyl, {[(C₁-C₆)-alkylacyl]-methoxy}carbonyl or [(arylacyl)-methoxy]carbonyl,
R¹ is (C₁-C₆)-alkyl, (C₁-C₆)-acyloxy substituted (C₂-C₆)alkyl, cyano(C₁-C₆)alkyl, arylmethyl, arylacyl (C₁-C₆)alkyl, 2-(C₁-C₆)alkoxyl-2-oxoethyl, O-interrupted (C₂-C₆)-alkyl, S-interrupted (C₂-C₆)-alkyl, (C₃-C₄)-alkenyl, (C₃-C₄)-alkynyl, 3-(C₁-C₆)alkoxyl-3-oxopropyl, 3-[0-interrupted (C₂-C₆)-alkoxyl]-3-oxopropyl, 2-(C₁-C₆)alkoxyl-2-oxoethyl, 2-[O-interrupted (C2-C₆)-alkoxyl]-2-oxoethyl, 2-{[(C₁-C₆)-alkylacyl]-methoxy}-2-oxoethyl, 3-{[(C₁-C₆)-alkylacyl]-methoxy}-3-oxopropyl, 2-[(arylacyl)-methoxy]-2-oxoethyl, 3-[(arylacyl)-methoxy]-3-oxopropyl, 3-{[(C₁-C₆)-alkoxylcarbonyl]-methoxy}-3-oxopropyl, 3-(arylmethoxy)-3-oxopropyl, 3-[(arylacyl)-methoxy]-3-oxopropyl, 2-(cyanomethoxy)-2-oxoethyl, 3-(cyanomethoxy)-3-oxopropyl, 2-{[(C₁-C₆)-alkoxylcarbonyl]methoxy}-2-oxoethyl, 1-[(C₁-C₆)-alkyl]-3-[(C₁-C₆)-alkoxyl]- 3-oxopropyl, 1-[(C₁-C₆)-alkyl]-3-[O-interrupted (C₁-C₆)-alkoxyl]-3-oxopropyl, 1-[(C₁-C₆)-alkyl]-3-(arylmethoxyl)-3-oxopropyl, 1-[(C₁-C₆)-alkyl]-3-{[(C₁-C₆)-alkylacyl]methoxy}- 3-oxopropyl or 1-[(C₁-C₆)-alkyl]-3-{[(C₁-C₆)-alkoxylcarbonyl]-methoxy}-3-oxopropyl,
R² to R⁷ independent from each other is hydrogen, (C₁-C₆)alkyl, aryl, (C₁-C₈)alkoxyl, (C3-C₈)cycloalkyl, O-interrupted (C₂-C₆)alkyl, S-interrupted (C₂-C₆)alkyl or halogen,
R⁸ is hydrogen, (C₁-C₆)alkyl, (C₃-C₄)-alkenyl, (C₃-C₄)-alkynyl, arylmethyl, 2-(C₁-C₆)alkoxyl-2-oxoethyl, 2-(arylmethyl)-2-oxoethyl, cyano(C₁-C₆)alkyl, O-interrupted (C₁-C₆)alkyl or S-interrupted (C₁-C₆)alkyl
   and
Y is carbonyl.

In particularly preferred dyes of the formula (I-a):
T¹, T³ and T⁴ independent from each other is hydrogen, nitro, cyano, bromine, chlorine, benzoxyl, phenoxy, methoxy, ethoxy, methyl, trifluromethyl, ethyl, benzoyl, acetyl, hydroxyl, methoxycarbonyl, ethoxycarbonyl, methylsulfonyl, cyanomethylsulfonyl, acetonylsulfonyl, (2-ethoxy-2-oxoethyl)sulfonyl, (2-methoxy-2-oxoethyl)sulfonyl, phenylsulfonyloxy, phenoxylsulfonyl, ethoxylsulfonyl, phenoxylsulfonyl cyanomethoxylcarbonyl, (2-ethoxy-2-oxoethyl)carbonyl, (2-methoxy-2-oxoethyl)carbonyl, acetonylcarbonyl or phenacylcarbonyl,
T² is hydrogen, chlorine, bromine, methoxy, ethoxyl, phenoxy, benzoxy, nitro, methyl or ethyl,
R¹ is methyl, ethyl, propyl, isopropyl, n-butyl, isobutyl, benzyl, 2-methoxylethyl, 2-ethoxylethyl, 2-acetoxyethyl, 2-(propionyl)oxyethyl, 2-(benzoyl)oxyethyl, cyanomethyl, 2-cyanoethyl, 3-cyanopropyl, 3-(C₁-C₆)alkoxyl-3-oxopropyl, 3-[O-interrupted (C₂-C₆)-alkoxyl]-3-oxopropyl, 2-(C₁-C₆)alkyl-2-oxoethyl, 2-[O-interrupted (C₂-C₆)-alkoxyl]-2-oxoethyl, 2-(cyanomethoxy)-2-oxoethyl, 3-(cyanomethoxy)-3-oxopropyl, 2-[(arylacyl)methoxy] -2-oxoethyl, 3- [(arylacyl)methoxy] -3 -oxopropyl, 3 -(arylmethoxy)-3 - oxopropyl, 3-{[(C₁-C₆)alkylacyl]-methoxyl}-3-oxopropyl, 3-[(arylacyl)-methoxy]-3-oxopropyl, 3-{[(C₁-C₆)-alkoxycabonyl]methoxy}-3-oxopropyl, 2-{[(C₁-C₆)-alkoxycabonyl]methoxy}-2-oxoethyl or 2-{[(C₁-C₆)alkylacyl]-methoxyl}2-oxoethyl,
R² to R⁷ independent from each other is hydrogen, bromine, chlorine, ethyl or methyl,
R⁸ is hydrogen, methyl, ethyl, propyl, isopropyl, 2-ethoxy-2-oxoethyl or 2-methoxy-2-oxoethyl and
Y is carbonyl.

Examples of preferred dyes of the formula (I-a) are the compounds illustrated in Table 1 and mixtures thereof.

| **Table 1** | | | | | | | |
|---|---|---|---|---|---|---|---|
| 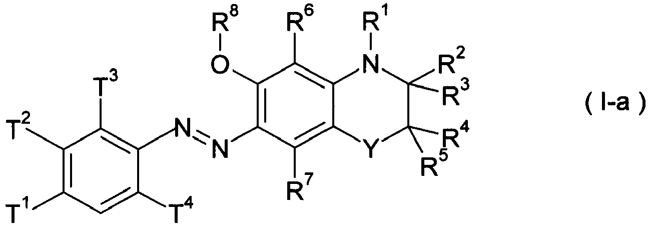 | | | | | | | |

| R³ to R⁷ is hydrogen, Y is carbonyl | | | | | | | |
|---|---|---|---|---|---|---|---|
| Ex pl. | T¹ | T² | T³ | T⁴ | R² | R⁸ | R¹ |
| 1 | NO₂ | H | CN | Br | H | H | -(CH₂)₂CO₂H |
| 2 | NO₂ | H | CN | Br | H | H | -(CH₂)₂CO₂CH₃ |
| 3 | NO₂ | H | CN | Br | H | H | -(CH₂)₂CO₂CH₂CH₃ |
| 4 | NO₂ | H | CN | Br | H | H | -(CH₂)₂CO₂(CH₂)₂CH₃ |
| 5 | NO₂ | H | CN | Br | H | H | -(CH₂)₂CO₂CH(CH₃)₂ |
| 6 | NO₂ | H | CN | Br | H | H | -(CH₂)₂CO₂(CH₂)₃CH₃ |
| 7 | NO₂ | H | CN | Br | H | H | -(CH₂)₂CO₂CH(CH₃)CH₂CH₃ |
| 8 | NO₂ | H | CN | Br | H | H | -(CH₂)₂CO₂CH₂C₆H₅ |
| 9 | NO₂ | H | CN | Br | H | H | -(CH₂)₂CO₂(CH₂)₂OCH₃ |
| 10 | NO₂ | H | CN | Br | H | H | -(CH₂)₂CO₂(CH₂)₂OCH₂CH₃ |
| 11 | NO₂ | H | CN | Br | H | H | -(CH₂)₂CO₂(CH₂)₃OCH₃ |
| 12 | NO₂ | H | CN | Br | H | H | -(CH₂)₂CO₂(CH₂)₃OCH₂CH₃ |
| 13 | NO₂ | H | CN | H | H | H | -(CH₂)₂CO₂H |
| 14 | NO₂ | H | CN | H | H | H | -(CH₂)₂CO₂CH₃ |
| 15 | NO₂ | H | CN | H | H | H | -(CH₂)₂CO₂CH₂CH₃ |
| 16 | NO₂ | H | CN | H | H | H | -(CH₂)₂CO₂(CH₂)₂CH₃ |
| 17 | NO₂ | H | CN | H | H | H | -(CH₂)₂CO₂CH(CH₃)₂ |
| 18 | NO₂ | H | CN | H | H | H | -(CH₂)₂CO₂(CH₂)₃CH₃ |
| 19 | NO₂ | H | CN | H | H | H | -(CH₂)₂CO₂CH(CH₃)CH₂CH₃ |
| 20 | NO₂ | H | CN | H | H | H | -(CH₂)₂CO₂CH₂C₆H₅ |
| 21 | NO₂ | H | CN | H | H | H | -(CH₂)₂CO₂(CH₂)₂OCH₃ |
| 22 | NO₂ | H | CN | H | H | H | -(CH₂)₂CO₂(CH₂)₂OCH₂CH₃ |
| 23 | NO₂ | H | CN | H | H | H | -(CH₂)₂CO₂(CH₂)₃OCH₃ |
| 24 | NO₂ | H | CN | H | H | H | -(CH₂)₂CO₂(CH₂)₃OCH₂CH₃ |
| 25 | NO₂ | H | NO₂ | H | H | H | -(CH₂)₂CO₂H |
| 26 | NO₂ | H | NO₂ | H | H | H | -(CH₂)₂CO₂CH₃ |
| 27 | NO₂ | H | NO₂ | H | H | H | -(CH₂)₂CO₂CH₂CH₃ |
| 28 | NO₂ | H | NO₂ | H | H | H | -(CH₂)₂CO₂(CH₂)₂CH₃ |
| 29 | NO₂ | H | NO₂ | H | H | H | -(CH₂)₂CO₂CH(CH₃)₂ |
| 30 | NO₂ | H | NO₂ | H | H | H | -(CH₂)₂CO₂(CH₂)₃CH₃ |
| 31 | NO₂ | H | NO₂ | H | H | H | -(CH₂)₂CO₂CH(CH₃)CH₂CH₃ |
| 32 | NO₂ | H | NO₂ | H | H | H | -(CH₂)₂CO₂CH₂C₆H₅ |
| 33 | NO₂ | H | NO₂ | H | H | H | -(CH₂)₂CO₂(CH₂)₂OCH₃ |
| 34 | NO₂ | H | NO₂ | H | H | H | -(CH₂)₂CO₂(CH₂)₂OCH₂CH₃ |
| 35 | NO₂ | H | NO₂ | H | H | H | -(CH₂)₂CO₂(CH₂)₃OCH₃ |
| 36 | NO₂ | H | NO₂ | H | H | H | -(CH₂)₂CO₂(CH₂)₃OCH₂CH₃ |
| 37 | NO₂ | H | NO₂ | Br | H | H | -(CH₂)₂CO₂H |
| 38 | NO₂ | H | NO₂ | Br | H | H | -(CH₂)₂CO₂CH₃ |
| 39 | NO₂ | H | NO₂ | Br | H | H | -(CH₂)₂CO₂CH₂CH₃ |
| 40 | NO₂ | H | NO₂ | Br | H | H | -(CH₂)₂CO₂(CH₂)₂CH₃ |
| 41 | NO₂ | H | NO₂ | Br | H | H | -(CH₂)₂CO₂CH(CH₃)₂ |
| 42 | NO₂ | H | NO₂ | Br | H | H | -(CH₂)₂C0₂(CH₂)₃CH₃ |
| 43 | NO₂ | H | NO₂ | Br | H | H | -(CH₂)₂CO₂CH(CH₃)CH₂CH₃ |
| 44 | NO₂ | H | NO₂ | Br | H | H | -(CH₂)₂CO₂CH₂C₆H₅ |
| 45 | NO₂ | H | NO₂ | Br | H | H | -(CH₂)₂CO₂(CH₂)₂OCH₃ |
| 46 | NO₂ | H | NO₂ | Br | H | H | -(CH₂)₂CO₂(CH₂)₂OCH₂CH₃ |
| 47 | NO₂ | H | NO₂ | Br | H | H | -(CH₂)₂CO₂(CH₂)₃OCH₃ |
| 48 | NO₂ | H | NO₂ | Br | H | H | -(CH₂)₂CO₂(CH₂)₃OCH₂CH₃ |
| 49 | NO₂ | H | NO₂ | Cl | H | H | -(CH₂)₂CO₂H |
| 50 | NO₂ | H | NO₂ | Cl | H | H | -(CH₂)₂CO₂CH₃ |
| 51 | NO₂ | H | NO₂ | Cl | H | H | -(CH₂)₂CO₂CH₂CH₃ |
| 52 | NO₂ | H | NO₂ | Cl | H | H | -(CH₂)₂CO₂(CH₂)₂CH₃ |
| 53 | NO₂ | H | NO₂ | Cl | H | H | -(CH₂)₂CO₂CH(CH₃)₂ |
| 54 | NO₂ | H | NO₂ | Cl | H | H | -(CH₂)₂CO₂(CH₂)₃CH₃ |
| 55 | NO₂ | H | NO₂ | Cl | H | H | -(CH₂)₂CO₂CH(CH₃)CH₂CH₃ |
| 56 | NO₂ | H | NO₂ | Cl | H | H | -(CH₂)₂CO₂CH₂C₆H₅ |
| 57 | NO₂ | H | NO₂ | Cl | H | H | -(CH₂)₂CO₂(CH₂)₂OCH₃ |
| 58 | NO₂ | H | NO₂ | Cl | H | H | -(CH₂)₂CO₂(CH₂)₂OCH₂CH₃ |
| 59 | NO₂ | H | NO₂ | Cl | H | H | -(CH₂)₂CO₂(CH₂)₃OCH₃ |
| 60 | NO₂ | H | NO₂ | Cl | H | H | -(CH₂)₂CO₂(CH₂)₃OCH₂CH₃ |
| 61 | NO₂ | H | NO₂ | CN | H | H | -(CH₂)₂CO₂H |
| 62 | NO₂ | H | NO₂ | CN | H | H | -(CH₂)₂CO₂CH₃ |
| 63 | NO₂ | H | NO₂ | CN | H | H | -(CH₂)₂CO₂CH₂CH₃ |
| 64 | NO₂ | H | NO₂ | CN | H | H | -(CH₂)₂CO₂(CH₂)₂CH₃ |
| 65 | NO₂ | H | NO₂ | CN | H | H | -(CH₂)₂CO₂CH(CH₃)₂ |
| 66 | NO₂ | H | NO₂ | CN | H | H | -(CH₂)₂CO₂(CH₂)₃CH₃ |
| 67 | NO₂ | H | NO₂ | CN | H | H | -(CH₂)₂CO₂CH(CH₃)CH₂CH₃ |
| 68 | NO₂ | H | NO₂ | CN | H | H | -(CH₂)₂CO₂CH₂C₆H₅ |
| 69 | NO₂ | H | NO₂ | CN | H | H | -(CH₂)₂CO₂(CH₂)₂OCH₃ |
| 70 | NO₂ | H | NO₂ | CN | H | H | -(CH₂)₂CO₂(CH₂)₂OCH₂CH₃ |
| 71 | NO₂ | H | NO₂ | CN | H | H | -(CH₂)₂CO₂(CH₂)₃OCH₃ |
| 72 | NO₂ | H | NO₂ | CN | H | H | -(CH₂)₂CO₂(CH₂)₃OCH₂CH₃ |
| 73 | NO₂ | H | CN | CN | H | H | -(CH₂)₂CO₂H |
| 74 | NO₂ | H | CN | CN | H | H | -(CH₂)₂CO₂CH₃ |
| 75 | NO₂ | H | CN | CN | H | H | -(CH₂)₂CO₂CH₂CH₃ |
| 76 | NO₂ | H | CN | CN | H | H | -(CH₂)₂CO₂(CH₂)₂CH₃ |
| 77 | NO₂ | H | CN | CN | H | H | -(CH₂)₂CO₂CH(CH₃)₂ |
| 78 | NO₂ | H | CN | CN | H | H | -(CH₂)₂CO₂(CH₂)₃CH₃ |
| 79 | NO₂ | H | CN | CN | H | H | -(CH₂)₂CO₂CH(CH₃)CH₂CH₃ |
| 80 | NO₂ | H | CN | CN | H | H | -(CH₂)₂CO₂CH₂C₆H₅ |
| 81 | NO₂ | H | CN | CN | H | H | -(CH₂)₂CO₂(CH₂)₂OCH₃ |
| 82 | NO₂ | H | CN | CN | H | H | -(CH₂)₂CO₂(CH₂)₂OCH₂CH₃ |
| 83 | NO₂ | H | CN | CN | H | H | -(CH₂)₂CO₂(CH₂)₃OCH₃ |
| 84 | NO₂ | H | CN | CN | H | H | -(CH₂)₂CO₂(CH₂)₃OCH₂CH₃ |
| 85 | Br | H | CN | CN | H | H | -(CH₂)₂CO₂H |
| 86 | Br | H | CN | CN | H | H | -(CH₂)₂CO₂CH₃ |
| 87 | Br | H | CN | CN | H | H | -(CH₂)₂CO₂CH₂CH₃ |
| 88 | Br | H | CN | CN | H | H | -(CH₂)₂CO₂(CH₂)₂CH₃ |
| 89 | Br | H | CN | CN | H | H | -(CH₂)₂CO₂CH(CH₃)₂ |
| 90 | Br | H | CN | CN | H | H | -(CH₂)₂CO₂(CH₂)₃CH₃ |
| 91 | Br | H | CN | CN | H | H | -(CH₂)₂CO₂CH(CH₃)CH₂CH₃ |
| 92 | Br | H | CN | CN | H | H | -(CH₂)₂CO₂CH₂C₆H₅ |
| 93 | Br | H | CN | CN | H | H | -(CH₂)₂CO₂(CH₂)₂OCH₃ |
| 94 | Br | H | CN | CN | H | H | -(CH₂)₂CO₂(CH₂)₂OCH₂CH₃ |
| 95 | Br | H | CN | CN | H | H | -(CH₂)₂CO₂(CH₂)₃OCH₃ |
| 96 | Br | H | CN | CN | H | H | -(CH₂)₂CO₂(CH₂)₃OCH₂CH₃ |
| 97 | -CO₂CH₃ | H | NO₂ | NO₂ | H | H | -(CH₂)₂CO₂H |
| 98 | -CO₂CH₃ | H | NO₂ | NO₂ | H | H | -(CH₂)₂CO₂CH₃ |
| 99 | -CO₂CH₃ | H | NO₂ | NO₂ | H | H | -(CH₂)₂CO₂CH₂CH₃ |
| 100 | -CO₂CH₃ | H | NO₂ | NO₂ | H | H | -(CH₂)₂CO₂(CH₂)₂CH₃ |
| 101 | -CO₂CH₃ | H | NO₂ | NO₂ | H | H | -(CH₂)₂CO₂CH(CH₃)₂ |
| 102 | -CO₂CH₃ | H | NO₂ | NO₂ | H | H | -(CH₂)₂CO₂(CH₂)₃CH₃ |
| 103 | -CO₂CH₃ | H | NO₂ | NO₂ | H | H | -(CH₂)₂CO₂CH(CH₃)CH₂CH₃ |
| 104 | -CO₂CH₃ | H | NO₂ | NO₂ | H | H | -(CH₂)₂CO₂CH₂C₆H₅ |
| 105 | -CO₂CH₃ | H | NO₂ | NO₂ | H | H | -(CH₂)₂CO₂(CH₂)₂OCH₃ |
| 106 | -CO₂CH₃ | H | NO₂ | NO₂ | H | H | -(CH₂)₂CO₂(CH₂)₂OCH₂CH₃ |
| 107 | -CO₂CH₃ | H | NO₂ | NO₂ | H | H | -(CH₂)₂CO₂(CH₂)₃OCH₃ |
| 108 | -CO₂CH₃ | H | NO₂ | NO₂ | H | H | -(CH₂)₂CO₂(CH₂)₃OCH₂CH₃ |
| 109 | -CO₂CH₂CH₃ | H | NO₂ | H | H | H | -(CH₂)₂CO₂H |
| 110 | -CO₂CH₂CH₃ | H | NO₂ | H | H | H | -(CH₂)₂CO₂CH₃ |
| 111 | -CO₂CH₂CH₃ | H | NO₂ | H | H | H | -(CH₂)₂CO₂CH₂CH₃ |
| 112 | -CO₂CH₂CH₃ | H | NO₂ | H | H | H | -(CH₂)₂CO₂(CH₂)₂CH₃ |
| 113 | -CO₂CH₂CH₃ | H | NO₂ | H | H | H | -(CH₂)₂CO₂CH(CH₃)₂ |
| 114 | -CO₂CH₂CH₃ | H | NO₂ | H | H | H | -(CH₂)₂CO₂(CH₂)₃CH₃ |
| 115 | -CO₂CH₂CH₃ | H | NO₂ | H | H | H | -(CH₂)₂CO₂CH(CH₃)CH₂CH₃ |
| 116 | -CO₂CH₂CH₃ | H | NO₂ | H | H | H | -(CH₂)₂CO₂CH₂C₆H₅ |
| 117 | -CO₂CH₂CH₃ | H | NO₂ | H | H | H | -(CH₂)₂CO₂(CH₂)₂OCH₃ |
| 118 | -CO₂CH₂CH₃ | H | NO₂ | H | H | H | -(CH₂)₂CO₂(CH₂)₂OCH₂CH₃ |
| 119 | -CO₂CH₂CH₃ | H | NO₂ | H | H | H | -(CH₂)₂CO₂(CH₂)₃OCH₃ |
| 120 | -CO₂CH₂CH₃ | H | NO₂ | H | H | H | -(CH₂)₂CO₂(CH₂)₃OCH₂CH₃ |
| 121 | -CO₂CH₃ | H | NO₂ | H | H | H | -(CH₂)₂CO₂H |
| 122 | -CO₂CH₃ | H | NO₂ | H | H | H | -(CH₂)₂CO₂CH₃ |
| 123 | -CO₂CH₃ | H | NO₂ | H | H | H | -(CH₂)₂CO₂CH₂CH₃ |
| 124 | -CO₂CH₃ | H | NO₂ | H | H | H | -(CH₂)₂CO₂(CH₂)₂CH₃ |
| 125 | -CO₂CH₃ | H | NO₂ | H | H | H | -(CH₂)₂CO₂CH(CH₃)₂ |
| 126 | -CO₂CH₃ | H | NO₂ | H | H | H | -(CH₂)₂CO₂(CH₂)₃CH₃ |
| 127 | -CO₂CH₃ | H | NO₂ | H | H | H | -(CH₂)₂CO₂CH(CH₃)CH₂CH₃ |
| 128 | -CO₂CH₃ | H | NO₂ | H | H | H | -(CH₂)₂CO₂CH₂C₆H₅ |
| 129 | -CO₂CH₃ | H | NO₂ | H | H | H | -(CH₂)₂CO₂(CH₂)₂OCH₃ |
| 130 | -CO₂CH₃ | H | NO₂ | H | H | H | -(CH₂)₂CO₂(CH₂)₂OCH₂CH₃ |
| 131 | -CO₂CH₃ | H | NO₂ | H | H | H | -(CH₂)₂CO₂(CH₂)₃OCH₃ |
| 132 | -CO₂CH₃ | H | NO₂ | H | H | H | -(CH₂)₂CO₂(CH₂)₃OCH₂CH₃ |
| 133 | C₆H₅CO- | H | NO₂ | H | H | H | -(CH₂)₂CO₂H |
| 134 | C₆H₅CO- | H | NO₂ | H | H | H | -(CH₂)₂CO₂CH₃ |
| 135 | C₆H₅CO- | H | NO₂ | H | H | H | -(CH₂)₂CO₂CH₂CH₃ |
| 136 | C₆H₅CO- | H | NO₂ | H | H | H | -(CH₂)₂CO₂(CH₂)₂CH₃ |
| 137 | C₆H₅CO- | H | NO₂ | H | H | H | -(CH₂)₂CO₂CH(CH₃)₂ |
| 138 | C₆H₅CO- | H | NO₂ | H | H | H | -(CH₂)₂CO₂(CH₂)₃CH₃ |
| 139 | C₆H₅CO- | H | NO₂ | H | H | H | -(CH₂)₂CO₂CH(CH₃)CH₂CH₃ |
| 140 | C₆H₅CO- | H | NO₂ | H | H | H | -(CH₂)₂CO₂CH₂C₆H₅ |
| 141 | C₆H₅CO- | H | NO₂ | H | H | H | -(CH₂)₂CO₂(CH₂)₂OCH₃ |
| 142 | C₆H₅CO- | H | NO₂ | H | H | H | -(CH₂)₂CO₂(CH₂)₂OCH₂CH₃ |
| 143 | C₆H₅CO- | H | NO₂ | H | H | H | -(CH₂)₂CO₂(CH₂)₃OCH₃ |
| 144 | C₆H₅CO- | H | NO₂ | H | H | H | -(CH₂)₂CO₂(CH₂)₃OCH₂CH₃ |
| 145 | NO₂ | H | C₆H₅CO- | H | H | H | -(CH₂)₂CO₂H |
| 146 | NO₂ | H | C₆H₅CO- | H | H | H | -(CH₂)₂CO₂CH₃ |
| 147 | NO₂ | H | C₆H₅CO- | H | H | H | -(CH₂)₂CO₂CH₂CH₃ |
| 148 | NO₂ | H | C₆H₅CO- | H | H | H | -(CH₂)₂CO₂(CH₂)₂CH₃ |
| 149 | NO₂ | H | C₆H₅CO- | H | H | H | -(CH₂)₂CO₂CH(CH₃)₂ |
| 150 | NO₂ | H | C₆H₅CO- | H | H | H | -(CH₂)₂CO₂(CH₂)₃CH₃ |
| 151 | NO₂ | H | C₆H₅CO- | H | H | H | -(CH₂)₂CO₂CH(CH₃)CH₂CH₃ |
| 152 | NO₂ | H | C₆H₅CO- | H | H | H | -(CH₂)₂CO₂CH₂C₆H₅ |
| 153 | NO₂ | H | C₆H₅CO- | H | H | H | -(CH₂)₂CO₂(CH₂)₂OCH₃ |
| 154 | NO₂ | H | C₆H₅CO- | H | H | H | -(CH₂)₂CO₂(CH₂)₂OCH₂CH₃ |
| 155 | NO₂ | H | C₆H₅CO- | H | H | H | -(CH₂)₂CO₂(CH₂)₃OCH₃ |
| 156 | NO₂ | H | C₆H₅CO- | H | H | H | -(CH₂)₂CO₂(CH₂)₃OCH₂CH₃ |
| 157 | NO₂ | H | CN | Cl | H | H | -(CH₂)₂CO₂H |
| 158 | NO₂ | H | CN | Cl | H | H | -(CH₂)₂CO₂CH₃ |
| 159 | NO₂ | H | CN | Cl | H | H | -(CH₂)₂CO₂CH₂CH₃ |
| 160 | NO₂ | H | CN | Cl | H | H | -(CH₂)₂CO₂(CH₂)₂CH₃ |
| 161 | NO₂ | H | CN | Cl | H | H | -(CH₂)₂CO₂CH(CH₃)₂ |
| 162 | NO₂ | H | CN | Cl | H | H | -(CH₂)₂CO₂(CH₂)₃CH₃ |
| 163 | NO₂ | H | CN | Cl | H | H | -(CH₂)₂CO₂CH(CH₃)CH₂CH₃ |
| 164 | NO₂ | H | CN | Cl | H | H | -(CH₂)₂CO₂CH₂C₆H₅ |
| 165 | NO₂ | H | CN | Cl | H | H | -(CH₂)₂CO₂(CH₂)₂OCH₃ |
| 166 | NO₂ | H | CN | Cl | H | H | -(CH₂)₂CO₂(CH₂)₂OCH₂CH₃ |
| 167 | NO₂ | H | CN | Cl | H | H | -(CH₂)₂CO₂(CH₂)₃OCH₃ |
| 168 | NO₂ | H | CN | Cl | H | H | -(CH₂)₂CO₂(CH₂)₃OCH₂CH₃ |
| 169 | NO₂ | H | Cl | Cl | H | H | -(CH₂)₂CO₂H |
| 170 | NO₂ | H | Cl | Cl | H | H | -(CH₂)₂CO₂CH₃ |
| 171 | NO₂ | H | Cl | Cl | H | H | -(CH₂)₂CO₂CH₂CH₃ |
| 172 | NO₂ | H | Cl | Cl | H | H | -(CH₂)₂CO₂(CH₂)₂CH₃ |
| 173 | NO₂ | H | Cl | Cl | H | H | -(CH₂)₂CO₂CH(CH₃)₂ |
| 174 | NO₂ | H | Cl | Cl | H | H | -(CH₂)₂CO₂(CH₂)₃CH₃ |
| 175 | NO₂ | H | Cl | Cl | H | H | -(CH₂)₂CO₂CH(CH₃)CH₂CH₃ |
| 176 | NO₂ | H | Cl | Cl | H | H | -(CH₂)₂CO₂CH₂C₆H₅ |
| 177 | NO₂ | H | Cl | Cl | H | H | -(CH₂)₂CO₂(CH₂)₂OCH₃ |
| 178 | NO₂ | H | Cl | Cl | H | H | -(CH₂)₂CO₂(CH₂)₂OCH₂CH₃ |
| 179 | NO₂ | H | Cl | Cl | H | H | -(CH₂)₂CO₂(CH₂)₃OCH₃ |
| 180 | NO₂ | H | Cl | Cl | H | H | -(CH₂)₂CO₂(CH₂)₃OCH₂CH₃ |
| 181 | NO₂ | H | Cl | Br | H | H | -(CH₂)₂CO₂H |
| 182 | NO₂ | H | Cl | Br | H | H | -(CH₂)₂CO₂CH₃ |
| 183 | NO₂ | H | Cl | Br | H | H | -(CH₂)₂CO₂CH₂CH₃ |
| 184 | NO₂ | H | Cl | Br | H | H | -(CH₂)₂CO₂(CH₂)₂CH₃ |
| 185 | NO₂ | H | Cl | Br | H | H | -(CH₂)₂CO₂CH(CH₃)₂ |
| 186 | NO₂ | H | Cl | Br | H | H | -(CH₂)₂CO₂(CH₂)₃CH₃ |
| 187 | NO₂ | H | Cl | Br | H | H | -(CH₂)₂CO₂CH(CH₃)CH₂CH₃ |
| 188 | NO₂ | H | Cl | Br | H | H | -(CH₂)₂CO₂CH₂C₆H₅ |
| 189 | NO₂ | H | Cl | Br | H | H | -(CH₂)₂CO₂(CH₂)₂OCH₃ |
| 190 | NO₂ | H | Cl | Br | H | H | -(CH₂)₂CO₂(CH₂)₂OCH₂CH₃ |
| 191 | NO₂ | H | Cl | Br | H | H | -(CH₂)₂CO₂(CH₂)₃OCH₃ |
| 192 | NO₂ | H | Cl | Br | H | H | -(CH₂)₂CO₂(CH₂)₃OCH₂CH₃ |
| 193 | NO₂ | H | Br | Br | H | H | -(CH₂)₂CO₂H |
| 194 | NO₂ | H | Br | Br | H | H | -(CH₂)₂CO₂CH₃ |
| 195 | NO₂ | H | Br | Br | H | H | -(CH₂)₂CO₂CH₂CH₃ |
| 196 | NO₂ | H | Br | Br | H | H | -(CH₂)₂CO₂(CH₂)₂CH₃ |
| 197 | NO₂ | H | Br | Br | H | H | -(CH₂)₂CO₂CH(CH₃)₂ |
| 198 | NO₂ | H | Br | Br | H | H | -(CH₂)₂CO₂(CH₂)₃CH₃ |
| 199 | NO₂ | H | Br | Br | H | H | -(CH₂)₂CO₂CH(CH₃)CH₂CH₃ |
| 200 | NO₂ | H | Br | Br | H | H | -(CH₂)₂CO₂CH₂C₆H₅ |
| 201 | NO₂ | H | Br | Br | H | H | -(CH₂)₂CO₂(CH₂)₂OCH₃ |
| 202 | NO₂ | H | Br | Br | H | H | -(CH₂)₂CO₂(CH₂)₂OCH₂CH₃ |
| 203 | NO₂ | H | Br | Br | H | H | -(CH₂)₂CO₂(CH₂)₃OCH₃ |
| 204 | NO₂ | H | Br | Br | H | H | -(CH₂)₂CO₂(CH₂)₃OCH₂CH₃ |
| 205 | -CO₂CH₂CH₃ | CH₃ | CN | CN | H | H | -(CH₂)₂CO₂H |
| 206 | -CO₂CH₂CH₃ | CH₃ | CN | CN | H | H | -(CH₂)₂CO₂CH₃ |
| 207 | -CO₂CH₂CH₃ | CH₃ | CN | CN | H | H | -(CH₂)₂CO₂CH₂CH₃ |
| 208 | -CO₂CH₂CH₃ | CH₃ | CN | CN | H | H | -(CH₂)₂CO₂(CH₂)₂CH₃ |
| 209 | -CO₂CH₂CH₃ | CH₃ | CN | CN | H | H | -(CH₂)₂CO₂CH(CH₃)₂ |
| 210 | -CO₂CH₂CH₃ | CH₃ | CN | CN | H | H | -(CH₂)₂CO₂(CH₂)₃CH₃ |
| 211 | -CO₂CH₂CH₃ | CH₃ | CN | CN | H | H | -(CH₂)₂CO₂CH(CH₃)CH₂CH₃ |
| 212 | -CO₂CH₂CH₃ | CH₃ | CN | CN | H | H | -(CH₂)₂CO₂CH₂C₆H₅ |
| 213 | -CO₂CH₂CH₃ | CH₃ | CN | CN | H | H | -(CH₂)₂CO₂(CH₂)₂OCH₃ |
| 214 | -CO₂CH₂CH₃ | CH₃ | CN | CN | H | H | -(CH₂)₂CO₂(CH₂)₂OCH₂CH₃ |
| 215 | -CO₂CH₂CH₃ | CH₃ | CN | CN | H | H | -(CH₂)₂CO₂(CH₂)₃OCH₃ |
| 216 | -CO₂CH₂CH₃ | CH₃ | CN | CN | H | H | -(CH₂)₂CO₂(CH₂)₃OCH₂CH₃ |
| 217 | NO₂ | H | H | OCH₃ | H | H | -(CH₂)₂CO₂H |
| 218 | NO₂ | H | H | OCH₃ | H | H | -(CH₂)₂CO₂CH₃ |
| 219 | NO₂ | H | H | OCH₃ | H | H | -(CH₂)₂CO₂CH₂CH₃ |
| 220 | NO₂ | H | H | -OCH₃ | H | H | -(CH₂)₂CO₂(CH₂)₂CH₃ |
| 221 | NO₂ | H | H | -OCH₃ | H | H | -(CH₂)₂CO₂CH(CH₃)₂ |
| 222 | NO₂ | H | H | -OCH₃ | H | H | -(CH₂)₂CO₂(CH₂)₃CH₃ |
| 223 | NO₂ | H | H | -OCH₃ | H | H | -(CH₂)₂CO₂CH(CH₃)CH₂CH₃ |
| 224 | NO₂ | H | H | -OCH₃ | H | H | -(CH₂)₂CO₂CH₂C₆H₅ |
| 225 | NO₂ | H | H | -OCH₃ | H | H | -(CH₂)₂CO₂(CH₂)₂OCH₃ |
| 226 | NO₂ | H | H | -OCH₃ | H | H | -(CH₂)₂CO₂(CH₂)₂OCH₂CH₃ |
| 227 | NO₂ | H | H | -OCH₃ | H | H | -(CH₂)₂CO₂(CH₂)₃OCH₃ |
| 228 | NO₂ | H | H | -OCH₃ | H | H | -(CH₂)₂CO₂(CH₂)₃OCH₂CH₃ |
| 229 | NO₂ | H | H | -OCH₃ | H | H | -(CH₂)₂CO₂H |
| 230 | NO₂ | H | H | -OCH₃ | H | H | -(CH₂)₂CO₂CH₃ |
| 231 | NO₂ | H | H | -OCH₃ | H | H | -(CH₂)₂CO₂CH₂CH₃ |
| 232 | NO₂ | H | H | -OCH₃ | H | H | -(CH₂)₂CO₂(CH₂)₂CH₃ |
| 233 | NO₂ | H | H | -OCH₃ | H | H | -(CH₂)₂CO₂CH(CH₃)₂ |
| 234 | NO₂ | H | H | -OCH₃ | H | H | -(CH₂)₂CO₂(CH₂)₃CH₃ |
| 235 | NO₂ | H | H | -OCH₃ | H | H | -(CH₂)₂CO₂CH(CH₃)CH₂CH₃ |
| 236 | NO₂ | H | H | -OCH₃ | H | H | -(CH₂)₂CO₂CH₂C₆H₅ |
| 237 | NO₂ | H | H | -OCH₃ | H | H | -(CH₂)₂CO₂(CH₂)₂OCH₃ |
| 238 | NO₂ | H | H | -OCH₃ | H | H | -(CH₂)₂CO₂(CH₂)₂OCH₂CH₃ |
| 239 | NO₂ | H | H | -OCH₃ | H | H | -(CH₂)₂CO₂(CH₂)₃OCH₃ |
| 240 | NO₂ | H | H | -OCH₃ | H | H | -(CH₂)₂CO₂(CH₂)₃OCH₂CH₃ |
| 241 | CF₃ | H | H | NO₂ | H | H | -(CH₂)₂CO₂H |
| 242 | CF₃ | H | H | NO₂ | H | H | -(CH₂)₂CO₂CH₃ |
| 243 | CF₃ | H | H | NO₂ | H | H | -(CH₂)₂CO₂CH₂CH₃ |
| 244 | CF₃ | H | H | NO₂ | H | H | -(CH₂)₂CO₂(CH₂)₂CH₃ |
| 245 | CF₃ | H | H | NO₂ | H | H | -(CH₂)₂CO₂CH(CH₃)₂ |
| 246 | CF₃ | H | H | NO₂ | H | H | -(CH₂)₂CO₂(CH₂)₃CH₃ |
| 247 | CF₃ | H | H | NO₂ | H | H | -(CH₂)₂CO₂CH(CH₃)CH₂CH₃ |
| 248 | CF₃ | H | H | NO₂ | H | H | -(CH₂)₂CO₂CH₂C₆H₅ |
| 249 | CF₃ | H | H | NO₂ | H | H | -(CH₂)₂CO₂(CH₂)₂OCH₃ |
| 250 | CF₃ | H | H | NO₂ | H | H | -(CH₂)₂CO₂(CH₂)₂OCH₂CH₃ |
| 251 | CF₃ | H | H | NO₂ | H | H | -(CH₂)₂CO₂(CH₂)₃OCH₃ |
| 252 | CF₃ | H | H | NO₂ | H | H | -(CH₂)₂CO₂(CH₂)₃OCH₂CH₃ |
| 253 | CF₃ | H | H | Cl | H | H | -(CH₂)₂CO₂H |
| 254 | CF₃ | H | H | Cl | H | H | -(CH₂)₂CO₂CH₃ |
| 255 | CF₃ | H | H | Cl | H | H | -(CH₂)₂CO₂CH₂CH₃ |
| 256 | CF₃ | H | H | Cl | H | H | -(CH₂)₂CO₂(CH₂)₂CH₃ |
| 257 | CF₃ | H | H | Cl | H | H | -(CH₂)₂CO₂CH(CH₃)₂ |
| 258 | CF₃ | H | H | Cl | H | H | -(CH₂)₂CO₂(CH₂)₃CH₃ |
| 259 | CF₃ | H | H | Cl | H | H | -(CH₂)₂CO₂CH(CH₃)CH₂CH₃ |
| 260 | CF₃ | H | H | Cl | H | H | -(CH₂)₂CO₂CH₂C₆H₅ |
| 261 | CF₃ | H | H | Cl | H | H | -(CH₂)₂CO₂(CH₂)₂OCH₃ |
| 262 | CF₃ | H | H | Cl | H | H | -(CH₂)₂CO₂(CH₂)₂OCH₂CH₃ |
| 263 | CF₃ | H | H | Cl | H | H | -(CH₂)₂CO₂(CH₂)₃OCH₃ |
| 264 | CF₃ | H | H | Cl | H | H | -(CH₂)₂CO₂(CH₂)₃OCH₂CH₃ |
| 265 | H | H | NO₂ | NO₂ | H | H | -(CH₂)₂CO₂H |
| 266 | H | H | NO₂ | NO₂ | H | H | -(CH₂)₂CO₂CH₃ |
| 267 | H | H | NO₂ | NO₂ | H | H | -(CH₂)₂CO₂CH₂CH₃ |
| 268 | H | H | NO₂ | NO₂ | H | H | -(CH₂)₂CO₂(CH₂)₂CH₃ |
| 269 | H | H | NO₂ | NO₂ | H | H | -(CH₂)₂CO₂CH(CH₃)₂ |
| 270 | H | H | NO₂ | NO₂ | H | H | -(CH₂)₂CO₂(CH₂)₃CH₃ |
| 271 | H | H | NO₂ | NO₂ | H | H | -(CH₂)₂CO₂CH(CH₃)CH₂CH₃ |
| 272 | H | H | NO₂ | NO₂ | H | H | -(CH₂)₂CO₂CH₂C₆H₅ |
| 273 | H | H | NO₂ | NO₂ | H | H | -(CH₂)₂CO₂(CH₂)₂OCH₃ |
| 274 | H | H | NO₂ | NO₂ | H | H | -(CH₂)₂CO₂(CH₂)₂OCH₂CH₃ |
| 275 | H | H | NO₂ | NO₂ | H | H | -(CH₂)₂CO₂(CH₂)₃OCH₃ |
| 276 | H | H | NO₂ | NO₂ | H | H | -(CH₂)₂CO₂(CH₂)₃OCH₂CH₃ |
| 277 | CH₃ | H | CN | CN | H | H | -(CH₂)₂CO₂H |
| 278 | CH₃ | H | CN | CN | H | H | -(CH₂)₂CO₂CH₃ |
| 279 | CH₃ | H | CN | CN | H | H | -(CH₂)₂CO₂CH₂CH₃ |
| 280 | CH₃ | H | CN | CN | H | H | -(CH₂)₂CO₂(CH₂)₂CH₃ |
| 281 | CH₃ | H | CN | CN | H | H | -(CH₂)₂CO₂CH(CH₃)₂ |
| 282 | CH₃ | H | CN | CN | H | H | -(CH₂)₂CO₂(CH₂)₃CH₃ |
| 283 | CH₃ | H | CN | CN | H | H | -(CH₂)₂CO₂CH(CH₃)CH₂CH₃ |
| 284 | CH₃ | H | CN | CN | H | H | -(CH₂)₂CO₂CH₂C₆H₅ |
| 285 | CH₃ | H | CN | CN | H | H | -(CH₂)₂CO₂(CH₂)₂OCH₃ |
| 286 | CH₃ | H | CN | CN | H | H | -(CH₂)₂CO₂(CH₂)₂OCH₂CH₃ |
| 287 | CH₃ | H | CN | CN | H | H | -(CH₂)₂CO₂(CH₂)₃OCH₃ |
| 288 | CH₃ | H | CN | CN | H | H | -(CH₂)₂CO₂(CH₂)₃OCH₂CH₃ |
| 289 | -SO₂CH₃ | H | Br | Br | H | H | -(CH₂)₂CO₂H |
| 290 | -SO₂CH₃ | H | Br | Br | H | H | -(CH₂)₂CO₂CH₃ |
| 291 | -SO₂CH₃ | H | Br | Br | H | H | -(CH₂)₂CO₂CH₂CH₃ |
| 292 | -SO₂CH₃ | H | Br | Br | H | H | -(CH₂)₂CO₂(CH₂)₂CH₃ |
| 293 | -SO₂CH₃ | H | Br | Br | H | H | -(CH₂)₂CO₂CH(CH₃)₂ |
| 294 | -SO₂CH₃ | H | Br | Br | H | H | -(CH₂)₂CO₂(CH₂)₃CH₃ |
| 295 | -SO₂CH₃ | H | Br | Br | H | H | -(CH₂)₂CO₂CH(CH₃)CH₂CH₃ |
| 296 | -SO₂CH₃ | H | Br | Br | H | H | -(CH₂)₂CO₂CH₂C₆H₅ |
| 297 | -SO₂CH₃ | H | Br | Br | H | H | -(CH₂)₂CO₂(CH₂)₂OCH₃ |
| 298 | -SO₂CH₃ | H | Br | Br | H | H | -(CH₂)₂CO₂(CH₂)₂OCH₂CH₃ |
| 299 | -SO₂CH₃ | H | Br | Br | H | H | -(CH₂)₂CO₂(CH₂)₃OCH₃ |
| 300 | -SO₂CH₃ | H | Br | Br | H | H | -(CH₂)₂CO₂(CH₂)₃OCH₂CH₃ |
| 301 | -SO₂CH₃ | H | NO₂ | H | H | H | -(CH₂)₂CO₂H |
| 302 | -SO₂CH₃ | H | NO₂ | Br | H | H | -(CH₂)₂CO₂CH₃ |
| 303 | -SO₂CH₃ | H | NO₂ | Br | H | H | -(CH₂)₂CO₂CH₂CH₃ |
| 304 | -SO₂CH₃ | H | NO₂ | Br | H | H | -(CH₂)₂CO₂(CH₂)₂CH₃ |
| 305 | -SO₂CH₃ | H | NO₂ | Br | H | H | -(CH₂)₂CO₂CH(CH₃)₂ |
| 306 | -SO₂CH₃ | H | NO₂ | Br | H | H | -(CH₂)₂CO₂(CH₂)₃CH₃ |
| 306 | -SO₂CH₃ | H | NO₂ | Br | H | H | -(CH₂)₂CO₂CH(CH₃)CH₂CH₃ |
| 308 | -SO₂CH₃ | H | NO₂ | Br | H | H | -(CH₂)₂CO₂CH₂C₆H₅ |
| 309 | -SO₂CH₃ | H | NO₂ | Br | H | H | -(CH₂)₂CO₂(CH₂)₂OCH₃ |
| 310 | -SO₂CH₃ | H | NO₂ | Br | H | H | -(CH₂)₂CO₂(CH₂)₂OCH₂CH₃ |
| 311 | -SO₂CH₃ | H | NO₂ | Br | H | H | -(CH₂)₂CO₂(CH₂)₃OCH₃ |
| 312 | -SO₂CH₃ | H | NO₂ | Br | H | H | -(CH₂)₂CO₂(CH₂)₃OCH₂CH₃ |
| 313 | NO₂ | H | -SO₂CH₃ | H | H | H | -(CH₂)₂CO₂H |
| 314 | NO₂ | H | -SO₂CH₃ | H | H | H | -(CH₂)₂CO₂CH₃ |
| 315 | NO₂ | H | -SO₂CH₃ | H | H | H | -(CH₂)₂CO₂CH₂CH₃ |
| 316 | NO₂ | H | -SO₂CH₃ | H | H | H | -(CH₂)₂CO₂(CH₂)₂CH₃ |
| 317 | NO₂ | H | -SO₂CH₃ | H | H | H | -(CH₂)₂CO₂CH(CH₃)₂ |
| 318 | NO₂ | H | -SO₂CH₃ | H | H | H | -(CH₂)₂CO₂(CH₂)₃CH₃ |
| 319 | NO₂ | H | -SO₂CH₃ | H | H | H | -(CH₂)₂CO₂CH(CH₃)CH₂CH₃ |
| 320 | NO₂ | H | -SO₂CH₃ | H | H | H | -(CH₂)₂CO₂CH₂C₆H₅ |
| 321 | NO₂ | H | -SO₂CH₃ | H | H | H | -(CH₂)₂CO₂(CH₂)₂OCH₃ |
| 322 | NO₂ | H | -SO₂CH₃ | H | H | H | -(CH₂)₂CO₂(CH₂)₂OCH₂CH₃ |
| 323 | NO₂ | H | -SO₂CH₃ | H | H | H | -(CH₂)₂CO₂(CH₂)₃OCH₃ |
| 324 | NO₂ | H | -SO₂CH₃ | H | H | H | -(CH₂)₂CO₂(CH₂)₃OCH₂CH₃ |
| 325 | -CO₂CH₃ | H | H | H | H | CH₃ | -(CH₂)₂CO₂CH₂CH₃ |
| 326 | -CO₂CH₃ | H | H | H | H | H | -(CH₂)₂CO₂CH₂CH₃ |
| 327 | Cl | H | H | NO₂ | H | H | -(CH₂)₂CO₂CH₂CH₃ |
| 328 | Cl | H | H | -OCH₃ | H | H | -(CH₂)₂CO₂CH₂CH₃ |
| 329 | CN | Cl | H | H | H | H | -(CH₂)₂CO₂CH₂CH₃ |
| 330 | CH₃ | H | H | NO₂ | H | H | -(CH₂)₂CO₂CH₂CH₃ |
| 331 | H | NO₂ | H | H | H | H | -(CH₂)₂CO₂CH₂CH₃ |
| 332 | NO₂ | H | Cl | H | H | H | -(CH₂)₂CO₂CH₂CH₃ |
| 333 | NO₂ | Cl | Cl | H | CH₃ | H | -CH(CH₃)CO₂CH₂CH₃ |
| 334 | NO₂ | H | H | H | H | H | -(CH₂)₂CO₂CH₂CN |
| 335 | NO₂ | H | H | H | H | H | -(CH₂)₂CO₂CH₂CO₂CH₃ |
| 336 | NO₂ | H | H | H | H | H | -(CH₂)₂CO₂CH₂CO₂C₂H₅ |
| 337 | NO₂ | H | H | H | H | H | -(CH₂)₂CO₂CH₂COCH₃ |
| 338 | NO₂ | H | H | H | H | H | -(CH₂)₂CO₂CH₂COC₂H₅ |
| 339 | NO₂ | H | H | H | H | H | -(CH₂)₂CO₂CH₂COC₆H₅ |
| 340 | NO₂ | H | Br | CF₃ | H | CH₃ | -(CH₂)₂CO₂CH₂CH₃ |
| 341 | NO₂ | H | CN | H | H | CH₃ | -(CH₂)₂CO₂CH₂CH₃ |
| 342 | NO₂ | H | NO₂ | H | H | CH₃ | -(CH₂)₂CO₂CH₃ |
| 343 | NO₂ | H | CN | CN | H | CH₃ | -(CH₂)₂CO₂CH₂CH₃ |
| 344 | NO₂ | H | CN | NO₂ | H | CH₃ | -(CH₂)₂CO₂CH₂CH₃ |
| 345 | NO₂ | H | Br | Cl | H | CH₃ | -(CH₂)₂CO₂CH₂CH₃ |

Preferred are dyes as described above having formula (I-b) 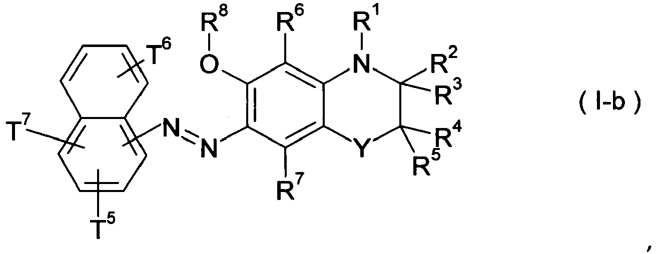 wherein
T⁵ to T⁷ independent from each other is hydrogen, (C₁-C₆)alkyl, (C₃-C₈)cycloalkyl, O-interrupted (C₁-C₆)-alkyl, S-interrupted (C₁-C₆)-alkyl, aryl, (C₁-C₆)alkoxyl, halogen, cyano, nitro, (C₁-C₆)-alkylacyl, arylacyl, (C₁-C₆)-alkoxycarbonyl, aryloxycarbonyl, *N*-(C₁-C₆)-alkyl carbamoyl, or *N,N*-bis(C₁-C₆)-alkyl carbamoyl,
whereby at least one of T⁵, T⁶ and T⁷ is not hydrogen,
R¹ is methyl, ethyl, propyl, isopropyl, n-butyl, isobutyl, benzyl, 2-methoxylethyl, 2-ethoxylethyl, 2-acetoxyethyl, 2-(propionyl)oxyethyl, 2-(benzoyl)oxyethyl, cyanomethyl, 2-cyanoethyl, 3-cyanopropyl, 3-(C₁-C₆)alkoxyl-3-oxopropyl, 3-[O-interrupted (C2-C6)-alkoxyl]-3-oxopropyl, 2-(C₁-C₆)alkyl-2-oxoethyl, 2-[O-interrupted (C2-C6)-alkoxyl]-2-oxoethyl, 2-(cyanomethoxy)-2-oxoethyl, 3-(cyanomethoxy)-3-oxopropyl, 2-[(arylacyl)methoxy] -2-oxoethyl, 3 - [(arylacyl)methoxy] -3 -oxopropyl, 3 -(arylmethoxy)-3-oxopropyl, 3-{[(C₁-C₆)alkylacyl]-methoxyl}-3-oxopropyl, 3 -[(arylacyl)-methoxy] -3-oxopropyl, 3-{[(C₁-C₆)-alkoxycabonyl]methoxy}-3-oxopropyl, 2-{[(C₁-C₆)-alkoxycabonyl]methoxy}-2-oxoethyl or 2-{[(C₁-C₆)alkylacyl]-methoxyl}-2-oxoethyl,
R² to R⁷ independent from each other is hydrogen, (C₁-C₆)alkyl, aryl, (C₁-C₈)alkoxyl, (C₃-C₈)cycloalkyl, O-interrupted (C₂-C₆)alkyl, S-interrupted (C₂-C₆)alkyl or halogen,
R⁸ is hydrogen, (C₁-C₆)alkyl, (C₃-C₄)-alkenyl, (C₃-C₄)-alkynyl, arylmethyl, 2-(C₁-C₆)alkoxyl-2-oxoethyl, 2-(arylmethyl)-2-oxoethyl, cyano(C₁-C₆)alkyl, O-interrupted (C₁-C₆)alkyl or S-interrupted (C₁-C₆)alkyl
   and
Y is carbonyl.

Examples of preferred dyes of the formula (I-b) are the compounds illustrated in Table 2, and mixtures thereof.

**Table 2**

| Example | Structure |
|---|---|
| 346 | 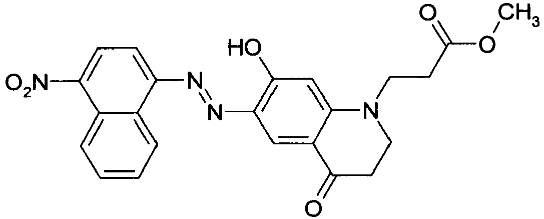 |
| 347 | 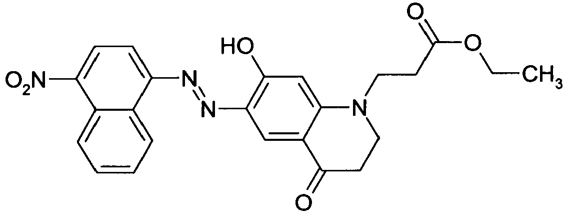 |
| 348 | 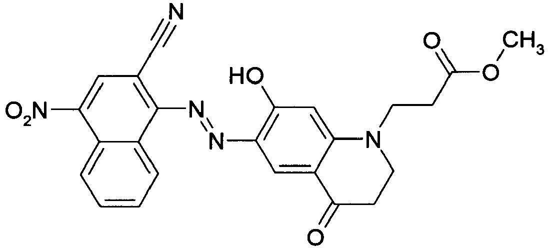 |
| 349 | 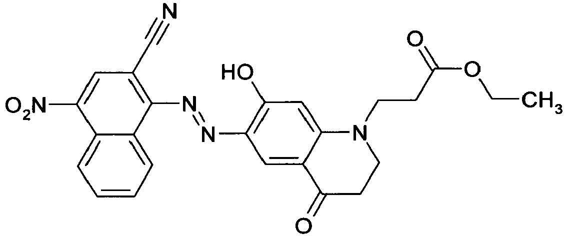 |

Another preferred aspect of the present invention are dyes as described above having formula (I-c) 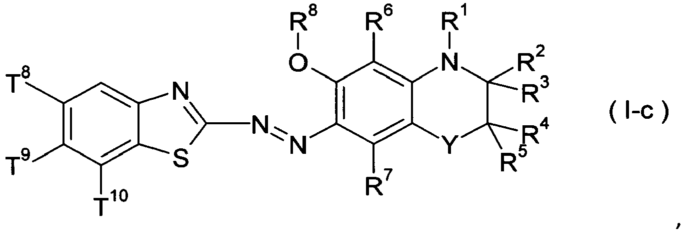 wherein
T⁸ and T¹⁰ independent from each other is hydrogen, chlorine or bromine,
T⁹ is hydrogen, (C₁-C₆)-alkylsulfonyl, thiocyano, (C₁-C₆)alkoxy, chlorine or nitro,
R¹ is methyl, ethyl, propyl, isopropyl, *n*-butyl, isobutyl, benzyl, 2-methoxylethyl, 2-ethoxylethyl, 2-acetoxyethyl, 2-(propionyl)oxyethyl, 2-(benzoyl)oxyethyl, cyanomethyl, 2-cyanoethyl, 3-cyanopropyl, 3-(C₁-C₆)alkoxyl-3-oxopropyl, 3-[O-interrupted (C₂-C₆)-alkoxyl]-3-oxopropyl, 2-(C₁-C₆)alkyl-2-oxoethyl, 2-[O-interrupted (C₂-C₆)-alkoxyl]-2-oxoethyl, 2-(cyanomethoxy)-2-oxoethyl, 3-(cyanomethoxy)-3-oxopropyl, 2-[(arylacyl)methoxy]-2-oxoethyl, 3-[(arylacyl)methoxy]-3-oxopropyl, 3-(arylmethoxy)-3-oxopropyl, 3-{[(Ci-C₆)alkylacyl]-methoxyl}-3-oxopropyl, 3-[(arylacyl)-methoxy]-3-oxopropyl, 3-{[(C₁-C₆)-alkoxycabonyl]methoxy}-3-oxopropyl, 2-{[(C₁-C₆)-alkoxycabonyl]methoxy}-2-oxoethyl or 2-{[(C₁-C₆)alkylacyl]-methoxyl}-2-oxoethyl,
R² to R⁷ independent from each other is hydrogen, (C₁-C₆)alkyl, aryl, (C₁-C₈)alkoxyl, (C₃-C₈)cycloalkyl, O-interrupted (C₂-C₆)alkyl, S-interrupted (C₂-C₆)alkyl orhalogen,
R⁸ is hydrogen, (C₁-C₆)alkyl, (C₃-C₄)-alkenyl, (C₃-C₄)-alkynyl, arylmethyl, 2-(C₁-C₆)alkoxyl-2-oxoethyl, 2-(arylmethyl)-2-oxoethyl, cyano(C₁-C₆)alkyl, O-interrupted (C₁-C₆)alkyl or S-interrupted (C₁-C₆)alkyl
   and
Y is carbonyl.

Examples of preferred dyes of the formula (I-c) are the compounds illustrated in Table 3 and mixtures thereof.

| **Table 3** | 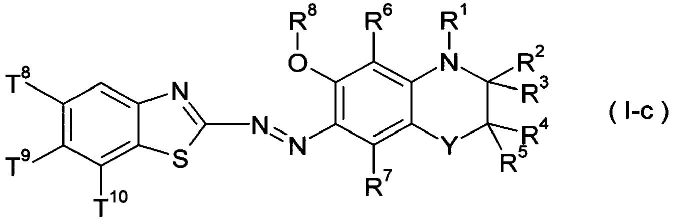 | | | | | |
|---|---|---|---|---|---|---|
| R³ to R⁷ is hydrogen, Y is carbonyl | | | | | | |
| Example | T⁸ | T⁹ | T¹⁰ | R² | R¹ | R⁸ |
| 350 | H | NO₂ | H | H | -(CH₂)₂CO₂H | H |
| 351 | H | NO₂ | H | H | -(CH₂)₂CO₂CH₃ | H |
| 352 | H | NO₂ | H | H | -(CH₂)₂CO₂CH₂CH₃ | H |
| 353 | H | NO₂ | H | H | -(CH₂)₂CO₂(CH₂)₂CH₃ | H |
| 354 | H | NO₂ | H | H | -(CH₂)₂CO₂CH(CH₃)₂ | H |
| 355 | H | NO₂ | H | H | -(CH₂)₂CO₂(CH₂)₃CH₃ | H |
| 356 | H | NO₂ | H | H | -(CH₂)₂CO₂CH(CH₃)CH₂CH₃ | H |
| 357 | H | NO₂ | H | H | -(CH₂)₂CO₂CH₂C₆H₅ | H |
| 358 | H | NO₂ | H | H | -(CH₂)₂CO₂(CH₂)₂OCH₃ | H |
| 359 | H | NO₂ | H | H | -(CH₂)₂CO₂(CH₂)₂OCH₂CH₃ | H |
| 360 | H | NO₂ | H | H | -(CH₂)₂CO₂(CH₂)₃OCH₃ | H |
| 361 | H | NO₂ | H | H | -(CH₂)₂CO₂(CH₂)₃OCH₂CH₃ | H |
| 362 | Cl | Cl | H | H | -(CH₂)₂CO₂H | H |
| 363 | Cl | Cl | H | H | -(CH₂)₂CO₂CH₃ | H |
| 364 | Cl | Cl | H | H | -(CH₂)₂CO₂CH₂CH₃ | H |
| 365 | Cl | Cl | H | H | -(CH₂)₂CO₂(CH₂)₂CH₃ | H |
| 366 | Cl | Cl | H | H | -(CH₂)₂CO₂CH(CH₃)₂ | H |
| 367 | Cl | Cl | H | H | -(CH₂)₂CO₂(CH₂)₃CH₃ | H |
| 368 | Cl | Cl | H | H | -(CH₂)₂CO₂CH(CH₃)CH₂CH₃ | H |
| 369 | Cl | Cl | H | H | -(CH₂)₂CO₂CH₂C₆H₅ | H |
| 370 | Cl | Cl | H | H | -(CH₂)₂CO₂(CH₂)₂OCH₃ | H |
| 371 | Cl | Cl | H | H | -(CH₂)₂CO₂(CH₂)₂OCH₂CH₃ | H |
| 372 | Cl | Cl | H | H | -(CH₂)₂CO₂(CH₂)₃OCH₃ | H |
| 373 | Cl | Cl | H | H | -(CH₂)₂CO₂(CH₂)₃OCH₂CH₃ | H |
| 374 | H | -OCH₃ | H | H | -(CH₂)₂CO₂H | H |
| 375 | H | -OCH₃ | H | H | -(CH₂)₂CO₂CH₃ | H |
| 376 | H | -OCH₃ | H | H | -(CH₂)₂CO₂CH₂CH₃ | H |
| 377 | H | -OCH₃ | H | H | -(CH₂)₂CO₂(CH₂)₂CH₃ | H |
| 378 | H | -OCH₃ | H | H | -(CH₂)₂CO₂CH(CH₃)₂ | H |
| 379 | H | -OCH₃ | H | H | -(CH₂)₂CO₂(CH₂)₃CH₃ | H |
| 380 | H | -OCH₃ | H | H | -(CH₂)₂CO₂CH(CH₃)CH₂CH₃ | H |
| 381 | H | -OCH₃ | H | H | -(CH₂)₂CO₂CH₂C₆H₅ | H |
| 382 | H | -OCH₃ | H | H | -(CH₂)₂CO₂(CH₂)₂OCH₃ | H |
| 383 | H | -OCH₃ | H | H | -(CH₂)₂CO₂(CH₂)₂OCH₂CH₃ | H |
| 384 | H | -OCH₃ | H | H | -(CH₂)₂CO₂(CH₂)₃OCH₃ | H |
| 385 | H | -OCH₃ | H | H | -(CH₂)₂CO₂(CH₂)₃OCH₂CH₃ | H |
| 386 | H | H | H | H | -(CH₂)₂CO₂H | H |
| 387 | H | H | H | H | -(CH₂)₂CO₂CH₃ | H |
| 388 | H | H | H | H | -(CH₂)₂CO₂CH₂CH₃ | H |
| 389 | H | H | H | H | -(CH₂)₂CO₂(CH₂)₂CH₃ | H |
| 390 | H | H | H | H | -(CH₂)₂CO₂CH(CH₃)₂ | H |
| 391 | H | H | H | H | -(CH₂)₂CO₂(CH₂)₃CH₃ | H |
| 392 | H | H | H | H | -(CH₂)₂CO₂CH(CH₃)CH₂CH₃ | H |
| 393 | H | H | H | H | -(CH₂)₂CO₂CH₂C₆H₅ | H |
| 394 | H | H | H | H | -(CH₂)₂CO₂(CH₂)₂OCH₃ | H |
| 395 | H | H | H | H | -(CH₂)₂CO₂(CH₂)₂OCH₂CH₃ | H |
| 396 | H | H | H | H | -(CH₂)₂CO₂(CH₂)₃OCH₃ | H |
| 397 | H | H | H | H | -(CH₂)₂CO₂(CH₂)₃OCH₂CH₃ | H |
| 398 | H | -SO₂CH₃ | H | H | -(CH₂)₂CO₂H | H |
| 399 | H | -SO₂CH₃ | H | H | -(CH₂)₂CO₂CH₃ | H |
| 400 | H | -SO₂CH₃ | H | H | -(CH₂)₂CO₂CH₂CH₃ | H |
| 401 | H | -SO₂CH₃ | H | H | -(CH₂)₂CO₂(CH₂)₂CH₃ | H |
| 402 | H | -SO₂CH₃ | H | H | -(CH₂)₂CO₂CH(CH₃)₂ | H |
| 403 | H | -SO₂CH₃ | H | H | -(CH₂)₂CO₂(CH₂)₃CH₃ | H |
| 404 | H | -SO₂CH₃ | H | H | -(CH₂)₂CO₂CH(CH₃)CH₂CH₃ | H |
| 405 | H | -SO₂CH₃ | H | H | -(CH₂)₂CO₂CH₂C₆H₅ | H |
| 406 | H | -SO₂CH₃ | H | H | -(CH₂)₂CO₂(CH₂)₂OCH₃ | H |
| 407 | H | -SO₂CH₃ | H | H | -(CH₂)₂CO₂(CH₂)₂OCH₂CH₃ | H |
| 408 | H | -SO₂CH₃ | H | H | -(CH₂)₂CO₂(CH₂)₃OCH₃ | H |
| 409 | H | -SO₂CH₃ | H | H | -(CH₂)₂CO₂(CH₂)₃OCH₂CH₃ | H |
| 410 | H | -SO₂CH₃ | H | H | -(CH₂)₂CO₂(CH₂)₃OCH₂CH₃ | -CH₃ |

Just another preferred embodiment of the present invention are dyes as described above having formula (I-d) 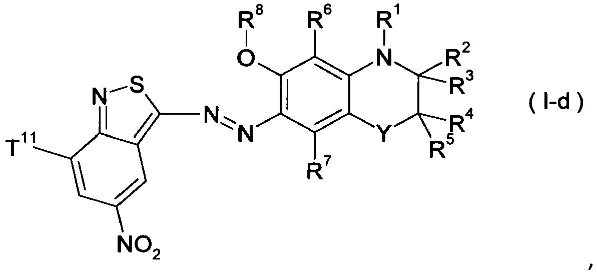 wherein
T¹¹ is hydrogen, nitro or bromine,
R¹ is methyl, ethyl, propyl, isopropyl, *n*-butyl, isobutyl, benzyl, 2-methoxylethyl, 2-ethoxylethyl, 2-acetoxyethyl, 2-(propionyl)oxyethyl, 2-(benzoyl)oxyethyl, cyanomethyl, 2-cyanoethyl, 3-cyanopropyl, 3-(C₁-C₆)alkoxyl-3-oxopropyl, 3-[O-interrupted (C₂-C₆)-alkoxyl]-3-oxopropyl, 2-(C₁-C₆)alkyl-2-oxoethyl, 2-[O-interrupted (C₂-C₆)-alkoxyl]-2-oxoethyl, 2-(cyanomethoxy)-2-oxoethyl, 3 -(cyanomethoxy)-3 -oxopropyl, 2-[(arylacyl)methoxy] -2-oxoethyl, 3-[(arylacyl)methoxy]-3-oxopropyl, 3-(arylmethoxy)-3-oxopropyl, 3-{[(C₁-C₆)alkylacyl]-methoxyl}-3-oxopropyl, 3-[(arylacyl)-methoxy]-3-oxopropyl, 3-{[(C₁-C₆)-alkoxycabonyl]methoxy}-3-oxopropyl, 2-{[(C₁-C₆)-alkoxycabonyl]methoxy}-2-oxoethyl or 2-{[(C₁-C₆)alkylacyl]-methoxyl}2-oxoethyl,
R² to R⁷ independent from each other is hydrogen, (C₁-C₆)alkyl, aryl, (C₁-C₈)alkoxyl, (C₃-C₈)cycloalkyl, O-interrupted (C₂-C₆)alkyl, S-interrupted (C₂-C₆)alkyl or halogen,
R⁸ is hydrogen, (C₁-C₆)alkyl, (C₃-C₄)-alkenyl, (C₃-C₄)-alkynyl, arylmethyl, 2-(C₁-C₆)alkoxyl-2-oxoethyl, 2-(arylmethyl)-2-oxoethyl, cyano(C₁-C₆)alkyl, O-interrupted (C₁-C₆)alkyl or S-interrupted (C₁-C₆)alkyl
   and
Y is carbonyl.

Examples of preferred dyes of the formula (I-d) are the compounds illustrated in Table 4 and mixtures thereof.

| **Table 4** | | | | |
|---|---|---|---|---|
| 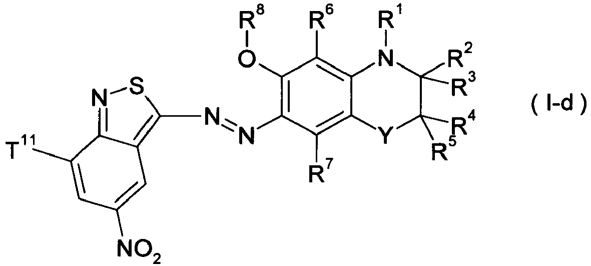 | | | | |
| R³ to R⁷ is hydrogen, Y is carbonyl | | | | |

| Example | T¹¹ | R² | R¹ | R⁸ |
|---|---|---|---|---|
| 411 | H | H | -(CH₂)₂CO₂H | H |
| 412 | H | H | -(CH₂)₂CO₂CH₃ | H |
| 413 | H | H | -(CH₂)₂CO₂CH₂CH₃ | H |
| 414 | H | H | -(CH₂)₂CO₂(CH₂)₂CH₃ | H |
| 415 | H | H | -(CH₂)₂CO₂CH(CH₃)₂ | H |
| 416 | H | H | -(CH₂)₂CO₂(CH₂)₃CH₃ | H |
| 417 | H | H | -(CH₂)₂CO₂CH(CH₃)CH₂CH₃ | H |
| 418 | H | H | -(CH₂)₂CO₂CH₂C₆H₅ | H |
| 419 | H | H | -(CH₂)₂CO₂(CH₂)₂OCH₃ | H |
| 420 | H | H | -(CH₂)₂CO₂(CH₂)₂OCH₂CH₃ | H |
| 421 | H | H | -(CH₂)₂CO₂(CH₂)₃OCH₃ | H |
| 422 | H | H | -(CH₂)₂CO₂(CH₂)₃OCH₂CH₃ | H |
| 423 | Br | H | -(CH₂)₂CO₂H | H |
| 424 | Br | H | -(CH₂)₂CO₂CH₃ | H |
| 425 | Br | H | -(CH₂)₂CO₂CH₂CH₃ | H |
| 426 | Br | H | -(CH₂)₂CO₂(CH₂)₂CH₃ | H |
| 427 | Br | H | -(CH₂)₂CO₂CH(CH₃)₂ | H |
| 428 | Br | H | -(CH₂)₂CO₂(CH₂)₃CH₃ | H |
| 429 | Br | H | -(CH₂)₂CO₂CH(CH₃)CH₂CH₃ | H |
| 430 | Br | H | -(CH₂)₂CO₂CH₂C₆H₅ | H |
| 431 | Br | H | -(CH₂)₂CO₂(CH₂)₂OCH₃ | H |
| 432 | Br | H | -(CH₂)₂CO₂(CH₂)₂OCH₂CH₃ | H |
| 433 | Br | H | -(CH₂)₂CO₂(CH₂)₃OCH₃ | H |
| 434 | Br | H | -(CH₂)₂CO₂(CH₂)₃OCH₂CH₃ | H |
| 435 | Br | -CH₃ | -CH(CH₃)CH₂CO₂(CH₂)₃OCH₂CH₃ | H |
| 436 | Br | H | -(CH₂)₂CO₂(CH₂)₃OCH₂CH₃ | -CH₃ |

Also preferred are dyes as described above having formula (I-e) 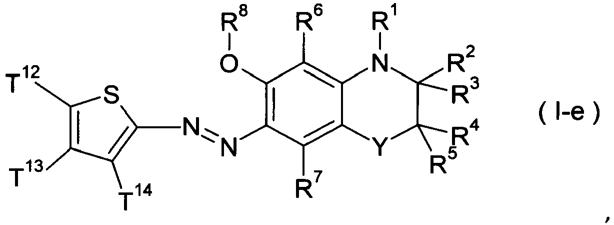 wherein
T¹² and T¹⁴ independent from each other, are nitro, cyano, formyl, acetyl or (C₁-C₆)-alkoxycarbonyl,
T¹³ is hydrogen, aryl, (C₁-C₆)-akyl or halogen,
R¹ is methyl, ethyl, propyl, isopropyl, *n*-butyl, isobutyl, benzyl, 2-methoxylethyl, 2-ethoxylethyl, 2-acetoxyethyl, 2-(propionyl)oxyethyl, 2-(benzoyl)oxyethyl, cyanomethyl, 2-cyanoethyl, 3-cyanopropyl, 3-(C₁-C₆)alkoxyl-3-oxopropyl, 3-[O-interrupted (C₂-C₆)-alkoxyl]-3-oxopropyl, 2-(C₁-C₆)alkyl-2-oxoethyl, 2-[O-interrupted (C₂-C₆)-alkoxyl]-2-oxoethyl, 2-(cyanomethoxy)-2-oxoethyl, 3-(cyanomethoxy)-3-oxopropyl, 2-[(arylacyl)methoxy]-2-oxoethyl, 3-[(arylacyl)methoxy]-3-oxopropyl, 3-(arylmethoxy)-3-oxopropyl, 3-{[(C₁-C₆)alkylacyl]-methoxyl}-3-oxopropyl, 3-[(arylacyl)-methoxy]-3-oxopropyl, 3-{[(C₁-C₆)-alkoxycabonyl]methoxy}-3-oxopropyl, 2-{[(C₁-C₆)-alkoxycabonyl]methoxy}-2-oxoethyl or 2-{[(C₁-C₆)alkylacyl]-methoxyl}-2-oxoethyl,
R² to R⁷ independent from each other is hydrogen, (C₁-C₆)alkyl, aryl, (C₁-C₈)alkoxyl, (C₃-C₈)cycloalkyl, O-interrupted (C₂-C₆)alkyl, S-interrupted (C₂-C₆)alkyl or halogen,
R⁸ is hydrogen, (C₁-C₆)alkyl, (C₃-C₄)-alkenyl, (C₃-C₄)-alkynyl, arylmethyl, 2-(C₁-C₆)alkoxyl-2-oxoethyl, 2-(arylmethyl)-2-oxoethyl, cyano(C₁-C₆)alkyl, O-interrupted (C₁-C₆)alkyl or S-interrupted (C₁-C₆)alkyl
   and
Y is carbonyl.

Examples of preferred dyes of the formula (I-e) are the compounds illustrated in Table 5 and mixtures thereof.

| **Table 5** | | | | | | |
|---|---|---|---|---|---|---|
| 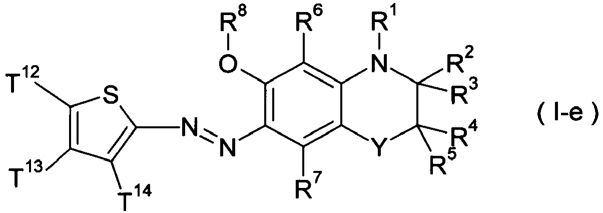 | | | | | | |

| R³ to R⁷ is hydrogen, Y is carbonyl | | | | | | |
|---|---|---|---|---|---|---|
| Exp 1. | T¹² | T¹⁴ | T¹³ | R² | R¹ | R⁸ |
| 437 | NO₂ | NO₂ | H | H | -(CH₂)₂CO₂H | H |
| 438 | NO₂ | NO₂ | H | H | -(CH₂)₂CO₂CH₃ | H |
| 439 | NO₂ | NO₂ | H | H | -(CH₂)₂CO₂CH₂CH₃ | H |
| 440 | NO₂ | NO₂ | H | H | -(CH₂)₂CO₂(CH₂)₂CH₃ | H |
| 441 | NO₂ | NO₂ | H | H | -(CH₂)₂CO₂CH(CH₃)₂ | H |
| 442 | NO₂ | NO₂ | H | H | -(CH₂)₂CO₂(CH₂)₃CH₃ | H |
| 443 | NO₂ | NO₂ | H | H | -(CH₂)₂CO₂CH(CH₃)CH₂CH₃ | H |
| 444 | NO₂ | NO₂ | H | H | -(CH₂)₂CO₂CH₂C₆H₅ | H |
| 445 | NO₂ | NO₂ | H | H | -(CH₂)₂CO₂(CH₂)₂OCH₃ | H |
| 446 | NO₂ | NO₂ | H | H | -(CH₂)₂CO₂(CH₂)₂OCH₂CH₃ | H |
| 447 | NO₂ | NO₂ | H | H | -(CH₂)₂CO₂(CH₂)₃OCH₃ | H |
| 448 | NO₂ | NO₂ | H | H | -(CH₂)₂CO₂(CH₂)₃OCH₂CH₃ | H |
| 449 | CN | CN | H | H | -(CH₂)₂CO₂H | H |
| 450 | CN | CN | CH₃ | H | -(CH₂)₂CO₂CH₃ | H |
| 451 | CN | CN | CH₃ | H | -(CH₂)₂CO₂CH₂CH₃ | H |
| 452 | CN | CN | CH₃ | H | -(CH₂)₂CO₂(CH₂)₂CH₃ | H |
| 453 | CN | CN | CH₃ | H | -(CH₂)₂CO₂CH(CH₃)₂ | H |
| 454 | CN | CN | CH₃ | H | -(CH₂)₂CO₂(CH₂)₃CH₃ | H |
| 455 | CN | CN | CH₃ | H | -(CH₂)₂CO₂CH(CH₃)CH₂CH₃ | H |
| 456 | CN | CN | CH₃ | H | -(CH₂)₂CO₂CH₂C₆H₅ | H |
| 457 | CN | CN | CH₃ | H | -(CH₂)₂CO₂(CH₂)₂OCH₃ | H |
| 458 | CN | CN | CH₃ | H | -(CH₂)₂CO₂(CH₂)₂OCH₂CH₃ | H |
| 459 | CN | CN | CH₃ | H | -(CH₂)₂CO₂(CH₂)₃OCH₃ | H |
| 460 | CN | CN | CH₃ | H | -(CH₂)₂CO₂(CH₂)₃OCH₂CH₃ | H |
| 461 | -CHO | CN | Cl | H | -(CH₂)₂CO₂H | H |
| 462 | -CHO | CN | Cl | H | -(CH₂)₂CO₂CH₃ | H |
| 463 | -CHO | CN | Cl | H | -(CH₂)₂CO₂CH₂CH₃ | H |
| 464 | -CHO | CN | Cl | H | -(CH₂)₂CO₂(CH₂)₂CH₃ | H |
| 465 | -CHO | CN | Cl | H | -(CH₂)₂CO₂CH(CH₃)₂ | H |
| 466 | -CHO | CN | Cl | H | -(CH₂)₂CO₂(CH₂)₃CH₃ | H |
| 467 | -CHO | CN | Cl | H | -(CH₂)₂CO₂CH(CH₃)CH₂CH₃ | H |
| 468 | -CHO | CN | Cl | H | -(CH₂)₂CO₂CH₂C₆H₅ | H |
| 469 | -CHO | CN | Cl | H | -(CH₂)₂CO₂(CH₂)₂OCH₃ | H |
| 470 | -CHO | CN | Cl | H | -(CH₂)₂CO₂(CH₂)₂OCH₂CH₃ | H |
| 471 | -CHO | CN | Cl | H | -(CH₂)₂CO₂(CH₂)₃OCH₃ | H |
| 472 | -CHO | CN | Cl | H | -(CH₂)₂CO₂(CH₂)₃OCH₂CH₃ | H |
| 473 | -CO₂CH₂CH₃ | CN | CH₃ | H | -(CH₂)₂CO₂H | H |
| 474 | -CO₂CH₂CH₃ | CN | CH₃ | H | -(CH₂)₂CO₂CH₃ | H |
| 475 | -CO₂CH₂CH₃ | CN | CH₃ | H | -(CH₂)₂CO₂CH₂CH₃ | H |
| 476 | -CO₂CH₂CH₃ | CN | CH₃ | H | -(CH₂)₂CO₂(CH₂)₂CH₃ | H |
| 477 | -CO₂CH₂CH₃ | CN | CH₃ | H | -(CH₂)₂CO₂CH(CH₃)₂ | H |
| 478 | -CO₂CH₂CH₃ | CN | CH₃ | H | -(CH₂)₂CO₂(CH₂)₃CH₃ | H |
| 479 | -CO₂CH₂CH₃ | CN | CH₃ | H | -(CH₂)₂CO₂CH(CH₃)CH₂CH₃ | H |
| 480 | -CO₂CH₂CH₃ | CN | CH₃ | H | -(CH₂)₂CO₂CH₂C₆H₅ | H |
| 481 | -CO₂CH₂CH₃ | CN | CH₃ | H | -(CH₂)₂CO₂(CH₂)₂OCH₃ | H |
| 482 | -CO₂CH₂CH₃ | CN | CH₃ | H | -(CH₂)₂CO₂(CH₂)₂OCH₂CH₃ | H |
| 483 | -CO₂CH₂CH₃ | CN | CH₃ | H | -(CH₂)₂CO₂(CH₂)₃OCH₃ | H |
| 484 | -CO₂CH₂CH₃ | CN | CH₃ | H | -(CH₂)₂CO₂(CH₂)₃OCH₂CH₃ | H |
| 485 | CN | -CO₂CH₂CH₃ | CH₃ | H | -(CH₂)₂CO₂H | H |
| 486 | CN | -CO₂CH₂CH₃ | CH₃ | H | -(CH₂)₂CO₂CH₃ | H |
| 487 | CN | -CO₂CH₂CH₃ | CH₃ | H | -(CH₂)₂CO₂CH₂CH₃ | H |
| 488 | CN | -CO₂CH₂CH₃ | CH₃ | H | -(CH₂)₂CO₂(CH₂)₂CH₃ | H |
| 489 | CN | -CO₂CH₂CH₃ | CH₃ | H | -(CH₂)₂CO₂CH(CH₃)₂ | H |
| 490 | CN | -CO₂CH₂CH₃ | CH₃ | H | -(CH₂)₂CO₂(CH₂)₃CH₃ | H |
| 491 | CN | -CO₂CH₂CH₃ | CH₃ | H | -(CH₂)₂CO₂CH(CH₃)CH₂CH₃ | H |
| 492 | CN | -CO₂CH₂CH₃ | CH₃ | H | -(CH₂)₂CO₂CH₂C₆H₅ | H |
| 493 | CN | -CO₂CH₂CH₃ | CH₃ | H | -(CH₂)₂CO₂(CH₂)₂OCH₃ | H |
| 494 | CN | -CO₂CH₂CH₃ | CH₃ | H | -(CH₂)₂CO₂(CH₂)₂OCH₂CH₃ | H |
| 495 | CN | -CO₂CH₂CH₃ | CH₃ | H | -(CH₂)₂CO₂(CH₂)₃OCH₃ | H |
| 496 | CN | -CO₂CH₂CH₃ | CH₃ | H | -(CH₂)₂CO₂(CH₂)₃OCH₂CH₃ | H |
| 497 | NO₂ | -CO₂CH₂CH₃ | H | H | -(CH₂)₂CO₂H | H |
| 498 | NO₂ | -CO₂CH₂CH₃ | H | H | -(CH₂)₂CO₂CH₃ | H |
| 499 | NO₂ | -CO₂CH₂CH₃ | H | H | -(CH₂)₂CO₂CH₂CH₃ | H |
| 500 | NO₂ | -CO₂CH₂CH₃ | H | H | -(CH₂)₂CO₂(CH₂)₂CH₃ | H |
| 501 | NO₂ | -CO₂CH₂CH₃ | H | H | -(CH₂)₂CO₂CH(CH₃)₂ | H |
| 502 | NO₂ | -CO₂CH₂CH₃ | H | H | -(CH₂)₂CO₂(CH₂)₃CH₃ | H |
| 503 | NO₂ | -CO₂CH₂CH₃ | H | H | -(CH₂)₂CO₂CH(CH₃)CH₂CH₃ | H |
| 504 | NO₂ | -CO₂CH₂CH₃ | H | H | -(CH₂)₂CO₂CH₂C₆H₅ | H |
| 505 | NO₂ | -CO₂CH₂CH₃ | H | H | -(CH₂)₂CO₂(CH₂)₂OCH₃ | H |
| 506 | NO₂ | -CO₂CH₂CH₃ | H | H | -(CH₂)₂CO₂(CH₂)₂OCH₂CH₃ | H |
| 507 | NO₂ | -CO₂CH₂CH₃ | H | H | -(CH₂)₂CO₂(CH₂)₃OCH₃ | H |
| 508 | NO₂ | -CO₂CH₂CH₃ | H | H | -(CH₂)₂CO₂(CH₂)₃OCH₂CH₃ | H |
| 509 | NO₂ | -CO₂CH₂CH₃ | H | CH₃ | -CH(CH₃)CO₂(CH₂)₂CH₃ | H |
| 510 | NO₂ | -CO₂CH₂CH₃ | H | H | -(CH₂)₂CO₂(CH₂)₃OCH₂CH₃ | CH₃ |

Also preferred are dyes as described above having formula (I-f) 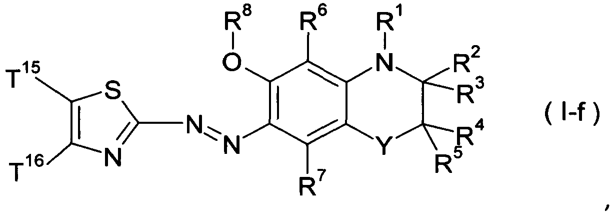 wherein
T¹⁵ and T¹⁶ independent from each other is hydrogen, halogen, (C₁-C₆)-alkyl, nitro, cyano, formyl, acetyl, (C₁-C₆)-alkoxycarbonyl, O-interrupted (C₁-C₆)-alkyl, S-interrupted (C₁-C₆)-alkyl or aryl,
R¹ is methyl, ethyl, propyl, isopropyl, *n*-butyl, isobutyl, benzyl, 2-methoxylethyl, 2-ethoxylethyl, 2-acetoxyethyl, 2-(propionyl)oxyethyl, 2-(benzoyl)oxyethyl, cyanomethyl, 2-cyanoethyl, 3-cyanopropyl, 3-(C₁-C₆)alkoxyl-3-oxopropyl, 3-[O-interrupted (C₂-C₆)-alkoxyl]-3-oxopropyl, 2-(C₁-C₆)alkyl-2-oxoethyl, 2-[O-interrupted (C₂-C₆)-alkoxyl]-2-oxoethyl, 2-(cyanomethoxy)-2-oxoethyl, 3-(cyanomethoxy)-3-oxopropyl, 2-[(arylacyl)methoxy]-2-oxoethyl, 3-[(arylacyl)methoxy]-3-oxopropyl, 3-(arylmethoxy)-3-oxopropyl, 3-{[(C₁-C₆)alkylacyl]-methoxyl}-3-oxopropyl, 3-[(arylacyl)-methoxy]-3-oxopropyl, 3-{[(C₁-C₆)-alkoxycabonyl]methoxy}-3-oxopropyl, 2-{[(C₁-C₆)-alkoxycabonyl]methoxy}-2-oxoethyl or 2-{[(C₁-C₆)alkylacyl]-methoxyl}-2-oxoethyl,
R² to R⁷ independent from each other is hydrogen, (C₁-C₆)alkyl, aryl, (C₁-C₈)alkoxyl, (C₃-C₈)cycloalkyl, O-interrupted (C₂-C₆)alkyl, S-interrupted (C₂-C₆)alkyl or halogen,
R⁸ is hydrogen, (C₁-C₆)alkyl, (C₃-C₄)-alkenyl, (C₃-C₄)-alkynyl, arylmethyl, 2-(C₁-C₆)alkoxyl-2-oxoethyl, 2-(arylmethyl)-2-oxoethyl, cyano(C₁-C₆)alkyl, O-interrupted (C₁-C₆)alkyl or S-interrupted (C₁-C₆)alkyl
   and
Y is carbonyl.

Examples of preferred dyes of the formula (I-f) are the compounds illustrated in Table 6 and mixtures thereof.

| **Table 6** | | | | | |
|---|---|---|---|---|---|
| 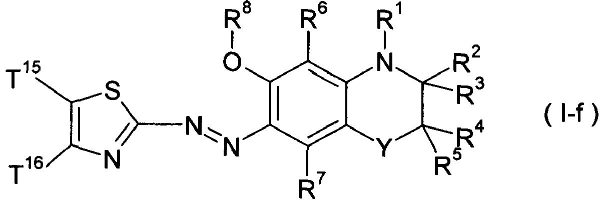 | | | | | |
| R³ to R⁷ is hydrogen, Y is carbonyl | | | | | |

| Example | T¹⁵ | T¹⁶ | R² | R¹ | R⁸ |
|---|---|---|---|---|---|
| 511 | NO₂ | H | H | -(CH₂)₂CO₂H | H |
| 512 | NO₂ | H | H | -(CH₂)₂CO₂CH₃ | H |
| 513 | NO₂ | H | H | -(CH₂)₂CO₂CH₂CH₃ | H |
| 514 | NO₂ | H | H | -(CH₂)₂CO₂(CH₂)₂CH₃ | H |
| 515 | NO₂ | H | H | -(CH₂)₂CO₂CH(CH₃)₂ | H |
| 516 | NO₂ | H | H | -(CH₂)₂CO₂(CH₂)₃CH₃ | H |
| 517 | NO₂ | H | H | -(CH₂)₂CO₂CH(CH₃)CH₂CH₃ | H |
| 518 | NO₂ | H | H | -(CH₂)₂CO₂CH₂C₆H₅ | H |
| 519 | NO₂ | H | H | -(CH₂)₂CO₂(CH₂)₂OCH₃ | H |
| 520 | NO₂ | H | H | -(CH₂)₂CO₂(CH₂)₂OCH₂CH₃ | H |
| 521 | NO₂ | H | H | -(CH₂)₂CO₂(CH₂)₃OCH₃ | H |
| 522 | NO₂ | H | H | -(CH₂)₂CO₂(CH₂)₃OCH₂CH₃ | H |
| 523 | -CHO | Cl | H | -(CH₂)₂CO₂H | H |
| 524 | -CHO | Cl | H | -(CH₂)₂CO₂CH₃ | H |
| 525 | -CHO | Cl | H | -(CH₂)₂CO₂CH₂CH₃ | H |
| 526 | -CHO | Cl | H | -(CH₂)₂CO₂(CH₂)₂CH₃ | H |
| 527 | -CHO | Cl | H | -(CH₂)₂CO₂CH(CH₃)₂ | H |
| 528 | -CHO | Cl | H | -(CH₂)₂CO₂(CH₂)₃CH₃ | H |
| 529 | -CHO | Cl | H | -(CH₂)₂CO₂CH(CH₃)CH₂CH₃ | H |
| 530 | -CHO | Cl | H | -(CH₂)₂CO₂CH₂C₆H₅ | H |
| 531 | -CHO | Cl | H | -(CH₂)₂CO₂(CH₂)₂OCH₃ | H |
| 532 | -CHO | Cl | H | -(CH₂)₂CO₂(CH₂)₂OCH₂CH₃ | H |
| 533 | -CHO | Cl | H | -(CH₂)₂CO₂(CH₂)₃OCH₃ | H |
| 534 | -CHO | Cl | H | -(CH₂)₂CO₂(CH₂)₃OCH₂CH₃ | H |
| 535 | -CHO | Cl | -CH₃ | -CH(CH₃)CO₂CH₂CH₃ | H |
| 536 | -CHO | Cl | H | -(CH₂)₂CO₂CH₂CH₃ | -CH₃ |

Another preferred group of dyes are dyes as described above having formula (I-g) 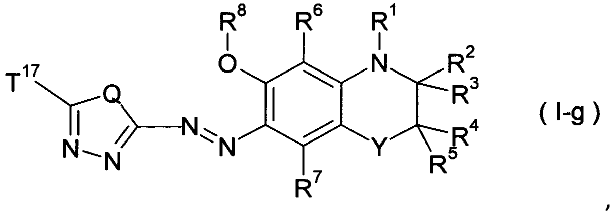 wherein
T¹⁷ is aryl or (C₁-C₆)-thioalkoxyl,
Q is oxygen or sulphur,
R¹ is methyl, ethyl, propyl, isopropyl, *n*-butyl, isobutyl, benzyl, 2-methoxylethyl, 2-ethoxylethyl, 2-acetoxyethyl, 2-(propionyl)oxyethyl, 2-(benzoyl)oxyethyl, cyanomethyl, 2-cyanoethyl, 3-cyanopropyl, 3-(C₁-C₆)alkoxyl-3-oxopropyl, 3-[O-interrupted (C₂-C₆)-alkoxyl]-3-oxopropyl, 2-(C₁-C₆)alkyl-2-oxoethyl, 2-[O-interrupted (C₂-C₆)-alkoxyl]-2-oxoethyl, 2-(cyanomethoxy)-2-oxoethyl, 3-(cyanomethoxy)-3-oxopropyl, 2-[(arylacyl)methoxy] -2-oxoethyl, 3 -[(arylacyl)methoxy] -3 -oxopropyl, 3-(arylmethoxy)-3-oxopropyl, 3-{[(C₁-C₆)alkylacyl]-methoxyl}-3-oxopropyl, 3-[(arylacyl)-methoxy]-3-oxopropyl, 3-{[(C₁-C₆)-alkoxycabonyl]methoxy}-3-oxopropyl, 2-{[(C₁-C₆)-alkoxycabonyl]methoxy} -2-oxoethyl or 2-{[(C₁-C₆)alkylacyl]-methoxyl}-2-oxoethyl,
R² to R⁷ independent from each other is hydrogen, (C₁-C₆)alkyl, aryl, (C₁-C₈)alkoxyl, (C₃-C₈)cycloalkyl, O-interrupted (C₂-C₆)alkyl, S-interrupted (C₂-C₆)alkyl, halogen,
R⁸ is hydrogen, (C₁-C₆)alkyl, (C₃-C₄)-alkenyl, (C₃-C₄)-alkynyl, arylmethyl, 2-(C₁-C₆)alkoxyl-2-oxoethyl, 2-(arylmethyl)-2-oxoethyl, cyano(C₁-C₆)alkyl, O-interrupted (C₁-C₆)alkyl or S-interrupted (C₁-C₆)alkyl
   and
Y is carbonyl.

Examples of preferred dyes of the formula (I-g) are the compounds illustrated in Table 7 and mixtures thereof.

| **Table 7** | | | | |
|---|---|---|---|---|
| 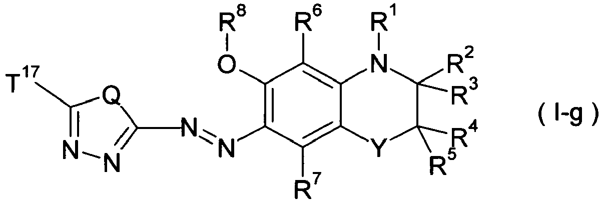 | | | | |

| R³ to R⁷ is hydrogen, Y is carboxyl and Q is sulphur | | | | |
|---|---|---|---|---|
| Example | T¹⁷ | R¹ | R² | R⁸ |
| 537 | -SCH₂CH₃ | -CH₂CH₃ | H | H |
| 538 | -SCH₂CH₃ | -CH₂C₆H₅ | H | H |
| 539 | -SCH₂CH₃ | -(CH₂)₂CN | H | H |
| 540 | -SCH₂CH₃ | -CH₃ | H | H |
| 541 | -SCH₂CH₃ | -CH₂CH₃ | -CH₃ | H |
| 542 | -SCH₂CH₃ | -(CH₂)₂CH₃ | H | H |
| 543 | -SCH₂CH₃ | -CH(CH₃)₂ | H | H |
| 544 | -SCH₂CH₃ | -(CH₂)₃CH₃ | H | H |
| 545 | -SCH₂CH₃ | -CH(CH₃)CH₂CH₃ | H | H |
| 546 | -SCH₂CH₃ | -CH₂CO₂CH₃ | H | H |
| 547 | -SCH₂CH₃ | -CH₂CO₂CH₂CH₃ | H | H |
| 548 | -SCH₂CH₃ | -(CH₂)₂CO₂H | H | H |
| 549 | -SCH₂CH₃ | -(CH₂)₂CO₂CH₃ | H | H |
| 550 | -SCH₂CH₃ | -(CH₂)₂CO₂CH₂CH₃ | H | H |
| 551 | -SCH₂CH₃ | -(CH₂)₂CO₂(CH₂)₂CH₃ | H | H |
| 552 | -SCH₂CH₃ | -(CH₂)₂CO₂CH(CH₃)₂ | H | H |
| 553 | -SCH₂CH₃ | -(CH₂)₂CO₂(CH₂)₃CH₃ | H | H |
| 554 | -SCH₂CH₃ | -(CH₂)₂CO₂CH(CH₃)CH₂CH₃ | H | H |
| 555 | -SCH₂CH₃ | -(CH₂)₂CO₂CH₂C₆H₅ | H | H |
| 556 | -SCH₂CH₃ | -(CH₂)₂CO₂(CH₂)₂OCH₃ | H | H |
| 557 | -SCH₂CH₃ | -(CH₂)₂CO₂(CH₂)₂OCH₂CH₃ | H | H |
| 558 | -SCH₂CH₃ | -(CH₂)₂CO₂(CH₂)₃OCH₃ | H | H |
| 559 | -SCH₂CH₃ | -(CH₂)₂CO₂(CH₂)₃OCH₂CH₃ | H | H |
| 560 | -SCH₂CH₃ | -CH(CH₃)CO₂CH₂CH₃ | -CH₃ | H |
| 561 | -SCH₂CH₃ | -(CH₂)₂CO₂CH₂CH₃ | H | -CH₃ |

Also preferred are dyes as described above having formula (I-h) 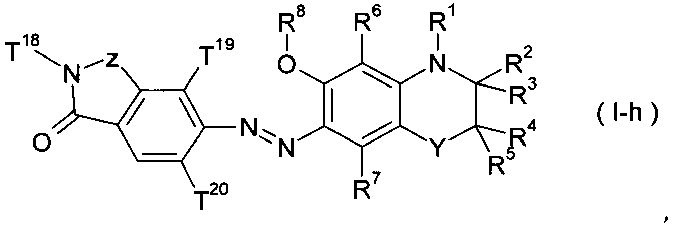 wherein
T¹⁸ is (C₁-C₆)alkyl, (C₁-C₆)-acyloxy substituted (C₁-C₆)alkyl, cyano(C₁-C₆)alkyl, arylmethyl, arylcarbonyl substituted (C₁-C₆)alkyl, 2-(C₁-C₆)alkoxyl-2-oxoethyl, O-interrupted (C₁-C₆)-alkyl, S-interrupted (C₁-C₆)-alkyl or aryl,
T¹⁹ and T²⁰ independent from each other are hydrogen, halogen, cyano, nitro or trifluoromethyl,
Z is a carbonyl or a sulfonyl rest,
R¹ is methyl, ethyl, propyl, isopropyl, *n*-butyl, isobutyl, benzyl, 2-methoxylethyl, 2-ethoxylethyl, 2-acetoxyethyl, 2-(propionyl)oxyethyl, 2-(benzoyl)oxyethyl, cyanomethyl, 2-cyanoethyl, 3-cyanopropyl, 3-(C₁-C₆)alkoxyl-3-oxopropyl, 3-[O-interrupted (C₂-C₆)-alkoxyl]-3-oxopropyl, 2-(C₁-C₆)alkyl-2-oxoethyl, 2-[O-interrupted (C₂-C₆)-alkoxyl]-2-oxoethyl, 2-(cyanomethoxy)-2-oxoethyl, 3-(cyanomethoxy)-3-oxopropyl, 2-[(arylacyl)methoxy] -2-oxoethyl, 3-[(arylacyl)methoxy]-3-oxopropyl, 3 -(arylmethoxy)-3-oxopropyl, 3-{[(C₁-C₆)alkylacyl]-methoxyl}-3-oxopropyl, 3-[(arylacyl)-methoxy]-3-oxopropyl, 3-{[(C₁-C₆)-alkoxycabonyl]methoxy}-3-oxopropyl, 2-{[(C₁-C₆)-alkoxycabonyl]methoxy}-2-oxoethyl or 2-{[(C₁-C₆)alkylacyl]-methoxyl}-2-oxoethyl,
R² to R⁷ independent from each other is hydrogen, (C₁-C₆)alkyl, aryl, (C₁-C₈)alkoxyl, (C₃-C₈)cycloalkyl, O-interrupted (C₂-C₆)alkyl, S-interrupted (C₂-C₆)alkyl or halogen,
R⁸ is hydrogen, (C₁-C₆)alkyl, (C₃-C₄)-alkenyl, (C₃-C₄)-alkynyl, arylmethyl, 2-(C₁-C₆)alkoxyl-2-oxoethyl, 2-(arylmethyl)-2-oxoethyl, cyano(C₁-C₆)alkyl, O-interrupted (C₁-C₆)alkyl or S-interrupted (C₁-C₆)alkyl
   and
Y is carbonyl.

Examples of preferred dyes of the formula (I-h) are the compounds illustrated in Table 8 and mixtures thereof.

| **Table 8** | | | | | | |
|---|---|---|---|---|---|---|
| 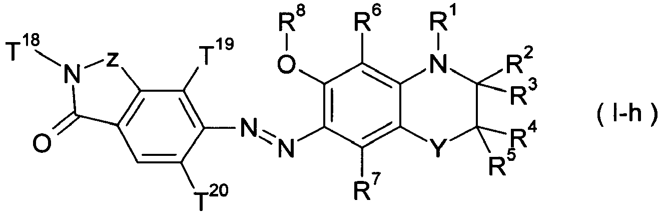 | | | | | | |

| R³ to R⁷ is hydrogen, Y and Z are carbonyl | | | | | | |
|---|---|---|---|---|---|---|
| Example | T¹⁸ | T¹⁹ | T²⁰ | R² | R¹ | R⁸ |
| 562 | -CH₂CH₃ | CN | CN | H | -(CH₂)₂CO₂CH₂CH₃ | H |
| 563 | -CH₂CH₃ | CN | CN | H | -(CH₂)₂CO₂CH₃ | H |
| 564 | -CH₂CH₃ | Br | Br | H | -(CH₂)₂CO₂CH₂CH₃ | H |
| 565 | -CH₂CH₃ | Br | Br | H | -(CH₂)₂CO₂CH₃ | H |
| 566 | -CH₂CH₃ | H | Br | H | -(CH₂)₂CO₂CH₂CH₃ | H |
| 567 | -CH₂CH₃ | H | Br | H | -(CH₂)₂CO₂CH₃ | H |
| 568 | -CH₂CH₃ | H | CN | H | -(CH₂)₂CO₂CH₂CH₃ | H |
| 569 | -CH₂CH₃ | H | CN | H | -(CH₂)₂CO₂CH₃ | H |
| 570 | -CH₂CH₃ | H | H | H | -(CH₂)₂CO₂CH₂CH₃ | H |
| 571 | -CH₂CH₃ | H | H | H | -(CH₂)₂CO₂CH₃ | H |

Preferred dyes according to the present invention are also those as described above having formula (I-i) 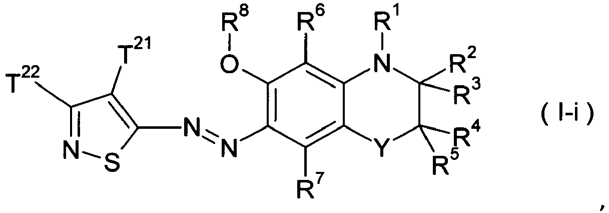 wherein
T²¹ is cyano, nitro or (C₁-C₆)-alkoxycarbonyl,
T²² is (C₁-C₆)-alkyl, hydrogen, halogen or aryl,
R¹ is methyl, ethyl, propyl, isopropyl, *n*-butyl, isobutyl, benzyl, 2-methoxylethyl, 2-ethoxylethyl, 2-acetoxyethyl, 2-(propionyl)oxyethyl, 2-(benzoyl)oxyethyl, cyanomethyl, 2-cyanoethyl, 3-cyanopropyl, 3-(C₁-C₆)alkoxyl-3-oxopropyl, 3-[O-interrupted (C₂-C₆)-alkoxyl]-3-oxopropyl, 2-(C₁-C₆)alkyl-2-oxoethyl, 2-[O-interrupted (C₂-C₆)-alkoxyl]-2-oxoethyl, 2-(cyanomethoxy)-2-oxoethyl, 3-(cyanomethoxy)-3-oxopropyl, 2-[(arylacyl)methoxy] -2-oxoethyl, 3 -[(arylacyl)methoxy] -3 -oxopropyl, 3 -(arylmethoxy)-3-oxopropyl, 3-{ [(C₁-C₆)alkylacyl]-methoxyl}-3-oxopropyl, 3-[(arylacyl)-methoxy]-3-oxopropyl, 3-{[(C₁-C₆)-alkoxycabonyl]methoxy}-3-oxopropyl, 2-{[(C₁-C₆)-alkoxycabonyl]methoxy}-2-oxoethyl or 2-{[(C₁-C₆)alkylacyl]-methoxyl}-2-oxoethyl,
R² to R⁷ independent from each other is hydrogen, (C₁-C₆)alkyl, aryl, (C₁-C₈)alkoxyl, (C₃-C₈)cycloalkyl, O-interrupted (C₂-C₆)alkyl, S-interrupted (C₂-C₆)alkyl or halogen,
R⁸ is hydrogen, (C₁-C₆)alkyl, (C₃-C₄)-alkenyl, (C₃-C₄)-alkynyl, arylmethyl, 2-(C₁-C₆)alkoxyl-2-oxoethyl, 2-(arylmethyl)-2-oxoethyl, cyano(C₁-C₆)alkyl, O-interrupted (C₁-C₆)alkyl or S-interrupted (C₁-C₆)alkyl
   and
Y is carbonyl.

Examples of preferred dyes of the formula (I-i) are the compounds illustrated in Table 9 and mixtures thereof.

| **Table 9** | | | | | |
|---|---|---|---|---|---|
| 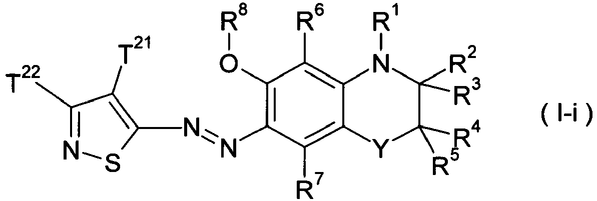 | | | | | |
| R³ to R⁷ is hydrogen, Y is carbonyl | | | | | |

| Example | T²¹ | T²² | R² | R¹ | R⁸ |
|---|---|---|---|---|---|
| 572 | CN | -C₆H₅ | H | -CH₂CH₃ | H |
| 573 | CN | -C₆H₅ | H | -(CH₂)₂CO₂CH₃ | H |
| 574 | CN | -C₆H₅ | H | -(CH₂)₂CN | H |
| 575 | CN | Cl | H | -CH₂CH₃ | H |
| 576 | CN | Cl | H | -(CH₂)₂CO₂CH₂CH₃ | H |
| 577 | CN | Cl | H | -(CH₂)₂CN | H |
| 578 | CN | Cl | H | -(CH₂)₂CN | -CH₃ |

Another preferred embodiment of the present invention are dyes as described above having formula (I-j) 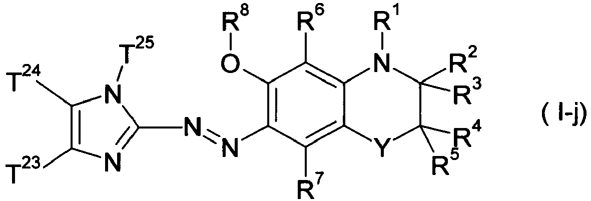 wherein
T²³ and T²⁴ independent from each other is cyano, nitro, halogen, aryl or trifluoromethyl,
T²⁵ is hydrogen, (C₁-C₆)-alkyl, O-interrupted (C₁-C₆)-alkyl, S-interrupted (C₁-C₆)-alkyl or aryl,
R¹ is methyl, ethyl, propyl, isopropyl, *n*-butyl, isobutyl, benzyl, 2-methoxylethyl, 2-ethoxylethyl, 2-acetoxyethyl, 2-(propionyl)oxyethyl, 2-(benzoyl)oxyethyl, cyanomethyl, 2-cyanoethyl, 3-cyanopropyl, 3-(C₁-C₆)alkoxyl-3-oxopropyl, 3-[O-interrupted (C₂-C₆)-alkoxyl]-3-oxopropyl, 2-(C₁-C₆)alkyl-2-oxoethyl, 2-[O-interrupted(C₂-C₆)-alkoxyl]-2-oxoethyl, 2-(cyanomethoxy)-2-oxoethyl, 3-(cyanomethoxy)-3-oxopropyl, 2-[(arylacyl)methoxy]-2-oxoethyl, 3-[(arylacyl)methoxy]-3-oxopropyl, 3-(arylmethoxy)-3-oxopropyl, 3-{[(C₁-C₆)alkylacyl]-methoxyl}-3-oxopropyl, 3-[(arylacyl)-methoxy]-3-oxopropyl, 3-{[(C₁-C₆)-alkoxycabonyl]methoxy}-3-oxopropyl, 2-{[(C₁-C₆)-alkoxycabonyl]methoxy}-2-oxoethyl or 2-{[(C₁-C₆)alkylacyl]-methoxyl}-2-oxoethyl,
Each of R² to R⁷, independent from each other, is hydrogen, (C₁-C₆)alkyl, aryl, (C₁-C₈)alkoxyl, (C₃-C₈)cycloalkyl, O-interrupted (C₂-C₆)alkyl, S-interrupted (C₂-C₆)alkyl or halogen,
R⁸ is hydrogen, (C₁-C₆)alkyl, (C₃-C₄)-alkenyl, (C₃-C₄)-alkynyl, arylmethyl, 2-(C₁-C₆)alkoxyl-2-oxoethyl, 2-(arylmethyl)-2-oxoethyl, cyano(C₁-C₆)alkyl, O-interrupted (C₁-C₆)alkyl or S-interrupted (C₁-C₆)alkyl
   and
Y is carbonyl.

Examples of preferred dyes of the formula (I-j) are the compounds illustrated in Table 10, and mixtures thereof

| **Table 10** | | | | | | |
|---|---|---|---|---|---|---|
| 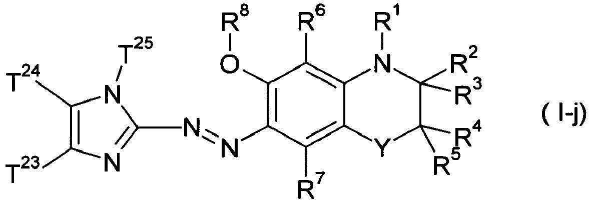 | | | | | | |

| R³ to R⁷ is hydrogen, Y is carbonyl | | | | | | |
|---|---|---|---|---|---|---|
| Example | T²³ | T²⁴ | T²⁵ | R² | R¹ | R⁸ |
| 579 | CN | CN | -CH₂CN | H | -(CH₂)₂CO₂H | H |
| 580 | CN | CN | -CH₂CN | H | -(CH₂)₂CO₂CH₃ | H |
| 581 | CN | CN | -CH₂CN | H | -(CH₂)₂CO₂CH₂CH₃ | H |
| 582 | CN | CN | -CH₂CN | H | -(CH₂)₂CO₂(CH₂)₂CH₃ | H |
| 583 | CN | CN | -CH₂CN | H | -(CH₂)₂CO₂CH(CH₃)₂ | H |
| 584 | CN | CN | -CH₂CN | H | -(CH₂)₂CO₂(CH₂)₃CH₃ | H |
| 585 | CN | CN | -CH₂CN | H | -(CH₂)₂CO₂CH(CH₃)CH₂CH₃ | H |
| 586 | CN | CN | -CH₂CN | H | -(CH₂)₂CO₂CH₂C₆H₅ | H |
| 587 | CN | CN | -CH₂CN | H | -(CH₂)₂CO₂(CH₂)₂OCH₃ | H |
| 588 | CN | CN | -CH₂CN | H | -(CH₂)₂CO₂(CH₂)₂OCH₂CH₃ | H |
| 589 | CN | CN | -CH₂CN | H | -(CH₂)₂CO₂(CH₂)₃OCH₃ | H |
| 590 | CN | CN | -CH₂CN | H | -(CH₂)₂CO₂(CH₂)₃OCH₂CH₃ | H |
| 591 | CN | CN | -CH₂CN | H | -(CH₂)₂CO₂CH₂CH₃ | CH₃ |

Another group of preferred dyes are dyes as described above having formula (I-k) 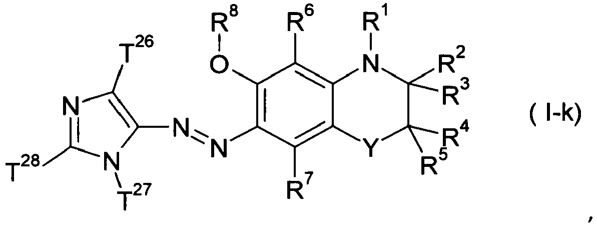 wherein
T²⁶ is cyano, nitro, aryl or trifluoromethyl,
T²⁷ is hydrogen, aryl or (C₁-C₆)-alkyl,
T²⁸ is hydrogen, (C₁-C₆)-acyloxy substituted (C₁-C₆)alkyl, cyano(C₁-C₆)alkyl, aryl, O-interrupted (C₁-C₆)-alkyl or S-interrupted (C₁-C₆)-alkyl,
R¹ is methyl, ethyl, propyl, isopropyl, *n*-butyl, isobutyl, benzyl, 2-methoxylethyl, 2-ethoxylethyl, 2-acetoxyethyl, 2-(propionyl)oxyethyl, 2-(benzoyl)oxyethyl, cyanomethyl, 2-cyanoethyl, 3-cyanopropyl, 3-(C₁-C₆)alkoxyl-3-oxopropyl, 3-[O-interrupted (C₂-C₆)-alkoxyl]-3-oxopropyl, 2-(C₁-C₆)alkyl-2-oxoethyl, 2-[O-interrupted (C₂-C₆)-alkoxyl]-2-oxoethyl, 2-(cyanomethoxy)-2-oxoethyl, 3 -(cyanomethoxy)-3-oxopropyl, 2- [(arylacyl)methoxy] -2-oxoethyl, 3-[(arylacyl)methoxy]-3-oxopropyl, 3-(arylmethoxy)-3-oxopropyl, 3-{[(C₁-C₆)alkylacyl]-methoxyl}-3-oxopropyl, 3-[(arylacyl)-methoxy]-3-oxopropyl, 3-{[(C₁-C₆)-alkoxycabonyl]methoxy}-3-oxopropyl, 2-{[(C₁-C₆)-alkoxycabonyl]methoxy}-2-oxoethyl or 2-{[(C₁-C₆)alkylacyl]-methoxyl}-2-oxoethyl,
R² to R⁷ independent from each other is hydrogen, (C₁-C₆)alkyl, aryl, (C₁-C₈)alkoxyl, (C₃-C₈)cycloalkyl, O-interrupted (C₂-C₆)alkyl, S-interrupted (C₂-C₆)alkyl or halogen,
R⁸ is hydrogen, (C₁-C₆)alkyl, (C₃-C₄)-alkenyl, (C₃-C₄)-alkynyl, arylmethyl, 2-(C₁-C₆)alkoxyl-2-oxoethyl, 2-(arylmethyl)-2-oxoethyl, cyano(C₁-C₆)alkyl, O-interrupted (C₁-C₆)alkyl or S-interrupted (C₁-C₆)alkyl
   and
Y is carbonyl.

Examples of preferred dyes of the formula (I-k) are the compounds illustrated in Table 11 and mixtures thereof.

| **Table 11** | | | | | | |
|---|---|---|---|---|---|---|
| 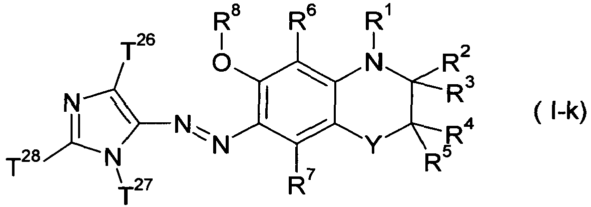 | | | | | | |

| R³ to R⁷ is hydrogen, Y is carbonyl | | | | | | |
|---|---|---|---|---|---|---|
| Example | T²⁶ | T²⁷ | T²⁸ | R² | R¹ | R⁸ |
| 592 | NO₂ | CH₃ | H | H | -(CH₂)₂CO₂H | H |
| 593 | NO₂ | CH₃ | H | H | -(CH₂)₂CO₂CH₃ | H |
| 594 | NO₂ | CH₃ | H | H | -(CH₂)₂CO₂CH₂CH₃ | H |
| 595 | NO₂ | CH₃ | H | H | -(CH₂)₂CO₂(CH₂)₂CH₃ | H |
| 596 | NO₂ | CH₃ | H | H | -(CH₂)₂CO₂CH(CH₃)₂ | H |
| 597 | NO₂ | CH₃ | H | H | -(CH₂)₂CO₂(CH₂)₃CH₃ | H |
| 598 | NO₂ | CH₃ | H | H | -(CH₂)₂CO₂CH(CH₃)CH₂CH₃ | H |
| 599 | NO₂ | CH₃ | H | H | -(CH₂)₂CO₂CH₂C₆H₅ | H |
| 600 | NO₂ | CH₃ | H | H | -(CH₂)₂CO₂(CH₂)₂OCH₃ | H |
| 601 | NO₂ | CH₃ | H | H | -(CH₂)₂CO₂(CH₂)₂OCH₂CH₃ | H |
| 602 | NO₂ | CH₃ | H | H | -(CH₂)₂CO₂(CH₂)₃OCH₃ | H |
| 603 | NO₂ | CH₃ | H | H | -(CH₂)₂CO₂(CH₂)₃OCH₂CH₃ | H |
| 604 | NO₂ | CH₃ | H | H | -(CH₂)₂CO₂CH₂CH₃ | CH₃ |

Preferred are dyes as described above having formula (I-l) 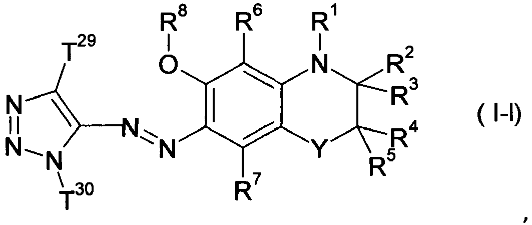 wherein
T²⁹ is cyano, nitro, aryl or trifluoromethyl,
T³⁰ is hydrogen, (C₁-C₆)-alkyl, cyano(C₁-C₆)alkyl, O-interrupted (C₁-C₆)alkyl, S-interrupted (C₁-C₆)alkyl, aryl, benzyl, (C₁-C₆)-acyloxy substituted (C₁-C₆)alkyl or cyano(C₁-C₆)alkyl,
R¹ is methyl, ethyl, propyl, isopropyl, *n*-butyl, isobutyl, benzyl, 2-methoxylethyl, 2-ethoxylethyl, 2-acetoxyethyl, 2-(propionyl)oxyethyl, 2-(benzoyl)oxyethyl, cyanomethyl, 2-cyanoethyl, 3-cyanopropyl, 3-(C₁-C₆)alkoxyl-3-oxopropyl, 3-[O-interrupted (C₂-C₆)-alkoxyl]-3-oxopropyl, 2-(C₁-C₆)alkyl-2-oxoethyl, 2-[O-interrupted (C₂-C₆)-alkoxyl]-2-oxoethyl, 2-(cyanomethoxy)-2-oxoethyl, 3-(cyanomethoxy)-3 -oxopropyl, 2-[(arylacyl)methoxy] -2-oxoethyl, 3-[(arylacyl)methoxy]-3-oxopropyl, 3-(arylmethoxy)-3-oxopropyl, 3-{[(C₁-C₆)alkylacyl]-methoxyl}-3 -oxopropyl, 3-[(arylacyl)-methoxy]-3-oxopropyl, 3-{[(C₁-C₆)-alkoxycabonyl]methoxy}-3-oxopropyl, 2-{[(C₁-C₆)-alkoxycabonyl]methoxy}-2-oxoethyl or 2-{[(C₁-C₆)alkylacyl]-methoxyl}-2-oxoethyl,
R² to R⁷ independent from each other is hydrogen, (C₁-C₆)alkyl, aryl, (C₁-C₈)alkoxyl, (C₃-C₈)cycloalkyl, O-interrupted (C₂-C₆)alkyl, S-interrupted (C₂-C₆)alkyl or halogen,
R⁸ is hydrogen, (C₁-C₆)alkyl, (C₃-C₄)-alkenyl, (C₃-C₄)-alkynyl, arylmethyl, 2-(C₁-C₆)alkoxyl-2-oxoethyl, 2-(arylmethyl)-2-oxoethyl, cyano(C₁-C₆)alkyl, O-interrupted (C₁-C₆)alkyl or S-interrupted (C₁-C₆)alkyl
   and
Y is carbonyl.

Examples of preferred dyes of the formula (1-1) are the compounds illustrated in Table 12 and mixtures thereof.

| **Table 12** | | | | | |
|---|---|---|---|---|---|
| 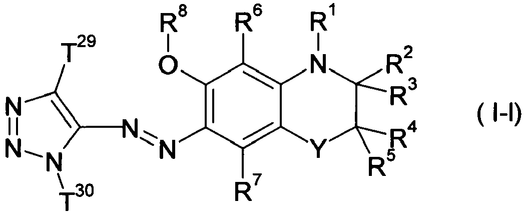 | | | | | |

| R³ to R⁷ is hydrogen, Y is carbonyl | | | | | |
|---|---|---|---|---|---|
| Example | T²⁹ | T³⁰ | R² | R¹ | R⁸ |
| 605 | CN | -CH₂C₆H₅ | H | -(CH₂)₂CO₂CH₂CH₃ | H |
| 606 | CN | -CH₂C₆H₅ | H | -(CH₂)₂CO₂CH₃ | H |
| 607 | CN | -CH₂C₆H₅ | H | -(CH₂)₂CO₂CH₃ | H |
| 608 | CN | -CH₂C₆H₅ | H | -(CH₂)₂CO₂CH₂CH₃ | H |
| 609 | CN | -CH₂C₆H₅ | H | -(CH₂)₂CO₂CH₂CH₃ | -CH₃ |

Preferred are also dyes as described above having formula (I-m) 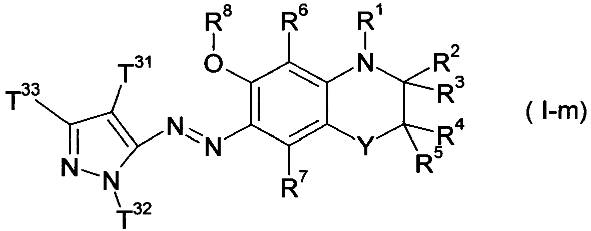 wherein
T³¹ is cyano, nitro, aryl or trifluoromethyl,
T³² is hydrogen, (C₁-C₆)-alkyl, O-interrupted (C₁-C₆)-alkyl, S-interrupted (C₁-C₆)-alkyl, cyano(C₁-C₆)alkyl, arylmethyl, cyano(C₁-C₆)alky or (C₁-C₆)-acyloxy substituted (C₁-C₆)alkyl, T³³ is hydrogen or (C₁-C₆)alkyl,
R¹ is methyl, ethyl, propyl, isopropyl, *n*-butyl, isobutyl, benzyl, 2-methoxylethyl, 2-ethoxylethyl, 2-acetoxyethyl, 2-(propionyl)oxyethyl, 2-(benzoyl)oxyethyl, cyanomethyl, 2-cyanoethyl, 3-cyanopropyl, 3-(C₁-C₆)alkoxyl-3-oxopropyl, 3-[O-interrupted (C₂-C₆)-alkoxyl]-3-oxopropyl, 2-(C₁-C₆)alkyl-2-oxoethyl, 2-[O-interrupted (C₂-C₆)-alkoxyl]-2-oxoethyl, 2-(cyanomethoxy)-2-oxoethyl, 3-(cyanomethoxy)-3-oxopropyl, 2-[(arylacyl)methoxy]-2-oxoethyl, 3-[(arylacyl)methoxy]-3-oxopropyl, 3-(arylmethoxy)-3-oxopropyl, 3-{[(C₁-C₆)alkylacyl]-methoxyl}-3-oxopropyl, 3-[(arylacyl)-methoxy]-3-oxopropyl, 3-{[(C₁-C₆)-alkoxycabonyl]methoxy}-3-oxopropyl, 2-{[(C₁-C₆)-alkoxycabonyl]methoxy}-2-oxoethyl or 2-{[(C₁-C₆)alkylacyl]-methoxyl}-2-oxoethyl,
R² to R⁷ independent from each other is hydrogen, (C₁-C₆)alkyl, aryl, (C₁-C₈)alkoxyl, (C₃-C8)cycloalkyl, O-interrupted (C₂-C₆)alkyl, S-interrupted (C₂-C₆)alkyl or halogen,
R⁸ is hydrogen, (C₁-C₆)alkyl, (C₃-C₄)-alkenyl, (C₃-C₄)-alkynyl, arylmethyl, 2-(C₁-C₆)alkoxyl-2-oxoethyl, 2-(arylmethyl)-2-oxoethyl, cyano(C₁-C₆)alkyl, O-interrupted (C₁-C₆)alkyl or S-interrupted (C₁-C₆)alkyl
   and
Y is carbonyl.

Examples of preferred dyes of the formula (I-m) are the compounds illustrated in Table 13 and mixtures thereof.

| **Table 13** | | | | | | |
|---|---|---|---|---|---|---|
| 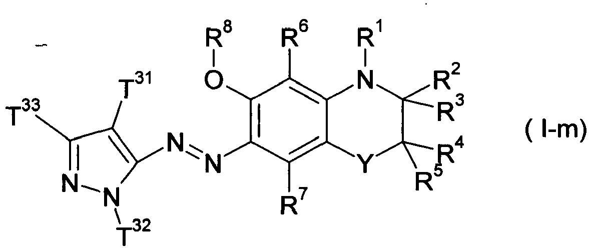 | | | | | | |

| R³ to R⁷ is hydrogen, Y is carbonyl | | | | | | |
|---|---|---|---|---|---|---|
| Example | T³¹ | T³² | T³³ | R² | R¹ | R⁸ |
| 610 | CN | H | H | H | -CH₂CH₃ | H |
| 611 | CN | H | CN | H | -(CH₂)₂CO₂CH₃ | H |
| 612 | CN | H | CN | H | -(CH₂)₂CN | H |
| 613 | CN | -C₆H₅ | CN | H | -CH₂CH₃ | H |
| 614 | CN | -C₆H₅ | CN | H | -(CH₂)₂CO₂CH₂CH₃ | H |
| 615 | CN | -C₆H₅ | CN | H | -(CH₂)₂CN | H |
| 616 | CN | -C₆H₅ | CN | H | -(CH₂)₂CN | -CH₃ |

Another preferred group of dyes are those as described above having formula (I-n) 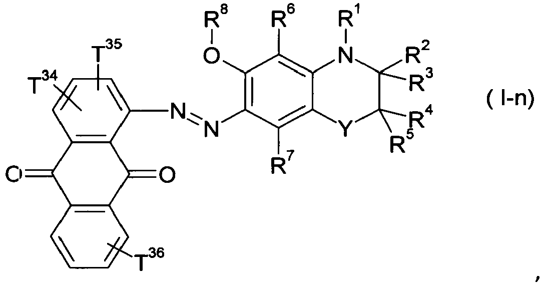 wherein
T³⁴, T³⁵ and T³⁶ independent from each other is hydrogen, halogen, nitro, cyano, hydroxyl, (C₁-C₆)-alkoxylcarbonyl, (C₁-C₆)-alkyl or acyl,
R¹ is methyl, ethyl, propyl, isopropyl, *n*-butyl, isobutyl, benzyl, 2-methoxylethyl, 2-ethoxylethyl, 2-acetoxyethyl, 2-(propionyl)oxyethyl, 2-(benzoyl)oxyethyl, cyanomethyl, 2-cyanoethyl, 3-cyanopropyl, 3-(C₁-C₆)alkoxyl-3-oxopropyl, 3-[O-interrupted (C₂-C₆)-alkoxyl]-3-oxopropyl, 2-(C₁-C₆)alkyl-2-oxoethyl, 2-[O-interrupted (C₂-C₆)-alkoxyl]-2-oxoethyl, 2-(cyanomethoxy)-2-oxoethyl, 3-(cyanomethoxy)-3-oxopropyl, 2-[(arylacyl)methoxy] -2-oxoethyl, 3-[(arylacyl)methoxy] -3 -oxopropyl, 3 -(arylmethoxy)-3-oxopropyl, 3-{[(C₁-C₆)alkylacyl]-methoxyl}-3-oxopropyl, 3-[(arylacyl)-methoxy]-3-oxopropyl, 3-{[(C₁-C₆)-alkoxycabonyl]methoxy}-3-oxopropyl, 2-{[(C₁-C₆)-alkoxycabonyl]methoxy}-2-oxoethyl or 2-{[(C₁-C₆)alkylacyl]-methoxyl}-2-oxoethyl,
R² to R⁷ independent from each other is hydrogen, (C₁-C₆)alkyl, aryl, (C₁-C₈)alkoxyl, (C₃-C₈)cycloalkyl, O-interrupted (C₂-C₆)alkyl, S-interrupted (C₂-C₆)alkyl or halogen,
R⁸ is hydrogen, (C₁-C₆)alkyl, (C₃-C₄)-alkenyl, (C₃-C₄)-alkynyl, arylmethyl, 2-(C₁-C₆)alkoxyl-2-oxoethyl, 2-(arylmethyl)-2-oxoethyl, cyano(C₁-C₆)alkyl, O-interrupted (C₁-C₆)alkyl or S-interrupted (C₁-C₆)alkyl
   and
Y is carbonyl.

Examples of preferred dyes of the formula (I-n) are the compounds illustrated in Table 14, and mixtures thereof.

**Table 14**

| Example | Structure |
|---|---|
| 617 | 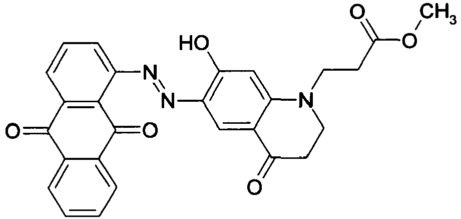 |
| 618 | 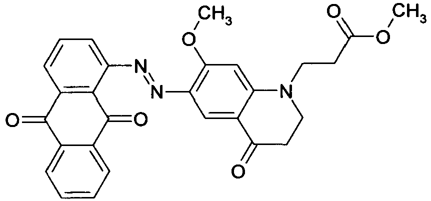 |
| 619 | 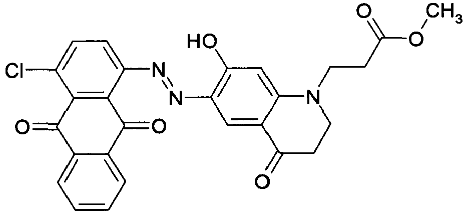 |
| 620 | 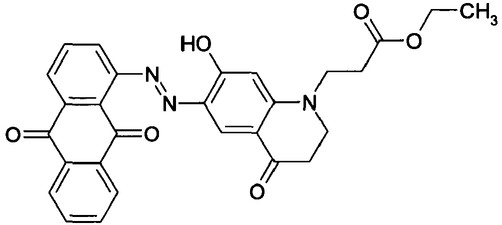 |

Just another preferred group of dyes are the dyes as described above having formula (I-o) 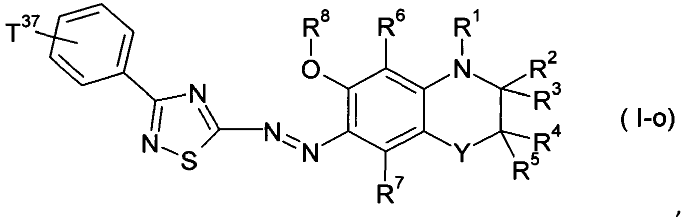 wherein
T³⁷ is nitro, hydrogen or halogen,
R¹ is methyl, ethyl, propyl, isopropyl, *n*-butyl, isobutyl, benzyl, 2-methoxylethyl, 2-ethoxylethyl, 2-acetoxyethyl, 2-(propionyl)oxyethyl, 2-(benzoyl)oxyethyl, cyanomethyl, 2-cyanoethyl, 3-cyanopropyl, 3-(C₁-C₆)alkoxyl-3-oxopropyl, 3-[O-interrupted (C₂-C₆)-alkoxyl]-3-oxopropyl, 2-(C₁-C₆)alkyl-2-oxoethyl, 2-[O-interrupted (C₂-C₆)-alkoxyl]-2-oxoethyl, 2-(cyanomethoxy)-2-oxoethyl, 3-(cyanomethoxy)-3-oxopropyl, 2-[(arylacyl)methoxy]-2-oxoethyl, 3-[(arylacyl)methoxy]-3-oxopropyl, 3-(arylmethoxy)-3-oxopropyl, 3-{[(C₁-C₆)alkylacyl]-methoxyl}-3-oxopropyl, 3-[(arylacyl)-methoxy]-3-oxopropyl, 3-{[(C₁-C₆)-alkoxycabonyl]methoxy}-3-oxopropyl, 2-{[(C₁-C₆)-alkoxycabonyl]methoxy}-2-oxoethyl or 2-{[(C₁-C₆)alkylacyl]-methoxyl}-2-oxoethyl,
R² to R⁷ independent from each other is hydrogen, (C₁-C₆)alkyl, aryl, (C₁-C₈)alkoxyl, (C₃-C₈)cycloalkyl, O-interrupted (C₂-C₆)alkyl, S-interrupted (C₂-C₆)alkyl or halogen,
R⁸ is hydrogen, (C₁-C₆)alkyl, (C₃-C₄)-alkenyl, (C₃-C₄)-alkynyl, arylmethyl, 2-(C₁-C₆)alkoxyl-2-oxoethyl, 2-(arylmethyl)-2-oxoethyl, cyano(C₁-C₆)alkyl, O-interrupted (C₁-C₆)alkyl or S-interrupted (C₁-C₆)alkyl
   and
Y is carbonyl.

Examples of preferred dyes of the formula (I-o) are the compounds illustrated in Table 15, and mixtures thereof

**Table 15**

| Example | Structure |
|---|---|
| 621 | 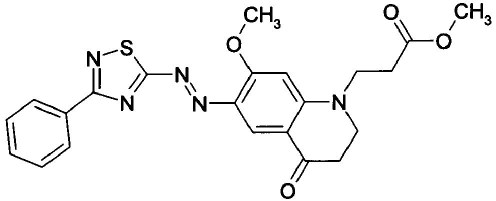 |
| 622 | 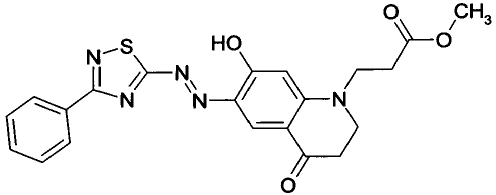 |
| 623 | 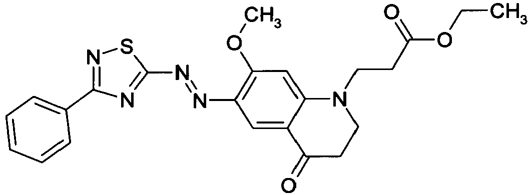 |
| 624 | 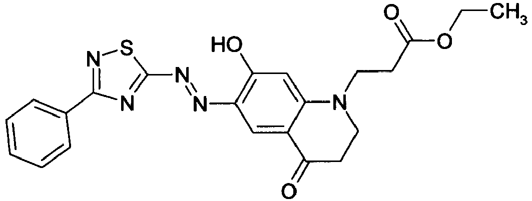 |

The present invention also provides a process for the production of dyes having formula (I) and mixtures thereof, comprising
a) diazotization of a compound of the general formula (XD)

   D-NH₂ (XD),

   wherein
   D is as defined above, and
b) reacting the obtained corresponding diazonium salt with a compound of formula (XC) 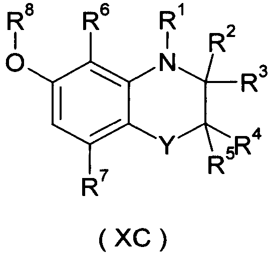 wherein R¹ to R⁸ and Y are each as defined above.

The diazotization of the compounds of the general formula (XD) is generally effected in a known manner, for example using sodium nitrite in an aqueous medium rendered acidic, for example with hydrochloric or sulfuric acid, or using nitrosylsulfuric acid in dilute sulfuric acid, phosphoric acid or in a mixture of acetic acid and propionic acid. The preferred temperature range is between 0 °C and 15 °C.

The coupling of the diazotized compounds onto the compounds of the general formula (XD) is generally likewise effected in a known manner, for example in an acidic, aqueous, aqueous-organic or organic medium, particularly advantageously at temperatures below 10 °C. Acids used are in particular sulfuric acid, acetic acid or propionic acid.

The compounds of the general formulae (XC) and (XD) are known and can be prepared by known methods (e.g. EP 1 154 774, WO 2009/036351, J. Chem. Soc., 1925, 127, 2303, Khim. Geterotsil., 1996, 32(4), 523).

The present invention's dyes of the general formula (I) are very useful for dyeing and printing hydrophobic materials, the dyeings and prints obtained being notable for level hues and high service fastnesses. Deserving of particular mention are excellent wash fastnesses and very good sublimation fastnesses.

The present invention thus also provides for the use of the dyes of the general formula I for dyeing and printing hydrophobic materials, i.e., processes for dyeing or printing such materials in a conventional manner wherein one or more dyes of the general formula (I) according to the present invention are used as a colorant.

The hydrophobic materials mentioned may be of synthetic or semisynthetic origin. Useful hydrophobic materials include for example secondary cellulose acetate, cellulose triacetate, polyamides and, in particular, high molecular weight polyesters. Materials of high molecular weight polyester are in particular those based on polyethylene glycol terephthalates.

The hydrophobic synthetic materials can be present in the form of sheet or threadlike constructions and can have been processed, for example, into yams or into woven or knit textile materials. Preference is given to fibrous textile materials, which may also be present in the form of micro fibers for example.

The dyeing in accordance with the use provided by the present invention can be carried out in a conventional manner, preferably from an aqueous dispersion, if appropriate in the presence of carriers, at between 80 to about 110 °C by the exhaust process or by the HT process in a dyeing autoclave at 110 to 140° C, but also by the so-called thermo-fix process, in which the fabric is padded with the dyeing liquor and subsequently fixed/set at about 180 to 230 °C.

Organic and inorganic acids such as acetic acid, succinic acid, boric acid or phosphoric acid are included to set a pH in the range from 4 to 5, preferably 4.5. It is advantageous to buffer the pH setting and to add a sufficient amount of a buffering system. The acetic acid/sodium acetate system is an example of an advantageous buffering system.

Furthermore, the dyes and dye mixtures according to the invention are also well suited to dyeing hydrophobic fiber materials from supercritical carbon dioxide.

To use the dye or dye mixture in textile printing, the requisite amounts of the abovementioned dye formulations are kneaded in a conventional manner together with thickeners, for example alkali metal alginates or the like, and if appropriate further additives, for example fixation accelerants, wetting agents and oxidizing agents, to give print pastes.

The present invention also provides inks for digital textile printing by the inkjet process, comprising a present invention dye of the general formula (I).

The inks of the present invention are preferably aqueous and comprise one or more of the present invention's dyes of the general formula (I), for example in amounts of 0.1% to 50% by weight, preferably in amounts of 0.5% to 30% by weight and more preferably in amounts of 1% to 15% by weight based on the total weight of the ink. They further comprise in particular from 0.1% to 20% by weight of a dispersant. Suitable dispersants are known to one skilled in the art, are commercially available and include for example sulfonated or sulfomethylated lignins, condensation products of aromatic sulfonic acids and formaldehyde, condensation products of substituted or unsubstituted phenol and formaldehyde, polyacrylates and corresponding copolymers, modified polyurethanes and reaction products of alkylene oxides with alkylatable compounds, for example fatty alcohols, fatty amines, fatty acids, carboxamides and substituted or unsubstituted phenols.

The inks of the present invention may further comprise customary additives, for example viscosity moderators to set viscosities in the range from 1.5 to 40.0 mPas in the temperature range of 20 to 50° C. Preferred inks have a viscosity in the range from 1.5 to 20 mPas and particularly preferred inks have a viscosity in the range from 1.5 to 15 mPas.

Useful viscosity moderators include rheological additives, for example polyvinyl-caprolactam, polyvinylpyrrolidone and also their copolymers, polyetherpolyol, associative thickeners, polyureas, sodium alginates, modified galactomannans, polyetherurea, polyurethane and nonionic cellulose ethers.

By way of further additives, the inks of the present invention may include surface-active substances to set surface tensions in the range from 20 to 65 mN/m, which are if appropriate adapted depending on the process used (thermal or piezo technology). Useful surface-active substances include for example surfactants of any kind, preferably nonionic surfactants, butyldiglycol and 1,2 hexanediol.

The inks may further include customary additives, for example chemical species to inhibit fungal and bacterial growth in amounts from 0.01% to 1% by weight based on the total weight of the ink.

The inks of the present invention can be prepared in conventional manner by mixing the components in water.

The examples below serve to illustrate the invention. Parts and percentages are by weight unless noted otherwise. The relationship between parts by weight and parts by volume is that of the kilogram to the liter.

### Synthesis Example 1:

### Preparation of substance 38

5.24 g of 6-bromo-2,4-dinitroaniline were introduced into a mixture of 9.8 ml of sulfuric acid (96 %ic), 0.5 ml of water and 3.5 ml of nitrosylsulfuric acid (40 %ic) at 30 to 35°C. After 3 hours of stirring at 30 to 35 °C, excess nitrite was destroyed with amidosulfonic acid. The diazonium salt solution thus obtained was expediently added dropwise to a mixture of 4.9 g of 2,3-dihydro-7-hydroxyquinolin-4-keto-1-propionyl methyl ester, 50 ml of methanol and 200 g of ice. After stirring for one hour the solids were filtered off with suction, washed with water and dried to leave 7.9 g of the dye example 38.

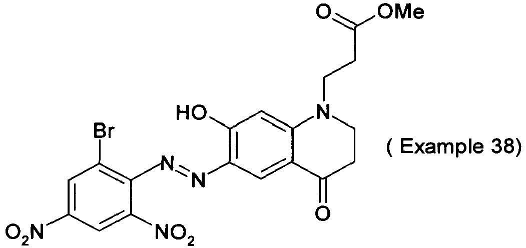

### Synthesis Example 2:

### Preparation of substance 450

8.16 g of 5-amino-3-methyl-thiophene-2,4-dicarbonitrile were introduced into a mixture of 40 mL of acetic acid, 10 ml of Propionic acid and 9.01 ml of nitrosylsulfuric acid (40%) at 0 to 5°C. After 3 hours of stirring at 0 to 5 °C, excess nitrite was destroyed with amidosulfonic acid. The diazonium salt solution thus obtained was expediently added dropwise to a mixture of 12.46 g of 2,3-dihydro-7-hydroxyquinolin-4-keto-1-propionyl methyl ester, 100 ml of methanol and 300 g of ice. After stirring for one hour the solids were filtered off with suction, washed with water and dried to leave 13.0 g of the dye example 450.

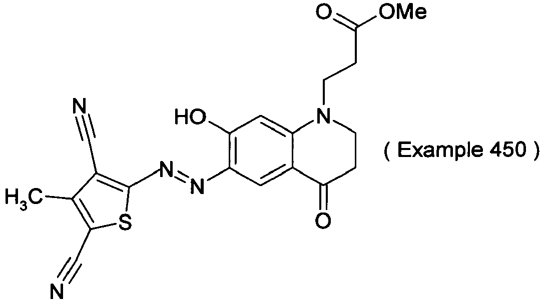

### Synthesis Example 3:

### Preparation of substance 342

8.69 g of 2,4-dinitroaniline were introduced into a mixture of 20 ml of sulfuric acid (96 %ic), 1.2 ml of water and 8.55 ml of nitrosylsulfuric acid (40%) at 30 to 35 °C. After 3 hours of stirring at 30 to 35°C, excess nitrite was destroyed with amidosulfonic acid. The diazonium salt solution thus obtained was expediently added dropwise to a mixture of 13.2 g of 2,3-dihydro-7-methoxyquinolin-4-keto-1-propionyl methyl ester, 100 ml of methanol and 300 g of ice. After stirring for one hour the solids were filtered off with suction, washed with water and dried to leave 16.3 g of the Dye example 342.

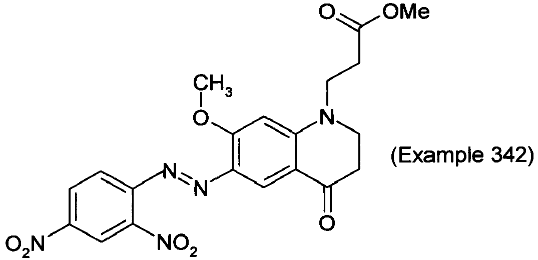

Through analogy, all the dyes of the present invention can be synthesized according to the methods described above.

### Application Example 1:

3 g of the dye example 38 were ground in a glass beads mill together with 3 g dispersing reagent and 94 g water, which was converted into 3 % aqueous dispersion. Using that formulation, a 1% dyeing (based on dye and substrate) is produced on polyester fabric by high temperature exhaust process at 130 °C and was reduction cleared. The rubine dyeing so obtained has very good in-use fastness properties, especially excellent fastness to washing.

### Application Example 2:

3 g of the dye example 450 were ground in a glass beads mill together with 3 g dispersing reagent and 94 g water, which was converted into 3 % aqueous dispersion. Using that formulation, a 1% dyeing (based on dye and substrate) was produced on polyester fabric by high temperature exhaust process at 130 °C and was reduction cleared. The bright rubine dyeing so obtained has very good in-use fastness properties, especially excellent fastness to washing.

### Application Example 3:

A textile fabric consisting of polyester was padded with liquor consisting of 50 g/l of 8 % sodium alginate solution, 100 g/l 8 to 12 % carob flour ether solution and 5 g/l of monosodium phosphate in water and then dried. The wet pickup was 70 %. The textile thus pretreated was then printed with an aqueous ink prepared in accordance with the procedure described above and containing 3.5 % of the dye example 38,
2.5% of Disperbyk® 190 dispersant,
30% of 1,5-pentanediol,
5% of the diethylene glycol monomethyl ether,
0.01% of Mergal® K9N biocide and
58.99% of water
using a drop-on-demand (piezo) in jet print head. The print was fully dried. Fixing was effected by means of superheated steam at 175 °C for 7 minutes. The print was subsequently subjected to an alkaline reduction clear, rinsed warm and then dried.

### Application Example 4:

A textile fabric consisting of polyester was padded with liquor consisting of 50 g/l of 8 % sodium alginate solution, 100 g/l 8 to 12 % carob flour ether solution and 5 g/l of monosodium phosphate in water and then dried. The wet pickup was 70 %. The textile thus pretreated was then printed with an aqueous ink prepared in accordance with the procedure described above and containing 3.5 % of the dye example 450,
2.5% of Disperbyk® 190 dispersant,
30% of 1,5-pentanediol,
5% of the diethylene glycol monomethyl ether,
0.01% of Mergal® K9N biocide and
58.99% of water
using a drop-on-demand (piezo) in jet print head. The print was fully dried. Fixing was effected by means of superheated steam at 175 °C for 7 minutes. The print was subsequently subjected to an alkaline reduction clear, rinsed warm and then dried.

### Comparison Example 1:

### a)

The build-up on standard 100 % Polyester was measured for a dye of the present invention [Example 15] and for a commonly used dye [C.I.Disperse Red 369, formula (xc1)]. The results are compared and are as shown below:

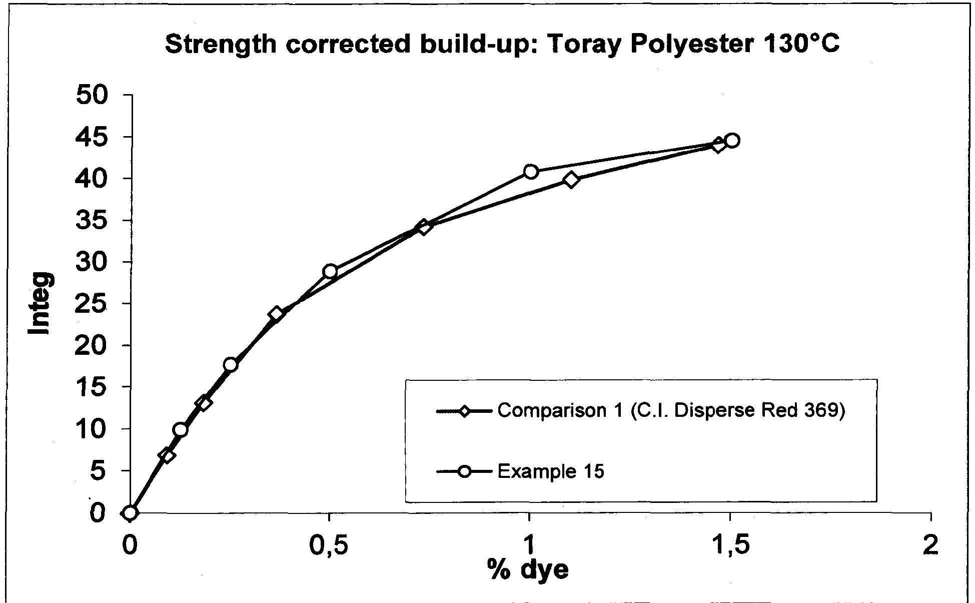

The dye according to Example 15 of the present invention has build-up on Toray polyester at 130 °C which is similar to the commercial product C.I. Disperse Red 369 (Comparison 1).

### b)

The wet fastness on standard 100 % polyester (exhaust dyed 130 °C, post-set 30" 180 °C) was measured for the same dye of the present invention [Example 15] as in a) and for a the same commonly used dye [C.I.Disperse Red 369, formula (xc1)]. The results are compared and are given in the table below:

| **Exhaust dyed 100% PES Toray** | AATCC TM61 2A 49°C | | ISO 105-C06 B2 | | Alk perspiration ISO 105 E04 | | Acid perspiration ISO 105 E04 | |
|---|---|---|---|---|---|---|---|---|
| | PES | PA | PES | PA | PES | PA | PES | PA |
| Comparison 1 (C.I.Disperse Red 369) | 4 | 3-4 | 4 | 4 | 3-4 | 3-4 | 4 | 3 |
| Example 15 | 5 | 5 | 5 | 5 | 5 | 5 | 5 | 4-5 |

From the data it is clear that the inventive dye of Example 15 has superior wet fastness properties compared to C.I. Disperse Red 369.

Thus it can be concluded that the inventive dyes have better wet fastness properties than the industry standard whilst they also maintain the same build-up properties.

### Comparision Example 2:

### a)

The build-up on 100 % polyester microfiber at 130 °C was measured for a dye of the present invention [Example 15] and for a commonly used dye [formula (xc2)]. The results are compared and are given below:

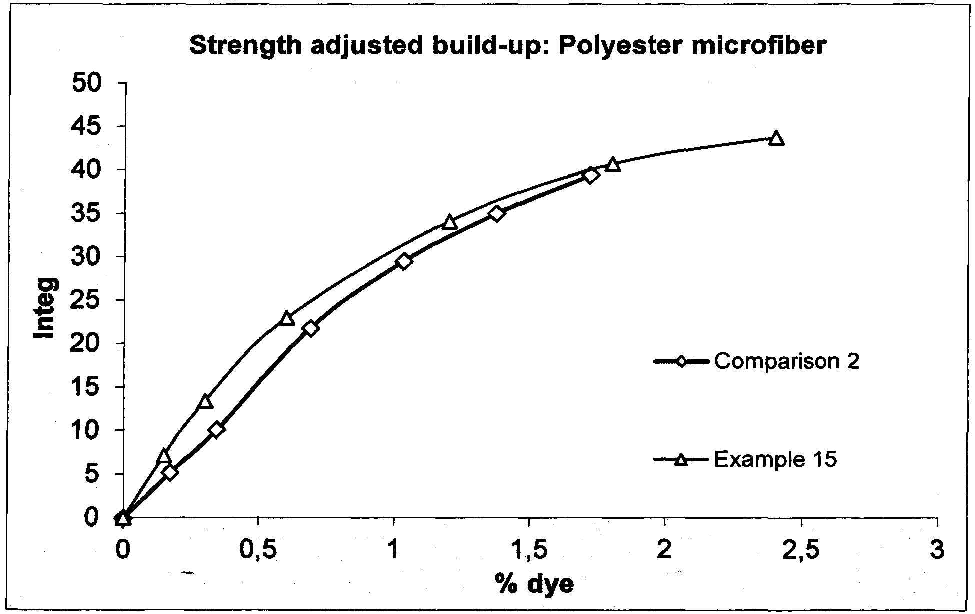

It can be seen that the dyes of the invention have a better build-up on polyester microfiber than the other industry standard for this colour.

### b)

The wet fastness on polyester/cotton (dyed by Pad-Dry-Thermofix method 220 °C) of the same dyes as in a), i.e. [Expl. 15] and [formula xc2] (comparison 2) was determined. Also the test was applied to the [C.I. Disperse Red 369] from Comparison Example 1 (comparison 3).

| **Pad-dry-thermofix 220°C** | Combi test | |
|---|---|---|
| **Polyester/cotton 65/35** | | |
| | PES | CTN |
| Comparison 2 | 4 | 5 |
| Comparison 3 (C.I. Disperse Red 356) | 2-3 | 5 |
| Example 15 | 4-5 | 5 |

The inventive dye of Example 15 has better fastness compared to the dyes Comparison 2 and Comparison 3 (C.I. Disperse Red 356) in the Combination fastness test for polyester/cotton workwear (dry heat ISO 105 P01 5' 190°C, followed by wash ISO 105-C05 4hrs 95°C). Other wet fastness properties are similar.

It can therefore be summarized that the dyes of the present invention show the same or even better build-up properties than the dyes now used and at the same time have improved fastness properties.

## Claims

1. Dyes of the formula (I) and mixtures thereof: 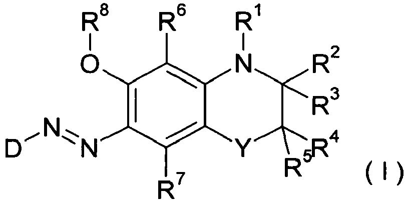 wherein
Y is a group of general formula (II) 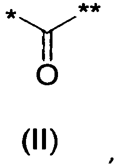
R¹ is alkyl, alkenyl, cycloalkyl, aryl, heteroaryl, heterocycloalkyl
or
alkyl interrupted by one or more heteroatoms selected from the group consisting of oxygen and sulphur
or
alkyl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N*-diaryl-amino, *N*-alkyl-*N*-aryl-amino, *N-*monocycloalkyl-amino, *N*,*N-*dicycloalkyl-amino, *N*-monoalkyl-monocycloalkyl-amino, *N,N-*monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxythiocarbonyl, acyloxy, aryloyloxy, carbamoyl, *N-*monocycloalkyl-carbamoyl, N-monoalkyl-carbamoyl, *N,N-*dicycloalkyl-carbamoyl, *N,N*-dialkyl-carbamoyl, *N*-monoaryl-carbamoyl, *N,N-*diaryl-carbamoyl, *N-*monocycloalkyl-*N*-monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N-*monocycloalkyl-*N*-monoarylsulfamoyl, *N-*monoalkyl-*N-*monoarylsulfamoyl, arylsulphonyloxy, alkylsulphonyloxy, aryloxysulfonyl, alkoxylsulfonyl and COOR¹² or alkyl interrupted by one or more heteroatoms selected from the group consisting of oxygen and sulphur and substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N*-diaryl-amino, *N*-alkyl-*N*-aryl-amino, *N-*monocycloalkyl-amino, *N,N*-dicycloalkyl-amino, *N*-monoalkyl-monocycloalkyl-amino, *N,N-*monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxythiocarbonyl, acyloxy, aryloyloxy, carbamoyl, *N-*monocycloalkyl-carbamoyl, *N*-monoalkyl-carbamoyl, *N,N*-dicycloalkyl-carbamoyl, *N,N*-dialkyl-carbamoyl, *N*-monoaryl-carbamoyl, *N,N-*diaryl-carbamoyl, *N-*monocycloalkyl-*N*-monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N,N*-dicycloalkylsulfamoyl, *N,N*-dialkyl-sulfamoyl, *N*-monoaryl-sulfamoyl, *N,N-*diaryl-sulfamoyl, *N-*monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹², or
aryl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N*-diaryl-amino, *N*-alkyl-*N*-aryl-amino, *N-*monocycloalkyl-amino, *N,N*-dicycloalkyl-amino, *N*-monoalkyl-monocycloalkyl-amino, *N,N*-monoaryl-monocycloalkyl-amino, *N*-acylamino, *N*-alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, N-monoaryl-carbamoyl, *N,N-*diaryl-carbamoyl, *N-*monocycloalkyl-*N*-monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, N,N-dialkyl-sulfamoyl, *N-*monoaryl-sulfamoyl, *N,N*-diaryl-sulfamoyl, *N-*monocycloalkyl-*N*-monoarylsulfamoyl, *N-*monoalkyl-N-monoarylsulfamoyl, arylsulphonyloxy, alkylsulphonyloxy, aryloxysulfonyl, alkoxylsulfonyl and COOR¹²,
R² to R⁸ independent of each other is hydrogen, alkyl, aryl, alkoxyl, cycloalkyl, halogen
or
alkyl interrupted by one or more heteroatoms selected from the group consisting of oxygen and sulphur
or
alkyl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N*-diaryl-amino, *N*-alkyl-*N*-aryl-amino, *N-*monocycloalkyl-amino, *N,N-*dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N*,*N*-dicycloalkyl-carbamoyl, *N,N-*dialkyl-carbamoyl, *N*-monoaryl-carbamoyl, *N,N-*diaryl-carbamoyl, *N-*monocycloalkyl-*N*-monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N-*monocycloalkyl-*N*-monoarylsulfamoyl, *N-*monoalkyl-*N-*monoarylsulfamoyl, arylsulphonyloxy, alkylsulphonyloxy, aryloxysulfonyl, alkoxylsulfonyl and COOR¹², or
alkyl interrupted by one or more heteroatoms selected from the group consisting of oxygen and sulphur and substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N*-diaryl-amino, *N-*alkyl-*N-*aryl-amino, *N-*monocycloalkyl-amino, *N*,*N*-dicycloalkyl-amino, *N*-monoalkyl-monocycloalkyl-amino, *N,N-*monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, *N*-monoalkyl-carbamoyl, *N,N-*dicycloalkyl-carbamoyl, *N,N-*dialkyl-carbamoyl, *N*-monoaryl-carbamoyl, *N,N-*diaryl-carbamoyl, *N-*monocycloalkyl-*N*-monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N-*diaryl-sulfamoyl, *N-*monocycloalkyl-*N*-monoarylsulfamoyl, *N-*monoalkyl-*N-*monoarylsulfamoyl, arylsulphonyloxy, alkylsulphonyloxy, aryloxysulfonyl, alkoxylsulfonyl and COOR¹²
or
aryl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N*-diaryl-amino, *N*-alkyl-*N*-aryl-amino, *N-*monocycloalkyl-amino, *N*,*N-*dicycloalkyl-amino, *N*-monoalkyl-monocycloalkyl-amino, *N,N*-monoaryl-monocycloalkyl-amino, *N*-acylamino, *N-*alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, N-monoaryl-carbamoyl, *N,N*-diaryl-carbamoyl, *N-*monocycloalkyl-*N*-monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, N,N-dialkyl-sulfamoyl, *N*-monoaryl-sulfamoyl, *N,N*-diaryl-sulfamoyl, *N-*monocycloalkyl-*N*-monoarylsulfamoyl, *N-*monoalkyl-N-monoarylsulfamoyl, arylsulphonyloxy, alkylsulphonyloxy, aryloxysulfonyl, alkoxylsulfonyl and COOR¹²,
D represents a group of formula (VI) wherein 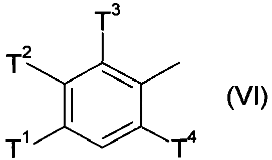 T¹ to T⁴ independent from each other is
hydrogen, alkyl, cycloalkyl, alkenyl, alkynyl, aryl, alkoxyl, halogen, cyano, nitro, acyl, aryloyl, arylsulfonyl, alkylsulfonyl, *N*-monoalkyl-sulfamoyl, *N,N*-dialkyl-sulfamoyl, alkoxylcarbonyl, aryloxycarbonyl, *N*-monoalkyl-carbamoyl, *N,N*-dialkyl-carbamoyl, aryloyloxy, acyloxy, aryloxy, thiocyano, hydroxyl, arylmethoxy, arylsulphonyloxy, alkylsulphonyloxy, aryloxysulfonyl, - [(alkoxycarbonyl)-methoxyl]carbonyl, [(aryloxycarbonyl)-methoxyl]carbonyl, [(alkylacyl)-methoxy]carbonyl, [(arylacyl)-methoxy]carbonyl, trifluoromethyl
or
alkyl interrupted by one or more heteroatoms selected from the group consisting of oxygen and sulfur
or
alkyl substituted by one or more substituents selected from the group consisting of hydroxy, aryl, cycloalkyl, alkoxy, thioalkoxy, amino, *N*-monoalkyl-amino, *N,N-*dialkyl-amino, *N-*monoaryl-amino, *N,N-*diaryl-amino, *N*-alkyl-*N*-aryl-amino, *N-*monocycloalkyl-amino, N, *N-*dicycloalkyl-amino, *N*-monoalkyl-monocycloalkyl-amino, *N,N-*monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, *N*-monoaryl-carbamoyl, *N,N*-diaryl-carbamoyl, N-monocycloalkyl-*N*-monoarylcarbamoyl, *N*-monoalkyl-*N*-monoaryl-carbamoyl, sulfamoyl, N-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N-*diaryl-sulfamoyl, *N-*monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹² or
alkyl interrupted by one or more heteroatoms selected from the group consisting of oxygen and sulphur and substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N-*diaryl-amino, *N*-alkyl-*N*-aryl-amino, *N-*monocycloalkyl-amino, *N,N-*dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, *N*-monoalkyl-carbamoyl, *N,N*-dicycloalkyl-carbamoyl, *N,N*-dialkyl-carbamoyl, *N*-monoaryl-carbamoyl, *N,N*-diaryl-carbamoyl, *N-*monocycloalkyl-*N-*monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹² or
aryl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N*-diaryl-amino, *N*-alkyl-*N*-aryl-amino, *N-*monocycloalkyl-amino, *N,N*-dicycloalkyl-amino, *N*-monoalkyl-monocycloalkyl-amino, *N,N*-monoaryl-monocycloalkyl-amino, *N*-acylamino, *N*-alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, N-monoaryl-carbamoyl, *N,N*-diaryl-carbamoyl, *N-*monocycloalkyl-*N-*monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, N,N-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-N-monoarylsulfamoyl, arylsulphonyloxy, alkylsulphonyloxy, aryloxysulfonyl, alkoxylsulfonyl and COOR¹², whereby at least one of T¹, T², T³ and T⁴ is not hydrogen
or
D represents a group of formula (VII) wherein 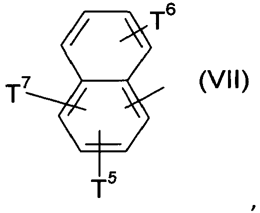
T⁵ to T⁷ independent from each other is
hydrogen, alkyl, cycloalkyl, alkenyl, alkynyl, aryl, alkoxyl, halogen, cyano, nitro, acyl, arylacyl, alkoxycarbonyl, aryloxycarbonyl, [(alkoxycarbonyl)-methoxyl]carbonyl, [(aryloxycarbonyl)-methoxyl]carbonyl, [(alkylacyl)-methoxy]carbonyl, [(arylacyl)-methoxy]carbonyl, *N*-monoalkyl-carbamoyl, *N,N*-dialkyl-carbamoyl
or
alkyl interrupted by one or more heteroatoms selected from the group consisting of oxygen and sulfur
or
alkyl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N-*diaryl-amino, *N*-alkyl-*N*-aryl-amino, *N-*monocycloalkyl-amino, *N,N-*dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N-*dialkyl-carbamoyl, *N*-monoaryl-carbamoyl, *N,N*-diaryl-carbamoyl, N-monocycloalkyl-*N*-monoarylcarbamoyl, *N*-monoalkyl-*N*-monoaryl-carbamoyl, sulfamoyl, N-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N-*diaryl-sulfamoyl, *N-*monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹² or
alkyl interrupted by one or more heteroatoms selected from the group consisting of oxygen and sulphur and substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N*-diaryl-amino, *N*-alkyl-*N*-aryl-amino, *N-*monocycloalkyl-amino, *N*,*N-*dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, *N*-monoalkyl-carbamoyl, *N,N*-dicycloalkyl-carbamoyl, *N,N*-dialkyl-carbamoyl, *N*-monoaryl-carbamoyl, *N,N-*diaryl-carbamoyl, *N-*monocycloalkyl-*N*-monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N-*diaryl-sulfamoyl, *N-*monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹² or
aryl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N*-diaryl-amino, *N-*alkyl-*N-*aryl-amino, *N-*monocycloalkyl-amino, *N*,*N*-dicycloalkyl-amino, *N*-monoalkyl-monocycloalkyl-amino, *N,N*-monoaryl-monocycloalkyl-amino, *N*-acylamino, *N*-alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, N-monoaryl-carbamoyl, *N,N*-diaryl-carbamoyl, *N-*monocycloalkyl-*N-*monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, N,N-dialkyl-sulfamoyl, *N-*monoaryl-sulfamoyl, *N,N*-diaryl-sulfamoyl, *N-*monocycloalkyl-*N*-monoarylsulfamoyl, *N*-monoalkyl-N-monoarylsulfamoyl, arylsulphonyloxy, alkylsulphonyloxy, aryloxysulfonyl, alkoxylsulfonyl and COOR¹²,
whereby at least one of T⁵, T⁶ and T⁷ is not hydrogen,
or
D represents a group of the formula (VIII) 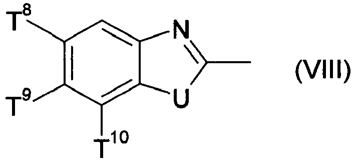 wherein
U is oxygen, sulphur or N-R¹⁵,
T⁸ and T¹⁰ independent of each other is hydrogen, fluorine, chlorine or bromine,
T⁹ is hydrogen, alkylsulfonyl, thiocyano, alkoxy, halogen or nitro,
R¹⁵ is alkyl, aryl or cycloalkyl,
or
D represents a group of the formula (IX) 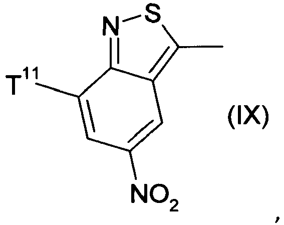 wherein
T¹¹ is hydrogen, nitro or halogen,
or
D represents a group of the formula (X) 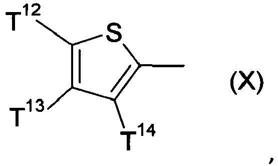 wherein
T¹² and T¹⁴ independent from each other is nitro, cyano, acyl or alkoxycarbonyl,
T¹³ is hydrogen, aryl, alkyl, halogen
or
alkyl interrupted by one or more heteroatoms selected from the group consisting of oxygen and sulfur
or
alkyl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N-*diaryl-amino, *N*-alkyl-*N*-aryl-amino, *N-*monocycloalkyl-amino, *N,N*-dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N-*dialkyl-carbamoyl, *N*-monoaryl-carbamoyl, *N,N-*diaryl-carbamoyl, N-monocycloalkyl-*N*-monoarylcarbamoyl, *N*-monoalkyl-*N*-monoaryl-carbamoyl, sulfamoyl, N-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹² or
alkyl interrupted by one or more heteroatoms selected from the group consisting of oxygen and sulphur and substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N-*diaryl-amino, *N*-alkyl-*N*-aryl-amino, *N-*monocycloalkyl-amino, *N*,*N*-dicycloalkyl-amino, *N*-monoalkyl-monocycloalkyl-amino, *N,N-*monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, *N*-monoalkyl-carbamoyl, *N,N*-dicycloalkyl-carbamoyl, *N,N*-dialkyl-carbamoyl, *N*-monoaryl-carbamoyl, *N,N*-diaryl-carbamoyl, *N-*monocycloalkyl-*N*-monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N-*diaryl-sulfamoyl, *N-*monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹² or
aryl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N*-diaryl-amino, *N*-alkyl-*N*-aryl-amino, *N-*monocycloalkyl-amino, *N*,*N-*dicycloalkyl-amino, *N*-monoalkyl-monocycloalkyl-amino, *N,N*-monoaryl-monocycloalkyl-amino, *N*-acylamino, *N*-alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N-*dialkyl-carbamoyl, N-monoaryl-carbamoyl, *N,N*-diaryl-carbamoyl, *N-*monocycloalkyl-*N*-monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, N,N-dialkyl-sulfamoyl, *N*-monoaryl-sulfamoyl, *N,N*-diaryl-sulfamoyl, *N-*monocycloalkyl-*N-*monoarylsulfamoyl, *N-*monoalkyl-N-monoarylsulfamoyl, arylsulphonyloxy, alkylsulphonyloxy, aryloxysulfonyl, alkoxylsulfonyl and COOR¹²,
or
D represents a group of the formula (XI) 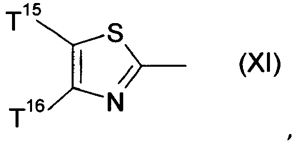 wherein
T¹⁵ and T¹⁶ independent from each other is hydrogen, halogen, alkyl, nitro, cyano, acyl, alkoxycarbonyl, aryl
or
alkyl interrupted by one or more heteroatoms selected from the group consisting of oxygen and sulfur
or
alkyl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N*-diaryl-amino, *N*-alkyl-*N*-aryl-amino, *N-*monocycloalkyl-amino, *N,N-*dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N-*dialkyl-carbamoyl, *N*-monoaryl-carbamoyl, *N,N-*diaryl-carbamoyl, N-monocycloalkyl-*N-*monoarylcarbamoyl, *N*-monoalkyl-*N*-monoaryl-carbamoyl, sulfamoyl, N-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N-*diaryl-sulfamoyl, *N-*monocycloalkyl-*N-*monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹² or
alkyl interrupted by one or more heteroatoms selected from the group consisting of oxygen and sulphur and substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N-*diaryl-amino, *N*-alkyl-*N*-aryl-amino, *N-*monocycloalkyl-amino, *N*,*N-*dicycloalkyl-amino, *N*-monoalkyl-monocycloalkyl-amino, *N,N-*monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N-*monocycloalkyl-carbamoyl, *N*-monoalkyl-carbamoyl, *N,N*-dicycloalkyl-carbamoyl, *N,N-*dialkyl-carbamoyl, *N*-monoaryl-carbamoyl, *N,N*-diaryl-carbamoyl, *N-*monocycloalkyl-*N*-monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹² or
aryl substituted by one or more substituents selected from the group consisting of hydroxy, aryl, cycloalkyl, alkoxy, thioalkoxy, amino, *N*-monoalkyl-amino, *N,N-*dialkyl-amino, *N-*monoaryl-amino, *N,N*-diaryl-amino, *N*-alkyl-*N*-aryl-amino, *N-*monocycloalkyl-amino, *N,N*-dicycloalkyl-amino, *N*-monoalkyl-monocycloalkyl-amino, *N,N*-monoaryl-monocycloalkyl-amino, *N*-acylamino, *N*-alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N-*dicycloalkyl-carbamoyl, *N,N*-dialkyl-carbamoyl, N-monoaryl-carbamoyl, *N,N-*diaryl-carbamoyl, *N-*monocycloalkyl-*N*-monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, N,N-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-N-monoarylsulfamoyl, arylsulphonyloxy, alkylsulphonyloxy, aryloxysulfonyl, alkoxylsulfonyl and COOR¹²
whereby at least one of T¹⁵ and T¹⁶ is not hydrogen,
or
D represents a group of the formula (XII) 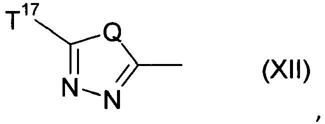 wherein
T¹⁷ is aryl, thioalkoxyl
or
aryl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N*-diaryl-amino, *N*-alkyl-*N*-aryl-amino, *N-*monocycloalkyl-amino, *N,N*-dicycloalkyl-amino, *N-*monoalkyl-monocycloalkyl-amino, *N,N*-monoaryl-monocycloalkyl-amino, *N*-acylamino, *N*-alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, N-monoaryl-carbamoyl, *N,N*-diaryl-carbamoyl, *N-*monocycloalkyl-*N*-monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, N,N-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-N-monoarylsulfamoyl, arylsulphonyloxy, alkylsulphonyloxy, aryloxysulfonyl, alkoxylsulfonyl and COOR¹²,
Q is oxygen or sulphur,
or
D represents a group of the formula (XIII) 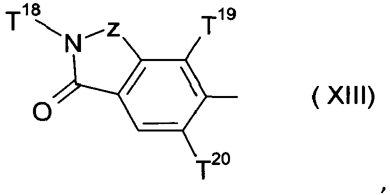 wherein
T¹⁸ is alkyl, alkenyl, aryl
or
alkyl interrupted by one or more heteroatoms selected from the group consisting of oxygen and sulfur
or
alkyl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N*-diaryl-amino, *N*-alkyl-*N*-aryl-amino, *N-*monocycloalkyl-amino, *N*,*N*-dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, *N*-monoaryl-carbamoyl, *N,N*-diaryl-carbamoyl, N-monocycloalkyl-*N*-monoarylcarbamoyl, *N*-monoalkyl-*N*-monoaryl-carbamoyl, sulfamoyl, N-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹² or
alkyl interrupted by one or more heteroatoms selected from the group consisting of oxygen and sulphur and substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N*-diaryl-amino, *N*-alkyl-*N*-aryl-amino, *N-*monocycloalkyl-amino, *N,N*-dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, *N*-monoalkyl-carbamoyl, *N,N*-dicycloalkyl-carbamoyl, *N,N*-dialkyl-carbamoyl, *N*-monoaryl-carbamoyl, *N,N*-diaryl-carbamoyl, *N-*monocycloalkyl-*N*-monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl, and COOR¹² or
aryl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N*-diaryl-amino, *N*-alkyl-*N*-aryl-amino, *N-*monocycloalkyl-amino, *N, N*-dicycloalkyl-amino, *N*-monoalkyl-monocycloalkyl-amino, *N,N*-monoaryl-monocycloalkyl-amino, *N*-acylamino, *N*-alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N-*dialkyl-carbamoyl, N-monoaryl-carbamoyl, *N,N*-diaryl-carbamoyl, *N-*monocycloalkyl-*N*-monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, N,N-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-N-monoarylsulfamoyl, arylsulphonyloxy, alkylsulphonyloxy, aryloxysulfonyl, alkoxylsulfonyl and COOR¹²,
T¹⁹ and T²⁰ independent from each other is hydrogen, halogen, cyano, nitro or trifluoromethyl,
Z is a carbonyl or a sulfonyl rest,
or
D represents a group of the formula (XIV) 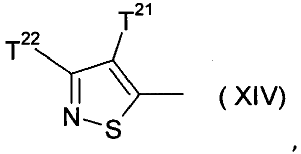 wherein
T²¹ is cyano, nitro or alkoxycarbonyl,
T²² is alkyl, hydrogen, halogen, aryl
or
alkyl interrupted by one or more heteroatoms selected from the group consisting of oxygen and sulfur
or
alkyl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N-*diaryl-amino, *N*-alkyl-*N*-aryl-amino, *N-*monocycloalkyl-amino, *N, N*-dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, *N*-monoaryl-carbamoyl, *N,N*-diaryl-carbamoyl, N-monocycloalkyl-*N*-monoarylcarbamoyl, *N*-monoalkyl-*N*-monoaryl-carbamoyl, sulfamoyl, N-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹² or
alkyl interrupted by one or more heteroatoms selected from the group consisting of oxygen or sulphur and substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N*-diaryl-amino, *N*-alkyl-*N*-aryl-amino, *N-*monocycloalkyl-amino, N, *N*-dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, *N*-monoalkyl-carbamoyl, *N,N*-dicycloalkyl-carbamoyl, *N,N*-dialkyl-carbamoyl, *N*-monoaryl-carbamoyl, *N,N*-diaryl-carbamoyl, *N-*monocycloalkyl-*N*-monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹² or
aryl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N-*diaryl-amino, *N*-alkyl-*N*-aryl-amino, *N-*monocycloalkyl-amino, *N,N*-dicycloalkyl-amino, *N*-monoalkyl-monocycloalkyl-amino, *N,N*-monoaryl-monocycloalkyl-amino, *N*-acylamino, *N*-alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, N-monoaryl-carbamoyl, *N,N*-diaryl-carbamoyl, *N-*monocycloalkyl-*N*-monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, N,N-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-N-monoarylsulfamoyl, arylsulphonyloxy, alkylsulphonyloxy, aryloxysulfonyl, alkoxylsulfonyl and COOR¹²,
or
D represents a group of the formula (XV) 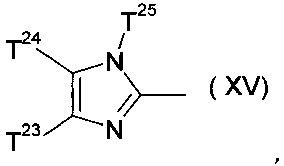 wherein
T²³ and T²⁴ independent from each other is cyano, nitro, halogen, aryl or trifluoromethyl,
T²⁵ is hydrogen, alkyl, alkenyl, aryl
or
alkyl interrupted by one or more heteroatoms selected from the group consisting of oxygen and sulphur
or
alkyl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N-*diaryl-amino, *N*-alkyl-*N*-aryl-amino, *N-*monocycloalkyl-amino, *N,N*-dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, *N*-monoaryl-carbamoyl, *N,N*-diaryl-carbamoyl, N-monocycloalkyl-*N*-monoarylcarbamoyl, *N*-monoalkyl-*N*-monoaryl-carbamoyl, sulfamoyl, N-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²
or
alkyl interrupted by one or more heteroatoms selected from the group consisting of oxygen and sulphur and substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N*-diaryl-amino, *N*-alkyl-*N*-aryl-amino, *N-*monocycloalkyl-amino, *N,N-*dicycloalkyl-amino, *N*-monoalkyl-monocycloalkyl-amino, *N,N-*monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, *N*-monoalkyl-carbamoyl, *N,N*-dicycloalkyl-carbamoyl, *N,N*-dialkyl-carbamoyl, *N*-monoaryl-carbamoyl, *N,N*-diaryl-carbamoyl, *N-*monocycloalkyl-*N*-monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹² or
aryl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N*-diaryl-amino, *N*-alkyl-*N*-aryl-amino, *N-*monocycloalkyl-amino, *N, N*-dicycloalkyl-amino, *N*-monoalkyl-monocycloalkyl-amino, *N,N*-monoaryl-monocycloalkyl-amino, *N*-acylamino, *N*-alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, N-monoaryl-carbamoyl, *N,N*-diaryl-carbamoyl, *N-*monocycloalkyl-*N*-monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, N,N-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-N-monoarylsulfamoyl, arylsulphonyloxy, alkylsulphonyloxy, aryloxysulfonyl, alkoxylsulfonyl and COOR¹²
or
D represents a group of the formula (XVI) 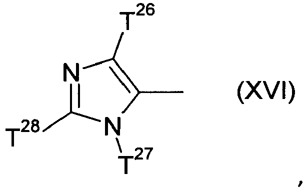 wherein
T²⁶ is cyano, nitro, aryl or trifluoromethyl,
T²⁷ is hydrogen, aryl, alkly
or
alkyl interrupted by one or more heteroatoms selected from the group consisting of oxygen and sulfur
or
alkyl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N*-diaryl-amino, *N*-alkyl-*N*-aryl-amino, *N-*monocycloalkyl-amino, *N,N*-dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, *N*-monoaryl-carbamoyl, *N,N*-diaryl-carbamoyl, N-monocycloalkyl-*N*-monoarylcarbamoyl, *N*-monoalkyl-*N*-monoaryl-carbamoyl, sulfamoyl, N-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²
or
alkyl interrupted by one or more heteroatoms selected from the group consisting of oxygen and sulphur and substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N-*diaryl-amino, *N*-alkyl-*N*-aryl-amino, *N-*monocycloalkyl-amino, *N,N*-dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, *N*-monoalkyl-carbamoyl, *N,N*-dicycloalkyl-carbamoyl, *N,N*-dialkyl-carbamoyl, *N*-monoaryl-carbamoyl, *N,N*-diaryl-carbamoyl, *N-*monocycloalkyl-*N*-monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²
or
aryl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N*-diaryl-amino, *N*-alkyl-*N*-aryl-amino, *N-*monocycloalkyl-amino, *N,N*-dicycloalkyl-amino, *N*-monoalkyl-monocycloalkyl-amino, *N,N*-monoaryl-monocycloalkyl-amino, *N*-acylamino, *N*-alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, N-monoaryl-carbamoyl, *N,N*-diaryl-carbamoyl, *N-*monocycloalkyl-*N*-monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, N,N-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-N-monoarylsulfamoyl, arylsulphonyloxy, alkylsulphonyloxy, aryloxysulfonyl, alkoxylsulfonyl and COOR¹²,
T²⁸ is hydrogen, alkyl, alkenyl, alkynyl, aryl, alkoxycarbonyl
or
alkyl interrupted by one or more heteroatoms selected from the group consisting of oxygen and sulfur
or
alkyl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N-*diaryl-amino, *N*-alkyl-*N*-aryl-amino, *N-*monocycloalkyl-amino, *N,N*-dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, N-monoaryl-carbamoyl, *N,N*-diaryl-carbamoyl, N-monocycloalkyl-*N*-monoarylcarbamoyl, *N*-monoalkyl-*N*-monoaryl-carbamoyl, sulfamoyl, N-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²
or
alkyl interrupted by one or more heteroatoms selected from the group consisting of oxygen and sulphur and substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N-*diaryl-amino, *N*-alkyl-*N*-aryl-amino, *N-*monocycloalkyl-amino, *N,N*-dicycloalkyl-amino, *N*-monoalkyl-monocycloalkyl-amino, *N,N*-monoaryl-monocycloalkyl-amino, *N*-acylamino, *N*-alkylsulfonyl-amino,
halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, *N*-monoalkyl-carbamoyl, *N,N*-dicycloalkyl-carbamoyl, *N,N-*dialkyl-carbamoyl, *N*-monoaryl-carbamoyl, *N,N*-diaryl-carbamoyl, *N-*monocycloalkyl-*N*-monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹² or
aryl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N*-diaryl-amino, *N*-alkyl-*N*-aryl-amino, *N-*monocycloalkyl-amino, *N,N*-dicycloalkyl-amino, *N*-monoalkyl-monocycloalkyl-amino, *N,N*-monoaryl-monocycloalkyl-amino, *N*-acylamino, *N*-alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N-*dialkyl-carbamoyl, N-monoaryl-carbamoyl, *N,N*-diaryl-carbamoyl, *N-*monocycloalkyl-*N*-monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, N,N-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹², or
D represents a group of the formula (XVII) 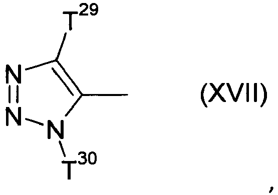 wherein
T²⁹ is cyano, nitro, aryl or trifluoromethyl,
T³⁰ is hydrogen, alkyl, alkenyl, alkenyl, alkynyl, aryl, arylmethyl, alkoxycarbonyl
or
alkyl interrupted by one or more heteroatoms selected from the group consisting of oxygen and sulfur
or
alkyl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N-*diaryl-amino, *N*-alkyl-*N*-aryl-amino, *N-*monocycloalkyl-amino, *N,N*-dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N-*dialkyl-carbamoyl, *N*-monoaryl-carbamoyl, *N,N*-diaryl-carbamoyl, N-monocycloalkyl-*N*-monoarylcarbamoyl, *N*-monoalkyl-*N*-monoaryl-carbamoyl, sulfamoyl, N-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²
or
alkyl interrupted by one or more heteroatoms selected from the group consisting of oxygen and sulphur and substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N-*diaryl-amino, *N*-alkyl-*N*-aryl-amino, *N-*monocycloalkyl-amino, *N,N*-dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, *N*-monoalkyl-carbamoyl, *N,N*-dicycloalkyl-carbamoyl, *N,N*-dialkyl-carbamoyl, *N*-monoaryl-carbamoyl, *N,N*-diaryl-carbamoyl, *N-*monocycloalkyl-*N*-monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹² or
aryl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N*-diaryl-amino, *N*-alkyl-*N*-aryl-amino, *N-*monocycloalkyl-amino, *N,N*-dicycloalkyl-amino, *N*-monoalkyl-monocycloalkyl-amino, *N,N*-monoaryl-monocycloalkyl-amino, *N*-acylamino, *N*-alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N-*dialkyl-carbamoyl, N-monoaryl-carbamoyl, *N,N*-diaryl-carbamoyl, *N-*monocycloalkyl-*N*-monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, N,N-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-N-monoarylsulfamoyl, arylsulphonyloxy, alkylsulphonyloxy, aryloxysulfonyl, alkoxylsulfonyl and COOR¹²,
or
D represents a group of the formula (XVIII) 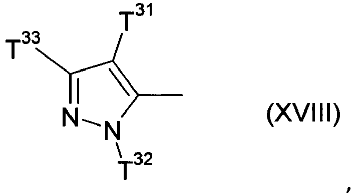 wherein
T³¹ is cyano, nitro, aryl, trifluoromethyl
or
aryl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N*-diaryl-amino, *N*-alkyl-*N*-aryl-amino, *N-*monocycloalkyl-amino, *N,N*-dicycloalkyl-amino, *N*-monoalkyl-monocycloalkyl-amino, *N,N*-monoaryl-monocycloalkyl-amino, *N*-acylamino, *N*-alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, N-monoaryl-carbamoyl, *N*,*N*-diaryl-carbamoyl, *N-*monocycloalkyl-*N*-monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, N,N-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-N-monoarylsulfamoyl, arylsulphonyloxy, alkylsulphonyloxy, aryloxysulfonyl, alkoxylsulfonyl and COOR¹²,
T³² is hydrogen, alkyl, alkenyl, alkynyl, aryl, alkoxycarbonyl
or
alkyl interrupted by one or more heteroatoms selected from the group consisting of oxygen and sulphur
or
alkyl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N*-diaryl-amino, *N*-alkyl-*N*-aryl-amino, *N-*monocycloalkyl-amino, *N,N*-dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N-*dialkyl-carbamoyl, *N*-monoaryl-carbamoyl, *N,N*-diaryl-carbamoyl, N-monocycloalkyl-*N*-monoarylcarbamoyl, *N*-monoalkyl-*N*-monoaryl-carbamoyl, sulfamoyl, N-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹² or
alkyl interrupted by one or more heteroatoms selected from the group consisting of oxygen and sulphur and substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N*-diaryl-amino, *N*-alkyl-*N*-aryl-amino, *N-*monocycloalkyl-amino, *N,N*-dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, *N*-monoalkyl-carbamoyl, *N,N*-dicycloalkyl-carbamoyl, *N,N*-dialkyl-carbamoyl, *N*-monoaryl-carbamoyl, *N,N*-diaryl-carbamoyl, *N-*monocycloalkyl-*N*-monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹² or
aryl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N-*diaryl-amino, *N*-alkyl-*N*-aryl-amino, *N-*monocycloalkyl-amino, *N,N*-dicycloalkyl-amino, *N*-monoalkyl-monocycloalkyl-amino, *N,N*-monoaryl-monocycloalkyl-amino, *N*-acylamino, *N*-alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, N-monoaryl-carbamoyl, *N,N*-diaryl-carbamoyl, *N-*monocycloalkyl-*N*-monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, N,N-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-N-monoarylsulfamoyl, arylsulphonyloxy, alkylsulphonyloxy, aryloxysulfonyl, alkoxylsulfonyl and COOR¹²,
T³³ is hydrogen, alkyl
or
alkyl interrupted by one or more heteroatoms selected from the group consisting of oxygen and sulphur
or
alkyl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N-*diaryl-amino, *N*-alkyl-*N*-aryl-amino, *N-*monocycloalkyl-amino, *N*,*N*-dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, *N*-monoaryl-carbamoyl, *N,N*-diaryl-carbamoyl, N-monocycloalkyl-*N*-monoarylcarbamoyl, *N*-monoalkyl-*N*-monoaryl-carbamoyl, sulfamoyl, N-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹² or
alkyl interrupted by one or more heteroatoms selected from the group consisting of oxygen and sulphur and substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N-*diaryl-amino, *N*-alkyl-*N*-aryl-amino, *N-*monocycloalkyl-amino, *N,N*-dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, *N*-monoalkyl-carbamoyl, *N,N*-dicycloalkyl-carbamoyl, *N,N-*dialkyl-carbamoyl, N-monoaryl-carbamoyl, *N,N*-diaryl-carbamoyl, *N-*monocycloalkyl-*N*-monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹² or
aryl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N-*diaryl-amino, *N*-alkyl-*N*-aryl-amino, *N-*monocycloalkyl-amino, *N,N*-dicycloalkyl-amino, *N*-monoalkyl-monocycloalkyl-amino, *N,N*-monoaryl-monocycloalkyl-amino, *N*-acylamino, *N*-alkylsulfonyl-amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N*-dialkyl-carbamoyl, N-monoaryl-carbamoyl, *N,N-*diaryl-carbamoyl, *N-*monocycloalkyl-*N*-monoarylcarbamoyl, N-monoalkyl-N-monoaryl-carbamoyl, sulfamoyl, *N*-monocycloalkyl-sulfamoyl, *N*-monoalkyl-sulfamoyl, N,N-dicycloalkyl-sulfamoyl, *N,N*-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²,
or
D represents a group of the formula (XIX) 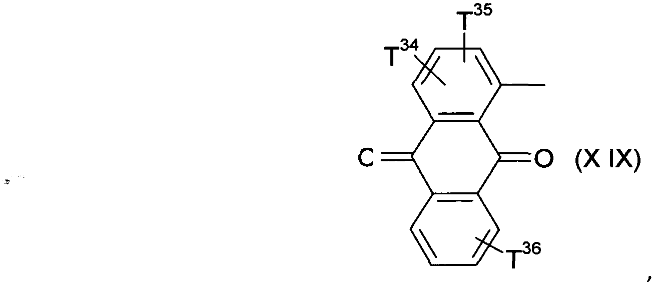 wherein
T³⁴, T³⁵ and ^{T36} independent from each other is hydrogen, halogen, nitro, cyano, hydroxyl, alkoxylcarbonyl, alkyl, acyl
or
alkyl substituted by one or more substituents selected from the group consisting of hydroxy, aryl, cycloalkyl, alkoxy, thioalkoxy, amino, *N*-monoalkyl-amino, *N,N-*dialkyl-amino, *N*-monoaryl-amino, *N,N*-diaryl-amino, *N*-alkyl-*N*-aryl-amino, *N-*monocycloalkyl-amino, *N,N*-dicycloalkyl-amino, *N*-monoalkyl-monocycloalkyl-amino, *N,N-*monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, N-monoalkyl-carbamoyl, *N,N*-dicycloalkyl-carbamoyl, *N,N-*dialkyl-carbamoyl, *N*-monoaryl-carbamoyl, *N,N*-diaryl-carbamoyl, N-monocycloalkyl-*N*-monoarylcarbamoyl, *N*-monoalkyl-*N*-monoaryl-carbamoyl, sulfamoyl, N-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹² or
alkyl interrupted by one or more heteroatoms selected from the group consisting of oxygen and sulphur and substituted by one or more substituents selected from the group consisting of hydroxy, aryl, cycloalkyl, alkoxy, thioalkoxy, amino, *N*-monoalkyl-amino, *N,N*-dialkyl-amino, *N*-monoaryl-amino, *N,N*-diaryl-amino, *N*-alkyl-*N*-aryl-amino, *N-*monocycloalkyl-amino, *N*,*N*-dicycloalkyl-amino, *N*-monoalkyl-monocycloalkyl-amino, *N,N*-monoaryl-monocycloalkyl-amino, *N*-acylamino, *N*-alkylsulfonyl-amino, halogen, cyano, thiocyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, *N*-monocycloalkyl-carbamoyl, *N*-monoalkyl-carbamoyl, *N,N*-dicycloalkyl-carbamoyl, *N,N*-dialkyl-carbamoyl, *N*-monoaryl-carbamoyl, *N,N*-diaryl-carbamoyl, *N-*monocycloalkyl-*N*-monoarylcarbamoyl, *N*-monoalkyl-*N*-monoaryl-carbamoyl, sulfamoyl, *N*-monocycloalkyl-sulfamoyl, *N*-monoalkyl-sulfamoyl, *N,N*-dicycloalkyl-sulfamoyl, *N,N*-dialkyl-sulfamoyl, *N*-monoaryl-sulfamoyl, *N,N*-diaryl-sulfamoyl, *N-*monocycloalkyl-*N*-monoarylsulfamoyl, *N*-monoalkyl-*N*-monoarylsulfamoyl, arylsulphonyloxy, alkylsulphonyloxy, aryloxysulfonyl, alkoxylsulfonyl and COOR¹²,
or
D represents a group of the formula (XX) 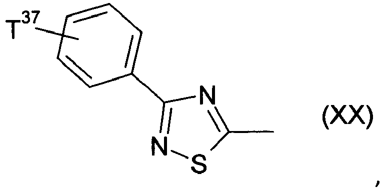 wherein
T³⁷ is hydrogen, halogen or nitro,
R¹² is hydrogen, alkyl, alkenyl, cycloalkyl, aryl, heteroaryl, heterocycloalkyl
or
alkyl interrupted by one or more heteroatoms selected from the group consisting of oxygen and sulfur
or
alkyl substituted by one or more substituents selected from the group consisting of hydroxy, aryl, cycloalkyl, alkoxy, thioalkoxy, halogen, cyano, amino, thiocyano, nitro, acyl, thioacyl, alkylsulfonyl, aryloyl, trifluoromethyl, heteroaryl, heterocycloalkyl, alkoxythiocarbonyl, acyloxy, aryloyloxy, carbamoyl, sulfamoyl, arylsulphonyloxy, alkylsulphonyloxy, aryloxysulfonyl and alkoxylsulfonyl
or
alkyl interrupted by one or more heteroatoms selected from the group consisting of oxygen and sulphur and substituted by one or more substituents selected from the group consisting of hydroxy, aryl, cycloalkyl, alkoxy, thioalkoxy, halogen, cyano, amino, thiocyano, nitro, acyl, thioacyl, alkylsulfonyl, aryloyl, trifluoromethyl, heteroaryl, heterocycloalkyl, alkoxythiocarbonyl, acyloxy, aryloyloxy, carbamoyl, sulfamoyl, arylsulphonyloxy, alkylsulphonyloxy, aryloxysulfonyl and alkoxylsulfonyl
or
aryl substituted by one or more substituents selected from the group consisting of hydroxy, aryl, cycloalkyl, alkoxy, thioalkoxy, amino, thiocyano, halogen, cyano, nitro, acyl, thioacyl, alkylsulfonyl, aryloyl, trifluoromethyl, heteroaryl, heterocycloalkyl, alkoxycarbonyl, alkoxythiocarbonyl, acyloxy, aryloyloxy, carbamoyl, sulfamoyl, arylsulphonyloxy, alkylsulphonyloxy, aryloxysulfonyl and alkoxylsulfonyl,
with the proviso that no fused-ring formation is allowed among R² to R¹⁴.

2. Dyes according to claim 1 having formula (I-a) 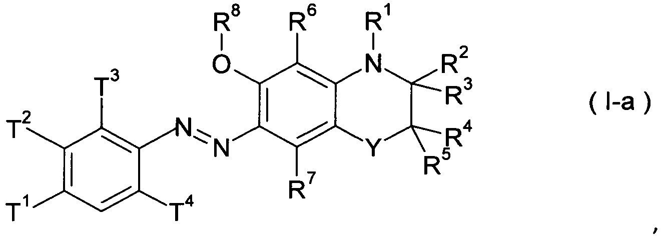 wherein
each of T¹ to T⁴ independent from each other is hydrogen, (C₁-C₆)-alkyl, (C₃-C₆)-cycloalkyl, substituted (C₁-C₆)-alkyl, (C₃-C₄)-alkenyl, (C₃-C₄)-alkynyl, aryl, O-interrupted (C₁-C₆)-alkyl, S-interrupted (C₁-C₆)-alkyl, (C₁-C₈)-alkoxyl, (C₁-C₆)-cycloalkyl, halogen, cyano, nitro, arylsulfonyl, arylsulfonyloxy, (C₁-C₆)-alkylsulfonyl, (C₁-C₆)-alkoxylsulfonyl, *N*-(C₁-C₆)-alkylsulfamoyl, *N,N*-bis(C₁-C₆)alkylsulfamoyl, *N-*(C₁-C₆)-alkyl carbamoyl, *N*-bis(C₁-C₆)alkyl-carbamoyl, (C₁-C₆)-alkylacyl, arylacyl, (C₁-C₆)-alkoxylcarbonyl, aryloxylcarbonyl, thiocyano, hydroxyl, aryloxy, arylmethoxyl, aryloyloxy, arylsulfonyloxy, {[(C₁-C₆)-alkoxylcarbonyl]methoxyl} carbonyl, [(aryloxycarbonyl)methoxy]carbonyl, {[(C₁-C₆)-alkylacyl]-methoxy}carbonyl or [(arylacyl)-methoxy]carbonyl,
R¹ is (C₁-C₆)-alkyl, (C₁-C₆)-acyloxy substituted (C₂-C₆)alkyl, cyano(C₁-C₆)alkyl, arylmethyl, arylacyl (C₁-C₆)alkyl, 2-(C₁-C₆)alkoxyl-2-oxoethyl, O-interrupted (C₂-C₆)-alkyl, S-interrupted (C₂-C₆)-alkyl, (C₃-C₄)-alkenyl, (C₃-C₄)-alkynyl, 3-(C₁-C₆)alkoxyl-3-oxopropyl, 3-[O-interrupted (C₂-C₆)-alkoxyl]-3-oxopropyl, 2-(C₁-C₆)alkoxyl-2-oxoethyl, 2-[O-interrupted (C₂-C₆)-alkoxyl]-2-oxoethyl, 2-{[(C₁-C₆)-alkylacyl]-methoxy}-2-oxoethyl, 3-{[(C₁-C₆)-alkylacyl]-methoxy}-3-oxopropyl,2-[(arylacyl)-methoxy]-2-oxoethyl, 3-[(arylacyl)-methoxy]-3-oxopropyl, 3-{[(C₁-C₆)-alkoxylcarbonyl]-methoxy}-3-oxopropyl, 3-(arylmethoxy)-3-oxopropyl,3-[(arylacyl)-methoxy]-3-oxopropyl, 2-(cyanomethoxy)-2-oxoethyl, 3-(cyanomethoxy)-3-oxopropyl, 2-{[(C₁-C₆)-alkoxylcarbonyl]methoxy)-2-oxoethyl, 1-[(C₁-C₆)-alkyl]-3-[(C₁-C₆)-alkoxyl]- 3-oxopropyl, 1-[(C₁-C₆)-alkyl]-3-[O-interrupted (C₁-C₆)-alkoxyl]-3-oxopropyl, 1-[(C₁-C₆)-alkyl]-3-(arylmethoxyl)-3-oxopropyl, 1-[(C₁-C₆)-alkyl]-3-{[(C₁-C₆)-alkylacyl]methoxy}-3-oxopropyl or 1-[(C₁-C₆)-alkyl]-3-{[(C₁-C₆)-alkoxylcarbonyl] -methoxy} -3 -oxopropyl,
R² to R⁷ independent from each other is hydrogen, (C₁-C₆)alkyl, aryl, (C₁-C₈)alkoxyl, (C₃-C₈)cycloalkyl, O-interrupted (C₂-C₆)alkyl, S-interrupted (C₂-C₆)alkyl or halogen,
R⁸ is hydrogen, (C₁-C₆)alkyl, (C₃-C₄)-alkenyl, (C₃-C₄)-alkynyl, arylmethyl, 2-(C₁-C₆)alkoxyl-2-oxoethyl, 2-(arylmethyl)-2-oxoethyl, cyano(C₁-C₆)alkyl, O-interrupted (C₁-C₆)alkyl or S-interrupted (C₁-C₆)alkyl
and
Y is carbonyl.

3. Dyes according to claim 1 or 2 having formula (I-a), wherein
T¹, T³ and T⁴ independent from each other is hydrogen, nitro, cyano, bromine, chlorine, benzoxyl, phenoxy, methoxy, ethoxy, methyl, trifluromethyl, ethyl, benzoyl, acetyl, hydroxyl, methoxycarbonyl, ethoxycarbonyl, methylsulfonyl, cyanomethylsulfonyl, acetonylsulfonyl, (2-ethoxy-2-oxoethyl)sulfonyl, (2-methoxy-2-oxoethyl)sulfonyl, phenylsulfonyloxy, phenoxylsulfonyl, ethoxylsulfonyl, phenoxylsulfonyl cyanomethoxylcarbonyl, (2-ethoxy-2-oxoethyl)carbonyl, (2-methoxy-2-oxoethyl)carbonyl, acetonylcarbonyl or phenacylcarbonyl,
T² is hydrogen, chlorine, bromine, methoxy, ethoxyl, phenoxy, benzoxy, nitro, methyl or ethyl,
R¹ is methyl, ethyl, propyl, isopropyl, *n*-butyl, isobutyl, benzyl, 2-methoxylethyl, 2-ethoxylethyl, 2-acetoxyethyl, 2-(propionyl)oxyethyl, 2-(benzoyl)oxyethyl, cyanomethyl, 2-cyanoethyl, 3-cyanopropyl, 3-(C₁-C₆)alkoxyl-3-oxopropyl, 3-[O-interrupted (C₂-C₆)-alkoxyl]-3-oxopropyl, 2-(C₁-C₆)alkyl-2-oxoethyl, 2-[O-interrupted (C₂-C₆)-alkoxyl]-2-oxoethyl, 2-(cyanomethoxy)-2-oxoethyl, 3-(cyanomethoxy)-3-oxopropyl, 2-[(arylacyl)methoxy]-2-oxoethyl, 3-[(arylacyl)methoxy]-3-oxopropyl, 3-(arylmethoxy)-3-oxopropyl, 3-{[(C₁-C₆)alkylacyl]-methoxyl}-3-oxopropyl,3-[(arylacyl)-methoxy]-3-oxopropyl,3-{[(C₁-C₆)-alkoxycabonyl]methoxy}-3-oxopropyl, 2-{[(C₁-C₆)-alkoxycabonyl]methoxy}-2-oxoethyl or 2-{[(C₁-C₆)alkylacyl]-methoxyl}2-oxoethyl,
R² to R⁷ independent from each other is hydrogen, bromine, chlorine, ethyl or methyl,
R⁸ is hydrogen, methyl, ethyl, propyl, isopropyl, 2-ethoxy-2-oxoethyl or 2-methoxy-2-oxoethyl
and
Y is carbonyl.

4. Dyes according to claim 1 having formula (I-b) 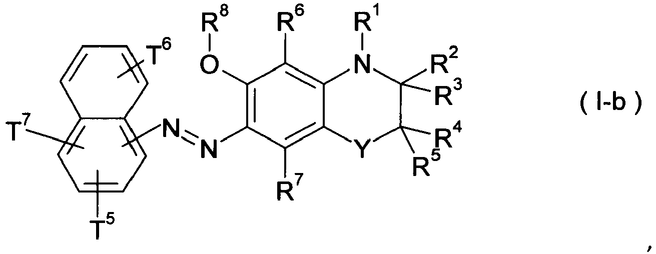 wherein
T⁵ to T⁷ independent from each other is hydrogen, (C₁-C₆)alkyl, (C₃-C₈)cycloalkyl, O-interrupted (C₁-C₆)-alkyl, S-interrupted (C₁-C₆)-alkyl, aryl, (C₁-C₆)alkoxyl, halogen, cyano, nitro, (C₁-C₆)-alkylacyl, arylacyl, (C₁-C₆)-alkoxycarbonyl, aryloxycarbonyl, N-(C₁-C₆)-alkyl carbamoyl, or *N,N-*bis(C₁-C₆)-alkyl carbamoyl,
whereby at least one of T⁵, T⁶ and T⁷ is not hydrogen,
R¹ is methyl, ethyl, propyl, isopropyl, n-butyl, isobutyl, benzyl, 2-methoxylethyl, 2-ethoxylethyl, 2-acetoxyethyl, 2-(propionyl)oxyethyl, 2-(benzoyl)oxyethyl, cyanomethyl, 2-cyanoethyl, 3-cyanopropyl, 3-(C₁-C₆)alkoxyl-3-oxopropyl, 3-[O-interrupted (C₂-C₆)-alkoxyl]-3-oxopropyl, 2-(C₁-C₆)alkyl-2-oxoethyl, 2-[O-interrupted (C₂-C₆)-alkoxyl]-2-oxoethyl, 2-(cyanomethoxy)-2-oxoethyl, 3-(cyanomethoxy)-3-oxopropyl, 2-[(arylacyl)methoxy]-2-oxoethyl, 3-[(arylacyl)methoxy] -3 -oxopropyl, 3-(arylmethoxy)-3-oxopropyl, 3-{[(C₁-C₆)alkylacyl]-methoxyl} -3-oxopropyl, 3-[(arylacyl)-methoxy]-3-oxopropyl, 3-{[(C₁-C₆)-alkoxycabonyl]methoxy}-3-oxopropyl, 2-{[(C₁-C₆)-alkoxycabonyl]methoxy}-2-oxoethyl or 2-{[(C₁-C₆)alkylacyl]-methoxyl}-2-oxoethyl,
R² to R⁷ independent from each other is hydrogen, (C₁-C₆)alkyl, aryl, (C₁-C₈)alkoxyl, (C₃-C₈)cycloalkyl, O-interrupted (C₂-C₆)alkyl, S-interrupted (C₂-C₆)alkyl or halogen, R⁸ is hydrogen, (C₁-C₆)alkyl, (C₃-C₄)-alkenyl, (C₃-C₄)-alkynyl, arylmethyl, 2-(C₁-C₆)alkoxyl-2-oxoethyl, 2-(arylmethyl)-2-oxoethyl, cyano(C₁-C₆)alkyl, O-interrupted (C₁-C₆)alkyl or S-interrupted (C₁-C₆)alkyl
and
Y is carbonyl.

5. Dyes according to claim 1 having formula (I-c) 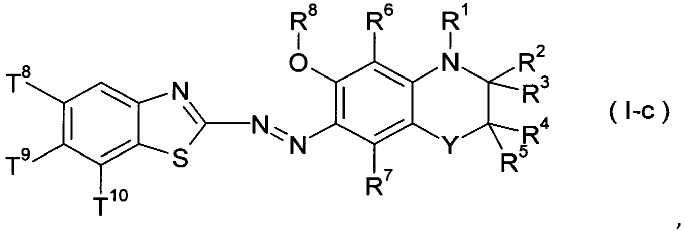 wherein
T⁸ and T¹⁰ independent from each other is hydrogen, chlorine or bromine,
T⁹ is hydrogen, (C₁-C₆)-alkylsulfonyl, thiocyano, (C₁-C₆)alkoxy, chlorine or nitro,
R¹ is methyl, ethyl, propyl, isopropyl, *n*-butyl, isobutyl, benzyl, 2-methoxylethyl, 2-ethoxylethyl, 2-acetoxyethyl, 2-(propionyl)oxyethyl, 2-(benzoyl)oxyethyl, cyanomethyl, 2-cyanoethyl, 3-cyanopropyl, 3-(C₁-C₆)alkoxyl-3-oxopropyl, 3-[O-interrupted (C₂-C₆)-alkoxyl]-3-oxopropyl, 2-(C₁-C₆)alkyl-2-oxoethyl, 2-[O-interrupted (C₂-C₆)-alkoxyl] -2-oxoethyl, 2-(cyanomethoxy)-2-oxoethyl, 3 -(cyanomethoxy)-3-oxopropyl, 2-[(arylacyl)methoxy]-2-oxoethyl, 3-[(arylacyl)methoxy]-3-oxopropyl, 3-(arylmethoxy)-3-oxopropyl, 3-{[(C₁-C₆)alkylacyl]-methoxyl} -3-oxopropyl, 3-[(arylacyl)-methoxy]-3-oxopropyl, 3-{[(C₁-C₆)-alkoxycabonyl]methoxy}-3-oxopropyl, 2-{[(C₁-C₆)-alkoxycabonyl]methoxy}-2-oxoethyl or 2-{[(C₁-C₆)alkylacyl]-methoxyl}-2-oxoethyl,
R² to R⁷ independent from each other is hydrogen, (C₁-C₆)alkyl, aryl, (C₁-C₈)alkoxyl, (C₃-C₈)cycloalkyl, O-interrupted (C₂-C₆)alkyl, S-interrupted (C₂-C₆)alkyl orhalogen, R⁸ is hydrogen, (C₁-C₆)alkyl, (C₃-C₄)-alkenyl, (C₃-C₄)-alkynyl, arylmethyl, 2-(C₁-C₆)alkoxyl-2-oxoethyl, 2-(arylmethyl)-2-oxoethyl, cyano(C₁-C₆)alkyl, O-interrupted (C₁-C₆)alkyl or S-interrupted (C₁-C₆)alkyl
and
Y is carbonyl.

6. Dyes according to claim 1 having formula (I-d) 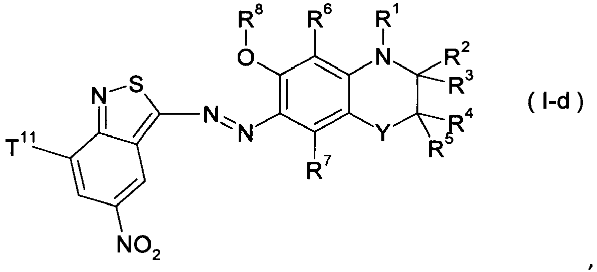 wherein
T¹¹ is hydrogen, nitro or bromine,
R¹ is methyl, ethyl, propyl, isopropyl, *n*-butyl, isobutyl, benzyl, 2-methoxylethyl, 2-ethoxylethyl, 2-acetoxyethyl, 2-(propionyl)oxyethyl, 2-(benzoyl)oxyethyl, cyanomethyl, 2-cyanoethyl, 3-cyanopropyl, 3-(C₁-C₆)alkoxyl-3-oxopropyl, 3-[O-interrupted (C₂-C₆)-alkoxyl]-3-oxopropyl, 2-(C₁-C₆)alkyl-2-oxoethyl, 2-[O-interrupted (C₂-C₆)-alkoxyl]-2-oxoethyl, 2-(cyanomethoxy)-2-oxoethyl, 3-(cyanomethoxy)-3-oxopropyl, 2-[(arylacyl)methoxy]-2-oxoethyl, 3-[(arylacyl)methoxy]-3-oxopropyl, 3-(arylmethoxy)-3-oxopropyl, 3-{[(C₁-C₆)alkylacyl]-methoxyl}-3-oxopropyl, 3-[(arylacyl)-methoxy]-3-oxopropyl, 3-{[(C₁-C₆)-alkoxycabonyl]methoxy}-3-oxopropyl, 2-{[(C₁-C₆)-alkoxycabonyl]methoxy}-2-oxoethyl or 2-{[(C₁-C₆)alkylacyl]-methoxyl} 2-oxoethyl,
R² to R⁷ independent from each other is hydrogen, (C₁-C₆)alkyl, aryl, (C₁-C₈)alkoxyl, (C₃-C₈)cycloalkyl, O-interrupted (C₂-C₆)alkyl, S-interrupted (C₂-C₆)alkyl or halogen, R⁸ is hydrogen, (C₁-C₆)alkyl, (C₃-C₄)-alkenyl, (C₃-C₄)-alkynyl, arylmethyl, 2-(C₁-C₆)alkoxyl-2-oxoethyl, 2-(arylmethyl)-2-oxoethyl, cyano(C₁-C₆)alkyl, O-interrupted (C₁-C₆)alkyl or S-interrupted (C₁-C₆)alkyl
and
Y is carbonyl.

7. Dyes according to claim 1 having formula (I-e) 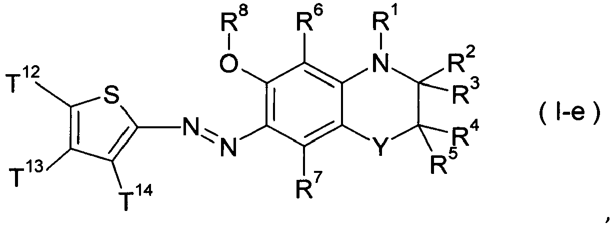 wherein
T¹² and T¹⁴ independent from each other, are nitro, cyano, formyl, acetyl or (C₁-C₆)-alkoxycarbonyl,
T¹³ is hydrogen, aryl, (C₁-C₆)-akyl or halogen,
R¹ is methyl, ethyl, propyl, isopropyl, *n*-butyl, isobutyl, benzyl, 2-methoxylethyl, 2-ethoxylethyl, 2-acetoxyethyl, 2-(propionyl)oxyethyl, 2-(benzoyl)oxyethyl, cyanomethyl, 2-cyanoethyl, 3-cyanopropyl, 3-(C₁-C₆)alkoxyl-3-oxopropyl, 3-[O-interrupted (C₂-C₆)-alkoxyl]-3-oxopropyl, 2-(C₁-C₆)alkyl-2-oxoethyl, 2-[O-interrupted (C₂-C₆)-alkoxyl]-2-oxoethyl, 2-(cyanomethoxy)-2-oxoethyl, 3-(cyanomethoxy)-3-oxopropyl, 2-[(arylacyl)methoxy]-2-oxoethyl, 3-[(arylacyl)methoxy]-3-oxopropyl, 3-(arylmethoxy)-3-oxopropyl, 3-{[(C₁-C₆)alkylacyl]-methoxyl}-3-oxopropyl, 3-[(arylacyl)-methoxy]-3-oxopropyl, 3-{[(C₁-C₆)-alkoxycabonyl]methoxy}-3-oxopropyl, 2-{[(C₁-C₆)-alkoxycabonyl]methoxy}-2-oxoethyl or 2-{[(C₁-C₆)alkylacyl]-methoxyl}-2-oxoethyl,
R² to R⁷ independent from each other is hydrogen, (C₁-C₆)alkyl, aryl, (C₁-C₈)alkoxyl, (C₃-C₈)cycloalkyl, O-interrupted (C₂-C₆)alkyl, S-interrupted (C₂-C₆)alkyl or halogen, R⁸ is hydrogen, (C₁-C₆)alkyl, (C₃-C₄)-alkenyl, (C₃-C₄)-alkynyl, arylmethyl, 2-(C₁-C₆)alkoxyl-2-oxoethyl, 2-(arylmethyl)-2-oxoethyl, cyano(C₁-C₆)alkyl, O-interrupted (C₁-C₆)alkyl or S-interrupted (C₁-C₆)alkyl
and
Y is carbonyl.

8. Dyes according to claim 1 having formula (I-f) 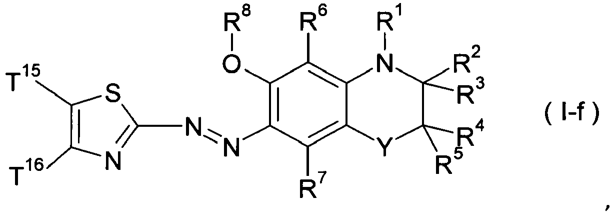 wherein
T¹⁵ and T¹⁶ independent from each other is hydrogen, halogen, (C₁-C₆)-alkyl, nitro, cyano, formyl, acetyl, (C₁-C₆)-alkoxycarbonyl, O-interrupted (C₁-C₆)-alkyl, S-interrupted (C₁-C₆)-alkyl or aryl,
R¹ is methyl, ethyl, propyl, isopropyl, *n*-butyl, isobutyl, benzyl, 2-methoxylethyl, 2-ethoxylethyl, 2-acetoxyethyl, 2-(propionyl)oxyethyl, 2-(benzoyl)oxyethyl, cyanomethyl, 2-cyanoethyl, 3-cyanopropyl, 3-(C₁-C₆)alkoxyl-3-oxopropyl, 3-[O-interrupted (C₂-C₆)-alkoxyl]-3-oxopropyl, 2-(C₁-C₆)alkyl-2-oxoethyl, 2-[O-interrupted (C₂-C₆)-alkoxyl]-2-oxoethyl, 2-(cyanomethoxy)-2-oxoethyl, 3-(cyanomethoxy)-3-oxopropyl, 2-[(arylacyl)methoxy]-2-oxoethyl, 3-[(arylacyl)methoxy]-3-oxopropyl, 3-(arylmethoxy)-3-oxopropyl, 3-{[(C₁-C₆)alkylacyl]-methoxyl}-3-oxopropyl, 3-[(arylacyl)-methoxy]-3-oxopropyl, 3-{[(C₁-C₆)-alkoxycabonyl]methoxy}-3-oxopropyl, 2-{[(C₁-C₆)-alkoxycabonyl]methoxy}-2-oxoethyl or 2-{[(C₁-C₆)alkylacyl]-methoxyl}-2-oxoethyl,
R² to R⁷ independent from each other is hydrogen, (C₁-C₆)alkyl, aryl, (C₁-C₈)alkoxyl, (C₃-C₈)cycloalkyl, O-interrupted (C₂-C₆)alkyl, S-interrupted (C₂-C₆)alkyl or halogen, R⁸ is hydrogen, (C₁-C₆)alkyl, (C₃-C₄)-alkenyl, (C₃-C₄)-alkynyl, arylmethyl, 2-(C₁-C₆)alkoxyl-2-oxoethyl, 2-(arylmethyl)-2-oxoethyl, cyano(C₁-C₆)alkyl, O-interrupted (C₁-C₆)alkyl or S-interrupted (C₁-C₆)alkyl
and
Y is carbonyl.

9. Dyes according to claim 1 having formula (I-g) 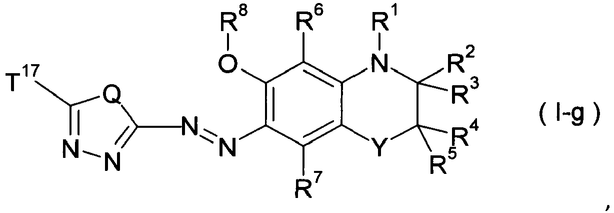 wherein
T¹⁷ is aryl or (C₁-C₆)-thioalkoxyl,
Q is oxygen or sulphur,
R¹ is methyl, ethyl, propyl, isopropyl, *n*-butyl, isobutyl, benzyl, 2-methoxylethyl, 2-ethoxylethyl, 2-acetoxyethyl, 2-(propionyl)oxyethyl, 2-(benzoyl)oxyethyl, cyanomethyl, 2-cyanoethyl, 3-cyanopropyl, 3-(C₁-C₆)alkoxyl-3-oxopropyl, 3-[O-interrupted (C₂-C₆)-alkoxyl]-3-oxopropyl, 2-(C₁-C₆)alkyl-2-oxoethyl, 2-[O-interrupted (C₂-C₆)-alkoxyl]-2-oxoethyl, 2-(cyanomethoxy)-2-oxoethyl, 3-(cyanomethoxy)-3-oxopropyl, 2-[(arylacyl)methoxy]-2-oxoethyl, 3-[(arylacyl)methoxy] -3 -oxopropyl, 3-(arylmethoxy)-3-oxopropyl, 3-{[(C₁-C₆)alkylacyl]-methoxyl}-3-oxopropyl, 3-[(arylacyl)-methoxy]-3-oxopropyl, 3-{[(C₁-C₆)-alkoxycabonyl]methoxy}-3-oxopropyl, 2-{[(C₁-C₆)-alkoxycabonyl]methoxy}-2-oxoethyl or 2-{[(C₁-C₆)alkylacyl]-methoxyl)-2-oxoethyl,
R² to R⁷ independent from each other is hydrogen, (C₁-C₆)alkyl, aryl, (C₁-C₈)alkoxyl, (C₃-C₈)cycloalkyl, O-interrupted (C₂-C₆)alkyl, S-interrupted (C₂-C₆)alkyl, halogen, R⁸ is hydrogen, (C₁-C₆)alkyl, (C₃-C₄)-alkenyl, (C₃-C₄)-alkynyl, arylmethyl, 2-(C₁-C₆)alkoxyl-2-oxoethyl, 2-(arylmethyl)-2-oxoethyl, cyano(C₁-C₆)alkyl, O-interrupted (C₁-C₆)alkyl or S-interrupted (C₁-C₆)alkyl
and
Y is carbonyl.

10. Dyes according to claim 1 having formula (I-h) 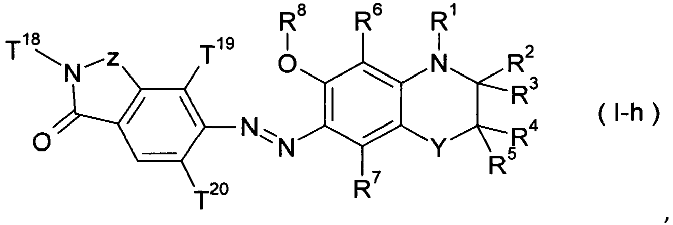 wherein
T¹⁸ is (C₁-C₆)alkyl, (C₁-C₆)-acyloxy substituted (C₁-C₆)alkyl, cyano(C₁-C₆)alkyl, arylmethyl, arylcarbonyl substituted (C₁-C₆)alkyl, 2-(C₁-C₆)alkoxyl-2-oxoethyl, O-interrupted (C₁-C₆)-alkyl, S-interrupted (C₁-C₆)-alkyl or aryl,
T¹⁹ and T²⁰ independent from each other are hydrogen, halogen, cyano, nitro or trifluoromethyl, _
Z is a carbonyl or a sulfonyl rest,
R¹ is methyl, ethyl, propyl, isopropyl, *n*-butyl, isobutyl, benzyl, 2-methoxylethyl, 2-ethoxylethyl, 2-acetoxyethyl, 2-(propionyl)oxyethyl, 2-(benzoyl)oxyethyl, cyanomethyl, 2-cyanoethyl, 3-cyanopropyl, 3-(C₁-C₆)alkoxyl-3-oxopropyl, 3-[O-interrupted (C₂-C₆)-alkoxyl]-3-oxopropyl, 2-(C₁-C₆)alkyl-2-oxoethyl, 2-[O-interrupted (C₂-C₆)-alkoxyl]-2-oxoethyl, 2-(cyanomethoxy)-2-oxoethyl, 3-(cyanomethoxy)-3-oxopropyl, 2-[(arylacyl)methoxy]-2-oxoethyl, 3-[(arylacyl)methoxy] -3-oxopropyl, 3-(arylmethoxy)-3-oxopropyl, 3-{[(C₁-C₆)alkylacyl]-methoxyl}-3-oxopropyl, 3-[(arylacyl)-methoxy]-3-oxopropyl, 3-{[(C₁-C₆)-alkoxycabonyl]methoxy}-3-oxopropyl, 2-{[(C₁-C₆)-alkoxycabonyl]methoxy}-2-oxoethyl or 2-{[(C₁-C₆)alkylacyl]-methoxyl}-2-oxoethyl,
R² to R⁷ independent from each other is hydrogen, (C₁-C₆)alkyl, aryl, (C₁-C₈)alkoxyl, (C₃-C₈)cycloalkyl, O-interrupted (C₂-C₆)alkyl, S-interrupted (C₂-C₆)alkyl or halogen,
R⁸ is hydrogen, (C₁-C₆)alkyl, (C₃-C₄)-alkenyl, (C₃-C₄)-alkynyl, arylmethyl, 2-(C₁-C₆)alkoxyl-2-oxoethyl, 2-(arylmethyl)-2-oxoethyl, cyano(C₁-C₆)alkyl, O-interrupted (C₁-C₆)alkyl or S-interrupted (C₁-C₆)alkyl
and
Y is carbonyl.

11. Dyes according to claim 1 having formula (I-i) 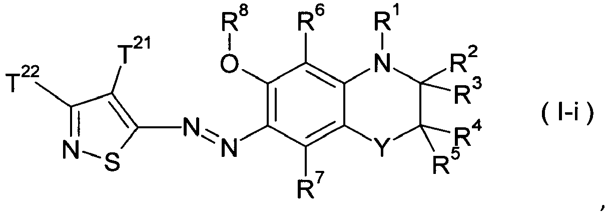 wherein
T²¹ is cyano, nitro or (C₁-C₆)-alkoxycarbonyl,
T²² is (C₁-C₆)-alkyl, hydrogen, halogen or aryl,
R¹ is methyl, ethyl, propyl, isopropyl, *n*-butyl, isobutyl, benzyl, 2-methoxylethyl, 2-ethoxylethyl, 2-acetoxyethyl, 2-(propionyl)oxyethyl, 2-(benzoyl)oxyethyl, cyanomethyl, 2-cyanoethyl, 3-cyanopropyl, 3-(C₁-C₆)alkoxyl-3-oxopropyl, 3-[O-interrupted (C₂-C₆)-alkoxyl]-3-oxopropyl, 2-(C₁-C₆)alkyl-2-oxoethyl, 2-[O-interrupted (C₂-C₆)-alkoxyl]-2-oxoethyl, 2-(cyanomethoxy)-2-oxoethyl, 3-(cyanomethoxy)-3-oxopropyl, 2-[(arylacyl)methoxy]-2-oxoethyl, 3-[(arylacyl)methoxy]-3-oxopropyl, 3-(arylmethoxy)-3-oxopropyl, 3-{[(C₁-C₆)alkylacyl]-methoxyl}-3-oxopropyl, 3-[(arylacyl)-methoxy]-3-oxopropyl, 3-{[(C₁-C₆)-alkoxycabonyl]methoxy}-3-oxopropyl, 2-{[(C₁-C₆)-alkoxycabonyl]methoxy}-2-oxoethyl or 2-{[(C₁-C₆)alkylacyl]-methoxyl}-2-oxoethyl,
R² to R⁷ independent from each other is hydrogen, (C₁-C₆)alkyl, aryl, (C₁-C₈)alkoxyl, (C₃-C₈)cycloalkyl, O-interrupted (C₂-C₆)alkyl, S-interrupted (C₂-C₆)alkyl or halogen,
R⁸ is hydrogen, (C₁-C₆)alkyl, (C₃-C₄)-alkenyl, (C₃-C₄)-alkynyl, arylmethyl, 2-(Ci-C₆)alkoxyl-2-oxoethyl, 2-(arylmethyl)-2-oxoethyl, cyano(C₁-C₆)alkyl, O-interrupted (C₁-C₆)alkyl or S-interrupted (C₁-C₆)alkyl
and
Y is carbonyl.

12. Dyes according to claim 1 having formula (I-j) 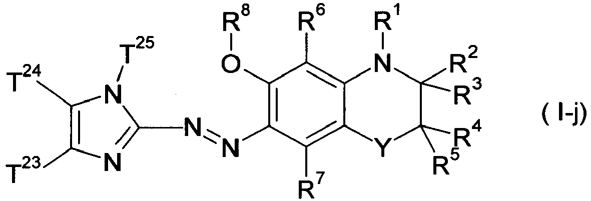 , wherein
T²³ and T²⁴ independent from each other is cyano, nitro, halogen, aryl or trifluoromethyl,
T²⁵ is hydrogen, (C₁-C₆)-alkyl, O-interrupted (C₁-C₆)-alkyl, S-interrupted (C₁-C₆)-alkyl or aryl,
R¹ is methyl, ethyl, propyl, isopropyl, *n*-butyl, isobutyl, benzyl, 2-methoxylethyl, 2-ethoxylethyl, 2-acetoxyethyl, 2-(propionyl)oxyethyl, 2-(benzoyl)oxyethyl, cyanomethyl, 2-cyanoethyl, 3-cyanopropyl, 3-(C₁-C₆)alkoxyl-3-oxopropyl, 3-[O-interrupted (C₂-C₆)-alkoxyl]-3-oxopropyl, 2-(C₁-C₆)alkyl-2-oxoethyl, 2-[O-interrupted (C₂-C₆)-alkoxyl]-2-oxoethyl, 2-(cyanomethoxy)-2-oxoethyl, 3-(cyanomethoxy)-3-oxopropyl, 2-[(arylacyl)methoxy]-2-oxoethyl, 3-[(arylacyl)methoxy]-3-oxopropyl, 3-(arylmethoxy)-3-oxopropyl, 3-{[(C₁-C₆)alkylacyl]-methoxyl}-3-oxopropyl, 3-[(arylacyl)-methoxy]-3-oxopropyl, 3-{[(C₁-C₆)-alkoxycabonyl]methoxy}-3-oxopropyl, 2-{[(C₁-C₆)-alkoxycabonyl]methoxy}-2-oxoethyl or 2-{[(C₁-C₆)alkylacyl]-methoxyl}-2-oxoethyl,
Each of R² to R⁷, independent from each other, is hydrogen, (C₁-C₆)alkyl, aryl, (C₁-C₈)alkoxyl, (C₃-C₈)cycloalkyl, O-interrupted (C₂-C₆)alkyl, S-interrupted (C₂-C₆)alkyl or halogen,
R⁸ is hydrogen, (C₁-C₆)alkyl, (C₃-C₄)-alkenyl, (C₃-C₄)-alkynyl, arylmethyl, 2-(C₁-C₆)alkoxyl-2-oxoethyl, 2-(arylmethyl)-2-oxoethyl, cyano(C₁-C₆)alkyl, O-interrupted (C₁-C₆)alkyl or S-interrupted (C₁-C₆)alkyl
and
Y is carbonyl.

13. Dyes according to claim 1 having formula (I-k) 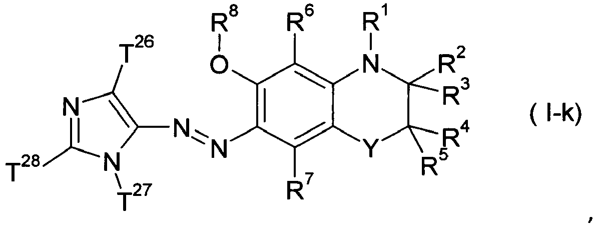 wherein
T²⁶ is cyano, nitro, aryl or trifluoromethyl,
T²⁷ is hydrogen, aryl or (C₁-C₆)-alkyl,
T²⁸ is hydrogen, (C₁-C₆)-acyloxy substituted (C₁-C₆)alkyl, cyano(C₁-C₆)alkyl, aryl, O-interrupted (C₁-C₆)-alkyl or S-interrupted (C₁-C₆)-alkyl,
R¹ is methyl, ethyl, propyl, isopropyl, *n*-butyl, isobutyl, benzyl, 2-methoxylethyl, 2-ethoxylethyl, 2-acetoxyethyl, 2-(propionyl)oxyethyl, 2-(benzoyl)oxyethyl, cyanomethyl, 2-cyanoethyl, 3-cyanopropyl, 3-(C₁-C₆)alkoxyl-3-oxopropyl, 3-[O-interrupted (C₂-C₆)-alkoxyl]-3-oxopropyl, 2-(C₁-C₆)alkyl-2-oxoethyl, 2-[O-interrupted (C₂-C₆)-alkoxyl]-2-oxoethyl, 2-(cyanomethoxy)-2-oxoethyl, 3-(cyanomethoxy)-3-oxopropyl, 2-[(arylacyl)methoxy]-2-oxoethyl, 3-[(arylacyl)methoxy]-3-oxopropyl, 3-(arylmethoxy)-3-oxopropyl, 3-{[(C₁-C₆)alkylacyl]-methoxyl}-3-oxopropyl, 3-[(arylacyl)-methoxy]-3-oxopropyl, 3-([(C₁-C₆)-alkoxycabonyl]methoxyl-3-oxopropyl, 2-{[(C₁-C₆)-alkoxycabonyl]methoxy}-2-oxoethyl or 2-{[(C₁-C₆)alkylacyl]-methoxyl}-2-oxoethyl,
R² to R⁷ independent from each other is hydrogen, (C₁-C₆)alkyl, aryl, (C₁-C₈)alkoxyl, (C₃-C₈)cycloalkyl, O-interrupted (C₂-C₆)alkyl, S-interrupted (C₂-C₆)alkyl or halogen,
R⁸ is hydrogen, (C₁-C₆)alkyl, (C₃-C₄)-alkenyl, (C₃-C₄)-alkynyl, arylmethyl, 2-(C₁-C₆)alkoxyl-2-oxoethyl, 2-(arylmethyl)-2-oxoethyl, cyano(C₁-C₆)alkyl, O-interrupted (C₁-C₆)alkyl or S-interrupted (C₁-C₆)alkyl
and
Y is carbonyl.

14. Dyes according to claim 1 having formula (1-1) 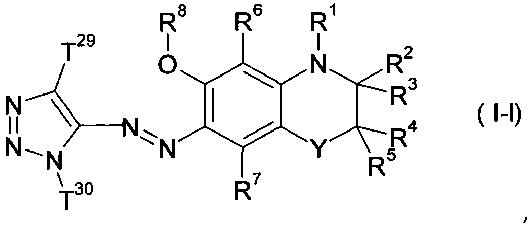 wherein
T²⁹ is cyano, nitro, aryl or trifluoromethyl,
T³⁰ is hydrogen, (C₁-C₆)-alkyl, cyano(C₁-C₆)alkyl, O-interrupted (C₁-C₆)alkyl, S-interrupted (C₁-C₆)alkyl, aryl, benzyl, (C₁-C₆)-acyloxy substituted (C₁-C₆)alkyl or cyano(C₁-C₆)alkyl,
R¹ is methyl, ethyl, propyl, isopropyl, *n*-butyl, isobutyl, benzyl, 2-methoxylethyl, 2-ethoxylethyl, 2-acetoxyethyl, 2-(propionyl)oxyethyl, 2-(benzoyl)oxyethyl, cyanomethyl, 2-cyanoethyl, 3-cyanopropyl, 3-(C₁-C₆)alkoxyl-3-oxopropyl, 3-[O-interrupted (C₂-C₆)-alkoxyl]-3-oxopropyl, 2-(C₁-C₆)alkyl-2-oxoethyl, 2-[O-interrupted (C₂-C₆)-alkoxyl]-2-oxoethyl, 2-(cyanomethoxy)-2-oxoethyl, 3-(cyanomethoxy)-3-oxopropyl, 2-[(arylacyl)methoxy]-2-oxoethyl, 3-[(arylacyl)methoxy]-3-oxopropyl, 3-(arylmethoxy)-3-oxopropyl, 3-{[(C₁-C₆)alkylacyl]-methoxyl}-3-oxopropyl, 3-[(arylacyl)-methoxy]-3-oxopropyl, 3-{[(C₁-C₆)-alkoxycabonyl]methoxy}-3-oxopropyl, 2-{[(C₁-C₆)-alkoxycabonyl]methoxy}-2-oxoethyl or 2-{[(C₁-C₆)alkylacyl]-methoxyl}-2-oxoethyl,
R² to R⁷ independent from each other is hydrogen, (C₁-C₆)alkyl, aryl, (C₁-C₈)alkoxyl, (C₃-C₈)cycloalkyl, O-interrupted (C₂-C₆)alkyl, S-interrupted (C₂-C₆)alkyl or halogen,
R⁸ is hydrogen, (C₁-C₆)alkyl, (C₃-C₄)-alkenyl, (C₃-C₄)-alkynyl, arylmethyl, 2-(C₁-C₆)alkoxyl-2-oxoethyl, 2-(arylmethyl)-2-oxoethyl, cyano(C₁-C₆)alkyl, O-interrupted (C₁-C₆)alkyl or S-interrupted (C₁-C₆)alkyl
and
Y is carbonyl.

15. Dyes according to claim 1 having formula (I-m) 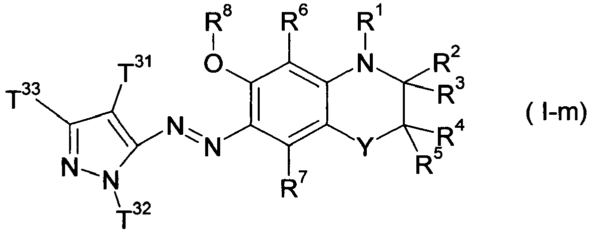 wherein
T³¹ is cyano, nitro, aryl or trifluoromethyl,
T³² is hydrogen, (C₁-C₆)-alkyl, O-interrupted (C₁-C₆)-alkyl, S-interrupted (C₁-C₆)-alkyl, cyano(C₁-C₆)alkyl, arylmethyl, cyano(C₁-C₆)alky or (C₁-C₆)-acyloxy substituted (C₁-C₆)alkyl,
T³³ is hydrogen or (C₁-C₆)alkyl,
R¹ is methyl, ethyl, propyl, isopropyl, *n*-butyl, isobutyl, benzyl, 2-methoxylethyl, 2-ethoxylethyl, 2-acetoxyethyl, 2-(propionyl)oxyethyl, 2-(benzoyl)oxyethyl, cyanomethyl, 2-cyanoethyl, 3-cyanopropyl, 3-(C₁-C₆)alkoxyl-3-oxopropyl, 3-[O-interrupted (C₂-C₆)-alkoxyl]-3-oxopropyl, 2-(C₁-C₆)alkyl-2-oxoethyl, 2-[O-interrupted (C₂-C₆)-alkoxyl]-2-oxoethyl, 2-(cyanomethoxy)-2-oxoethyl, 3-(cyanomethoxy)-3-oxopropyl, 2-[(arylacyl)methoxy]-2-oxoethyl, 3-[(arylacyl)methoxy] -3 -oxopropyl, 3-(arylmethoxy)-3-oxopropyl, 3-{[(C₁-C6)alkylacyl]-methoxyl}-3-oxopropyl, 3-[(arylacyl)-methoxy]-3-oxopropyl, 3-{[(C₁-C₆)-alkoxycabonyl]methoxy}-3-oxopropyl, 2-{[(C₁-C₆)-alkoxycabonyl]methoxy}-2-oxoethyl or 2-{[(C₁-C₆)alkylacyl]-methoxyl}-2-oxoethyl,
R² to R⁷ independent from each other is hydrogen, (C₁-C₆)alkyl, aryl, (C₁-C₈)alkoxyl, (C₃-C₈)cycloalkyl, O-interrupted (C₂-C₆)alkyl, S-interrupted (C₂-C₆)alkyl or halogen,
R⁸ is hydrogen, (C₁-C₆)alkyl, (C₃-C₄)-alkenyl, (C₃-C₄)-alkynyl, arylmethyl, 2-(C₁-C₆)alkoxyl-2-oxoethyl, 2-(arylmethyl)-2-oxoethyl, cyano(C₁-C₆)alkyl, O-interrupted (C₁-C₆)alkyl or S-interrupted (C₁-C₆)alkyl
and
Y is carbonyl.

16. Dyes according to claim 1 having formula (I-n) 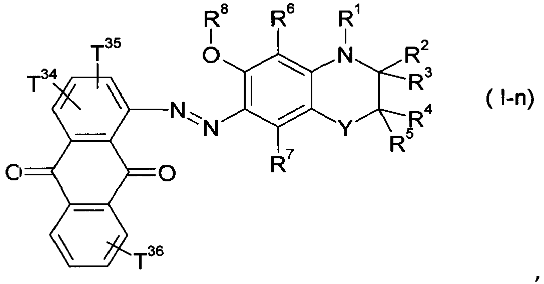 wherein
T³⁴, T³⁵ and T³⁶ independent from each other is hydrogen, halogen, nitro, cyano, hydroxyl, (C₁-C₆)-alkoxylcarbonyl, (C₁-C₆)-alkyl or acyl,
R¹ is methyl, ethyl, propyl, isopropyl, *n*-butyl, isobutyl, benzyl, 2-methoxylethyl, 2-ethoxylethyl, 2-acetoxyethyl, 2-(propionyl)oxyethyl, 2-(benzoyl)oxyethyl, cyanomethyl, 2-cyanoethyl, 3-cyanopropyl, 3-(C₁-C₆)alkoxyl-3-oxopropyl, 3-[O-interrupted (C₂-C₆)-alkoxyl]-3-oxopropyl, 2-(C₁-C₆)alkyl-2-oxoethyl, 2-[O-interrupted (C₂-C₆)-alkoxyl]-2-oxoethyl, 2-(cyanomethoxy)-2-oxoethyl, 3-(cyanomethoxy)-3-oxopropyl, 2-[(arylacyl)methoxy]-2-oxoethyl, 3-[(arylacyl)methoxy]-3-oxopropyl, 3-(arylmethoxy)-3-oxopropyl, 3-{[(C₁-C₆)alkylacyl]-methoxyl}-3-oxopropyl, 3-[(arylacyl)-methoxy]-3-oxopropyl, 3-{[(C₁-C₆)-alkoxycabonyl]methoxy}-3-oxopropyl, 2-{[(C₁-C₆)-alkoxycabonyl]methoxy}-2-oxoethyl or 2-{[(C₁-C₆)alkylacyl]-methoxyl}-2-oxoethyl,
R² to R⁷ independent from each other is hydrogen, (C₁-C₆)alkyl, aryl, (C₁-C₈)alkoxyl, (C₃-C₈)cycloalkyl, O-interrupted (C₂-C₆)alkyl, S-interrupted (C₂-C₆)alkyl or halogen, R⁸ is hydrogen, (C₁-C₆)alkyl, (C₃-C₄)-alkenyl, (C₃-C₄)-alkynyl, arylmethyl, 2-(Ci-C₆)alkoxyl-2-oxoethyl, 2-(arylmethyl)-2-oxoethyl, cyano(C₁-C₆)alkyl, O-interrupted (C₁-C₆)alkyl or S-interrupted (C₁-C₆)alkyl
and
Y is carbonyl.

17. Dyes according to claim 1 having formula (I-o) 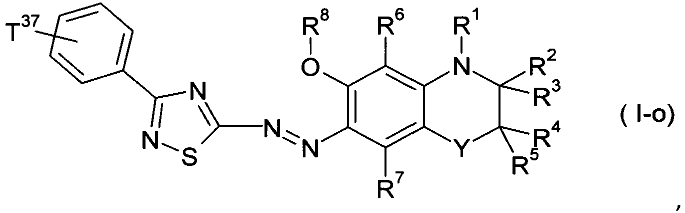 wherein
T³⁷ is nitro, hydrogen or halogen,
R¹ is methyl, ethyl, propyl, isopropyl, *n*-butyl, isobutyl, benzyl, 2-methoxylethyl, 2-ethoxylethyl, 2-acetoxyethyl, 2-(propionyl)oxyethyl, 2-(benzoyl)oxyethyl, cyanomethyl, 2-cyanoethyl, 3-cyanopropyl, 3-(C₁-C₆)alkoxyl-3-oxopropyl, 3-[O-interrupted (C₂-C₆)-alkoxyl]-3-oxopropyl, 2-(C₁-C₆)alkyl-2-oxoethyl, 2-[O-interrupted (C₂-C₆)-alkoxyl]-2-oxoethyl, 2-(cyanomethoxy)-2-oxoethyl, 3-(cyanomethoxy)-3-oxopropyl, 2-[(arylacyl)methoxy]-2-oxoethyl, 3-[(arylacyl)methoxy]-3-oxopropyl, 3-(arylmethoxy)-3-oxopropyl, 3-{[(C₁-C6)alkylacyl]-methoxyl}-3-oxopropyl, 3-[(arylacyl)-methoxy]-3-oxopropyl, 3-{[(C₁-C6)-alkoxycabonyl]methoxy}-3-oxopropyl, 2-{[(C₁-C₆)-alkoxycabonyl]methoxy}-2-oxoethyl or 2-{[(C₁-C₆)alkylacyl]-methoxyl)-2-oxoethyl,
R² to R⁷ independent from each other is hydrogen, (C₁-C₆)alkyl, aryl, (C₁-C₈)alkoxyl, (C₃-C₈)cycloalkyl, O-interrupted (C₂-C₆)alkyl, S-interrupted (C₂-C₆)alkyl or halogen,
R⁸ is hydrogen, (C₁-C₆)alkyl, (C₃-C₄)-alkenyl, (C₃-C₄)-alkynyl, arylmethyl, 2-(C₁-C₆)alkoxyl-2-oxoethyl, 2-(arylmethyl)-2-oxoethyl, cyano(C₁-C₆)alkyl, O-interrupted (C₁-C₆)alkyl or S-interrupted (C₁-C₆)alkyl
and
Y is carbonyl.

18. Process for the production of dyes having formula (I) and mixtures thereof, comprising
a) diazotization of a compound of the general formula (XD)
D-NH₂ (XD),
wherein
D is as defined in claim 1, and
b) reacting the obtained corresponding diazonium salt with a compound of formula (XC) 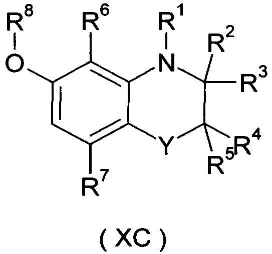 wherein R¹ to R⁸ and Y are each as defined in claim 1.

19. Use of the dyes according to claim 1 for dyeing and printing hydrophobic materials.

## Patentansprüche

1. Farbstoffe der Formel (I) und Mischungen davon: 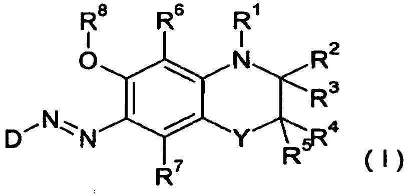 wobei
Y für eine Gruppe der allgemeinen Formel (II) 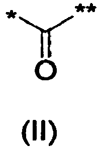 steht,
R¹ für Alkyl, Alkenyl, Cycloalkyl, Aryl, Heteroaryl, Heterocycloalkyl
oder
Alkyl, das durch ein oder mehrere Heteroatome aus der Gruppe bestehend aus Sauerstoff und Schwefel unterbrochen ist,
oder
Alkyl, das durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N*-Monoarylamino, *N,N*-Diarylamino, *N*-Alkyl-*N*-arylamino, *N*-Monocycloalkylamino, *N,N*-Dicycloalkylamino, *N*-Monoalkylmonocycloalkylamino, *N,N*-Monoarylmonocycloalkylamino, *N*-Acylamino, *N*-Alkylsulfonylamino, Halogen, Cyano, Thiocyano, Nitro, Acyl, Thioacyl, Alkylsulfonyl, Aryloyl, Trifluormethyl, Heteroaryl, Heterocycloalkyl, Alkoxythiocarbonyl, Acyloxy, Aryloyloxy, Carbamoyl, *N-*Monocycloalkylcarbamoyl, *N*-Monoalkylcarbamoyl, *N,N*-Dicycloalkylcarbamoyl, *N,N*-Dialkylcarbamoyl, N-Monoarylcarbamoyl, *N,N*-Diarylcarbamoyl, *N-*Monocycloalkyl-*N*-monoarylcarbamoyl, *N*-Monoalkyl-*N-*monoarylcarbamoyl, Sulfamoyl, *N-*Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N-*Monocycloalkyl-*N*-monoarylsulfamoyl, *N*-Monoalkyl-*N-*monoarylsulfamoyl, Arylsulfonyloxy, Alkylsulfonyloxy, Aryloxysulfonyl, Alkoxylsulfonyl und COOR¹² substituiert ist,
oder
Alkyl, das durch ein oder mehrere Heteroatome aus der Gruppe bestehend aus Sauerstoff und Schwefel unterbrochen ist und durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N-*Monoarylamino, *N,N*-Diarylamino, *N*-Alkyl-*N-*arylamino, *N*-Monocycloalkylamino, *N,N-*Dicycloalkylamino, *N*-Monoalkylmonocycloalkylamino, *N,N*-Monoarylmonocycloalkylamino, *N*-Acylamino, *N-*Alkylsulfonylamino, Halogen, Cyano, Thiocyano, Nitro, Acyl, Thioacyl, Alkylsulfonyl, Aryloyl, Trifluormethyl, Heteroaryl, Heterocycloalkyl, Alkoxythiocarbonyl, Acyloxy, Aryloyloxy, Carbamoyl, *N*-Monocycloalkylcarbamoyl, *N-*Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-*N*-monoarylcarbamoyl, *N*-Monoalkyl-*N*-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-N-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
oder
Aryl, das durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N*-Monoarylamino, *N,N*-Diarylamino, *N*-Alkyl-*N*-arylamino, *N*-Monocycloalkylamino, *N,N*-Dicycloalkylamino, *N*-Monoalkylmonocycloalkylamino, *N,N*-Monoarylmonocycloalkylamino, *N*-Acylamino, *N*-Alkylsulfonylamino, Thiocyano, Halogen, Cyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N-*Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-*N*-monoarylcarbamoyl, *N*-Monoalkyl-N-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-N-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
steht,
R²-bis R⁹ unabhängig voneinander für Wasserstoff, Alkyl, Aryl, Alkoxyl, Cycloalkyl, Halogen
oder
Alkyl, das durch ein oder mehrere Heteroatome aus der Gruppe bestehend aus Sauerstoff und Schwefel unterbrochen ist,
oder
Alkyl, das durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N*-Monoarylamino, *N,N*-Diarylamino, *N*-Alkyl-*N*-arylamino, *N*-Monocycloalkylamino, *N,N*-Dicycloalkylamino, *N*-Monoalkylmonocycloalkylamino, *N,N*-Monoarylmonocycloalkylamino, *N*-Acylamino, *N*-Alkylsulfonylamino, Halogen, Cyano, Thiocyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N-*Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-N-monoarylcarbamoyl, *N*-Monoalkyl-*N*-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-*N*-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
oder
Alkyl, das durch ein oder mehrere Heteroatome aus der Gruppe bestehend aus Sauerstoff und Schwefel unterbrochen ist und durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N-*Monoarylamino, *N,N*-Diarylamino, *N*-Alkyl-*N-*arylamino, *N*-Monocycloalkylamino, *N,N-*Dicycloalkylamino, *N*-Monoalkylmonocycloalkylamino, *N,N*-Monoarylmonocycloalkylamino, *N*-Acylamino, *N-*Alkylsulfonylamino, Halogen, Cyano, Thiocyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N*-Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-*N*-monoarylcarbamoyl, *N*-Monoalkyl-N-monoarylcarbamoyl, Sulfamoyl, *N*-Monocycloalkylsulfamoyl, *N*-Monoalkylsulfamoyl, *N*,N-Dicycloalkylsulfamoyl, *N,N*-Dialkylsulfamoyl, *N*-Monoarylsulfamoyl, *N,N*-Diarylsulfamoyl, *N*-Monocycloalkyl-N-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
oder
Aryl, das durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N*-Monoarylamino, *N,N*-Diarylamino, *N*-Alkyl-*N*-arylamino, *N*-Monocycloalkylamino, *N,N*-Dicycloalkylamino, *N*-Monoalkylmonocycloalkylamino, *N,N*-Monoarylmonocycloalkylamino, N-Acylamino, *N*-Alkylsulfonylamino, Thiocyano, Halogen, Cyano, Nitro, Acyl, Thioacyl, Alkylsulfonyl, Aryloyl, Trifluormethyl, - Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N-*Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-N-monoarylcarbamoyl, *N*-Monoalkyl-*N*-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N*,*N*-Diarylsulfamoyl, *N*-Monocycloalkyl-N-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
stehen,
D für eine Gruppe der Formel (VI) 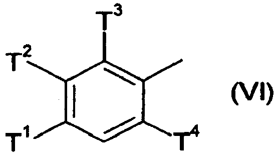 steht, wobei
T¹ bis T⁴ unabhängig voneinander für Wasserstoff, Alkyl, Cycloalkyl, Alkenyl, Alkinyl, Aryl, Alkoxyl, Halogen, Cyano, Nitro, Acyl, Aryloyl, Arylsulfonyl, Alkylsulfonyl, *N*-Monoalkylsulfamoyl, *N,N*-Dialkylsulfamoyl, Alkoxylcarbonyl, Aryloxycarbonyl, *N*-Monoalkylcarbamoyl, *N,N-*Dialkylcarbamoyl, Aryloyloxy, Acyloxy, Aryloxy, Thiocyano, Hydroxyl, Arylmethoxy, Arylsulfonyloxy, Alkylsulfonyloxy, Aryloxysulfonyl,-[(Alkoxycarbonyl)methoxyl]carbonyl, -[(Aryloxycarbonyl)methoxyl]carbonyl, [(Alkylacyl)-methoxy]carbonyl, [(Arylacyl)methoxy]carbonyl, Trifluormethyl
oder
Alkyl, das durch ein oder mehrere Heteroatome aus der Gruppe bestehend aus Sauerstoff und Schwefel unterbrochen ist,
oder
Alkyl, das durch einen oder mehrere Substituenten aus der Gruppe bestehend aus Hydroxy, Aryl, Cycloalkyl, Alkoxy, Thioalkoxy, Amino, *N*-Monoalkylamino, - *N,N*-Dialkylamino, *N*-Monoarylamino, *N,N*-Diarylamino, *N*-Alkyl-*N*-arylamino, *N*-Monocycloalkylamino, *N,N*-Dicycloalkylamino, *N*-Monoalkylmonocycloalkylamino, *N,N*-Monoarylmonocycloalkylamino, *N*-Acylamino, *N*-Alkylsulfonylamino, Halogen, Cyanö, Thiocyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N-*Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-*N*-monoarylcarbamoyl, *N*-Monoalkyl-*N*-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-*N*-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
oder
Alkyl, das durch ein oder mehrere Heteroatome aus der Gruppe bestehend aus Sauerstoff und Schwefel unterbrochen ist und durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N-*Monoarylamino, *N,N*-Diarylamino, *N*-Alkyl-*N-*arylamino, *N*-Monocycloalkylamino, *N,N-*Dicycloalkylamino, *N*-Monoalkylmonocycloalkylamino, *N,N*-Monoarylmonocycloalkylamino, *N*-Acylamino, N-Alkylsulfonylamino, Halogen, Cyano, Thiocyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N*-Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-*N*-monoarylcarbamoyl, *N*-Monoalkyl-*N*-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-N-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
oder
Aryl, das durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N*-Monoarylamino, *N,N*-Diarylamino, *N*-Alkyl-*N*-arylamino, *N*-Monocycloalkylamino, *N,N*-Dicycloalkylamino, *N*-Monoalkylmonocycloalkylamino, *N,N*-Monoarylmonocycloalkylamino, *N*-Acylamino, *N*-Alkylsulfonylamino, Thiocyano, Halogen, Cyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N-*Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-*N*-monoarylcarbamoyl, *N*-Monoalkyl-*N*-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-*N*-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
stehen, wobei mindestens eine der Variablen T¹, T², T³ und bis T⁴ nicht für Wasserstoff steht,
oder
D für eine Gruppe der Formel (VII) 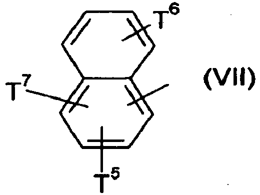 steht, wobei
T⁵ bis T⁷ unabhängig voneinander für Wasserstoff, Alkyl, Cycloalkyl, Alkenyl, Alkinyl, Aryl, Alkoxyl, Halogen, Cyano, Nitro, Acyl, Arylacyl, Alkoxycarbonyl, Aryloxycarbonyl, [(Alkoxycarbonyl)methoxyl]carbonyl, -[(Aryloxycarbonyl)methoxyl]carbonyl, [(Alkylacyl)-methoxy]carbonyl, [(Arylacyl)methoxy]carbonyl, *N-*Monoalkylcarbamoyl, *N,N*-Dialkylcarbamoyl
oder
Alkyl, das durch ein oder mehrere Heteroatome aus der Gruppe bestehend aus Sauerstoff und Schwefel unterbrochen ist,
oder
Alkyl, das durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N*-Monoarylamino, *N,N*-Diarylamino, *N*-Alkyl-*N*-arylamino, *N*-Monocycloalkylamino, *N,N*-Dicycloalkylamino, *N*-Monoalkylmonocycloalkylamino, *N,N*-Monoarylmonocycloalkylamino, *N*-Acylamino, *N*-Alkylsulfonylamino, Halogen, Cyano, Thiocyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N-*Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-*N*-monoarylcarbamoyl, *N*-Monoalkyl-*N*-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-*N*-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
oder
Alkyl, das durch ein oder mehrere Heteroatome aus der Gruppe bestehend aus Sauerstoff und Schwefel unterbrochen ist und durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N-*Monöarylamino, *N,N*-Diarylamino, *N*-Alkyl-*N-*arylamino, *N*-Monocycloalkylamino, *N,N-*Dicycloalkylamino, *N*-Monoalkylmonocycloalkylamino, *N,N*-Monoarylmonocycloalkylamino, *N*-Acylamino, *N-*Alkylsulfonylamino, Halogen, Cyano, Thiocyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N*-Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-N-monoarylcarbamoyl, *N*-Monoalkyl-N-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-*N*-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
oder
Aryl, das durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N*-Monoarylamino, *N,N*-Diarylamino, *N*-Alkyl-N-arylamino, *N*-Monocycloalkylamino, *N,N*-Dicycloalkylamino, *N*-Monoalkylmonocycloalkylamino, *N,N*-Monoarylmonocycloalkylamino, *N*-Acylamino, *N*-Alkylsulfonylamino, Thiocyano, Halogen, Cyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N-*Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-*N*-monoarylcarbamoyl, *N*-Monoalkyl-*N*-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-*N*-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
stehen, wobei mindestens eine der Variablen T⁵, T⁶ und T⁷ nicht für Wasserstoff steht,
oder
D für eine Gruppe der Formel (VIII) 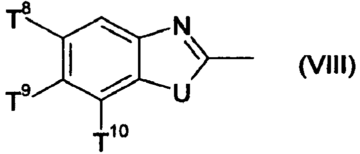 steht, wobei
U für Sauerstoff, Schwefel oder N-R¹⁵ steht,
T⁸ und T¹⁰ unabhängig voneinander für Wasserstoff, Fluor, Chlor oder Brom stehen,
T⁹ für Wasserstoff, Alkylsulfonyl, Thiocyano, Alkoxy, Halogen oder Nitro steht,
R¹⁵ für Alkyl, Aryl oder Cycloalkyl steht,
oder
D für eine Gruppe der Formel (XI) 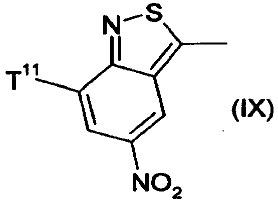 steht, wobei
T¹¹ für Wasserstoff, Nitro oder Halogen steht, oder
D für eine Gruppe der Formel (X) 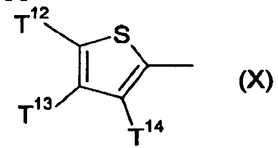 steht, wobei
T¹² und T¹⁴ unabhängig voneinander für Nitro, Cyano, Acyl oder Alkoxycarbonyl stehen,
T¹³ für Wasserstoff, Aryl, Alkyl, Halogen
oder
Alkyl, das durch ein oder mehrere Heteroatome aus der Gruppe bestehend aus Sauerstoff und Schwefel unterbrochen ist,
oder
Alkyl, das durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N*-Monoarylamino, *N,N*-Diarylamino, *N*-Alkyl-*N*-arylamino, *N*-Monocycloalkylamino, *N,N*-Dicycloalkylamino, *N*-Monoalkylmonocycloalkylamino, *N,N*-Monoarylmonocycloalkylamino, *N*-Acylamino, *N*-Alkylsulfonylamino, Halogen, Cyano, Thiocyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N-*Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-*N*-monoarylcarbamoyl, *N*-Monoalkyl-*N*-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-*N*-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
oder
Alkyl, das durch ein oder mehrere Heteroatome aus der Gruppe bestehend aus Sauerstoff und Schwefel
unterbrochen ist und durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N-*Monoarylamino, *N,N*-Diarylamino, *N*-Alkyl-N-arylamino, *N*-Monocycloalkylamino, *N,N-*Dicycloalkylamino, *N*-Monoalkylmonocycloalkylamino, *N,N*-Monoarylmonocycloalkylamino, *N*-Acylamino, *N-*Alkylsulfonylamino, Halogen, Cyano, Thiocyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Äcyloxy, Aryloyloxy, Carbamoyl, *N*-Monocycloalkylcarbamoyl, *N*-Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-*N*-monoarylcarbamoyl, *N*-Monoalkyl-N-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-N-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
oder
Aryl, das durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N*-Monoarylamino, *N,N*-Diarylamino, *N*-Alkyl-N-arylamino, *N*-Monocycloalkylamino, *N,N*-Dicycloalkylamino, *N*-Monoalkylmonocycloalkylamino, *N,N*-Monoarylmonocycloalkylamino, *N*-Acylamino, *N*-Alkylsulfonylamino, Thiocyano, Halogen, Cyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N-*Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-*N*-monoarylcarbamoyl, *N*-Monoalkyl-*N*-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-*N*-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
steht,
oder
D für eine Gruppe der Formel (XI) 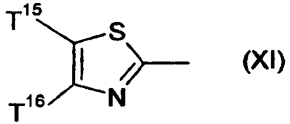 steht, wobei
T¹⁵ und T¹⁶ unabhängig voneinander für Wasserstoff, Halogen, Alkyl, Nitro, Cyano, Acyl, Alkoxycarbonyl, Aryl
oder
Alkyl, das durch ein oder mehrere Heteroatome aus der Gruppe bestehend aus Sauerstoff und Schwefel unterbrochen ist,
oder
Alkyl, das durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N*-Monoarylamino, *N,N*-Diarylamino, *N*-Alkyl-N-arylamino, *N*-Monocycloalkylamino, *N,N*-Dicycloalkylamino, *N*-Monoalkylmonocycloalkylamino, *N,N*-Monoarylmonocycloalkylamino, *N*-Acylamino, *N*-Alkylsulfonylamino, Halogen, Cyano, Thiocyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N-*Monoalkylcarbamoyl, *N,N*-Dicycloalkylcarbamoyl, N,N-Dialkylcarbamoyl, *N*-Monoarylcarbamoyl, *N,N-*Diarylcarbamoyl, N*-*Monocycloalkyl-*N*-monoarylcarbamoyl, *N*-Monoalkyl-*N*-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-N-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
oder
Alkyl, das durch ein oder mehrere Heteroatome aus der Gruppe bestehend aus Sauerstoff und Schwefel unterbrochen ist und durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N-*Monoarylamino, *N,N*-Diarylamino, *N*-Alkyl-*N-*arylamino, *N*-Monocycloalkylamino, *N,N-*Dicycloalkylamino, *N*-Monoalkylmonocycloalkylamino, *N,N*-Monoarylmonocycloalkylamino, *N*-Acylamino, *N-*Alkylsulfonylamino, Halogen, Cyano, Thiocyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N*-Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-*N*-monoarylcarbamoyl, *N*-Monoalkyl-*N*-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-N-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
oder
Aryl, das durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N*-Monoarylamino, *N*,*N*-Diarylamino, *N*-Alkyl-N-arylamino, *N*-Monocycloalkylamino, *N*,*N*-Dicycloalkylamino, *N*-Monoalkylmonocycloalkylamino, *N,N*-Monoarylmonocycloalkylamino, *N*-Acylamino, *N*-Alkylsulfonylamino, Thiocyano, Halogen, Cyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N-*Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-N-monoarylcarbamoyl, *N*-Monoalkyl-N-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-*N*-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
stehen, wobei mindestens eine der Variablen T¹⁵ und T¹⁶ nicht für Wasserstoff steht,
oder
D für eine Gruppe der Formel (XII) 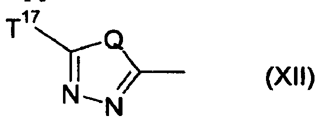 steht, wobei
T¹⁷ für Aryl, Thioalkoxyl
oder
Aryl, das durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N*-Monoarylamino, *N,N*-Diarylamino, *N*-Alkyl-N-arylamino, *N*-Monocycloalkylamino, *N,N*-Dicycloalkylamino, *N*-Monoalkylmonocycloalkylamino, *N,N*-Monoarylmonocycloalkylamino, *N*-Acylamino, *N*-Alkylsulfonylamino, Thiocyano, Halogen, Cyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N-*Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-*N*-monoarylcarbamoyl, *N*-Monoalkyl-N-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-N-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
steht,
Q für Sauerstoff oder Schwefel steht,
oder
D für eine Gruppe der Formel (XIII) 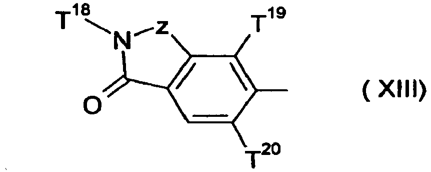 steht, wobei
T¹⁸ für Alkyl, Alkenyl, Aryl
oder
Alkyl, das durch ein oder mehrere Heteroatome aus der Gruppe bestehend aus Sauerstoff und Schwefel unterbrochen ist,
oder
Alkyl, das durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N*-Monoarylamino, *N,N*-Diarylamino, *N*-Alkyl-*N*-arylamino, *N*-Monocycloalkylamino, *N,N*-Dicycloalkylamino, *N*-Monoalkylmonocycloalkylamino, *N,N*-Monoarylmonocycloalkylamino, *N*-Acylamino, *N*-Alkylsulfonylamino, Halogen, Cyano, Thiocyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N-*Monoalkylcarbamoyl, *N,N-*Dicycloalkylcarbamoyl, *N,N*-Dialkylcarbamoyl, *N*-Monoarylcarbamoyl, *N,N-*Diarylcarbamoyl, *N*-Monocycloalkyl-*N*-monoarylcarbamoyl, *N*-Monoalkyl-*N*-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-*N*-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
oder
Alkyl, das durch ein oder mehrere Heteroatome aus der Gruppe bestehend aus Sauerstoff und Schwefel unterbrochen ist und durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N-*Monoarylamino, *N,N*-Diarylamino, *N*-Alkyl-*N-*arylamino, *N*-Monocycloalkylamino, *N,N-*Dicycloalkylamino, *N*-Monoalkylmonocycloalkylamino, *N,N*-Monoarylmonocycloalkylamino, *N*-Acylamino, *N-*Alkylsulfonylamino, Halogen, Cyano, Thiocyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N*-Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-*N*-monoarylcarbamoyl, *N*-Monoalkyl-*N*-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-*N*-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
oder
Aryl, das durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N*-Monoarylamino, *N,N*-Diarylamino, *N*-Alkyl-*N*-arylamino, *N*-Monocycloalkylamino, *N,N*-Dicycloalkylamino, *N*-Monoalkylmonocycloalkylamino, *N,N*-Monoarylmonocycloalkylamino, *N*-Acylamino, *N*-Alkylsulfonylamino, Thiocyano, Halogen, Cyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N-*Monoalkylcarbamoyl, *N,N*-Dicycloalkylcarbamoyl, *N,N*-Dialkylcarbamoyl, N-Monoarylcarbamoyl, *N,N-*Diarylcarbamoyl, *N*-Monocycloalkyl-*N*-monoarylcarbamoyl, *N*-Monoalkyl-*N*-monoarylcarbamoyl, Sulfamoyl, *N*-Monocycloalkylsulfamoyl, *N*-Monoalkylsulfamoyl, *N,N-*Dicycloalkylsulfamoyl, *N,N*-Dialkylsulfamoyl, *N*-Monoarylsulfamoyl, *N,N*-Diarylsulfamoyl, *N*-Monocycloalkyl-N-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
steht,
T¹⁹ und T²⁰ unabhängig voneinander für Wasserstoff, Halogen, Cyano, Nitro oder Trifluormethyl stehen,
Z für einen Carbonyl- oder Sulfonylrest steht, oder
D für eine Gruppe der Formel (XIV) 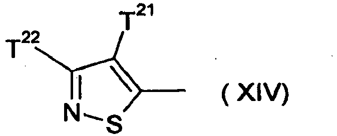 steht, wobei
T²¹ für Cyano, Nitro oder Alkoxycarbonyl steht,
T²² für Alkyl, Wasserstoff, Halogen, Aryl
oder
Alkyl, das durch ein oder mehrere Heteroatome aus der Gruppe bestehend aus Sauerstoff und Schwefel unterbrochen ist,
oder
Alkyl, das durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N*-Monoarylamino, *N,N*-Diarylamino, *N*-Alkyl-*N*-arylamino, *N*-Monocycloalkylamino, *N,N*-Dicycloalkylamino, *N*-Monoalkylmonocycloalkylamino, *N,N*-Monoarylmonocycloalkylamino, *N*-Acylamino, *N*-Alkylsulfonylamino, Halogen, Cyano, Thiocyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N-*Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-*N*-monoarylcarbamoyl., *N*-Monoalkyl-N-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-*N*-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
oder
Alkyl, das durch ein oder mehrere Heteroatome aus der Gruppe bestehend aus Sauerstoff und Schwefel unterbrochen ist und durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N-*Monoarylamino, *N,N*-Diarylamino, *N*-Alkyl-*N-*arylamino, *N*-Monocycloalkylamino, *N,N-*Dicycloalkylamino, *N*-Monoalkylmonocycloalkylamino, *N,N*-Monoarylmonocycloalkylamino, *N*-Acylamino, *N-*Alkylsulfonylamino, Halogen, Cyano, Thiocyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N*-Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-*N*-monoarylcarbamoyl, *N*-Monoalkyl-*N*-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-*N*-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
oder
Aryl, das durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N*-Monoarylamino, *N,N*-Diarylamino, *N*-Alkyl-*N*-arylamino, *N*-Monocycloalkylamino, *N,N*-Dicycloalkylamino, *N*-Monoalkylmonocycloalkylamino, *N,N*-Monoarylmonocycloalkylamino, *N*-Acylamino, *N*-Alkylsulfonylamino, Thiocyano, Halogen, Cyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N-*Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-*N*-monoarylcarbamoyl, *N*-Monoalkyl-N-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-*N*-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
steht,
oder
D für eine Gruppe der Formel (XV) 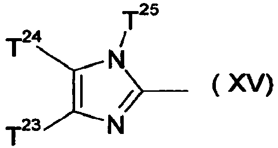 steht, wobei
T²³ und T²⁴ unabhängig voneinander für Cyano, Nitro, Halogen, Aryl oder Trifluormethyl stehen,
T²⁵ für Wasserstoff, Alkyl, Alkenyl, Aryl
oder
Alkyl, das durch ein oder mehrere Heteroatome aus der Gruppe bestehend aus Sauerstoff und Schwefel unterbrochen ist,
oder
Alkyl, das durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N*-Monoarylamino, *N,N*-Diarylamino, *N*-Alkyl-*N*-arylamino, *N*-Monocycloalkylamino, *N,N*-Dicycloalkylamino, *N*-Monoalkylmonocycloalkylamino, *N,N*-Monoarylmonocycloalkylamino, *N*-Acylamino, *N*-Alkylsulfonylamino, Halogen, Cyano, Thiocyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N-*Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-*N*-monoarylcarbamoyl, *N*-Monoalkyl-*N*-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-N-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
oder
Alkyl, das durch ein oder mehrere Heteroatome aus der Gruppe bestehend aus Sauerstoff und Schwefel unterbrochen ist und durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N-*Monoarylamino, *N,N*-Diarylamino, *N*-Alkyl-*N-*arylamino, *N*-Monocycloalkylamino, *N,N-*Dicycloalkylamino, *N*-Monoalkylmonocycloalkylamino, *N,N*-Monoarylmonocycloalkylamino, *N*-Acylamino, *N-*Alkylsulfonylamino, Halogen, Cyano, Thiocyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N*-Monoalkylcarbamoyl, *N,N*-Dicycloalkylcarbamoyl, *N,N-*Dialkylcarbamoyl, *N*-Monoarylcarbamoyl, *N,N-*Diarylcarbamoyl, *N*-Monocycloalkyl-N-monoarylcarbamoyl, *N*-Monoalkyl-N-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-*N*-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
oder
Aryl, das durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N*-Monoarylamino, *N,N*-Diarylamino, *N*-Alkyl-*N*-arylamino, *N*-Monocycloalkylamino, *N,N*-Dicycloalkylamino, *N*-Monoalkylmonocycloalkylamino, *N,N*-Monoarylmonocycloalkylamino, *N*-Acylamino, *N*-Alkylsulfonylamino, Thiocyano, Halogen, Cyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N-*Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-*N*-monoarylcarbamoyl, *N*-Monoalkyl-*N*-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-N-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
steht,
oder D für eine Gruppe der Formel (XVI) 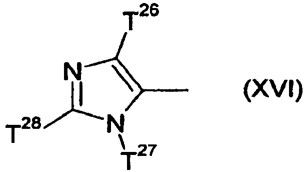 steht, wobei
T²⁶ für Cyano, Nitro, Aryl oder Trifluormethyl steht,
T²⁷ für Wasserstoff, Aryl, Alkyl
oder
Alkyl, das durch ein oder mehrere Heteroatome aus der Gruppe bestehend aus Sauerstoff und Schwefel unterbrochen ist,
oder
Alkyl, das durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N*-Monoarylamino, *N,N*-Diarylamino, *N*-Alkyl-N-arylamino, *N*-Monocycloalkylamino, *N,N*-Dicycloalkylamino, *N*-Monoalkylmonocycloalkylamino, *N,N*-Monoarylmonocycloalkylamino, *N*-Acylamino, *N*-Alkylsulfonylamino, Halogen, Cyano, Thiocyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N-*Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-*N*-monoarylcarbamoyl, *N*-Monoalkyl-*N*-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-*N*-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
oder
Alkyl, das durch ein oder mehrere Heteroatome aus der Gruppe bestehend aus Sauerstoff und Schwefel unterbrochen ist und durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N-*Monoarylamino, *N,N*-Diarylamino, *N*-Alkyl-*N-*arylamino, *N*-Monocycloalkylamino, *N,N-*Dicycloalkylamino, *N*-Monoalkylmonocycloalkylamino, *N,N*-Monoarylmonocycloalkylamino, *N*-Acylamino, *N-*Alkylsulfonylamino, Halogen, Cyano, Thiocyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N*-Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-N-monoarylcarbamoyl, *N*-Monoalkyl-N-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-*N*-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
oder
Aryl, das durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N*-Monoarylamino, *N,N*-Diarylamino, *N*-Alkyl-*N*-arylamino, *N*-Monocycloalkylamino, *N,N*-Dicycloalkylamino, *N*-Monoalkylmonocycloalkylamino, *N,N*-Monoarylmonocycloalkylamino, *N*-Acylamino, *N*-Alkylsulfonylamino, Thiocyano, Halogen, Cyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N-*Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-N-monoarylcarbamoyl, *N*-Monoalkyl-N-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-*N*-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
steht,
T²⁸ für Wasserstoff, Alkyl, Alkenyl, Alkinyl, Aryl, Alkoxycarbonyl
oder
Alkyl, das durch ein oder mehrere Heteroatome aus der Gruppe bestehend aus Sauerstoff und Schwefel unterbrochen ist,
oder
Alkyl, das durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N*-Monoarylamino, *N,N*-Diarylamino, *N*-Alkyl-*N*-arylamino, *N*-Monocycloalkylamino, *N,N*-Dicycloalkylamino, *N*-Monoalkylmonocycloalkylamino, *N,N*-Monoarylmonocycloalkylamino, *N*-Acylamino, *N*-Alkylsulfonylamino, Halogen, Cyano, Thiocyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N-*Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, N-Monocycloalkyl-*N*-monoarylcarbamoyl, N-Monoalkyl-*N*-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-*N*-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
oder
Alkyl, das durch ein oder mehrere Heteroatome aus der Gruppe bestehend aus Sauerstoff und Schwefel unterbrochen ist und durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N-*Monoarylamino, *N,N*-Diarylamino, *N*-Alkyl-*N-*arylamino, *N*-Monocycloalkylamino, *N,N-*Dicycloalkylamino, *N*-Monoalkylmonocycloalkylamino, *N,N*-Monoarylmonocycloalkylamino, *N*-Acylamino, *N-*Alkylsulfonylamino, Halogen, Cyano, Thiocyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N*-Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-N-monoarylcarbamoyl, *N*-Monoalkyl-N-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-*N*-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
oder
Aryl, das durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N*-Monoarylamino, *N,N*-Diarylamino, *N*-Alkyl-*N*-arylamino, *N*-Monocycloalkylamino, *N,N*-Dicycloalkylamino, *N*-Monoalkylmonocycloalkylamino, *N,N*-Monoarylmonocycloalkylamino, *N*-Acylamino, *N*-Alkylsulfonylamino, Thiocyano, Halogen, Cyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N-*Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-*N*-monoarylcarbamoyl, *N*-Monoalkyl-*N*-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-*N*-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
steht,
oder
D für eine Gruppe der Formel (XVII) 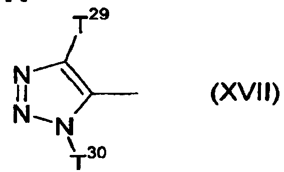 steht, wobei
T²⁹ für Cyano, Nitro, Aryl oder Trifluormethyl steht,
T³⁰ für Wasserstoff, Alkyl, Alkenyl, Alkenyl, Alkinyl, Aryl, Arylmethyl, Alkoxycarbonyl
oder
Alkyl, das durch ein oder mehrere Heteroatome aus der Gruppe bestehend aus Sauerstoff und Schwefel unterbrochen ist,
oder
Alkyl, das durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N*-Monoarylamino, *N,N*-Diarylamino, *N*-Alkyl-*N*-arylamino, *N*-Monocycloalkylamino, *N,N*-Dicycloalkylamino, *N*-Monoalkylmonocycloalkylamino, *N,N*-Monoarylmonocycloalkylamino, *N*-Acylamino, *N*-Alkylsulfonylamino, Halogen, Cyano, Thiocyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, -Carbamoyl, *N*-Monocycloalkylcarbamoyl, *N-*Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-*N*-monoarylcarbamoyl, *N*-Monoalkyl-*N*-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-*N*-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
oder
Alkyl, das durch ein oder mehrere Heteroatome aus der Gruppe bestehend aus Sauerstoff und Schwefel unterbrochen ist und durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N-*Monoarylamino, *N,N*-Diarylamino, *N*-Alkyl-*N-*arylamino, *N*-Monocycloalkylamino, *N,N-*Dicycloalkylamino, *N*-Monoalkylmonocycloalkylamino, *N,N*-Monoarylmonocycloalkylamino, *N*-Acylamino, *N-*Alkylsulfonylamino, Halogen, Cyano, Thiocyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N*-Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-*N*-monoarylcarbamoyl, *N*-Monoalkyl-N-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N-*Diarylsulfamoyl, *N*-Monocycloalkyl-N-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
oder
Aryl, das durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N*-Monoarylamino, *N,N*-Diarylamino, *N*-Alkyl-*N*-arylamino, *N*-Monocycloalkylamino, *N,N*-Dicycloalkylamino, *N*-Monoalkylmonocycloalkylamino, *N,N*-Monoarylmonocycloalkylamino, *N*-Acylamino, *N*-Alkylsulfonylamino, Thiocyano, Halogen, Cyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N-*Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-*N*-monoarylcarbamoyl, *N*-Monoalkyl-*N*-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-*N*-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
steht,
oder
D für eine Gruppe der Formel (XVIII) 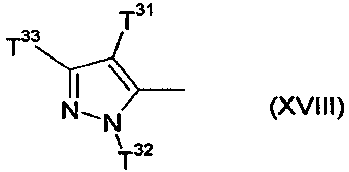 steht, wobei
T³¹ für Cyano, Nitro, Aryl, Trifluormethyl
oder
Aryl, das durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N*-Monoarylamino, *N,N*-Diarylamino, *N*-Alkyl-*N*-arylamino, *N*-Monocycloalkylamino, *N,N*-Dicycloalkylamino, *N*-Monoalkylmonocycloalkylamino, *N,N*-Monoarylmonocycloalkylamino, *N*-Acylamino, *N*-Alkylsulfonylamino, Thiocyano, Halogen, Cyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N-*Monoalkylcarbamoyl, *N,N-*Dicycloalkylcarbamoyl, *N,N*-Dialkylcarbamoyl, *N*-Monoarylcarbamoyl, *N,N-*Diarylcarbamoyl, *N*-Monocycloalkyl-*N*-monoarylcarbamoyl, *N*-Monoalkyl-N-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-*N*-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
steht,
T³² für Wasserstoff, Alkyl, Alkenyl, Alkinyl, Aryl, Alkoxycarbonyl
oder
Alkyl, das durch ein oder mehrere Heteroatome aus der Gruppe bestehend aus Sauerstoff und Schwefel unterbrochen ist,
oder
Alkyl, das durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N*-Monoarylamino, *N,N*-Diarylamino, *N*-Alkyl-N-arylamino, *N*-Monocycloalkylamino, *N,N*-Dicycloalkylamino, *N*-Monoalkylmonocycloalkylamino, *N,N*-Monoarylmonocycloalkylamino, *N*-Acylamino, *N*-Alkylsulfonylamino, Halogen, Cyano, Thiocyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N-*Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-*N*-monoarylcarbamoyl, *N*-Monoalkyl-*N*-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-*N*-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
oder
Alkyl, das durch ein oder mehrere Heteroatome aus der Gruppe bestehend aus Sauerstoff und Schwefel unterbrochen ist und durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N-*Monoarylamino, *N,N*-Diarylamino, *N*-Alkyl-*N-*arylamino, *N*-Monocycloalkylamino, *N,N-*Dicycloalkylamino, *N*-Monoalkylmonocycloalkylamino, *N,N*-Monoarylmonocycloalkylamino, *N*-Acylamino, *N-*Alkylsulfonylamino, Halogen, Cyano, Thiocyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N*-Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-N-monoarylcarbamoyl, *N*-Monoalkyl-N-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-*N*-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
oder
Aryl, das durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N*-Monoarylamino, *N,N*-Diarylamino, *N*-Alkyl-N-arylamino, *N*-Monocycloalkylamino, *N,N*-Dicycloalkylamino, *N*-Monoalkylmonocycloalkylamino, *N,N*-Monoarylmonocycloalkylamino, *N*-Acylamino, *N*-Alkylsulfonylamino, Thiocyano, Halogen, Cyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N-*Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-*N*-monoarylcarbamoyl, *N*-Monoalkyl-*N*-monoarylcarbamoyl, Sulfamoyl, *N*-Monocycloalkylsulfamoyl, *N*-Monoalkylsulfamoyl, *N,N-*Dicycloalkylsulfamoyl, *N,N*-Dialkylsulfamoyl, *N*-Monoarylsulfamoyl, *N,N*-Diarylsulfamoyl, *N*-Monocycloalkyl-*N*-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
T³³ für Wasserstoff, Alkyl
oder
Alkyl, das durch ein oder mehrere Heteroatome aus der Gruppe bestehend aus Sauerstoff und Schwefel unterbrochen ist,
oder
Alkyl, das durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N*-Monoarylamino, *N,N*-Diarylamino, *N*-Alkyl-*N*-arylamino, *N*-Monocycloalkylamino, *N,N*-Dicycloalkylamino, *N*-Monoalkylmonocycloalkylamino, *N,N*-Monoarylmonocycloalkylamino, *N*-Acylamino, *N*-Alkylsulfonylamino, Halogen, Cyano, Thiocyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N-*Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-*N*-monoarylcarbamoyl, *N*-Monoalkyl-*N*-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-*N*-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
oder
Alkyl, das durch ein oder mehrere Heteroatome aus der Gruppe bestehend aus Sauerstoff und Schwefel unterbrochen ist und durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N-*Monoarylamino, *N,N*-Diarylamino, *N*-Alkyl-*N-*arylamino, *N*-Monocycloalkylamino, *N,N-*Dicycloalkylamino, *N*-Monoalkylmonocycloalkylamino, *N,N*-Monoarylmonocycloalkylamino, *N*-Acylamino, *N-*Alkylsulfonylamino, Halogen, Cyano, Thiocyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N*-Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-*N*-monoarylcarbamoyl, *N*-Monoalkyl-N-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-N-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
oder
Aryl, das durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N*-Monoarylamino, *N,N*-Diarylamino, *N*-Alkyl-N-arylamino, *N*-Monocycloalkylamino, *N,N*-Dicycloalkylamino, *N*-Monoalkylmonocycloalkylamino, *N,N*-Monoarylmonocycloalkylamino, *N*-Acylamino, *N*-Alkylsulfonylamino, Thiocyano, Halogen, Cyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, N*-*Monocycloalkylcarbamoyl, *N-*Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-*N*-monoarylcarbamoyl, *N*-Monoalkyl-*N*-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-*N*-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
steht,
oder
D für eine Gruppe der Formel (XIX) 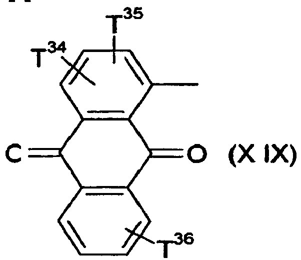 steht, wobei
T³⁴, T³⁵ und T³⁶ unabhängig voneinander für Wasserstoff, Halogen, Nitro, Cyano, Hydroxyl, Alkoxylcarbonyl, Alkyl, Acyl
oder
Alkyl, das durch einen oder mehrere Substituenten aus der Gruppe bestehend aus . Hydroxy, Aryl, Cycloalkyl, Alkoxy, Thioalkoxy, Amino, *N*-Monoalkylamino, *N,N*-Dialkylamino, *N*-Monoarylamino, *N,N-*Diarylamino, *N*-Alkyl-*N*-arylamino, *N*-Monocycloalkylamino, *N,N*-Dicycloalkylamino, *N*-Monoalkylmonocycloalkylamino, *N,N*-Monoarylmonocycloalkylamino, *N*-Acylamino, *N*-Alkylsulfonylamino, Halogen, Cyano, Thiocyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, *N-*Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-N-monoarylcarbamoyl, *N*-Monoalkyl-*N*-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-*N*-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
oder
Alkyl, das durch ein oder mehrere Heteroatome aus der Gruppe bestehend aus Sauerstoff und Schwefel unterbrochen ist und durch einen oder mehrere Substituenten aus der Gruppe bestehend aus Hydroxy, Aryl, Cycloalkyl, Alkoxy, Thioalkoxy, Amino, *N*-Monoalkylamino, *N,N*-Dialkylamino, *N-*Monoarylamino, *N,N*-Diarylamino, *N*-Alkyl-*N-*arylamino, *N*-Monocycloalkylamino, *N,N-*Dicycloalkylamino, *N*-Monoalkylmonocycloalkylamino, *N,N*-Monoarylmonocycloalkylamino, *N*-Acylamino, *N-*Alkylsulfonylamino, Halogen, Cyano, Thiocyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, *N*-Monocycloalkylcarbamoyl, N-Monoalkylcarbamoyl, *N,N*-Dicycloalkylcarbamoyl, *N,N*-Dialkylcarbamoyl, *N*-Monoarylcarbamoyl, *N,N-*Diarylcarbamoyl, *N*-Monocycloalkyl-N-monoarylcarbamoyl, *N*-Monoalkyl-N-monoarylcarbamoyl, Sulfamoyl, *N*-Monocycloalkylsulfamoyl, *N*-Monoalkylsulfamoyl, *N,N*-Dicycloalkylsulfamoyl, *N,N*-Dialkylsulfamoyl, *N*-Monoarylsulfamoyl, *N,N*-Diarylsulfamoyl, *N*-Monocycloalkyl-*N*-monoarylsulfamoyl, *N*-Monoalkyl-*N*-monoarylsulfamoyl, Arylsulfonyloxy, Alkylsulfonyloxy, Aryloxysulfonyl, Alkoxylsulfonyl und COOR¹² substituiert ist,
stehen,
oder
D für eine Gruppe der Formel (XX) 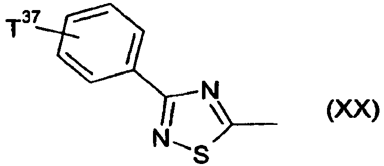 steht, wobei
T³⁷ für Wasserstoff, Halogen oder Nitro steht,
R¹² für Wasserstoff, Alkyl, Alkenyl, Cycloalkyl, Aryl, Heteroaryl, Heterocycloalkyl
oder
Alkyl, das durch ein oder mehrere Heteroatome aus der Gruppe bestehend aus Sauerstoff und Schwefel unterbrochen ist,
oder
Alkyl, das durch einen oder mehrere Substituenten aus der Gruppe bestehend aus Hydroxy, Aryl, Cycloalkyl, Alkoxy, Thioalkoxy, Halogen, Cyano, Amino, Thiocyano, Nitro, Acyl, Thioacyl, Alkylsulfonyl, Aryloyl, Trifluormethyl, Heteroaryl, Heterocycloalkyl, Alkoxythiocarbonyl, Acyloxy, Aryloyloxy, Carbamoyl, Sulfamoyl, Arylsulfonyloxy, Alkylsulfonyloxy, Aryloxysulfonyl und Alkoxylsulfonyl substituiert ist,
oder
Alkyl, das durch ein oder mehrere Heteroatome aus der Gruppe bestehend aus Sauerstoff und Schwefel unterbrochen ist und durch einen oder mehrere Substituenten aus der Gruppe bestehend aus Hydroxy, Aryl, Cycloalkyl, Alkoxy, Thioalkoxy, Halogen, Cyano, Amino, Thiocyano, Nitro, Acyl, Thioacyl, Alkylsulfonyl, Aryloyl, Trifluormethyl, Heteroaryl, Heterocycloalkyl, Alkoxythiocarbonyl, Acyloxy, Aryloyloxy, Carbamoyl, Sulfamoyl, Arylsulfonyloxy, Alkylsulfonyloxy, Aryloxysulfonyl und Alkoxylsulfonyl substituiert ist,
oder
Aryl, das durch einen oder mehrere Substituenten aus der Gruppe bestehend aus Hydroxy, Aryl, Cycloalkyl, Alkoxy, Thioalkoxy, Amino, Thiocyano, Halogen, Cyano, Nitro, Acyl, Thioacyl, Alkylsulfonyl, Aryloyl, Trifluormethyl, Heteroaryl, Heterocycloalkyl, Alkoxycarbonyl, Alkoxythiocarbonyl, Acyloxy, Aryloyloxy, Carbamoyl, Sulfamoyl, Arylsulfonyloxy, Alkylsulfonyloxy, Aryloxysulfonyl und Alkoxylsulfonyl substituiert ist,
steht,
mit der Maßgabe, dass unter R² bis R¹⁴ keine Bildung von anellierten Ringen erlaubt ist.

2. Farbstoffe nach Anspruch 1 mit der Formel (I-a) 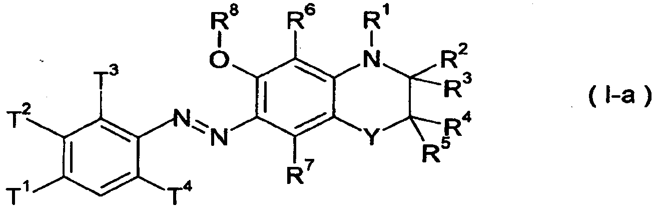 wobei
T¹ bis T⁴ jeweils unabhängig voneinander für Wasserstoff, (C₁-C₆)-Alkyl, (C₃-C₆)-Cycloalkyl, substituiertes (C₁-C₆)-Alkyl, (C₃-C₄)-Alkenyl, (C₃-C₄)-Alkinyl, Aryl, durch O unterbrochenes (C₁-C₆)-Alkyl, durch S unterbrochenes (C₁-C₆)-Alkyl, (C₁-C₈)-Alkoxyl, (C₁-C₆)-Cycloalkyl, Halogen, Cyano, Nitro, Arylsulfonyl, Arylsulfonyloxy, (C₁-C₆)-Alkylsulfonyl, (C₁-C₆)-Alkoxylsulfonyl, *N*-(C₁-C₆)-Alkylsulfamoyl, *N,N*-Bis(C₁-C₆)alkylsulfamoyl, N-(C₁-C₆)-Alkylcarbamoyl, *N,N*-Bis(C₁-C₆)alkylcarbamoyl, (C₁-C₆)-Alkylacyl, Arylacyl, (C₁-C₆)-Alkoxylcarbonyl, Aryloxylcarbonyl, Thiocyano, Hydroxyl, Aryloxy, Arylmethoxyl, Aryloyloxy, Arylsulfonyloxy, {[(C₁-C₆)-Alkoxylcarbonyl]methoxyl}carbonyl, [(Aryloxy-carbonyl)methoxy]carbonyl, {[(C₁-C₆)-Alkylacyl]-methoxy}carbonyl oder [(Arylacyl)methoxy]carbonyl stehen,
R¹ für (C₁-C₆)-Alkyl, durch (C₁-C₆)-Acyloxy substituiertes (C₂-C₆)-Alkyl, Cyano- (C₁-C₆)-alkyl, Arylmethyl, Arylacyl-(C₁-C₆)alkyl, 2-(C₁-C₆)-Alkoxyl-2-oxoethyl, durch O unterbrochenes (C₂-C₆)-Alkyl, durch S unterbrochenes (C₂-C₆)-Alkyl, (C₃-C₄)-Alkenyl, (C₃-C₄)-Alkinyl, 3-(C₁-C₆)-Alkoxyl-3-oxopropyl, 3-[durch O unterbrochenes (C₂-C₆)-Alkoxyl]-3-oxopropyl, 2-(C₁-C₆)-Alkoxyl-2-oxoethyl, 2-[durch O unterbrochenes (C₂-C₆)-Alkoxyl]-2-oxoethyl, 2-{[(C₁-C₆)-Alkylacyl]methoxy}-2-oxoethyl, 3-{[(C₁-C₆)-Alkylacyl]methoxy}-3-oxopropyl, 2-[(Arylacyl)methoxy]-2-oxoethyl, 3-[(Arylacyl)methoxy]-3-oxopropyl, 3-{[(C₁-C₆)-Alkoxylcarbonyl]methoxy}-3-oxopropyl, 3-(Arylmethoxy)-3-oxopropyl, 3-[(Arylacyl)methoxy]-3-oxopropyl, 2-(Cyanomethoxy)-2-oxoethyl, 3-(Cyanomethoxy)-3-oxopropyl, 2-{[(C₁-C₆)-Alkoxycarbonyl]methoxy}-2-oxoethyl, 1-[(C₁-C₆)-Alkyl]-3-[(C₁-C₆)-alkoxyl]-3-oxopropyl, 1-[(C₁-C₆)-Alkyl]-3-[durch O unterbrochenes (C₁-C₆)-alkoxyl]-3-oxopropyl, 1-[(C₁-C₆)-Alkyl]-3-(arylmethoxyl)-3-oxopropyl, 1-[(C₁-C₆)-Alkyl]-3-{[(C₁-C₆)-alkylacyl]methoxy}-3-oxopropyl oder 1-[(C₁-C₆)-Alkyl]-3-{[(C₁-C₆)-alkoxylcarbonyl]methoxy}-3-oxopropyl steht,
R² bis R⁷ unabhängig voneinander für Wasserstoff, (C₁-C₆)-Alkyl, Aryl, (C₁-C₈) -Alkoxyl, (C₃-C₈)-Cycloalkyl, durch O unterbrochenes (C₂-C₆)-Alkyl, durch S unterbrochenes (C₂-C₆)-Alkyl oder Halogen stehen,
R⁸ für Wasserstoff, (C₁-C₆)-Alkyl, (C₃-C₄)-Alkenyl, (C₃-C₄)-Alkinyl, Arylmethyl, 2- (C₁-C₆)-Alkoxyl-2-oxoethyl, 2-(Arylmethyl)-2-oxoethyl, Cyano-(C₁-C₆)-alkyl, durch O unterbrochenes (C₁-C₆)-Alkyl oder durch S unterbrochenes (C₁-C₆)-Alkyl steht
und
Y für Carbonyl steht.

3. Farbstoffe nach Anspruch 1 oder 2 mit der Formel (I-a), wobei
T¹, T³ und T⁴ unabhängig voneinander für Wasserstoff, Nitro, Cyano, Brom, Chlor, Benzoxyl, Phenoxy, Methoxy, Ethoxy, Methyl, Trifluormethyl, Ethyl, Benzoyl, Acetyl, Hydroxyl, Methoxycarbonyl, Ethoxycarbonyl, Methylsulfonyl, Cyanomethylsulfonyl, Acetonylsulfonyl, (2-Ethoxy-2-oxoethyl)sulfonyl, (2-Methoxy-2-oxoethyl)sulfonyl, Phenylsulfonyloxy, Phenoxylsulfonyl, Ethoxylsulfonyl, Phenoxylsulfonyl, Cyanomethoxylcarbonyl, (2-Ethoxy-2-oxoethyl)carbonyl, (2-Methoxy-2-oxoethyl)carbonyl, Acetonylcarbonyl oder Phenacylcarbonyl stehen, T² für Wasserstoff, Chlor, Brom, Methoxy, Ethoxyl, Phenoxy, Benzoxy, Nitro, Methyl oder Ethyl steht,
R¹ für Methyl, Ethyl, Propyl, Isopropyl, n-Butyl, Isobutyl, Benzyl, 2-Methoxylethyl, 2-Ethoxylethyl, 2-Acetoxyethyl, 2-(Propionyl)oxyethyl, 2-(Benzoyl)oxyethyl, Cyanomethyl, 2-Cyanoethyl, 3-Cyanopropyl, 3-(C₁-C₆)-Alkoxy-3-oxopropyl, 3-[durch O unterbrochenes (C₂-C₆)-Alkoxyl]-3-oxopropyl, 2-(C₁-C₆)-Alkyl-2-oxoethyl, 2-[durch O unterbrochenes (C₂-C₆)-Alkoxyl]-2-oxoethyl, 2-(Cyanomethoxy)-2-oxoethyl, 3-(Cyanomethoxy)-3-oxopropyl, 2-[(Arylacyl)methoxy]-2-oxoethyl, 3-[(Arylacyl)methoxy]-3-oxopropyl, 3-(Arylmethoxy)-3-oxopropyl, 3-{[(C₁-C₆)-Alkylacyl]methoxyl}-3-oxopropyl, 3-[(Arylacyl)methoxy]-3-oxopropyl, 3-{[(C₁-C₆)-Alkoxycarbonyl]methoxy}-3-oxopropyl, 2-{[(C₁-C₆)-Alkoxycarbonyl]methoxy}-2-oxoethyl oder 2-{[(C₁-C₆)-Alkylacyl]methoxyl}2-oxoethyl steht,
R² bis R⁷ unabhängig voneinander für Wasserstoff, Brom, Chlor, Ethyl oder Methyl stehen,
R⁸ für Wasserstoff, Methyl, Ethyl, Propyl, Isopropyl, 2-Ethoxy-2-oxoethyl oder 2-Methoxy-2-oxoethyl steht
und
Y für Carbonyl steht.

4. Farbstoffe nach Anspruch 1 mit der Formel (I-b) 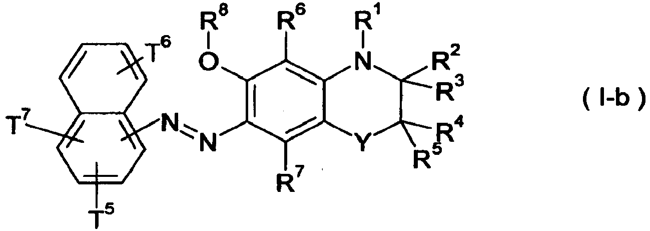 wobei
T⁵ bis T⁷ unabhängig voneinander für Wasserstoff, (C₁-C₆)-Alkyl, (C₃-C₆)-Cycloalkyl, durch, O unter-brochenes (C₁-C₆)-Alkyl, durch S unterbrochenes (C₁-C₆)-Alkyl, Aryl, (C₁-C₆)-Alkoxyl, Halogen, Cyano, Nitro, (C₁-C₆)-Alkylacyl, Arylacyl, (C₁-C₆)-Alkoxycarbonyl, Aryloxycarbonyl, *N*-(C₁-C₆)-Alkyl-carbamoyl oder *N,N*-Bis (C₁-C₆)-alkylcarbamoyl stehen,
wobei mindestens eine der Variablen T⁵, T⁶ und T⁷ nicht für Wasserstoff steht,
R¹ für Methyl, Ethyl, Propyl, Isopropyl, *n*-Butyl, Isobutyl, Benzyl, 2-Methoxylethyl, 2-Ethoxylethyl, 2-Acetoxyethyl, 2-(Propionyl)oxyethyl, 2-(Benzoyl)oxyethyl, Cyanomethyl, 2-Cyanoethyl, 3-Cyanopropyl, 3-(C₁-C₆)-Alkoxyl-3-oxopropyl, 3-[durch O unterbrochenes (C₂-C₆)-Alkoxyl]-3-oxopropyl, 2-(C₁-C₆)-Alkyl-2-oxoethyl, 2-[durch O unterbrochenes (C₂-C₆)-Alkoxyl]-2-oxoethyl, 2-(Cyanomethoxy)-2-oxoethyl, 3-(Cyanomethoxy)-3-oxopropyl, 2-[(Arylacyl)methoxy]-2-oxoethyl, 3-[(Arylacyl)methoxy]-3-oxopropyl, 3-(Arylmethoxy)-3-oxopropyl, 3-{[(C₁-C₆)-Alkylacyl]methoxyl}-3-oxopropyl, 3-[(Arylacyl)methoxy]-3-oxopropyl, 3-{[(C₁-C₆)-Alkoxycarbonyl]methoxy}-3-oxopropyl, 2-{[(C₁-C₆)-Alkoxycarbonyl]methoxy}-2-oxoethyl oder 2-{[(C₁-C₆)-Alkylacyl]methoxyl}2-oxoethyl steht,
R² bis R⁷ unabhängig voneinander für Wasserstoff, (C₁-C₆)-Alkyl, Aryl, (C₁-C₈) -Alkoxyl, (C₃-C₈)-Cycloalkyl, durch O unterbrochenes (C₂-C₆)-Alkyl, durch S unterbrochenes (C₂-C₆)-Alkyl oder Halogen stehen,
R⁸ für Wasserstoff, (C₁-C₆)-Alkyl, (C₃-C₄)-Alkenyl, (C₃-C₄)-Alkinyl, Arylmethyl, 2-(C₁-C₆)-Alkoxyl-2-oxoethyl, 2-(Arylmethyl)-2-oxoethyl, Cyano-(C₁-C₆)-alkyl, durch O unterbrochenes (C₁-C₆)-Alkyl oder durch S unterbrochenes (C₁-C₆)-Alkyl steht
und
Y für Carbonyl steht.

5. Farbstoffe nach Anspruch 1 mit der Formel (I-c) 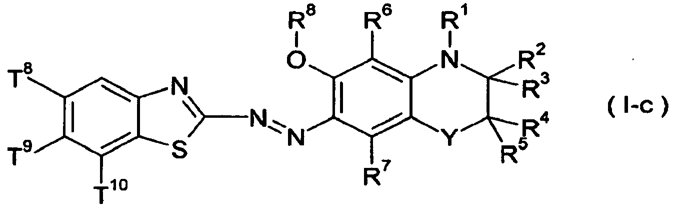 wobei
T⁸ und T¹⁰ unabhängig voneinander für Wasserstoff, Chlor oder Brom stehen,
T⁹ für Wasserstoff, (C₁-C₆)-Alkylsulfonyl, Thiocyano, (C₁-C₆)-Alkoxy, Chlor oder Nitro steht,
R¹ für -Methyl, Ethyl, Propyl, Isopropyl, n-Butyl, Isobutyl, Benzyl, 2-Methoxylethyl, 2-Ethoxylethyl, 2-Acetoxyethyl, 2-(Propionyl)oxyethyl, 2-(Benzoyl)oxyethyl, Cyanomethyl, 2-Cyanoethyl, 3-Cyanopropyl, 3-(C₁-C₆)-Alkoxy-3-oxopropyl, 3-[durch O unterbrochenes (C₂-C₆)-Alkoxyl]-3-oxopropyl, 2-(C₁-C₆)-Alkyl-2-oxoethyl, 2-[durch O unterbrochenes (C₂-C₆)-Alkoxyl]-2-oxoethyl, 2-(Cyanomethoxy)-2-oxoethyl, 3-(Cyanomethoxy)-3-oxopropyl, 2-[(Arylacyl)methoxy]-2-oxoethyl, 3-[(Arylacyl)methoxy]-3-oxopropyl, 3-(Arylmethoxy)-3-oxopropyl, 3-{[(C₁-C₆)-Alkylacyl]methoxyl}-3-oxopropyl, 3-[(Arylacyl)methoxy]-3-oxopropyl, 3-{[(C₁-C₆)-Alkoxycarbonyl]methoxy}-3-oxopropyl, 2-{[(C₁-C₆)-Alkoxycarbonyl]methoxy}-2-oxoethyl oder 2-{[(C₁-C₆)-Alkylacyl]methoxyl}2-oxoethyl steht,
R² bis R⁷ unabhängig voneinander für Wasserstoff, (C₁-C₆)-Alkyl, Aryl, (C₁-C₈) -Alkoxyl, (C₃-C₈)-Cycloalkyl, durch O unterbrochenes (C₂-C₆)-Alkyl, durch S unterbrochenes (C₂-C₆)-Alkyl oder Halogen stehen,
R⁸ für Wasserstoff, (C₁-C₆)-Alkyl, (C₃-C₄)-Alkenyl, (C₃-C₄)-Alkinyl, Arylmethyl, 2- (C₁-C₆)-Alkoxyl-2-oxoethyl, 2-(Arylmethyl)-2-oxoethyl, cyano-(C₁-C₆)-alkyl, durch O unterbrochenes (C₁-C₆)-Alkyl oder durch S unterbrochenes (C₁-C₆)-Alkyl steht
und
Y für Carbonyl steht.

6. Farbstoffe nach Anspruch 1 mit der Formel (I-d) 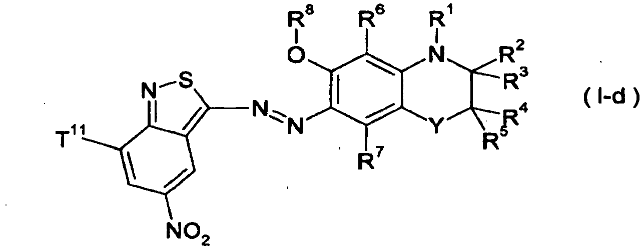 wobei
T¹¹ für Wasserstoff, Nitro oder Brom steht,
R¹ für Methyl, Ethyl, Propyl, Isopropyl, n-Butyl, Isobutyl, Benzyl, 2-Methoxylethyl, 2-Ethoxylethyl, 2-Acetoxyethyl, 2-(Propionyl)oxyethyl, 2-(Benzoyl)oxyethyl, Cyanomethyl, 2-Cyanoethyl, 3-Cyanopropyl, 3-(C₁-C₆)-Alkoxy-3-oxopropyl, 3-[durch O unterbrochenes (C₂-C₆)-Alkoxyl]-3-oxopropyl, 2-(C₁-C₆)-Alkyl-2-oxoethyl, 2-[durch O unterbrochenes (C₂-C₆)-Alkoxyl]-2-oxoethyl, 2-(Cyanomethoxy)-2-oxoethyl, 3-(Cyanomethoxy)-3-oxopropyl, 2-[(Arylacyl)methoxy]-2-oxoethyl, 3-[(Arylacyl)methoxy]-3-oxopropyl, 3-(Arylmethoxy)-3-oxopropyl, 3-{[(C₁-C₆)-Alkylacyl]methoxyl}-3-oxopropyl, 3-[(Arylacyl)methoxy]-3-oxopropyl, 3-{[(C₁-C₆)-Alkoxycarbonyl]methoxy}-3-oxopropyl, 2-{[(C₁-C₆)-Alkoxycarbonyl]methoxy}-2-oxoethyl oder 2-{[(C₁-C₆)-Alkylacyl]methoxyl}2-oxoethyl steht,
R² bis R⁷ unabhängig voneinander für Wasserstoff, (C₁-C₆)-Alkyl, Aryl, (C₁-C₈) -Alkoxyl, (C₃-C₈)-Cycloalkyl, durch O unterbrochenes (C₂-C₆)-Alkyl, durch S unterbrochenes (C₂-C₆)-Alkyl oder Halogen stehen,
R⁸ für Wasserstoff, (C₁-C₆)-Alkyl, (C₃-C₄)-Alkenyl, (C₃-C₄)-Alkinyl, Arylmethyl, 2-(C₁-C₆)-Alkoxyl-2-oxoethyl, 2-(Arylmethyl)-2-oxoethyl, Cyano-(C₁-C₆)-alkyl, durch O unterbrochenes (C₁-C₆)-Alkyl oder durch S unterbrochenes (C₁-C₆)-Alkyl steht
und
Y für Carbonyl steht.

7. Farbstoffe nach Anspruch 1 mit der Formel (I-e) 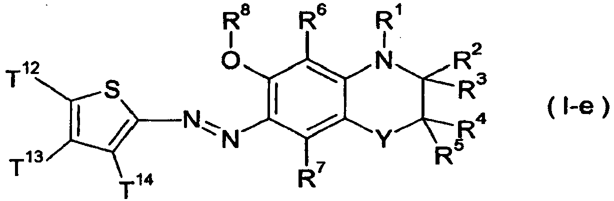 wobei
T¹² und T¹⁴ unabhängig voneinander für Nitro, Cyano, Formyl, Acetyl oder (C₁-C₆)-Alkoxycarbonyl stehen,
T¹³ für Wasserstoff, Aryl, (C₁-C₆)-Alkyl oder Halogen steht,
R¹ für Methyl, Ethyl, Propyl, Isopropyl, n-Butyl, Isobutyl, Benzyl, 2-Methoxylethyl, 2-Ethoxylethyl, 2-Acetoxyethyl, 2-(Propionyl)oxyethyl, 2-(Benzoyl)oxyethyl, Cyanomethyl, 2-Cyanoethyl, 3-Cyanopropyl, 3-(C₁-C₆)-Alkoxy-3-oxopropyl, 3-[durch O unterbrochenes (C₂-C₆)-Alkoxyl]-3-oxopropyl, 2-(C₁-C₆)-Alkyl-2-oxoethyl, 2-[durch O unterbrochenes (C₂-C₆)-Alkoxyl]-2-oxoethyl, 2-(Cyanomethoxy)-2-oxoethyl, 3-(Cyanomethoxy)-3-oxopropyl, 2-[(Arylacyl)methoxy]-2-oxoethyl, 3-[(Arylacyl)methoxy]-3-oxopropyl, 3-(Arylmethoxy)-3-oxopropyl, 3-{[(C₁-C₆)-Alkylacyl]methoxyl}-3-oxopropyl, 3-[(Arylacyl)methoxy]-3-oxopropyl, 3-{[(C₁-C₆)-Alkoxycarbonyl]methoxy}-3-oxopropyl, 2-{[(C₁-C₆)-Alkoxycarbonyl]methoxy}-2-oxoethyl oder 2-{[(C₁-C₆)-Alkylacyl]methoxyl}2-oxoethyl steht,
R² bis R⁷ unabhängig voneinander für Wasserstoff, (C₁-C₆)-Alkyl, Aryl, (C₁-C₈) -Alkoxyl, (C₃-C₈)-Cycloalkyl, durch O unterbrochenes (C₂-C₆)-Alkyl, durch S unterbrochenes (C₂-C₆)-Alkyl oder Halogen stehen,
R⁸ für Wasserstoff, (C₁-C₆) -Alkyl, (C₃-C₄)-Alkenyl, (C₃-C₄)-Alkinyl, Arylmethyl, 2- (C₁-C₆)-Alkoxyl-2-oxoethyl, 2-(Arylmethyl)-2-oxoethyl, Cyano-(C₁-C₆)-alkyl, durch O unterbrochenes (C₁-C₆)-Alkyl oder durch S unterbrochenes (C₁-C₆)-Alkyl steht
und
Y für Carbonyl steht.

8. Farbstoffe nach Anspruch 1 mit der Formel (I-f) 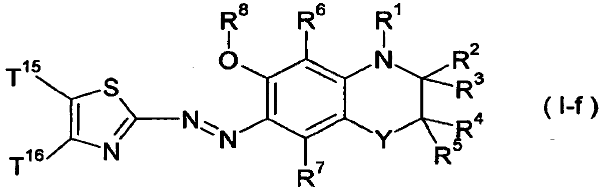 wobei
T¹⁵ und T¹⁶ unabhängig voneinander für Wasserstoff, Halogen, (C₁-C₆)-alkyl, Nitro, Cyano, Formyl, Acetyl, (C₁-C₆)-Alkoxycarbonyl, durch O unterbrochenes (C₁-C₆)-Alkyl, durch S unterbrochenes (C₁-C₆)-Alkyl oder Aryl stehen,
R¹ für Methyl, Ethyl, Propyl, Isopropyl, n-Butyl, Isobutyl, Benzyl, 2-Methoxylethyl, 2-Ethoxylethyl, 2-Acetoxyethyl, 2-(Propionyl)oxyethyl, 2-(Benzoyl)oxyethyl, Cyanomethyl, 2-Cyanoethyl, 3-Cyanopropyl, 3-(C₁-C₆)-Alkoxy-3-oxopropyl, 3-[durch O unterbrochenes (C₂-C₆)-Alkoxyl]-3-oxopropyl, 2-(C₁-C₆)-Alkyl-2-oxoethyl, 2-[durch O unterbrochenes (C₂-C₆)-Alkoxyl]-2-oxoethyl, 2-(Cyanomethoxy)-2-oxoethyl, 3-(Cyanomethoxy)-3-oxopropyl, 2-[(Arylacyl)methoxy]-2-oxoethyl, 3-[(Arylacyl)methoxy]-3-oxopropyl, 3-(Arylmethoxy)-3-oxopropyl, 3-{[(C₁-C₆)-Alkylacyl]methoxyl}-3-oxopropyl, 3-[(Arylacyl)methoxy]-3-oxopropyl, 3-([(C₁-C₆)-Alkoxycarbonyl]methoxy}-3-oxopropyl, 2-{[(C₁-C₆)-Alkoxycarbonyl]methoxy}-2-oxoethyl oder 2-{[(C₁-C₆)-Alkylacyl]methoxyl}2-oxoethyl steht,
R² bis R⁷ unabhängig voneinander für Wasserstoff, (C₁-C₆)-Alkyl, Aryl, (C₁-C₈) -Alkoxyl, (C₃-C₈)-Cycloalkyl, durch O unterbrochenes (C₂-C₆)-Alkyl, durch S unterbrochenes (C₂-C₆)-Alkyl oder Halogen stehen,
R⁸ für Wasserstoff, (C₁-C₆)-Alkyl, (C₃-C₄)-Alkenyl, (C₃-C₄)-Alkinyl, Arylmethyl, 2- (C₁-C₆)-Alkoxyl-2-oxoethyl, 2-(Arylmethyl)-2-oxoethyl, Cyano-(C₁-C₆)-alkyl, durch O unterbrochenes (C₁-C₆)-Alkyl oder durch S unterbrochenes (C₁-C₆)-Alkyl steht
und
Y für Carbonyl steht.

9. Farbstoffe nach Anspruch 1 mit der Formel (I-g) 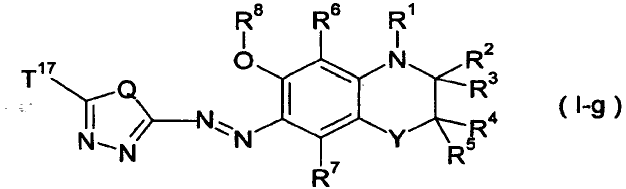 wobei
T¹⁷ für Aryl oder (C₁-C₆)-Thioalkoxyl steht,
Q für Sauerstoff oder Schwefel steht,
R¹ für Methyl, Ethyl, Propyl, Isopropyl, n-Butyl, Isobutyl, Benzyl, 2-Methoxylethyl, 2-Ethoxylethyl, 2-Acetoxyethyl, 2-(Propionyl)oxyethyl, 2-(Benzoyl)oxyethyl, Cyanomethyl, 2-Cyanoethyl, 3-Cyanopropyl, 3-(C₁-C₆)-Alkoxy-3-oxopropyl, 3-[durch O unterbrochenes (C₂-C₆)-Alkoxyl]-3-oxopropyl, 2-(C₁-C₆)-Alkyl-2-oxoethyl, 2-[durch O unterbrochenes (C₂-C₆)-Alkoxyl]-2-oxoethyl, 2-(Cyanomethoxy)-2-oxoethyl, 3-(Cyanomethoxy)-3-oxopropyl, 2-[(Arylacyl)methoxy]-2-oxoethyl, 3-[(Arylacyl)methoxy]-3-oxopropyl, 3-(Arylmethoxy)-3-oxopropyl, 3-{[(C₁-C₆)-Alkylacyl]methoxyl}-3-oxopropyl, 3-[(Arylacyl)methoxy]-3-oxopropyl, 3-{[(C₁-C₆)-Alkoxycarbonyl]methoxy}-3-oxopropyl, 2-{[(C₁-C₆)-Alkoxycarbonyl]methoxy}-2-oxoethyl oder 2-{[(C₁-C₆)-Alkylacyl]methoxyl}2-oxoethyl steht,
R² bis R⁷ unabhängig voneinander für Wasserstoff, (C₁-C₆)-Alkyl, Aryl, (C₁-C₈) -Alkoxyl, (C₃-C₈)-Cycloalkyl, durch O unterbrochenes (C₂-C₆)-Alkyl, durch S unterbrochenes (C₂-C₆)-Alkyl oder Halogen stehen,
R⁸ für Wasserstoff, (C₁-C₆)-Alkyl, (C₃-C₄)-Alkenyl, (C₃-C₄)-Alkinyl, Arylmethyl, 2- (C₁-C₆)-Alkoxyl-2-oxoethyl, 2-(Arylmethyl)-2-oxoethyl, Cyano-(C₁-C₆)-alkyl, durch O unterbrochenes (C₁-C₆)-Alkyl oder durch S unterbrochenes (C₁-C₆)-Alkyl steht
und
Y für Carbonyl steht.

10. Farbstoffe nach Anspruch 1 mit der Formel (I-h) 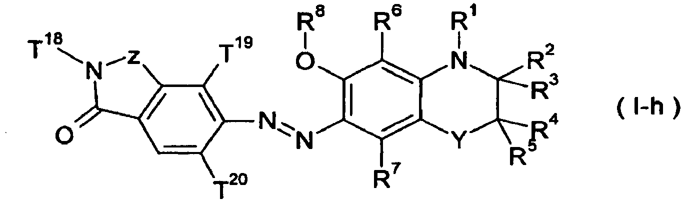 wobei
T¹⁸ für (C₁-C₆)-Alkyl, durch (C₁-C₆)-Acyloxy substituiertes (C₁-C₆)-Alkyl, Cyano- (C₁-C₆) -alkyl, Arylmethyl, durch Arylcarbonyl substituiertes (C₁-C₆)-Alkyl, 2- (C₁-C₆)-Alkoxyl-2-oxoethyl, durch O unterbrochenes (C₁-C₆)-Alkyl, durch S unterbrochenes (C₁-C₆)-Alkyl oder Aryl steht,
T¹⁹ und T²⁰ unabhängig voneinander für Wasserstoff, Halogen, Cyano, Nitro oder Trifluormethyl stehen,
Z für einen Carbonyl- oder Sulfonylrest steht,
R¹ für Methyl, Ethyl, Propyl, Isopropyl, n-Butyl, Isobutyl, Benzyl, 2-Methoxylethyl, 2-Ethoxylethyl, 2-Acetoxyethyl, 2-(Propionyl)oxyethyl, 2-(Benzoyl)oxyethyl, Cyanomethyl, 2-Cyanoethyl, 3-Cyanopropyl, 3-(C₁-C₆)-Alkoxy-3-oxopropyl, 3-[durch O unterbrochenes (C₂-C₆)-Alkoxyl]-3-oxopropyl, 2-(C₁-C₆)-Alkyl-2-oxoethyl, 2-[durch O unterbrochenes (C₂-C₆)-Alkoxyl]-2-oxoethyl, 2-(Cyanomethoxy)-2-oxoethyl, 3-(Cyanomethoxy)-3-oxopropyl, 2-[(Arylacyl)methoxy]-2-oxoethyl, 3-[(Arylacyl)methoxy]-3-oxopropyl, 3-(Arylmethoxy)-3-oxopropyl, 3-{[(C₁-C₆)-Alkylacyl]methoxyl}-3-oxopropyl, 3-[(Arylacyl)methoxy]-3-oxopropyl, 3-{[(C₁-C₆)-Alkoxycarbonyl]methoxy}-3-oxopropyl, 2-{[(C₁-C₆)-Alkoxycarbonyl]methoxy}-2-oxoethyl oder 2-{[(C₁-C₆)-Alkylacyl]methoxyl}2-oxoethyl steht,
R² bis R⁷ unabhängig voneinander für Wasserstoff, (C₁-C₆)-Alkyl, Aryl, (C₁-C₈) -Alkoxyl, (C₃-C₈)-Cycloalkyl, durch O unterbrochenes (C₂-C₆)-Alkyl, durch S unterbrochenes (C₂-C₆)-Alkyl oder Halogen stehen,
R⁸ für Wasserstoff, (C₁-C₆)-Alkyl, (C₃-C₄)-Alkenyl, (C₃-C₄)-Alkinyl, Arylmethyl, 2- (C₁-C₆)-Alkoxyl-2-oxoethyl, 2-(Arylmethyl)-2-oxoethyl, Cyano-(C₁-C₆)-alkyl, durch O unterbrochenes (C₁-C₆)-Alkyl oder durch S unterbrochenes (C₁-C₆)-Alkyl steht
und
Y für Carbonyl steht.

11. Farbstoffe nach Anspruch 1 mit der Formel (I-i) 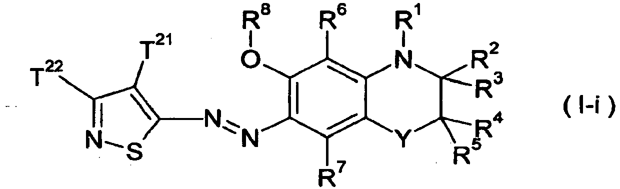 wobei
T²¹ für Cyano, Nitro oder (C₁-C₆)-Alkoxycarbonyl steht,
T²² für (C₁-C₆)-Alkyl, Wasserstoff, Halogen oder Aryl steht,
R¹ für Methyl, Ethyl, Propyl, Isopropyl, n-Butyl, Isobutyl, Benzyl, 2-Methoxylethyl, 2-Ethoxylethyl, 2-Acetoxyethyl, 2-(Propionyl)oxyethyl, 2-(Benzoyl)oxyethyl, Cyanomethyl, 2-Cyanoethyl, 3-Cyanopropyl, 3-(C₁-C₆)-Alkoxy-3-oxopropyl, 3-[durch O unterbrochenes (C₂-C₆)-Alkoxyl]-3-oxopropyl, 2-(C₁-C₆) -Alkyl-2-oxoethyl, 2-[durch O unterbrochenes (C₂-C₆)-Alkoxyl]-2-oxoethyl, 2-(Cyanomethoxy)-2-oxoethyl, 3-(Cyanomethoxy)-3-oxopropyl, 2-[(Arylacyl)methoxy]-2-oxoethyl, 3-[(Arylacyl)methoxy]-3-oxopropyl, 3-(Arylmethoxy)-3-oxopropyl, 3-{[(C₁-C₆)-Alkylacyl]methoxyl}-3-oxopropyl, 3-[(Arylacyl)methoxy]-3-oxopropyl, 3-{[(C₁-C₆)-Alkoxycarbonyl]methoxy}-3-oxopropyl, 2-{[(C₁-C₆)-Alkoxycarbonyl]methoxy}-2-oxoethyl oder 2-{[(C₁-C₆)-Alkylacyl]methoxyl}2-oxoethyl steht,
R² bis R⁷ unabhängig voneinander für Wasserstoff, (C₁-C₆) -Alkyl, Aryl, (C₁-C₈) -Alkoxyl, (C₃-C₈)-Cycloalkyl, durch O unterbrochenes (C₂-C₆)-Alkyl, durch S unterbrochenes (C₂-C₆)-Alkyl oder Halogen stehen,
R⁸ für Wasserstoff, (C₁-C₆)-Alkyl, (C₃-C₄)-Alkenyl, (C₃-C₄)-Alkinyl, Arylmethyl, 2-(C₁-C₆)-Alkoxyl-2-oxoethyl, 2-(Arylmethyl)-2-oxoethyl, Cyano-(C₁-C₆)-alkyl, durch O unterbrochenes (C₁-C₆)-Alkyl oder durch S unterbrochenes (C₁-C₆)-Alkyl steht
und
Y für Carbonyl steht.

12. Farbstoffe nach Anspruch 1 mit der Formel (I-j) 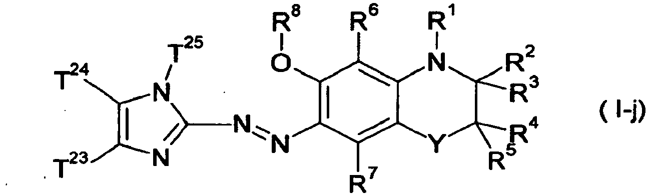 wobei
T²³ und T²⁴ unabhängig voneinander für Cyano, Nitro, Halogen, Aryl oder Trifluormethyl stehen,
T²⁵ für Wasserstoff, (C₁-C₆)-Alkyl, durch O unterbrochenes (C₁-C₆)-Alkyl, durch S unterbrochenes (C₁-C₆)-Alkyl oder Aryl steht,
R¹ für Methyl, Ethyl, Propyl, Isopropyl, n-Butyl, Isobutyl, Benzyl, 2-Methoxylethyl, 2-Ethoxylethyl, 2-Acetoxyethyl, 2-(Propionyl)oxyethyl, 2-(Benzoyl)oxyethyl, Cyanomethyl, 2-Cyanoethyl, 3-Cyanopropyl, 3-(C₁-C₆)-Alkoxy-3-oxopropyl, 3-[durch O unterbrochenes (C₂-C₆)-Alkoxyl]-3-oxopropyl, 2-(C₁-C₆)-Alkyl-2-oxoethyl, 2-[durch O unterbrochenes (C₂-C₆)-Alkoxyl]-2-oxoethyl, 2-(Cyanomethoxy)-2-oxoethyl, 3-(Cyanomethoxy)-3-oxopropyl, 2-[(Arylacyl)methoxy]-2-oxoethyl, 3-[(Arylacyl)methoxy]-3-oxopropyl, 3-(Arylmethoxy)-3-oxopropyl, 3-{[(C₁-C₆)-Alkylacyl]methoxyl}-3-oxopropyl, 3-[(Arylacyl)methoxy]-3-oxopropyl, 3-{[(C₁-C₆)-Alkoxycarbonyl]methoxy}-3-oxopropyl, 2-{[(C₁-C₆)-Alkoxycarbonyl]methoxy}-2-oxoethyl oder 2-{[(C₁-C₆)-Alkylacyl]methoxyl}2-oxoethyl steht,
R² bis R⁷ jeweils unabhängig voneinander für Wasserstoff, (C₁-C₆)-Alkyl, Aryl, (C₁-C₈) -Alkoxyl, (C₃-C₈) -Cycloalkyl, durch O unterbrochenes (C₂-C₆)-Alkyl, durch S unterbrochenes (C₂-C₆)-Alkyl oder Halogen stehen,
R⁸ für Wasserstoff, (C₁-C₆)-Alkyl, (C₃-C₄)-Alkenyl, (C₃-C₄)-Alkinyl, Arylmethyl, 2- (C₁-C₆)-Alkoxyl-2-oxoethyl, 2-(Arylmethyl)-2-oxoethyl, Cyano-(C₁-C₆)-alkyl, durch O unterbrochenes (C₁-C₆)-Alkyl oder durch S unterbrochenes (C₁-C₆)-Alkyl steht
und
Y für Carbonyl steht.

13. Farbstoffe nach Anspruch 1 mit der Formel (I-k) 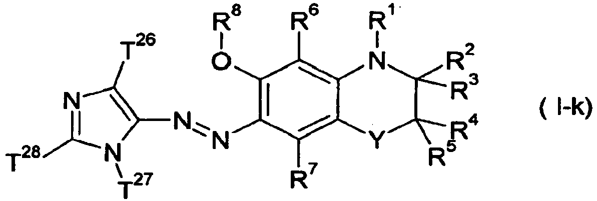 wobei
T²⁶ für Cyano, Nitro, Aryl oder Trifluormethyl steht,
T²⁷ für Wasserstoff, Aryl oder (C₁-C₆)-Alkyl steht,
T²⁸ für Wasserstoff, durch (C₁-C₆)-Acyloxy substituiertes (C₁-C₆)-Alkyl, Cyano- (C₁-C₆)-alkyl, Aryl, durch O unterbrochenes (C₁-C₆)-alkyl oder durch S unterbrochenes (C₁-C₆)-Alkyl steht,
R¹ für Methyl, Ethyl, Propyl, Isopropyl, n-Butyl, Isobutyl, Benzyl, 2-Methoxylethyl, 2-Ethoxylethyl, 2-Acetoxyethyl, 2-(Propionyl)oxyethyl, 2-(Benzoyl)oxyethyl, Cyanomethyl, 2-Cyanoethyl, 3-Cyanopropyl, 3-(C₁-C₆)-Alkoxy-3-oxopropyl, 3-[durch O unterbrochenes (C₂-C₆)-Alkoxyl]-3-oxopropyl, 2-(C₁-C₆)-Alkyl-2-oxoethyl, 2-[durch O unterbrochenes (C₂-C₆)-Alkoxyl]-2-oxoethyl, 2-(Cyanomethoxy)-2-oxoethyl, 3-(Cyanomethoxy)-3-oxopropyl, 2-[(Arylacyl)methoxy]-2-oxoethyl, 3-[(Arylacyl)methoxy]-3-oxopropyl, 3-(Arylmethoxy)-3-oxopropyl, 3-{[(C₁-C₆)-Alkylacyl]methoxyl}-3-oxopropyl, 3-[(Arylacyl)methoxy]-3-oxopropyl, 3-{[(C₁-C₆)-Alkoxycarbonyl]methoxy}-3-oxopropyl, 2-{[(C₁-C₆)-Alkoxycarbonyl]methoxy}-2-oxoethyl oder 2-{[(C₁-C₆)-Alkylacyl]methoxyl}2-oxoethyl steht,
R² bis R⁷ unabhängig voneinander für Wasserstoff, (C₁-C₆)-Alkyl, Aryl, (C₁-C₈)-Alkoxyl, (C₃-C₈)-Cycloalkyl, durch O unterbrochenes (C₂-C₆)-Alkyl, durch S unterbrochenes (C₂-C₆)-Alkyl oder Halogen stehen,
R⁸ für Wasserstoff, (C₁-C₆)-Alkyl, (C₃-C₄)-Alkenyl, (C₃-C₄)-Alkinyl, Arylmethyl, 2- (C₁-C₆)-Alkoxyl-2-oxoethyl, 2-(Arylmethyl)-2-oxoethyl, Cyano-(C₁-C₆)-alkyl, durch O unterbrochenes (C₁-C₆)-Alkyl oder durch S unterbrochenes (C₁-C₆)-Alkyl steht
und
Y für Carbonyl steht.

14. Farbstoffe nach Anspruch 1 mit der Formel (I-1) 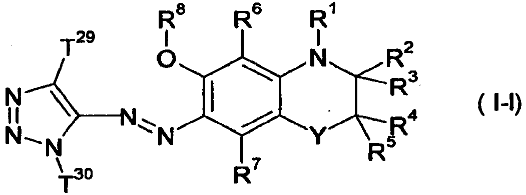 wobei
T²⁹ für Cyano, Nitro, Aryl oder Trifluormethyl steht,
T³⁰ für Wasserstoff, (C₁-C₆)-Alkyl, Cyano-(C₁-C₆)-alkyl, durch O unterbrochenes (C₁-C₆)-Alkyl, durch S unterbrochenes (C₁-C₆)-Alkyl, Aryl, Benzyl, durch (C₁-C₆)-Acyloxy substituiertes (C₁-C₆)-Alkyl oder Cyano-(C₁-C₆)-alkyl steht,
R¹ für Methyl, Ethyl, Propyl, Isopropyl, n-Butyl, Isobutyl, Benzyl, 2-Methoxylethyl, 2-Ethoxylethyl, 2-Acetoxyethyl, 2-(Propionyl)oxyethyl, 2-(Benzoyl)oxyethyl, Cyanomethyl, 2-Cyanoethyl, 3-Cyanopropyl, 3-(C₁-C₆)-Alkoxy-3-oxopropyl, 3-[durch O unterbrochenes (C₂-C₆)-Alkoxyl]-3-oxopropyl, 2-(C₁-C₆)-Alkyl-2-oxoethyl, 2-[durch O unterbrochenes (C₂-C₆)-Alkoxyl]-2-oxoethyl, 2-(Cyanomethoxy)-2-oxoethyl, 3-(Cyanomethoxy)-3-oxopropyl, 2-[(Arylacyl)methoxy]-2-oxoethyl, 3-[(Arylacyl)methoxy]-3-oxopropyl, 3-(Arylmethoxy)-3-oxopropyl, 3-{[(C₁-C₆)-Alkylacyl]methoxyl}-3-oxopropyl, 3-[(Arylacyl)methoxy]-3-oxopropyl, 3-{[(C₁-C₆)-Alkoxycarbonyl]methoxy}-3-oxopropyl, 2-{[(C₁-C₆)-Alkoxycarbonyl]methoxy}-2-oxoethyl oder 2-{[(C₁-C₆)-Alkylacyl]methoxyl}2-oxoethyl steht,
R² bis R⁷ unabhängig voneinander für Wasserstoff, (C₁-C₆)-Alkyl, Aryl, (C₁-C₈) -Alkoxyl, (C₃-C₈)-Cycloalkyl, durch O unterbrochenes (C₂-C₆)-Alkyl, durch S unterbrochenes (C₂-C₆)-Alkyl oder Halogen stehen,
R⁸ für Wasserstoff, (C₁-C₆)-Alkyl, (C₃-C₄)-Alkenyl, (C₃-C₄)-Alkinyl, Arylmethyl, 2- (C₁-C₆)-Alkoxyl-2-oxoethyl, 2-(Arylmethyl)-2-oxoethyl, Cyano-(C₁-C₆)-alkyl, durch O unterbrochenes (C₁-C₆)-Alkyl oder durch S unterbrochenes (C₁-C₆)-Alkyl steht
und
Y für Carbonyl steht.

15. Farbstoffe nach Anspruch 1 mit der Formel (I-m) 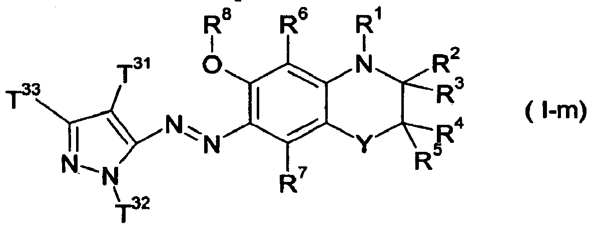 wobei
T³¹ für Cyano, Nitro, Aryl oder Trifluormethyl steht,
T³² für Wasserstoff, (C₁-C₆)-Alkyl, durch O unterbrochenes (C₁-C₆)-Alkyl, durch S unterbrochenes (C₁-C₆)-Alkyl, Cyano- (C₁-C₆)-alkyl, Arylmethyl, Cyano- (C₁-C₆)-alkyl oder durch (C₁-C₆)-Acyloxy substituiertes (C₁-C₆)-Alkyl steht,
T³³ für Wasserstoff oder (C₁-C₆)-Alkyl steht,
R¹ für Methyl, Ethyl, Propyl, Isopropyl, n-Butyl, Isobutyl, Benzyl, 2-Methoxylethyl, 2-Ethoxylethyl, 2-Acetoxyethyl, 2-(Propionyl)oxyethyl, 2-(Benzoyl)oxyethyl, Cyanomethyl, 2-Cyanoethyl, 3-Cyanopropyl, 3-(C₁-C₆)-Alkoxy-3-oxopropyl, 3-[durch O unterbrochenes (C₂-C₆)-Alkoxyl]-3-oxopropyl, 2-(C₁-C₆)-Alkyl-2-oxoethyl, 2-[durch O unterbrochenes (C₂-C₆)-Alkoxyl]-2-oxoethyl, 2-(Cyanomethoxy)-2-oxoethyl, 3-(Cyanomethoxy)-3-oxopropyl, 2-[(Arylacyl)methoxy]-2-oxoethyl, 3-[(Arylacyl)methoxy]-3-oxopropyl, 3-(Arylmethoxy)-3-oxopropyl, 3-{[(C₁-C₆)-Alkylacyl]methoxyl}-3-oxopropyl, 3-[(Arylacyl)methoxy]-3-oxopropyl, 3-{[(C₁-C₆)-Alkoxycarbonyl]methoxy}-3-oxopropyl, 2-{[(C₁-C₆)-Alkoxycarbonyl]methoxy}-2-oxoethyl oder 2-{[(C₁-C₆)-Alkylacyl]methoxyl}2-oxoethyl steht,
R² bis R⁷ unabhängig voneinander für Wasserstoff, (C₁-C₆)-Alkyl, Aryl, (C₁-C₈) -Alkoxyl, (C₃-C₈)-Cycloalkyl, durch O unterbrochenes (C₂-C₆)-Alkyl, durch S unterbrochenes (C₂-C₆)-Alkyl oder Halogen stehen,
R⁸ für Wasserstoff, (C₁-C₆)-Alkyl, (C₃-C₄)-Alkenyl, (C₃-C₄)-Alkinyl, Arylmethyl, 2- (C₁-C₆)-Alkoxyl-2-oxoethyl, 2-(Arylmethyl)-2-oxoethyl, Cyano-(C₁-C₆)-alkyl, durch O unterbrochenes (C₁-C₆)-Alkyl oder durch S unterbrochenes (C₁-C₆)-Alkyl steht
und
Y für Carbonyl steht.

16. Farbstoffe nach Anspruch 1 mit der Formel (I-n) 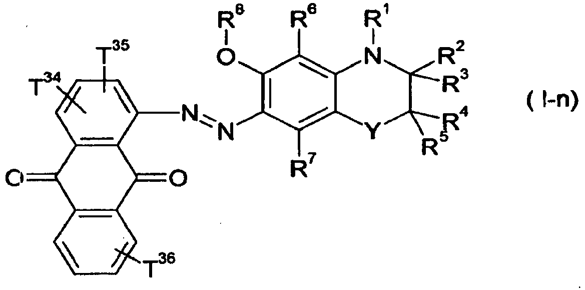 wobei
T³⁴, T³⁵ und T³⁶ unabhängig voneinander für Wasserstoff, Halogen, Nitro, Cyano, Hydroxyl, (C₁-C₆)-Alkoxylcarbonyl, (C₁-C₆)-Alkyl oder Acyl stehen,
R¹ für Methyl, Ethyl, Propyl, Isopropyl, n-Butyl, Isobutyl, Benzyl, 2-Methoxylethyl, 2-Ethoxylethyl, 2-Acetoxyethyl, 2-(Propionyl)oxyethyl, 2-(Benzoyl)oxyethyl, Cyanomethyl, 2-Cyanoethyl, 3-Cyanopropyl, 3-(C₁-C₆)-Alkoxy-3-oxopropyl, 3-[durch O unterbrochenes (C₂-C₆)-Alkoxyl]-3-oxopropyl, 2-(C₁-C₆)-Alkyl-2-oxoethyl, 2-[durch O unterbrochenes (C₂-C₆)-Alkoxyl]-2-oxoethyl, 2-(Cyanomethoxy)-2-oxoethyl, 3-(Cyanomethoxy)-3-oxopropyl, 2-[(Arylacyl)methoxy]-2-oxoethyl, 3-[(Arylacyl)methoxy]-3-oxopropyl, 3-(Arylmethoxy)-3-oxopropyl, 3-{[(C₁-C₆)-Alkylacyl]methoxyl}-3-oxopropyl, 3-[(Arylacyl)methoxy]-3-oxopropyl, 3-{[(C₁-C₆)-Alkoxycarbonyl]methoxy}-3-oxopropyl, 2-{[(C₁-C₆)-Alkoxycarbonyl]methoxy}-2-oxoethyl oder 2-{[(C₁-C₆)-Alkylacyl]methoxyl}2-oxoethyl steht,
R² bis R⁷ unabhängig voneinander für Wasserstoff, (C₁-C₆)-Alkyl, Aryl, (C₁-C₈) -Alkoxyl, (C₃-C₈)-Cycloalkyl, durch O unterbrochenes (C₂-C₆)-Alkyl, durch S unterbrochenes (C₂-C₆)-Alkyl oder Halogen stehen,
R⁸ für Wasserstoff, (C₁-C₆)-Alkyl, (C₃-C₄)-Alkenyl, (C₃-C₄)-Alkinyl, Arylmethyl, 2- (C₁-C₆)-Alkoxyl-2-oxoethyl, 2-(Arylmethyl)-2-oxoethyl, Cyano-(C₁-C₆)-alkyl, durch O unterbrochenes (C₁-C₆)-Alkyl oder durch S unterbrochenes (C₁-C₆)-Alkyl steht
und
Y für Carbonyl steht.

17. Farbstoffe nach Anspruch 1 mit der Formel (I-o) 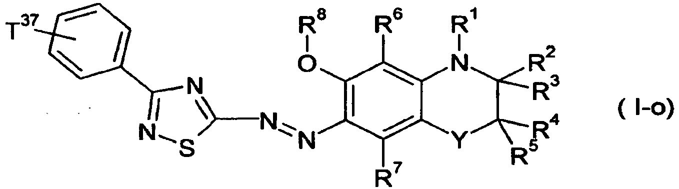 wobei
T³⁷ für Nitro, Wasserstoff oder Halogen steht,
R¹ für Methyl, Ethyl, Propyl, Isopropyl, n-Butyl, Isobutyl, Benzyl, 2-Methoxylethyl, 2-Ethoxylethyl, 2-Acetoxyethyl, 2-(Propionyl)oxyethyl, 2-(Benzoyl)oxyethyl, Cyanomethyl, 2-Cyanoethyl, 3-Cyanopropyl, 3-(C₁-C₆)-Alkoxy-3-oxopropyl, 3-[durch O unterbrochenes (C₂-C₆)-Alkoxyl]-3-oxopropyl, 2-(C₁-C₆)-Alkyl-2-oxoethyl, 2-[durch O unterbrochenes (C₂-C₆)-Alkoxyl]-2-oxoethyl, 2-(Cyanomethoxy)-2-oxoethyl, 3-(Cyanomethoxy)-3-oxopropyl, 2-[(Arylacyl)methoxy]-2-oxoethyl, 3-[(Arylacyl)methoxy]-3-oxopropyl, 3-(Arylmethoxy)-3-oxopropyl, 3-{[(C₁-C₆)-Alkylacyl]methoxyl}-3-oxopropyl, 3-[(Arylacyl)-methoxy]-3-oxopropyl, 3-{[(C₁-C₆)-Alkoxycarbonyl]-methoxy}-3-oxopropyl, 2-{[(C₁-C₆)-Alkoxycarbonyl]-methoxy}-2-oxoethyl oder 2-{[(C₁-C₆)-Alkylacyl]-methoxyl}2-oxoethyl steht,
R² bis R⁷ unabhängig voneinander für Wasserstoff, (C₁-C₆)-Alkyl, Aryl, (C₁-C₈) -Alkoxyl, (C₃-C₈)-Cycloalkyl, durch O unterbrochenes (C₂-C₆)-Alkyl, durch S unterbrochenes (C₂-C₆)-Alkyl oder Halogen stehen,
R⁸ für Wasserstoff, (C₁-C₆)-Alkyl, (C₃-C₄)-Alkenyl, (C₃-C₄)-Alkinyl, Arylmethyl, 2-(C₁-C₆) -Alkoxyl-2-oxoethyl, 2-(Arylmethyl)-2-oxoethyl, Cyano-(C₁-C₆)-alkyl, durch O unterbrochenes (C₁-C₆)-Alkyl oder durch S unterbrochenes (C₁-C₆)-Alkyl steht
und
Y für Carbonyl steht.

18. Verfahren zur Herstellung von Farbstoffen mit der Formel (I) und Mischungen davon, umfassend
a) Diazotierung einer Verbindung der allgemeinen Formel (XD)
D-NH₂ (XD),
wobei
D wie in Anspruch 1 definiert ist, und
b) Umsetzen des erhaltenen entsprechenden Diazoniumsalzes mit einer Verbindung der Formel (XC) 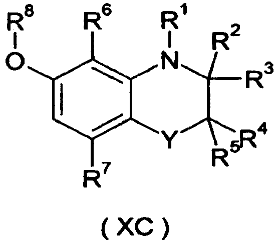 wobei R¹ bis R⁸ und Y jeweils wie in Anspruch 1 definiert sind.

19. Verwendung von Farbstoffen nach Anspruch 1 zum Färben und Bedrucken von hydrophoben Materialien.

## Revendications

1. Colorants de formule (I) et mélanges correspondants : 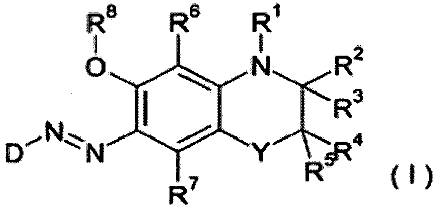
Y étant un groupe de formule générale (II) 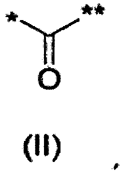
R¹ étant alkyle, alcényle, cycloalkyle, aryle, hétéroaryle, hétérocycloalkyle
ou
alkyle interrompu par un ou plusieurs hétéroatomes choisis dans le groupe constitué par oxygène et soufre
ou
alkyle substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, halogène, cyano, thiocyano, nitro, acyle, thioacyle, alkylsulfonyle, aryloyle, trifluorométhyle, hétéroaryle, hétérocycloalkyle, alcoxythiocarbonyle, acyloxy, aryloyloxy, carbamoyle, *N*-monocycloalkylcarbamoyle, *N-*monoalkylcarbamoyle, *N,N*-dicycloalkylcarbamoyle, *N,N-*dialkylcarbamoyle, *N*-monoarylcarbamoyle, *N,N-*diarylcarbamoyle, *N*-monocycloalkyl-*N*-monoarylcarbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, N,N-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹² ou
alkyle interrompu par un ou plusieurs hétéroatomes choisis dans le groupe constitué par oxygène et soufre et substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, halogène, cyano, thiocyano, nitro, acyle, thioacyle, alkylsulfonyle, aryloyle, trifluorométhyle, hétéroaryle, hétérocycloalkyle, alcoxythiocarbonyle, acyloxy, aryloyloxy, carbamoyle, *N*-monocycloalkylcarbamoyle, *N-*monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoaryl-carbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, N-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹²,
ou
aryle substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, thiocyano, halogène, cyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N*-monoalkylcarbamoyle, *N,N-*dicycloalkylcarbamoyle, *N,N*-dialkylcarbamoyle, *N*-monoarylcarbamoyle, *N,N-*diarylcarbamoyle, *N*-monocycloalkyl-*N*-monoarylcarbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹²,
R² à R⁸ indépendamment les uns des autres étant hydrogène, alkyle, aryle, alcoxyle, cycloalkyle, halogène
ou
alkyle interrompu par un ou plusieurs hétéroatomes choisis dans le groupe constitué par oxygène et soufre
ou
alkyle substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N-*dicycloalkylamino, *N*-monoalkyl-monocycloalkylamino, *N,N*-monoarylmonocycloalkyl-amino, *N*-acylamino, *N*-alkylsulfonylamino, halogène, cyano, thiocyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N-*monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoaryl-carbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹²,
ou
alkyle interrompu par un ou plusieurs hétéroatomes choisis dans le groupe constitué par oxygène et soufre et substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, halogène, cyano, thiocyano, nitro, acyle, thioacyle, alkylsulfonyle, aryloyle, trifluorométhyle,
hétéroaryle, hétérocycloalkyle, alcoxycarbonyle, alcoxythiocarbonyle, acyloxy, aryloyloxy, carbamoyle, *N*-monocycloalkylcarbamoyle, *N-*monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoaryl-carbamoyle, *N*-monoalkyl-*N*-monoarylcarbamoyle, sulfamoyle, *N*-monocycloalkylsulfamoyle, *N-*monoalkylsulfamoyle, *N,N-*dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹²
ou
aryle substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, thiocyano, halogène, cyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N*-monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoaryl-carbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy,
alkylsulfonyloxy, aryloxysulfonyle, alcoxylsulfonyle et COOR¹²,
D représentant un groupe de formule (VI) 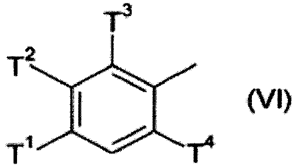
T¹ à T⁴ indépendamment les uns des autres étant hydrogène, alkyle, cycloalkyle, alcényle, alcynyle, aryle, alcoxyle, halogène, cyano, nitro, acyle, aryloyle, arylsulfonyle, alkylsulfonyle, *N-*monoalkylsulfamoyle, *N,N*-dialkylsulfamoyle, alcoxylcarbonyle, aryloxycarbonyle, *N*-monoalkyl-carbamoyle, *N,N*-dialkylcarbamoyle, aryloyloxy, acyloxy, aryloxy, thiocyano, hydroxyle, arylméthoxy, arylsulfonyloxy, alkylsulfonyloxy, aryloxysulfonyle, -[(alcoxycarbonyl)-méthoxyl]carbonyle, [(aryloxycarbonyl)méthoxyl]-carbonyle, [(alkylacyl)méthoxy]carbonyle, [(arylacyl)méthoxy]carbonyle, trifluorométhyle
ou
alkyle interrompu par un ou plusieurs hétéroatomes choisis dans le groupe constitué par oxygène et soufre
ou
alkyle substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, halogène, cyano, thiocyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N-*monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoaryl-carbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹² ou
alkyle interrompu par un ou plusieurs hétéroatomes choisis dans le groupe constitué par oxygène et soufre et substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, halogène, cyano, thiocyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N-*monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoaryl-carbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹²,
ou
aryle substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, thiocyano, halogène, cyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N*-monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoaryl-carbamoyle, *N*-monoalkyl-*N*-monoarylcarbamoyle, sulfamoyle, *N*-monocycloalkylsulfamoyle, *N-*monoalkylsulfamoyle, *N,N-*dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹²,
au moins un parmi T¹, T², T³ et T⁴ n'étant pas hydrogène
ou
D représentant un groupe de formule (VII) 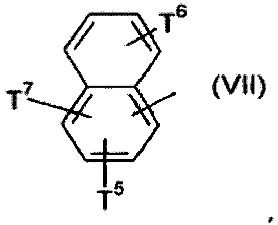
T⁵ à T⁷ indépendamment les uns des autres étant hydrogène, alkyle, cycloalkyle, alcényle, alcynyle, aryle, alcoxyle, halogène, cyano, nitro, acyle, arylacyle, alcoxylcarbonyle, aryloxycarbonyle, [(alcoxycarbonyl)méthoxyl]carbonyle, [(aryloxy-carbonyl)méthoxyl]carbonyle, [(alkylacyl)-méthoxy]carbonyle, [(arylacyl)méthoxy]carbonyle, *N-*monoalkylcarbamoyle, *N,N*-dialkylcarbamoyle
ou
alkyle interrompu par un ou plusieurs hétéroatomes choisis dans le groupe constitué par oxygène et soufre
ou
alkyle substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, halogène, cyano, thiocyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N-*monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoaryl-carbamoyle, *N*-monoalkyl-*N*-monoarylcarbamoyle, sulfamoyle, *N*-monocycloalkylsulfamoyle, *N-*monoalkylsulfamoyle, *N,N-*dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹² ou
alkyle interrompu par un ou plusieurs hétéroatomes choisis dans le groupe constitué par oxygène et soufre et substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, halogène, cyano, thiocyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N-*monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoaryl-carbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹²
ou
aryle substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, thiocyano, halogène, cyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N*-monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoaryl-carbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-mondarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹²,
au moins un parmi T⁵, T⁶ et T⁷ n'étant pas hydrogène, ou
D représentant un groupe de formule (VIII) 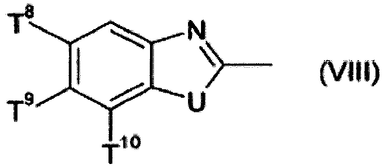
U étant oxygène, soufre ou N-R¹⁵,
T⁸ et T¹⁰ indépendamment l'un de l'autre étant hydrogène, fluor, chlore ou brome,
T⁹ étant hydrogène, alkylsulfonyle, thiocyano, alcoxy, halogène ou nitro,
R¹⁵ étant alkyle, aryle ou cycloalkyle,
ou
D représentant un groupe de formule (IX) 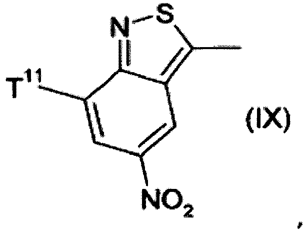 T¹¹ étant hydrogène, nitro ou halogène,
ou
D représentant un groupe de formule (X) 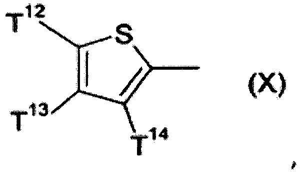
T¹² et T¹⁴ indépendamment l'un de l'autre étant nitro, cyano, acyle ou alcoxycarbonyle,
T¹³ étant hydrogène, aryle, alkyle, halogène
ou
alkyle interrompu par un ou plusieurs hétéroatomes choisis dans le groupe constitué par oxygène et soufre
ou
alkyle substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, halogène, cyano, thiocyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N-*monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoaryl-carbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹² ou
alkyle interrompu par un ou plusieurs hétéroatomes choisis dans le groupe constitué par oxygène et soufre et substitué par un ou plusieurs substituants choisis dans le groupe constitué par hydroxy, aryle, cycloalkyle, alcoxy, thioalcoxy, amino, *N*-mono-alkylamino, *N,N-*dialkylamino, *N*-monoarylamino, *N,N-*diarylamino, *N*-alkyl-N-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, halogène, cyano, thiocyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, N-monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoaryl-carbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹²
ou
aryle substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, thiocyano, halogène, cyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N*-monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoaryl-carbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹²,
ou
D représentant un groupe de formule (XI) 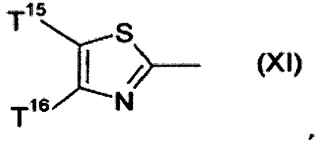
T¹⁵ et T¹⁶ indépendamment l'un de l'autre étant hydrogène, halogène, alkyle, nitro, cyano, acyle, alcoxycarbonyle, aryle
ou
alkyle interrompu par un ou plusieurs hétéroatomes choisis dans le groupe constitué par oxygène et soufre
ou
alkyle substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, halogène, cyano, thiocyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N-*monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoaryl-carbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹² ou
alkyle interrompu par un ou plusieurs hétéroatomes choisis dans le groupe constitué par oxygène et soufre et substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, halogène, cyano, thiocyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, N-monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoaryl-carbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹²
ou
aryle substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, thiocyano, halogène, cyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N*-monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoaryl-carbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹²
au moins un parmi T¹⁵ et T¹⁶ n'étant pas hydrogène, ou
D représentant un groupe de formule (XII) 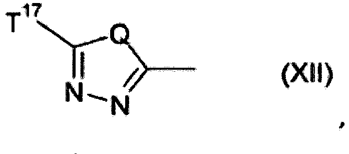
T¹⁷ étant aryle, thioalcoxyle
ou
aryle substitué par un ou plusieurs substituants choisis dans le groupe constitué par hydroxy, aryle, cycloalkyle, alcoxy, thioalcoxy, amino, *N*-mono-alkylamino, *N,N*-dialkylamino, N-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, thiocyano, halogène, cyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N*-monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoaryl-carbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹²,
Q étant oxygène ou soufre,
ou
D représentant un groupe de formule (XIII) 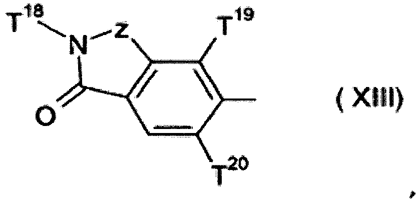
T¹⁸ étant alkyle, alcényle, aryle
ou
alkyle interrompu par un ou plusieurs hétéroatomes choisis dans le groupe constitué par oxygène et soufre
ou
alkyle substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-N-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, halogène, cyano, thiocyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N-*monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoaryl-carbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹² ou
alkyle interrompu par un ou plusieurs hétéroatomes choisis dans le groupe constitué par oxygène et soufre et substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, halogène, cyano, thiocyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N-*monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoaryl-carbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹²
ou
aryle substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-N-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, thiocyano, halogène, cyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N*-monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoaryl-carbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹²,
T¹⁹ et T²⁰ indépendamment l'un de l'autre étant hydrogène, halogène, cyano, nitro ou trifluorométhyle,
Z étant un carbonyle ou un radical sulfonyle,
ou
D représentant un groupe de formule (XIV) 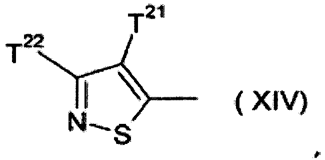
T²¹ étant cyano, nitro ou alcoxycarbonyle,
T²² étant alkyle, hydrogène, halogène, aryle
ou
alkyle interrompu par un ou plusieurs hétéroatomes choisis dans le groupe constitué par oxygène et soufre
ou
alkyle substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-N-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, halogène, cyano, thiocyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N-*monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoaryl-carbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹² ou
alkyle interrompu par un ou plusieurs hétéroatomes choisis dans le groupe constitué par oxygène ou soufre et substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, halogène, cyano, thiocyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N-*monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoaryl-carbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹²
ou
aryle substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, thiocyano, halogène, cyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N*-monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoarylcarbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹²,
ou
D représentant un groupe de formule (XV) 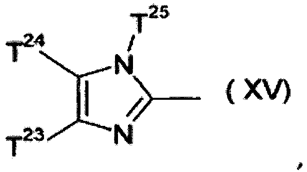
T²³ et T²⁴ indépendamment l'un de l'autre étant cyano, nitro, halogène, aryle ou trifluorométhyle,
T²⁵ étant hydrogène, alkyle, alcényle, aryle
ou
alkyle interrompu par un ou plusieurs hétéroatomes choisis dans le groupe constitué par oxygène et soufre
ou
alkyle substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, halogène, cyano, thiocyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N-*monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoaryl-carbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N-*dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹² ou
alkyle interrompu par un ou plusieurs hétéroatomes choisis dans le groupe constitué par oxygène et soufre et substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, halogène, cyano, thiocyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N-*monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoarylcarbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*moncarylsulfamoyle, *N*-monoalkyl-*N-*monoarylsulfamoyle, arylsulfonyloxy, alkylsulfonyloxy, aryloxysulfonyle, alcoxylsulfonyle et COOR¹²
ou
aryle substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N*,*N*-monoarylmonocycloalkyl-amino, *N*-acylamino, *N*-alkylsulfonylamino, thiocyano, halogène, cyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, N-monocycloalkylcarbamoyle, *N*-monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoarylcarbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N-*dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹²
ou
D représentant un groupe de formule (XVI) 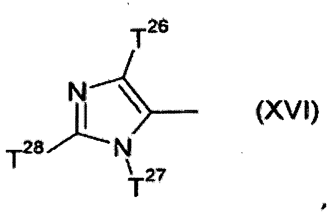
T²⁶ étant cyano, nitro, aryle ou trifluorométhyle,
T²⁷ étant hydrogène, aryle, alkyle
ou
alkyle interrompu par un ou plusieurs hétéroatomes choisis dans le groupe constitué par oxygène et soufre
ou
alkyle substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, halogène, cyano, thiocyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N-*monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N-*monoarylcarbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹² ou
alkyle interrompu par un ou plusieurs hétéroatomes choisis dans le groupe constitué par oxygène et soufre et substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N-*monoarylmonocycloalkyl-amino, *N*-acylamino, *N*-alkylsulfonylamino, halogène, cyano, thiocyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N-*monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoarylcarbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹²
ou
aryle substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, thiocyano, halogène, cyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N*-monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-N-monoarylcarbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹²,
T²⁸ étant hydrogène, alkyle, alcényle, alcynyle, aryle, alcoxycarbonyle
ou
alkyle interrompu par un ou plusieurs hétéroatomes choisis dans le groupe constitué par oxygène et soufre
ou
alkyle substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, halogène, cyano, thiocyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, N-monocycloalkylcarbamoyle, N-monoalkylcarbamoyle, N,N-dicycloalkylcarbamoyle, *N,N*-dialkylcarbamoyle, *N*-monoarylcarbamoyle, *N,N-*diarylcarbamoyle, *N*-monocycloalkyl-*N*-monoarylcarbamoyle, *N*-monoalkyl-N-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl*-N-*monoarylsulfamoyle, arylsulfonyloxy, alkylsulfonyloxy, aryloxysulfonyle, alcoxylsulfonyle et COOR¹² ou
alkyle interrompu par un ou plusieurs hétéroatomes choisis dans le groupe constitué par oxygène et soufre et substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-N-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, halogène, cyano, thiocyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N-*monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoarylcarbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl*-N-*monoarylsulfamoyle, arylsulfonyloxy, alkylsulfonyloxy, aryloxysulfonyle, alcoxylsulfonyle et COOR¹²
ou
aryle substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, thiocyano, halogène, cyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N*-monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoarylcarbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl*-N-*monoarylsulfamoyle, arylsulfonyloxy, alkylsulfonyloxy, aryloxysulfonyle, alcoxylsulfonyle et COOR¹²,
ou
D représentant un groupe de formule (XVII) 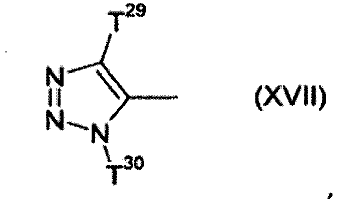
T²⁹ étant cyano, nitro, aryle ou trifluorométhyle,
T³⁰ étant hydrogène, alkyle, alcényle, alcynyle, aryle, arylméthyle, alcoxycarbonyle
ou
alkyle interrompu par un ou plusieurs hétéroatomes choisis dans le groupe constitué par oxygène et soufre
ou
alkyle substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, halogène, cyano, thiocyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N-*monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoarylcarbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl*-N-*monoarylsulfamoyle, arylsulfonyloxy, alkylsulfonyloxy, aryloxysulfonyle, alcoxylsulfonyle et COOR¹² ou
alkyle interrompu par un ou plusieurs hétéroatomes choisis dans le groupe constitué par oxygène et soufre et substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, halogène, cyano, thiocyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N-*monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoarylcarbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹²
ou
aryle substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N-*monoarylmonocycloalkyl-amino, *N*-acylamino, *N*-alkylsulfonylamino, thiocyano, halogène, cyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N*-monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoarylcarbamoyle, *N*-monoalkyl-*N-*monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹²,
ou
D représentant un groupe de formule (XVIII) 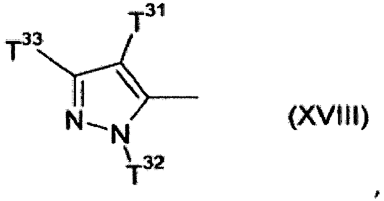
T³¹ étant cyano, nitro, aryle, trifluorométhyle
ou
aryle substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N-*dicycloalkylamino, *N*-monoalkyl-monocycloalkylamino, *N,N*-monoarylmonocycloalkyl-amino, *N*-acylamino, *N*-alkylsulfonylamino, thiocyano, halogène, cyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N*-monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoarylcarbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹²,
T³² étant hydrogène, alkyle, alcényle, alcynyle, aryle, alcoxycarbonyle
ou
alkyle interrompu par un ou plusieurs hétéroatomes choisis dans le groupe constitué par oxygène et soufre
ou
alkyle substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, halogène, cyano, thiocyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N-*monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoarylcarbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹² ou
alkyle interrompu par un ou plusieurs hétéroatomes choisis dans le groupe constitué par oxygène et soufre et substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, halogène, cyano, thiocyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N-*monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoarylcarbamoyle, *N*-monoalkyl-*N*-monoarylcarbamoyle, sulfamoyle, *N*-monocycloalkylsulfamoyle, *N-*monoalkylsulfamoyle, *N,N-*dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹²
ou
aryle substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N-*dicycloalkylamino, *N*-monoalkyl-monocycloalkylamino, *N,N*-monoarylmonocycloalkyl-amino, *N*-acylamino, *N*-alkylsulfonylamino, thiocyano, halogène, cyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N*-monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoarylcarbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹²,
T³³ étant hydrogène, alkyle
ou
alkyle interrompu par un ou plusieurs hétéroatomes choisis dans le groupe constitué par oxygène et soufre
ou
alkyle substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, halogène, cyano, thiocyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N-*monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl*-N*-monoarylcarbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹² ou
alkyle interrompu par un ou plusieurs hétéroatomes choisis dans le groupe constitué par oxygène et soufre et substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N-*dicycloalkylamino, *N*-monoalkyl-monocycloalkylamino, *N,N*-monoarylmonocycloalkyl-amino, *N*-acylamino, *N*-alkylsulfonylamino, halogène, cyano, thiocyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N-*monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoarylcarbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹²
ou
aryle substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, thiocyano, halogène, cyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, N-monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoarylcarbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹²,
ou
D représentant un groupe de formule (XIX) 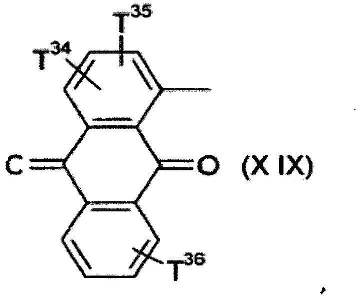 T³⁴, T³⁵ et T³⁶ indépendamment les uns des autres étant hydrogène, halogène, nitro, cyano, hydroxyle, alcoxylcarbonyle, alkyle, acyle
ou
alkyle substitué par un ou plusieurs substituants choisis dans le groupe constitué par hydroxy, aryle, cycloalkyle, alcoxy, thioalcoxy, amino, *N*-mono-alkylamino, *N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N*-monoarylmonocycloalkyl-amino, *N*-acylamino, *N*-alkylsulfonylamino, halogène, cyano, thiocyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N-*monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoarylcarbamoyle, *N*-monoalkyl-*N*-monoarylcarbamoyle, sulfamoyle, *N*-monocycloalkylsulfamoyle, *N-*monoalkylsulfamoyle, *N,N-*dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl*-N-*monoarylsulfamoyle, arylsulfonyloxy, alkylsulfonyloxy, aryloxysulfonyle, alcoxylsulfonyle et COOR¹² ou
alkyle interrompu par un ou plusieurs hétéroatomes choisis dans le groupe constitué par oxygène et soufre et substitué par un ou plusieurs substituants choisis dans le groupe constitué par hydroxy, aryle, cycloalkyle, alcoxy, thioalcoxy, amino, *N*-mono-alkylamino*, N,N*-dialkylamino, *N*-monoarylamino, *N,N-*diarylamino, *N*-alkyl-*N*-arylamino, *N*-monocyclo-alkylamino, *N,N*-dicycloalkylamino, *N*-monoalkyl-monocycloalkylamino, *N,N-*monoarylmonocycloalkylamino, *N*-acylamino, *N*-alkylsulfonylamino, halogène, cyano, thiocyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, *N*-monocycloalkylcarbamoyle, *N-*monoalkylcarbamoyle, *N,N*-dicycloalkylcarbamoyle, *N,N*-dialkylcarbamoyle, *N*-monoarylcarbamoyle, *N,N-*diarylcarbamoyle, *N*-monocycloalkyl-*N*-monoarylcarbamoyle, *N*-monoalkyl-*N*-monoarylcarbamoyle, sulfamoyle, *N*-monocycloalkylsulfamoyle, *N-*monoalkylsulfamoyle, *N,N*-dicycloalkylsulfamoyle, *N,N*-dialkylsulfamoyle, *N*-monoarylsulfamoyle, *N,N-*diarylsulfamoyle, *N*-monocycloalkyl-*N-*monoarylsulfamoyle, *N*-monoalkyl-*N-*monoarylsulfamoyle, arylsulfonyloxy, alkylsulfonyloxy, aryloxysulfonyle, alcoxylsulfonyle et COOR¹²,
ou
D représentant un groupe de formule (XX) 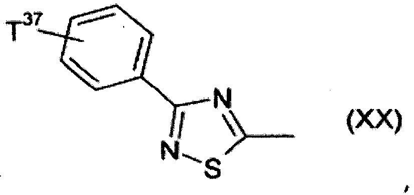
T³⁷ étant hydrogène, halogène ou nitro,
R¹² étant hydrogène, alkyle, alcényle, cycloalkyle, aryle, hétéroaryle, hétérocycloalkyle
ou
alkyle interrompu par un ou plusieurs hétéroatomes choisis dans le groupe constitué par oxygène et soufre
ou
alkyle substitué par un ou plusieurs substituants choisis dans le groupe constitué par hydroxy, aryle, cycloalkyle, alcoxy, thioalcoxy, halogène, cyano, amino, thiocyano, nitro, acyle, thioacyle, alkylsulfonyle, aryloyle, trifluorométhyle, hétéroaryle, hétérocycloalkyle, alcoxythiocarbonyle, acyloxy, aryloyloxy, carbamoyle, sulfamoyle, arylsulfonyloxy, alkylsulfonyloxy, aryloxysulfonyle et alcoxylsulfonyle
ou
alkyle interrompu par un ou plusieurs hétéroatomes choisis dans le groupe constitué par oxygène et soufre et substitué par un ou plusieurs substituants choisis dans le groupe constitué par hydroxy, aryle, cycloalkyle, alcoxy, thioalcoxy, halogène, cyano, amino, thiocyano, nitro, acyle, thioacyle, alkylsulfonyle, aryloyle, trifluorométhyle, hétéroaryle, hétérocycloalkyle, alcoxythiocarbonyle, acyloxy, aryloyloxy, carbamoyle, sulfamoyle, arylsulfonyloxy, alkylsulfonyloxy, aryloxysulfonyle et alcoxylsulfonyle
ou
aryle substitué par un ou plusieurs substituants choisis dans le groupe constitué par hydroxy, aryle, cycloalkyle, alcoxy, thioalcoxy, amino, thiocyano, halogène, cyano, nitro, acyle, thioacyle, alkylsulfonyle, aryloyle, trifluorométhyle, hétéroaryle, hétérocycloalkyle, alcoxycarbonyle, alcoxythiocarbonyle, acyloxy, aryloyloxy, carbamoyle, sulfamoyle, arylsulfonyloxy, alkylsulfonyloxy, aryloxysulfonyle et alcoxylsulfonyle,
étant entendu qu'aucune formation de cycle condensé n'est permise parmi R² à R¹⁴.

2. Colorants selon la revendication 1 possédant la formule (I-a) 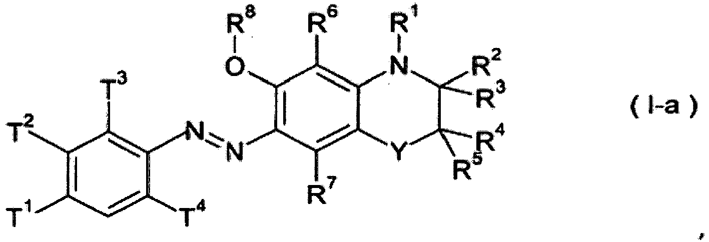
chacun de T¹ à T⁴ indépendamment les uns des autres étant hydrogène, (C₁-C₆) -alkyle, (C₃-C₆)-cycloalkyle, (C₁-C₆) -alkyle substitué, (C₃-C₄)-alcényle, (C₃-C₄)-alcynyle, aryle, (C₁-C₆) -alkyle interrompu par O, (C₁-C₆) -alkyle interrompu par S, (C₁-C₈) -alcoxyle, (C₁-C₆) -cycloalkyle, halogène, cyano, nitro, arylsulfonyle, arylsulfonyloxy, (C₁-C₆)-alkylsulfonyle, (C₁-C₆) -alcoxylsulfonyle, *N*-(C₁-C₆)-alkylsulfamoyle, *N,N*-bis (C₁-C₆) alkylsulfamoyle, *N*-(C₁-C₆) -alkylcarbamoyle, *N,N*-bis(C₁-C₆)alkylcarbamoyle, (C₁-C₆)-alkylacyle, arylacyle, (C₁-C₆)-alcoxylcarbonyle, aryloxylcarbonyle, thiocyano, hydroxyle, aryloxy, arylméthoxyle, aryloyloxy, arylsulfonyloxy, {[(C₁-C₆)-alcoxylcarbonyl]méthoxyl}carbonyle, [(aryloxy-carbonyl)méthoxy]carbonyle, {[(C₁-C₆)-alkylacyl]-méthoxy}carbonyle ou [(arylacyl)méthoxy]carbonyle, R¹ étant (C₁-C₆)-alkyle, (C₂-C₆)-alkyle substitué par (C₁-C₆)-acyloxy, cyano- (C₁-C₆) -alkyle, arylméthyle, arylacyl-(C₁-C₆)alkyle, 2-(C₁-C₆) -alcoxyl-2-oxoéthyle, (C₂-C₆)-alkyle interrompu par O, (C₂-C₆)-alkyle interrompu par S, (C₃-C₄)-alcényle, (C₃-C₄)-alcynyle, 3-(C₁-C₆)-alcoxyl-3-oxopropyle, 3-[(C₂-C₆)-alcoxyl interrompu par O]-3-oxopropyle, 2-(C₁-C₆)-alcoxyl-2-oxoéthyle, 2-[(C₂-C₆)-alcoxyl interrompu par O]-2-oxoéthyle, 2-{[(C₁-C₆)-alkylacyl]méthoxy}-2-oxoéthyle, 3-{[(C₁-C₆)-alkylacyl]méthoxy}-3-oxopropyle, 2-[(arylacyl)-méthoxy]-2-oxoéthyle, 3-[(arylacyl)méthoxy]-3-oxopropyle, 3-{[(C₁-C6)-alcoxylcarbonyl]méthoxy}-3-oxopropyle, 3-(arylméthoxy)-3-oxopropyle, 3-[(arylacyl)méthoxy]-3-oxopropyle, 2-(cyanométhoxy)-2-oxoéthyle, 3-(cyanométhoxy)-3-oxopropyle, 2-{[(C₁-C₆)-alcoxylcarbonyl]méthoxy}-2-oxoéthyle, 1-[(C₁-C₆)-alkyl]-3-[(C₁-C₆)-alcoxyl]-3-oxopropyle, 1-[(C₁-C₆) -alkyl]-3-[(C₁-C₆)-alcoxyl interrompu par O]-3-oxopropyle, 1-[(C₁-C₆)-alkyl]-3-(arylméthoxyl)-3-oxopropyle, 1-[(C₁-C₆)-alkyl]-3-{[(C₁-C₆)-alkylacyl]méthoxy}-3-oxopropyle ou 1-[(C₁-Cₑ)-alkyl]-3-{[(C₁-C₆)-alcoxylcarbonyl]méthoxy}-3-oxopropyle,
R² à R⁷ indépendamment les uns des autres étant hydrogène, (C₁-C₆) -alkyle, aryle, (C₁-C₈) -alcoxyle, (C₃-C₈)-cycloalkyle, (C₂-C₆)-alkyle interrompu par O, (C₂-C₆)-alkyle interrompu par S ou halogène,
R⁸ étant hydrogène, (C₁-C₆) -alkyle, (C₃-C₄)-alcényle, (C₃-C₄)-alcynyle, arylméthyle, 2-(C₁-C₆)-alcoxyl-2-oxoéthyle, 2-(arylméthyl)-2-oxoéthyle, cyano-(C₁-C₆)-alkyle, (C₁-C₆) -alkyle interrompu par O ou (C₁-C₆)-alkyle interrompu par S
et
Y étant carbonyle.

3. Colorants selon la revendication 1 ou 2 possédant la formule (I-a),
T¹, T³ et T⁴ indépendamment l'un de l'autre étant hydrogène, nitro, cyano, brome, chlore, benzoxyle, phénoxy, méthoxy, éthoxy, méthyle, trifluorométhyle, éthyle, benzoyle, acétyle, hydroxyle, méthoxycarbonyle, éthoxycarbonyle, méthylsulfonyle, cyanométhylsulfonyle, acétonylsulfonyle, (2-éthoxy-2-oxoéthyl)sulfonyle, (2-méthoxy-2-oxoéthyl)sulfonyle, phénylsulfonyloxy, phénoxylsulfonyle, éthoxylsulfonyle, phénoxylsulfonyle, cyanométhoxylcarbonyle, (2-éthoxy-2-oxoéthyl)carbonyle, (2-méthoxy-2-oxoéthyl)carbonyle, acétonylcarbonyle ou phénacylcarbonyle,
T² étant hydrogène, chlore, brome, méthoxy, éthoxyle, phénoxy, benzoxy, nitro, méthyle ou éthyle,
R¹ étant méthyle, éthyle, propyle, isopropyle, n-butyle, isobutyle, benzyle, 2-méthoxyléthyle, 2-éthoxyléthyle, 2-acétoxyéthyle, 2-(propionyl)oxyéthyle, 2-(benzoyl)oxyéthyle, cyanométhyle, 2-cyanoéthyle, 3-cyanopropyle, 3-(C₁-C₆)-alcoxyl-3-oxopropyle, 3-[(C₂-C₆)-alcoxyl interrompu par O]-3-oxopropyle, 2-(C₁-C₆)-alkyl-2-oxoéthyle, 2-[(C₂-C₆)-alcoxyl interrompu par O]-2-oxoéthyle, 2-(cyanométhoxy)-2-oxoéthyle, 3-(cyanométhoxy)-3-oxopropyle, 2-[(arylacyl)-méthoxy]-2-oxoéthyle, 3-[(arylacyl)méthoxy]-3-oxopropyle, 3-(arylméthoxy)-3-oxopropyle, 3-{[(C₁-C₆)-alkylacyl]méthoxyl}-3-oxopropyle, 3-[(arylacyl)méthoxy]-3-oxopropyle, 3-{[(C₁-C₆)-alcoxycarbonyl]méthoxy}-3-oxopropyle, 2-{[(C₁-C₆)-alcoxycarbonyl]méthoxy}-2-oxoéthyle ou 2-{[(C₁-C₆) - alkylacyl]méthoxyl}-2-oxoéthyle,
R² à R⁷ indépendamment les uns des autres étant hydrogène, brome, chlore, éthyle ou méthyle,
R⁸ étant hydrogène, méthyle, éthyle, propyle, isopropyle, 2-éthoxy-2-oxoéthyle ou 2-méthoxy-2-oxoéthyle
et
Y étant carbonyle.

4. Colorants selon la revendication 1 possédant la formule (I-b) 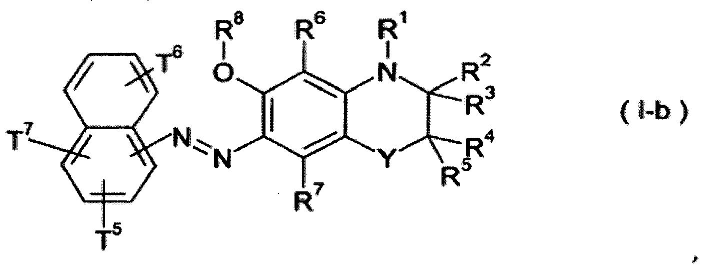
T⁵ à T⁷ indépendamment les uns des autres étant hydrogène, (C₁-C₆) -alkyle, (C₃-C₈) -cycloalkyle, (C₁-C₆)-alkyle interrompu par O, (C₁-C₆) -alkyle interrompu par S, aryle, (C₁-C₆)-alcoxyle, halogène, cyano, nitro, (C₁-C₆)-alkylacyle, arylacyle, (C₁-C₆)-alcoxylcarbonyle, aryloxycarbonyle, *N*-(C₁-C₆)-alkylcarbamoyle, ou *N,N*-bis(C₁-C₆)alkylcarbamoyle, au moins un parmi T⁵, T⁶ et T⁷ n'étant pas hydrogène, R¹ étant méthyle, éthyle, propyle, isopropyle, n-butyle, isobutyle, benzyle, 2-méthoxyléthyle, 2-éthoxyléthyle, 2-acétoxyéthyle, 2-(propionyl)oxyéthyle, 2-(benzoyl)oxyéthyle, cyanométhyle, 2-cyanoéthyle, 3-cyanopropyle, 3-(C₁-C₆)-alcoxyl-3-oxopropyle, 3-[(C₂-C₆)-alcoxyl interrompu par O]-3-oxopropyle, 2-(C₁-C₆)-alkyl-2-oxoéthyle, 2-[(C₂-C₆)-alcoxyl interrompu par O]-2-oxoéthyle, 2-(cyanométhoxy)-2-oxoéthyle, 3-(cyanométhoxy)-3-oxopropyle, 2-[(arylacyl)-méthoxy]-2-oxoéthyle, 3-[(arylacyl)méthoxy]-3-oxopropyle, 3-(arylméthoxy)-3-oxopropyle, 3-{[(C₁-C₆)-alkylacyl]méthoxyl}-3-oxopropyle, 3-[(arylacyl)méthoxy]-3-oxopropyle, 3-{[(C₁-C₆)-alcoxycarbonyl]méthoxy}-3-oxopropyle, 2-{[(C₁-C₆)-alcoxycarbonyl]méthoxy}-2-oxoéthyle ou 2-{[(C₁-C₆)-alkylacyl]méthoxyl}-2-oxoéthyle,
R² à R⁷ indépendamment les uns des autres étant hydrogène, (C₁-C₆) -alkyle, aryle, (C₁-C₆) -alcoxyle, (C₃-C₈)-cycloalkyle, (C₂-C₆)-alkyle interrompu par O, (C₂-C₆)-alkyle interrompu par S ou halogène,
R⁸ étant hydrogène, (C₁-C₆) -alkyle, (C₃-C₄)-alcényle, (C₃-C₄)-alcynyle, arylméthyle, 2-(C₁-C₆) -alcoxyl-2-oxoéthyle, 2-(arylméthyl)-2-oxoéthyle, cyano-(C₁-C₆)-alkyle, (C₁-C₆) -alkyle interrompu par O ou (C₁-C₆)-alkyle interrompu par S
et
Y étant carbonyle.

5. Colorants selon la revendication 1 possédant la formule (I-c) 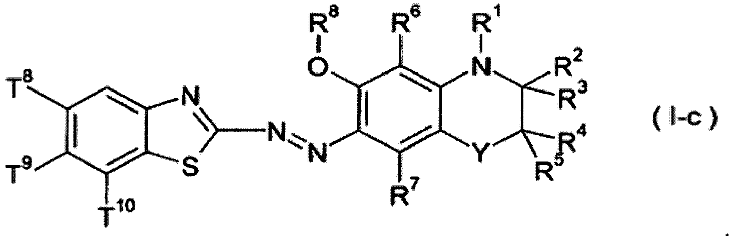
T⁸ et T¹⁰ indépendamment l'un de l'autre étant hydrogène, chlore ou brome,
T⁹ étant hydrogène, (C₁-C₆)-alkylsulfonyle, thiocyano, (C₁-C₆)-alcoxy, chlore ou nitro,
R¹ étant méthyle, éthyle, propyle, isopropyle, n-butyle, isobutyle, benzyle, 2-méthoxyléthyle, 2-éthoxyléthyle, 2-acétoxyéthyle, 2-(propionyl)oxyéthyle, 2-(benzoyl)oxyéthyle, cyanométhyle, 2-cyanoéthyle, 3-cyanopropyle, 3-(C₁-C₆)-alcoxyl-3-oxopropyle, 3-[(C₂-C₆)-alcoxyl interrompu par O]-3-oxopropyle, 2-(C₁-C₆)-alkyl-2-oxoéthyle, 2-[(C₂-C₆)-alcoxyl interrompu par O]-2-oxoéthyle, 2-(cyanométhoxy)-2-oxoéthyle, 3-(cyanométhoxy)-3-oxopropyle, 2-[(arylacyl)-méthoxy]-2-oxoéthyle, 3-[(arylacyl)méthoxy]-3-oxopropyle, 3-(arylméthoxy)-3-oxopropyle, 3-{[(C₁-C₆)-alkylacyl]méthoxyl}-3-oxopropyle, 3-[(arylacyl)méthoxy]-3-oxopropyle, 3-{[(C₁-C₆)-alcoxycarbonyl]méthoxy}-3-oxopropyle, 2-{[(C₁-C₆)-alcoxycarbonyl]méthoxy}-2-oxoéthyle ou 2-1[(C₁-C₆)-alkylacyl]méthoxyl}-2-oxoéthyle,
R² à R⁷ indépendamment les uns des autres étant hydrogène, (C₁-C₆) -alkyle, aryle, (C₁-C₈)-alcoxyle, (C₃-C₈)-cycloalkyle, (C₂-C₆)-alkyle interrompu par O, (C₂-C₆)-alkyle interrompu par S ou halogène,
R⁸ étant hydrogène, (C₁-C₆) -alkyle, (C₃-C₄)-alcényle, (C₃-C₄)-alcynyle, arylméthyle, 2-(C₁-C₆)-alcoxyl-2-oxoéthyle, 2-(arylméthyl)-2-oxoéthyle, cyano-(C₁-C₆)-alkyle, (C₁-C₆) -alkyle interrompu par O ou (C₁-C₆)-alkyle interrompu par S
et
Y étant carbonyle.

6. Colorants selon la revendication 1 possédant la formule (I-d) 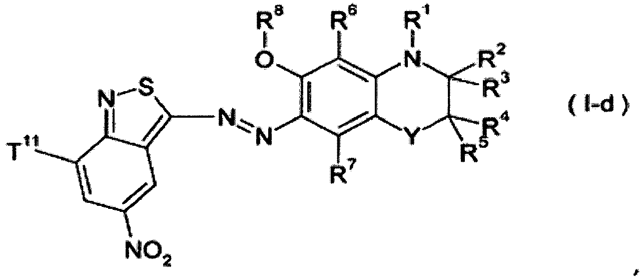
T¹¹ étant hydrogène, nitro ou brome,
R¹ étant méthyle, éthyle, propyle, isopropyle, n-butyle, isobutyle, benzyle, 2-méthoxyléthyle, 2-éthoxyléthyle, 2-acétoxyéthyle, 2-(propionyl)oxyéthyle, 2-(benzoyl)oxyéthyle, cyanométhyle, 2-cyanoéthyle, 3-cyanopropyle, 3-(C₁-C₆) -alcoxyl-3-oxopropyle, 3-[(C₂-C₆)-alcoxyl interrompu par O]-3-oxopropyle, 2-(C₁-C₆) -alkyl-2-oxoéthyle, 2-[(C₂-C₆)-alcoxyl interrompu par O]-2-oxoéthyle, 2-(cyanométhoxy)-2-oxoéthyle, 3-(cyanométhoxy)-3-oxopropyle, 2-[(arylacyl)-méthoxy]-2-oxoéthyle, 3-[(arylacyl)méthoxy]-3-oxopropyle, 3-(arylméthoxy)-3-oxopropyle, 3-{[(C₁-C₆)-alkylacyl]méthoxyl}-3-oxopropyle, 3-[(arylacyl)méthoxy]-3-oxopropyle, 3-{[(C₁-C₆)-alcoxycarbonyl]méthoxy}-3-oxopropyle, 2-{[(C₁-C₆)-alcoxycarbonyl]méthoxy}-2-oxoéthyle ou 2-{[(C₁-C₆)-alkylacyl]méthoxyl}-2-oxoéthyle,
R² à R⁷ indépendamment les uns des autres étant hydrogène, (C₁-C₆) -alkyle, aryle, (C₁-C₈) -alcoxyle, (C₃-C₈) -cycloalkyle, (C₂-C₆)-alkyle interrompu par O, (C₂-C₆)-alkyle interrompu par S ou halogène,
R⁸ étant hydrogène, (C₁-C₆) -alkyle, (C₃-C₄)-alcényle, (C₃-C₄)-alcynyle, arylméthyle, 2-(C₁-C₆)-alcoxyl-2-oxoéthyle, 2-(arylméthyl)-2-oxoéthyle, cyano-(C₁-C₆)-alkyle, (C₁-C₆) -alkyle interrompu par O ou (C₁-C₆)-alkyle interrompu par S
et
Y étant carbonyle.

7. Colorants selon la revendication 1 possédant la formule (I-e) 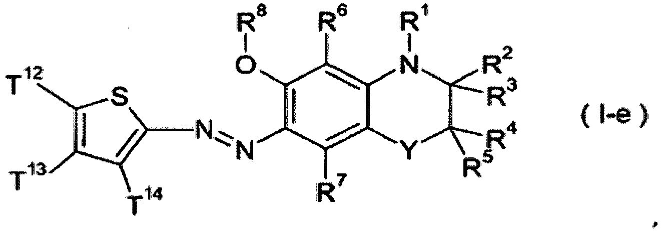
T¹² et T¹⁴ indépendamment l'un de l'autre étant nitro, cyano, formyle, acétyle ou (C₁-C₆)-alcoxycarbonyle,
T¹³ étant hydrogène, aryle, (C₁-C₆) -alkyle ou halogène,
R¹ étant méthyle, éthyle, propyle, isopropyle, *n-*butyle, isobutyle, benzyle, 2-méthoxyléthyle, 2-éthoxyléthyle, 2-acétoxyéthyle, 2-(propionyl)oxyéthyle, 2-(benzoyl)oxyéthyle, cyanométhyle, 2-cyanoéthyle, 3-cyanopropyle, 3-(C₁-C₆)-alcoxyl-3-oxopropyle, 3-[(C₂-C₆)-alcoxyl interrompu par O]-3-oxopropyle, 2-(C₁-C₆)-alkyl-2-oxoéthyle, 2-[(C₂-C₆)-alcoxyl interrompu par O]-2-oxoéthyle, 2-(cyanométhoxy)-2-oxoéthyle, 3-(cyanométhoxy)-3-oxopropyle, 2-[(arylacyl)-méthoxy]-2-oxoéthyle, 3-[(arylacyl)méthoxy]-3-oxopropyle, 3-(arylméthoxy)-3-oxopropyle, 3-{[(C₁-C₆)-alkylacyl]méthoxyl}-3-oxopropyle, 3-[(arylacyl)méthoxy]-3-oxopropyle, 3-{[(C₁-C₆)-alcoxycarbonyl]méthoxy}-3-oxopropyle, 2-{[(C₁-C₆)-alcoxycarbonyl]méthoxy}-2-oxoéthyle ou 2-{[(C₁-C₆)-alkylacyl]méthoxyl}-2-oxoéthyle,
R² à R⁷ indépendamment les uns des autres étant hydrogène, (C₁-C₆) -alkyle, aryle, (C₁-C₈) -alcoxyle, (C₃-C₈)-cycloalkyle, (C₂-C₆)-alkyle interrompu par O, (C₂-C₆)-alkyle interrompu par S ou halogène,
R⁸ étant hydrogène, (C₁-C₆) -alkyle, (C₃-C₄)-alcényle, (C₃-C₄)-alcynyle, arylméthyle, 2-(C₁-C₆)-alcoxyl-2-oxoéthyle, 2-(arylméthyl)-2-oxoéthyle, cyano-(C₁-C₆)-alkyle, (C₁-C₆) -alkyle interrompu par O ou (C₁-C₆)-alkyle interrompu par S
et
Y étant carbonyle.

8. Colorants selon la revendication 1 possédant la formule (I-f) 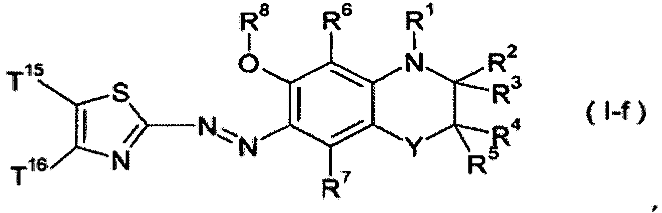
T¹⁵ et T¹⁶ indépendamment l'un de l'autre étant hydrogène, halogène, (C₁-C₆)-alkyle, nitro, cyano, formyle, acétyle, (C₁-C₆) -alcoxycarbonyle, (C₁-C₆)-alkyle interrompu par O, (C₁-C₆)-alkyle interrompu par S ou aryle,
R¹ étant méthyle, éthyle, propyle, isopropyle, n-butyle, isobutyle, benzyle, 2-méthoxyléthyle, 2-éthoxyléthyle, 2-acétoxyéthyle, 2-(propionyl)oxyéthyle, 2-(benzoyl)oxyéthyle, cyanométhyle, 2-cyanoéthyle, 3-cyanopropyle, 3-(C₁-C₆)-alcoxyl-3-oxopropyle, 3-[(C₂-C₆)-alcoxyl interrompu par O]-3-oxopropyle, 2-(C₁-C₆)-alkyl-2-oxoéthyle, 2-[(C₂-C₆)-alcoxyl interrompu par O]-2-oxoéthyle, 2-(cyanométhoxy)-2-oxoéthyle, 3-(cyanométhoxy)-3-oxopropyle, 2-[(arylacyl)-méthoxy]-2-oxoéthyle, 3-[(arylacyl)méthoxy]-3-oxopropyle, 3-(arylméthoxy)-3-oxopropyle, 3-{[(C₁-C₆)-alkylacyl]méthoxyl}-3-oxopropyle, 3-[(arylacyl)méthoxy]-3-oxopropyle, 3-{[(C₁-C₆)-alcoxycarbonyl]méthoxy}-3-oxopropyle, 2-{[(C₁-C₆)-alcoxycarbonyl]méthoxy}-2-oxoéthyle ou 2-{[(C₁-C₆)-alkylacyl]méthoxyl}-2-oxoéthyle,
R² à R⁷ indépendamment les uns des autres étant hydrogène, (C₁-C₆) -alkyle, aryle, (C₁-C₈)-alcoxyle, (C₃-C₈)-cycloalkyle, (C₂-C₆)-alkyle interrompu par O, (C₂-C₆)-alkyle interrompu par S ou halogène,
R⁸ étant hydrogène, (C₁-C₆) -alkyle, (C₃-C₄)-alcényle, (C₃-C₄)-alcynyle, arylméthyle, 2-(C₁-C₆)-alcoxyl-2-oxoéthyle, 2-(arylméthyl)-2-oxoéthyle, cyano-(C₁-C₆)-alkyle, (C₁-C₆) -alkyle interrompu par O ou (C₁-C₆)-alkyle interrompu par S
et
Y étant carbonyle.

9. Colorants selon la revendication 1 possédant la formule (I-g) 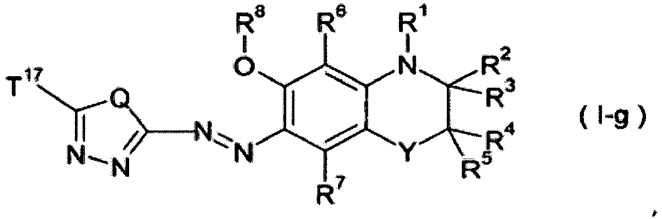
T¹⁷ étant aryle ou (C₁-C₆)-thioalcoxyle,
Q étant oxygène ou soufre,
R¹ étant méthyle, éthyle, propyle, isopropyle, n-butyle, isobutyle, benzyle, 2-méthoxyléthyle, 2-éthoxyléthyle, 2-acétoxyéthyle, 2-(propionyl)oxyéthyle, 2-(benzoyl)oxyéthyle, cyanométhyle, 2-cyanoéthyle, 3-cyanopropyle, 3-(C₁-C₆)-alcoxyl-3-oxopropyle, 3-[(C₂-C₆)-alcoxyl interrompu par O]-3-oxopropyle, 2-(C₁-C₆)-alkyl-2-oxoéthyle, 2-[(C₂-C₆)-alcoxyl interrompu par O]-2-oxoéthyle, 2-(cyanométhoxy)-2-oxoéthyle, 3-(cyanométhoxy)-3-oxopropyle, 2-[(arylacyl)-méthoxy]-2-oxoéthyle, 3-[(arylacyl)méthoxy]-3-oxopropyle, 3-(arylméthoxy)-3-oxopropyle, 3-{[(C₁-C₆)-alkylacyl]méthoxyl}-3-oxopropyle, 3-[(arylacyl)méthoxy]-3-oxopropyle, 3-{[(C₁-C₆)-alcoxycarbonyl]méthoxy}-3-oxopropyle, 2-{[(C₁-C₆)-alcoxycarbonyl]méthoxy}-2-oxoéthyle ou 2-{[(C₁-C₆)-alkylacyl]méthoxyl}-2-oxoéthyle,
R² à R⁷ indépendamment les uns des autres étant hydrogène, (C₁-C₆) -alkyle, aryle, (C₁-C₈) -alcoxyle, (C₃-C₈)-cycloalkyle, (C₂-C₆)-alkyle interrompu par O, (C₂-C₆)-alkyle interrompu par S, halogène,
R⁸ étant hydrogène, (C₁-C₆)-alkyle, (C₃-C₄)-alcényle, (C₃-C₄)-alcynyle, arylméthyle, 2-(C₁-C₆)-alcoxyl-2-oxoéthyle, 2-(arylméthyl)-2-oxoéthyle, cyano-(C₁-C₆)-alkyle, (C₁-C₆) -alkyle interrompu par O ou (C₁-C₆)-alkyle interrompu par S
et
Y étant carbonyle.

10. Colorants selon la revendication 1 possédant la formule (I-h) 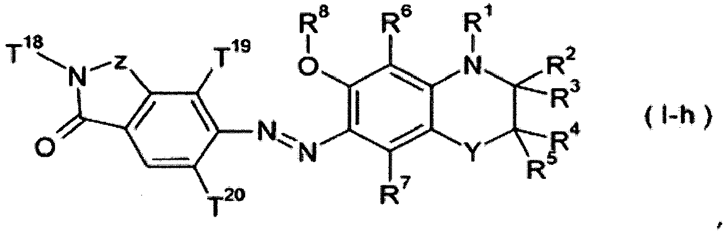
T¹⁸ étant (C₁-C₆) -alkyle, (C₂-C₆)-alkyle substitué par (C₁-C₆)-acyloxy, cyano-(C₁-C₆) -alkyle, arylméthyle, (C₁-C₆) alkyle substitué par arylcarbonyle, 2-(C₁-C₆)-alcoxyl-2-oxoéthyle, (C₁-C₆)-alkyle interrompu par O, (C₁-C₆) -alkyle interrompu par S ou aryle,
T¹⁹ et T²⁰ indépendamment l'un de l'autre étant hydrogène, halogène, cyano, nitro ou trifluorométhyle,
Z étant un carbonyle ou un radical sulfonyle,
R¹ étant méthyle, éthyle, propyle, isopropyle, n-butyle, isobutyle, benzyle, 2-méthoxyléthyle, 2-éthoxyléthyle, 2-acétoxyéthyle, 2-(propionyl)oxyéthyle, 2-(benzoyl)oxyéthyle, cyanométhyle, 2-cyanoéthyle, 3-cyanopropyle, 3-(C₁-C₆)-alcoxyl-3-oxopropyle, 3-[(C₂-C₆)-alcoxyl interrompu par O]-3-oxopropyle, 2-(C₁-C₆)-alkyl-2-oxoéthyle, 2-[(C₂-C₆)-alcoxyl interrompu par O]-2-oxoéthyle, 2-(cyanométhoxy)-2-oxoéthyle, 3-(cyanométhoxy)-3-oxopropyle, 2-[(arylacyl)-méthoxy]-2-oxoéthyle, 3-[(arylacyl)méthoxy]-3-oxopropyle, 3-(arylméthoxy)-3-oxopropyle, 3-{[(C₁-C₆)-alkylacyl]méthoxyl}-3-oxopropyle, 3-[(arylacyl)méthoxy]-3-oxopropyle, 3-{[(C₁-C₆)-alcoxycarbonyl]méthoxy}-3-oxopropyle, 2-{[(C₁-C₆)-alcoxycarbonyl]méthoxy}-2-oxoéthyle ou 2-{[(C₁-C₆)-alkylacyl]méthoxyl}-2-oxoéthyle,
R² à R⁷ indépendamment les uns des autres étant hydrogène, (C₁-C₆)-alkyle, aryle, (C₁-C₈) -alcoxyle, (C₃-C₈) -cycloalkyle, (C₂-C₆)-alkyle interrompu par O, (C₂-C₆)-alkyle interrompu par S ou halogène,
R⁸ étant hydrogène, (C₁-C₆)-alkyle, (C₃-C₄)-alcényle, (C₃-C₄)-alcynyle, arylméthyle, 2-(C₁-C₆)-alcoxyl-2-oxoéthyle, 2-(arylméthyl)-2-oxoéthyle, cyano-(C₁-C₆)-alkyle, (C₁-C₆) -alkyle interrompu par O ou (C₁-C₆)-alkyle interrompu par S
et
Y étant carbonyle.

11. Colorants selon la revendication 1 possédant la formule (I-i) 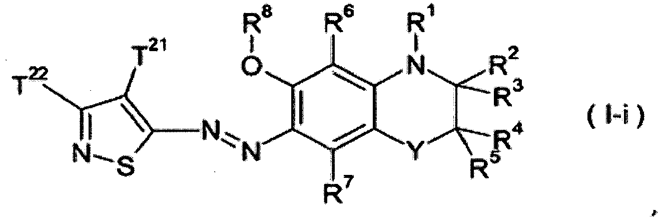
T²¹ étant cyano, nitro ou (C₁-C₆)-alcoxycarbonyle,
T²² étant (C₁-C₆)-alkyle, hydrogène, halogène ou aryle,
R¹ étant méthyle, éthyle, propyle, isopropyle, n-butyle, isobutyle, benzyle, 2-méthoxyléthyle, 2-éthoxyléthyle, 2-acétoxyéthyle, 2-(propionyl)oxyéthyle, 2-(benzoyl)oxyéthyle, cyanométhyle, 2-cyanoéthyle, 3-cyanopropyle, 3-(C₁-C₆)-alcoxyl-3-oxopropyle, 3-[(C₂-C₆)-alcoxyl interrompu par O]-3-oxopropyle, 2-(C₁-C₆)-alkyl-2-oxoéthyle, 2-[(C₂-C₆)-alcoxyl interrompu par O]-2-oxoéthyle, 2-(cyanométhoxy)-2-oxoéthyle, 3-(cyanométhoxy)-3-oxopropyle, 2-[(arylacyl)-méthoxy]-2-oxoéthyle, 3-[(arylacyl)méthoxy]-3-oxopropyle, 3-(arylméthoxy)-3-oxopropyle, 3-{[(C₁-C₆)-alkylacyl]méthoxyl}-3-oxopropyle, 3-[(arylacyl)méthoxy]-3-oxopropyle, 3-{[(C₁-C₆)-alcoxycarbonyl]méthoxy}-3-oxopropyle, 2-{[(C₁-C₆)-alcoxycarbonyl]méthoxy}-2-oxoéthyle ou 2-{[(C₁-C₆)-alkylacyl]méthoxyl}-2-oxoéthyle,
R² à R⁷ indépendamment les uns des autres étant hydrogène, (C₁-C₆) -alkyle, aryle, (C₁-C₈) -alcoxyle, (C₃-C₈) -cycloalkyle, (C₂-C₆)-alkyle interrompu par O, (C₂-C₆)-alkyle interrompu par S ou halogène,
R⁸ étant hydrogène, (C₁-C₆) -alkyle, (C₃-C₄)-alcényle, (C₃-C₄)-alcynyle, arylméthyle, 2-(C₁-C₆)-alcoxyl-2-oxoéthyle, 2-(arylméthyl)-2-oxoéthyle, cyano-(C₁-C₆)-alkyle, (C₁-C₆) -alkyle interrompu par O ou (C₁-C₆)-alkyle interrompu par S
et
Y étant carbonyle.

12. Colorants selon la revendication 1 possédant la formule (I-j) 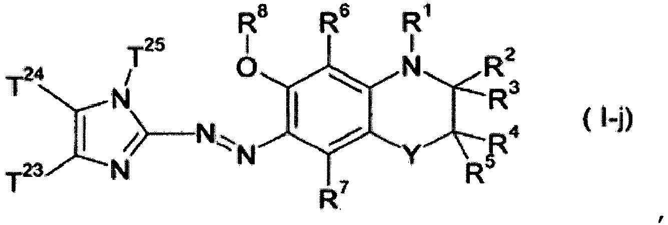
T²³ et T²⁴ indépendamment l'un de l'autre étant cyano, nitro, halogène, aryle ou trifluorométhyle,
T²⁵ étant hydrogène, (C₁-C₆)-alkyle, (C₁-C₆)-alkyle interrompu par O, (C₁-C₆)-alkyle interrompu par S ou aryle,
R¹ étant méthyle, éthyle, propyle, isopropyle, n-butyle, isobutyle, benzyle, 2-méthoxyléthyle, 2-éthoxyléthyle, 2-acétoxyéthyle, 2-(propionyl)oxyéthyle, 2-(benzoyl)oxyéthyle, cyanométhyle, 2-cyanoéthyle, 3-cyanopropyle, 3-(C₁-C₆)-alcoxyl-3-oxopropyle, 3-[(C₂-C₆)-alcoxyl interrompu par O]-3-oxopropyle, 2-(C₁-C₆)-alkyl-2-oxoéthyle, 2-[(C₂-C₆)-alcoxyl interrompu par O]-2-oxoéthyle, 2-(cyanométhoxy)-2-oxoéthyle, 3-(cyanométhoxy)-3-oxopropyle, 2-[(arylacyl)-méthoxy]-2-oxoéthyle, 3-[(arylacyl)méthoxy]-3-oxopropyle, 3-(arylméthoxy)-3-oxopropyle, 3-{[(C₁-C₆)-alkylacyl]méthoxyl}-3-oxopropyle, 3-[(arylacyl)méthoxy]-3-oxopropyle, 3-{[(C₁-C₆)-alcoxycarbonyl]méthoxy}-3-oxopropyle, 2-{[(C₁-C₆)-alcoxycarbonyl]méthoxy}-2-oxoéthyle ou 2-{[(C₁-C₆)-alkylacyl]méthoxyl}-2-oxoéthyle,
chacun de R² à R⁷, indépendamment les uns des autres étant hydrogène, (C₁-C₆) -alkyle, aryle, (C₁-C₈)-alcoxyle, (C₃-C₈)-cycloalkyle, (C₂-C₆)-alkyle interrompu par O, (C₂-C₆)-alkyle interrompu par S ou halogène,
R⁸ étant hydrogène, (C₁-C₆) -alkyle, (C₃-C₄)-alcényle, (C₃-C₄)-alcynyle, arylméthyle, 2-(C₁-C₆) -alcoxyl-2-oxoéthyle, 2-(arylméthyl)-2-oxoéthyle, cyano-(C₁-C₆)-alkyle, (C₁-C₆)-alkyle interrompu par O ou (C₁-C₆)-alkyle interrompu par S
et
Y étant carbonyle.

13. Colorants selon la revendication 1 possédant la formule (I-k) 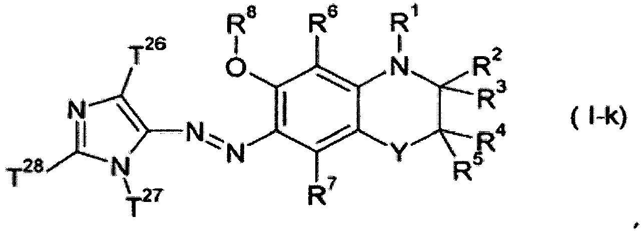
T²⁶ étant cyano, nitro, aryle ou trifluorométhyle,
T²⁷ étant hydrogène, aryle ou (C₁-C₆)-alkyle,
T²⁸ étant hydrogène, (C₁-C₆)-alkyle substitué par (C₁-C₆)-acyloxy, cyano- (C₁-C₆)-alkyle, aryle, (C₁-C₆)-alkyle interrompu par O ou (C₁-C₆) -alkyle interrompu par S,
R¹ étant méthyle, éthyle, propyle, isopropyle, n-butyle, isobutyle, benzyle, 2-méthoxyléthyle, 2-éthoxyléthyle, 2-acétoxyéthyle, 2-(propionyl)oxyéthyle, 2-(benzoyl)oxyéthyle, cyanométhyle, 2-cyanoéthyle, 3-cyanopropyle, 3-(C₁-C₆)-alcoxyl-3-oxopropyle, 3-[(C₂-C₆)-alcoxyl interrompu par O]-3-oxopropyle, 2-(C₁-C₆)-alkyl-2-oxoéthyle, 2-[(C₂-C₆)-alcoxyl interrompu par O]-2-oxoéthyle, 2-(cyanométhoxy)-2-oxoéthyle, 3-(cyanométhoxy)-3-oxopropyle, 2-[(arylacyl)-méthoxy]-2-oxoéthyle, 3-[(arylacyl)méthoxy]-3-oxopropyle, 3-(arylméthoxy)-3-oxopropyle, 3-{[(C₁-C₆)-alkylacyl]méthoxyl}-3-oxopropyle, 3-[(arylacyl)méthoxy]-3-oxopropyle, 3-{[(C₁-C₆)-alcoxycarbonyl]méthoxy}-3-oxopropyle, 2-{[(C₁-C₆)-alcoxycarbonyl]méthoxy}-2-oxoéthyle ou 2-{[(C₁-C₆)-alkylacyl]méthoxyl}-2-oxoéthyle,
R² à R⁷ indépendamment les uns des autres étant hydrogène, (C₁-C₆) -alkyle, aryle, (C₁-C₈)-alcoxyle, (C₃-C₈)-cycloalkyle, (C₂-C₆)-alkyle interrompu par O, (C₂-C₆)-alkyle interrompu par S ou halogène,
R⁸ étant hydrogène, (C₁-C₆)-alkyle, (C₃-C₄)-alcényle, (C₃-C₄)-alcynyle, arylméthyle, 2-(C₁-C₆)-alcoxyl-2-oxoéthyle, 2-(arylméthyl)-2-oxoéthyle, cyano-(C₁-C₆)-alkyle, (C₁-C₆)-alkyle interrompu par O ou (C₁-C₆)-alkyle interrompu par S
et
Y étant carbonyle.

14. Colorants selon la revendication 1 possédant la formule (I-l) 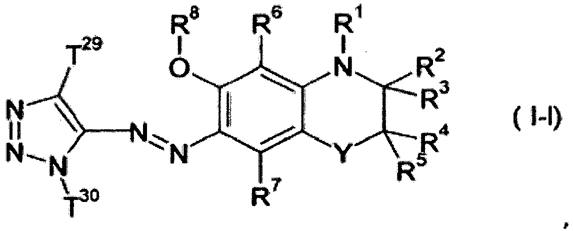
T²⁹ étant cyano, nitro, aryle ou trifluorométhyle,
T³⁰ étant hydrogène, (C₁-C₆) -alkyle, cyano- (C₁-C₆)-alkyle, (C₁-C₆)-alkyle interrompu par O, (C₁-C₆)-alkyle interrompu par S, aryle, benzyle, (C₁-C₆)-alkyle ou cyano- (C₁-C₆) -alkyle substitué par (C₁-C₆)-acyloxy,
R¹ étant méthyle, éthyle, propyle, isopropyle, n-butyle, isobutyle, benzyle, 2-méthoxyléthyle, 2-éthoxyléthyle, 2-acétoxyéthyle, 2-(propionyl)oxyéthyle, 2-(benzoyl)oxyéthyle, cyanométhyle, 2-cyanoéthyle, 3-cyanopropyle, 3-(C₁-C₆)-alcoxyl-3-oxopropyle, 3-[(C₂-C₆)-alcoxyl interrompu par O]-3-oxopropyle, 2-(C₁-C₆)-alkyl-2-oxoéthyle, 2-[(C₂-C₆)-alcoxyl interrompu par O]-2-oxoéthyle, 2-(cyanométhoxy)-2-oxoéthyle, 3-(cyanométhoxy)-3-oxopropyle, 2-[(arylacyl)-méthoxy]-2-oxoéthyle, 3-[(arylacyl)méthoxy]-3-oxopropyle, 3-(arylméthoxy)-3-oxopropyle, 3-{[(C₁-C₆)-alkylacyl]méthoxyl}-3-oxopropyle, 3-[(arylacyl)méthoxy]-3-oxopropyle, 3-{[(C₁-C₆)-alcoxycarbonyl]méthoxy}-3-oxopropyle, 2-{[(C₁-C₆)-alcoxycarbonyl]méthoxy}-2-oxoéthyle ou 2-{[(C₁-C₆)-alkylacyl]méthoxyl}-2-oxoéthyle,
R² à R⁷ indépendamment les uns des autres étant hydrogène, (C₁-C₆)-alkyle, aryle, (C₁-C₈)-alcoxyle, (C₃-C₈) -cycloalkyle, (C₂-C₆)-alkyle interrompu par O, (C₂-C₆)-alkyle interrompu par S ou halogène,
R⁸ étant hydrogène, (C₁-C₆)-alkyle, (C₃-C₄)-alcényle, (C₃-C₄)-alcynyle, arylméthyle, 2-(C₁-C₆)-alcoxyl-2-oxoéthyle, 2-(arylméthyl)-2-oxoéthyle, cyano-(C₁-C₆)-alkyle, (C₁-C₆)-alkyle interrompu par O ou (C₁-C₆)-alkyle interrompu par S
et
Y étant carbonyle.

15. Colorants selon la revendication 1 possédant la formule (I-m) 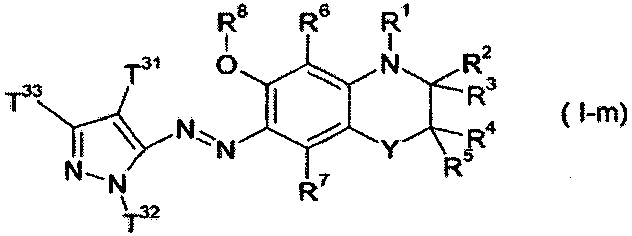
T³¹ étant cyano, nitro, aryle ou trifluorométhyle,
T³² étant hydrogène, (C₁-C₆) -alkyle, (C₁-C₆) -alkyle interrompu par O, (C₁-C₆) -alkyle interrompu par S, cyano-(C₁-C₆)-alkyle, arylméthyle, (C₁-C₆) -alkyle substitué par cyano- (C₁-C₆) -alkyle ou (C₁-C₆)-acyloxy,
T³³ étant hydrogène ou (C₁-C₆) -alkyle,
R¹ étant méthyle, éthyle, propyle, isopropyle, n-butyle, isobutyle, benzyle, 2-méthoxyléthyle, 2-éthoxyléthyle, 2-acétoxyéthyle, 2-(propionyl)oxyéthyle, 2-(benzoyl)oxyéthyle, cyanométhyle, 2-cyanoéthyle, 3-cyanopropyle, 3-(C₁-C₆)-alcoxyl-3-oxopropyle, 3-[(C₂-C₆)-alcoxyl interrompu par O]-3-oxopropyle, 2-(C₁-C₆) -alkyl-2-oxoéthyle, 2-[(C₂-C₆)-alcoxyl interrompu par O]-2-oxoéthyle, 2-(cyanométhoxy)-2-oxoéthyle, 3-(cyanométhoxy)-3-oxopropyle, 2-[(arylacyl)-méthoxy]-2-oxoéthyle, 3-[(arylacyl)méthoxy]-3-oxopropyle, 3-(arylméthoxy)-3-oxopropyle, 3-{[(C₁-C₆)-alkylacyl]méthoxyl}-3-oxopropyle, 3-[(arylacyl)méthoxy]-3-oxopropyle, 3-{[(C₁-C₆)-alcoxycarbonyl]méthoxy}-3-oxopropyle, 2-{[(C₁-C₆)-alcoxycarbonyl]méthoxy}-2-oxoéthyle ou 2-{[(C₁-C₆)-alkylacyl]méthoxyl}-2-oxoéthyle,
R² à R⁷ indépendamment les uns des autres étant hydrogène, (C₁-C₆) -alkyle, aryle, (C₁-C₈) -alcoxyle, (C₃-C₈)-cycloalkyle, (C₂-C₆)-alkyle interrompu par O, (C₂-C₆)-alkyle interrompu par S ou halogène,
R⁸ étant hydrogène, (C₁-C₆) -alkyle, (C₃-C₄)-alcényle, (C₃-C₄)-alcynyle, arylméthyle, 2-(C₁-C₆)-alcoxyl-2-oxoéthyle, 2-(arylméthyl)-2-oxoéthyle, cyano-(C₁-C₆)-alkyle, (C₁-C₆) -alkyle interrompu par O ou (C₁-C₆)-alkyle interrompu par S
et
Y étant carbonyle.

16. Colorants selon la revendication 1 possédant la formule (I-n) 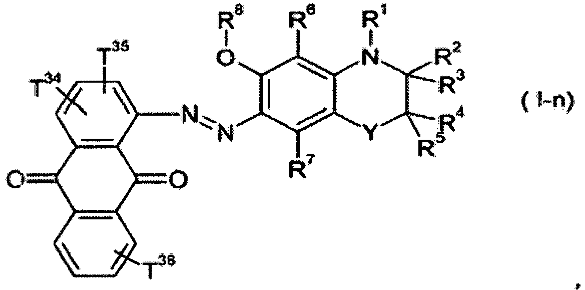
T³⁴, T³⁵ et T³⁶ indépendamment les uns des autres étant hydrogène, halogène, nitro, cyano, hydroxyle, (C₁-C₆)-alcoxylcarbonyle, (C₁-C₆)-alkyle ou acyle,
R¹ étant méthyle, éthyle, propyle, isopropyle, n-butyle, isobutyle, benzyle, 2-méthoxyléthyle, 2-éthoxyléthyle, 2-acétoxyéthyle, 2-(propionyl)oxyéthyle, 2-(benzoyl)oxyéthyle, cyanométhyle, 2-cyanoéthyle, 3-cyanopropyle, 3-(C₁-C₆)-alcoxyl-3-oxopropyle, 3-[(C₂-C₆)-alcoxyl interrompu par O]-3-oxopropyle, 2-(C₁-C₆) -alkyl-2-oxoéthyle, 2-[(C₂-C₆)-alcoxyl interrompu par O]-2-oxoéthyle, 2-(cyanométhoxy)-2-oxoéthyle, 3-(cyanométhoxy)-3-oxopropyle, 2-[(arylacyl)-méthoxy]-2-oxoéthyle, 3-[(arylacyl)méthoxy]-3-oxopropyle, 3-(arylméthoxy)-3-oxopropyle, 3-{[(C₁-C₆)-alkylacyl]méthoxyl}-3-oxopropyle, 3-[(arylacyl)méthoxy]-3-oxopropyle, 3-{[(C₁-C₆)-alcoxycarbonyl]méthoxy}-3-oxopropyle, 2-{[(C₁-C₆)-alcoxycarbonyl]méthoxy}-2-oxoéthyle ou 2-{[(C₁-C₆)-alkylacyl]méthoxyl}-2-oxoéthyle,
R² à R⁷ indépendamment les uns des autres étant hydrogène, (C₁-C₆)-alkyle, aryle, (C₁-C₈)-alcoxyle, (C₃-C₈)-cycloalkyle, (C₂-C₆)-alkyle interrompu par O, (C₂-C₆)-alkyle interrompu par S ou halogène,
R⁸ étant hydrogène, (C₁-C₆) -alkyle, (C₃-C₄)-alcényle, (C₃-C₄)-alcynyle, arylméthyle, 2-(C₁-C₆)-alcoxyl-2-oxoéthyle, 2-(arylméthyl)-2-oxoéthyle, cyano-(C₁-C₆)-alkyle, (C₁-C₆) -alkyle interrompu par O ou (C₁-C₆)-alkyle interrompu par S
et
Y étant carbonyle.

17. Colorants selon la revendication 1 possédant la formule (I-o) 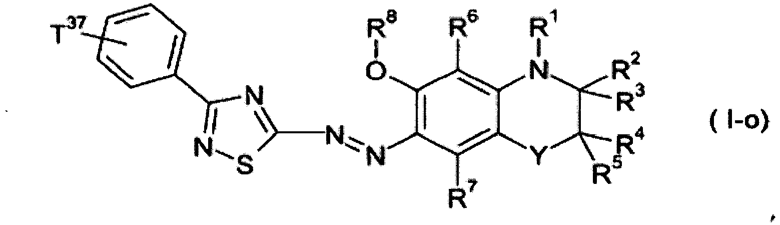
T³⁷ étant nitro, hydrogène ou halogène,
R¹ étant méthyle, éthyle, propyle, isopropyle, n-butyle, isobutyle, benzyle, 2-méthoxyléthyle, 2-éthoxyléthyle, 2-acétoxyéthyle, 2-(propionyl)oxyéthyle, 2-(benzoyl)oxyéthyle, cyanométhyle, 2-cyanoéthyle, 3-cyanopropyle, 3-(C₁-C₆)-alcoxyl-3-oxopropyle, 3-[(C₂-C₆)-alcoxyl interrompu par O]-3-oxopropyle, 2-(C₁-C₆)-alkyl-2-oxoéthyle, 2-[(C₂-C₆)-alcoxyl interrompu par O]-2-oxoéthyle, 2-(cyanométhoxy)-2-oxoéthyle, 3-(cyanométhoxy)-3-oxopropyle, 2-[(arylacyl)-méthoxy]-2-oxoéthyle, 3-[(arylacyl)méthoxy]-3-oxopropyle, 3-(arylméthoxy)-3-oxopropyle, 3-{[(C₁-C₆)-alkylacyl]methoxyl}-3-oxopropyle, 3-[(arylacyl)méthoxy]-3-oxopropyle, 3-{[(C₁-C₆)-alcoxycarbonyl]méthoxy}-3-oxopropyle, 2-{[(C₁-C₆)-alcoxycarbonyl]méthoxy}-2-oxoéthyle ou 2-{[(C₁-C₆)-alkylacyl]méthoxyl}-2-oxoéthyle,
R² à R⁷ indépendamment les uns des autres étant hydrogène, (C₁-C₆) -alkyle, aryle, (C₁-C₈)-alcoxyle, (C₃-C₈)-cycloalkyle, (C₂-C₆)-alkyle interrompu par O,
(C₂-C₆)-alkyle interrompu par S ou halogène,
R⁸ étant hydrogène, (C₁-C₆) -alkyle, (C₃-C₄)-alcényle, (C₃-C₄)-alcynyle, arylméthyle, 2-(C₁-C₆)-alcoxyl-2-oxoéthyle, 2-(arylméthyl)-2-oxoéthyle, cyano-(C₁-C₆)-alkyle, (C₁-C₆) -alkyle interrompu par O ou (C₁-C₆)-alkyle interrompu par S
et
Y étant carbonyle.

18. Procédé pour la production de colorants possédant la formule (I) et de mélanges correspondants, comprenant
a) la diazotation d'un composé de formule générale (XD)
**D-NH₂** **(XD),**
D étant tel que défini dans la revendication 1, et
b) la mise en réaction du sel de diazonium correspondant obtenu avec un composé de formule (XC) 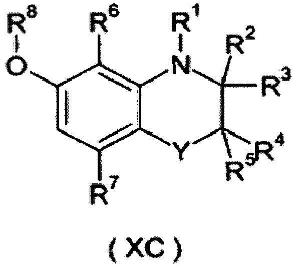 R¹ à R⁸ et Y étant chacun tels que définis dans la revendication 1.

19. Utilisation des colorants selon la revendication 1 pour la coloration et l'impression de matériaux hydrophobes.
